# EUROPEAN PATENT APPLICATION

(11) **EP 4 446 314 A2**
(43) Date of publication of application: **16.10.2024**
(21) Application number: 24190103.2
(22) Date of filing: 01.05.2020
(51) Int. Cl.: C07D 249/04

(54) **KCNT1 INHIBITORS AND METHODS OF USE**

(30) Priority: 03.05.2019 US 201962842855 P; 03.05.2019 US 201962842858 P; 03.05.2019 US 201962842861 P; 28.02.2020 US 202062982858 P
(62) Divisional of application: 20802501.5
(71) Applicant: Praxis Precision Medicines, Inc., Boston, MA 02110 (US)
(72) Inventor: MARTINEZ BOTELLA, Gabriel, Cambridge, 02142 (US); GRIFFIN, Andrew, Mark, Cambridge, 02142 (US); CHARIFSON, Paul, S., Cambridge, 02142 (US); REDDY, Kiran, Cambridge, 02142 (US); KAHLIG, Michael, Kristopher Mathieu, Cambridge, 02142 (US); MARRON, Brian, Edward, Cambridge, 02142 (US)
(74) Representative: Carpmaels & Ransford LLP

(57) **Abstract**

The present invention is directed to, in part, compounds and compositions useful for preventing and/or treating a neurological disease or disorder, a disease or condition relating to excessive neuronal excitability, and/or a gain-of-function mutation in a gene (e.g., KCNT1). Methods of treating a neurological disease or disorder, a disease or condition relating to excessive neuronal excitability, and/or a gain-of-function mutation in a gene such as KCNT1 are also provided herein.

## Description

### CROSS REFERENCE TO RELATED APPLICATIONS

This application claims priority to and the benefit of U.S. Provisional Patent Application Number 62/842,855 filed May 3, 2019, U.S. Provisional Patent Application Number 62/842,858 filed May 3, 2019, U.S. Provisional Patent Application Number 62/842,861 filed May 3, 2019, and U.S. Provisional Patent Application Number 62/982,858 filed February 28, 2020, the contents of each which are incorporated herein by reference in their entirety.

### BACKGROUND

KCNT1 encodes sodium-activated potassium channels known as Slack (Sequence like a calcium-activated K⁺ channel). These channels are found in neurons throughout the brain and can mediate a sodium-activated potassium current *I*_{KNa}. This delayed outward current can regulate neuronal excitability and the rate of adaption in response to maintained stimulation. Abnormal Slack activity have been associated with development of early onset epilepsies and intellectual impairment. Accordingly, pharmaceutical compounds that selectively regulate sodium-activated potassium channels, e.g., abnormal KCNT1, abnormal *I*_{KNa}, are useful in treating a neurological disease or disorder or a disease or condition related to excessive neuronal excitability and/or KCNT1 gain-of-function mutations.

### SUMMARY OF THE INVENTION

Described herein are compounds and compositions useful for preventing and/or treating a disease, disorder, or condition, e.g., a neurological disease or disorder, a disease, disorder, or condition associated with excessive neuronal excitability and/or a gain-of-function mutation in a gene, for example, KCNT1.

Thus, in one aspect, the present disclosure features a pharmaceutical composition comprising a compound of Formula **I-I:** 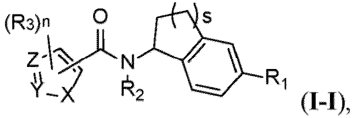
or a pharmaceutically acceptable salt thereof, wherein
   X is selected from the group consisting of NH, O, and S, wherein the hydrogen of NH may be substituted with R₃;
Y is selected from N and CH, wherein the hydrogen of CH may be substituted with R₃;
Z is selected from N and CH, wherein the hydrogen of CH may be substituted with R₃, or Z is C when Z is substituted with the -C(O)N(R₂)- moiety;
   R₁ is selected from the group consisting of -Cl, -F, and C₁₋₆alkyl substituted with one or more substituents independently selected from -Cl and -F;
   R₂ is hydrogen;
   each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆, - C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
n is selected from the group consisting of 0, 1, 2, 3, and 4;
   R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
   each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and s is 1 or 2;
and a pharmaceutically acceptable excipient.

In some embodiments, the compound of Formula **I-I** is a compound of Formula **I:** 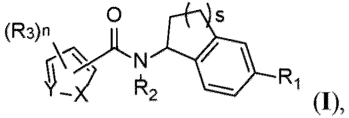 or a pharmaceutically acceptable salt thereof, wherein the variables are as defined for Formula **I-I.**

In some embodiments, the compound of Formula **I-I** is a compound of Formula **I-Ia** or Formula **I-Ia1:** 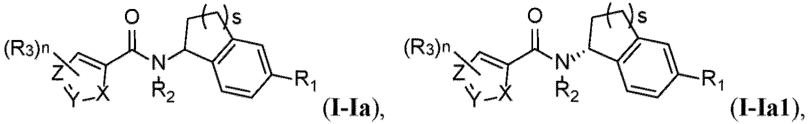 or a pharmaceutically acceptable salt thereof, wherein the variables are as defined for Formula **I-I.**

In some embodiments, the compound of Formula **I-I** is a compound of Formula **I-Ib** or Formula **I-Ib1**: 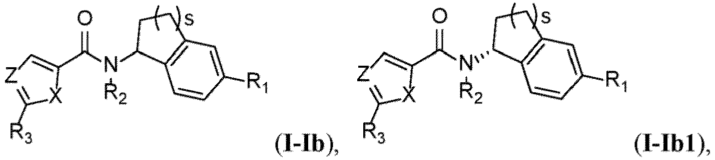 or a pharmaceutically acceptable salt thereof, wherein the variables are as defined for Formula **I-I.**

In some embodiments, the compound of Formula **I-I** is a compound of Formula **I-Ic** or Formula **I-Ic1**: 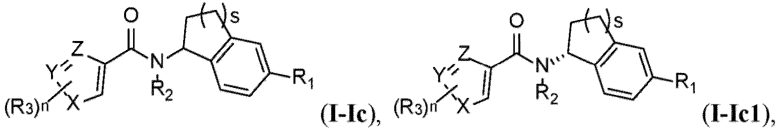 or a pharmaceutically acceptable salt thereof, wherein the variables are as defined for Formula **I-I.**

In some embodiments, the compound of Formula **I-I** is a compound of Formula **I-a** or Formula **I-a1**: 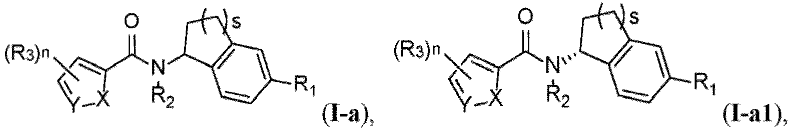 or a pharmaceutically acceptable salt thereof, wherein the variables are as defined for Formula **I-I.**

In some embodiments, the compound of Formula **I-I** is a compound of Formula **1-b** or Formula **I-b1:** 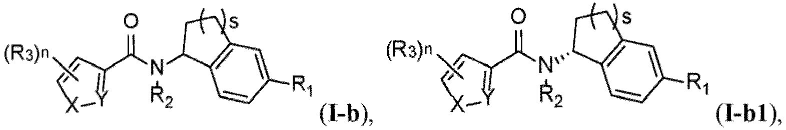 or a pharmaceutically acceptable salt thereof, wherein the variables are as defined for Formula **I-I.**

In some embodiments, the compound of Formula **I-I** is a compound of Formula **I-c** or Formula **I-c1**: 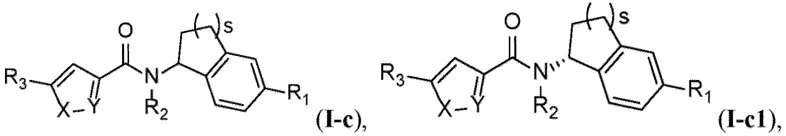 or a pharmaceutically acceptable salt thereof, wherein the variables are as defined for Formula **I-I.**

In some embodiments, the compound of Formula **I-I** is a compound of Formula **I-d** or Formula **I-d1**: 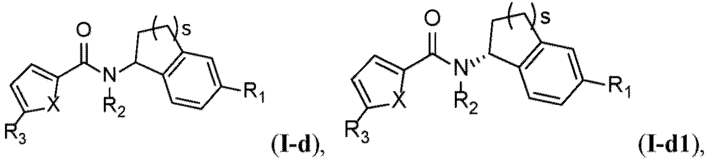 or a pharmaceutically acceptable salt thereof, wherein the variables are as defined for Formula **I-I.**

In another aspect, the present disclosure features a pharmaceutical composition comprising a compound of Formula **II:** 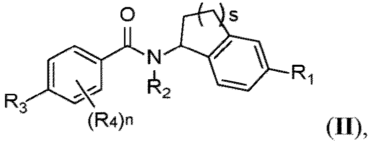
or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl, - NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and - S(O)₂-C₁₋₆alkyl, wherein the C₁₋₆alkyl is optionally substituted with one or more halogen or C₁₋₆alkoxy;
   each R₄ is independently selected from the group consisting of C₁₋₆alkyl, halogen, and -OH; wherein R₄ is substituted at the carbon adjacent to R₃ when R₄ is -OH;
n is selected from the group consisting of 0, 1, 2, 3, and 4;
   R₅, R₆, R₉, and R₁₀ are each independently hydrogen or C₁₋₆alkyl;
each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
   s is 1 or 2;
and a pharmaceutically acceptable excipient.

In some embodiments, the compound of Formula **II** is a compound of Formula **11-a,** Formula **II-a1**, or Formula **II-a2:** 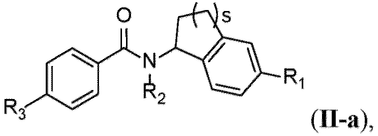 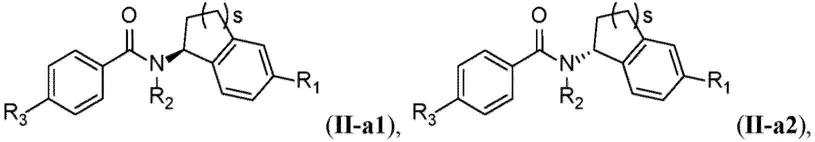 or a pharmaceutically acceptable salt thereof.

In some embodiments, the compound is a compound of Formula **II-b,** Formula **II-b1**, or Formula **II-b2:** 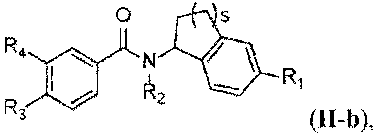 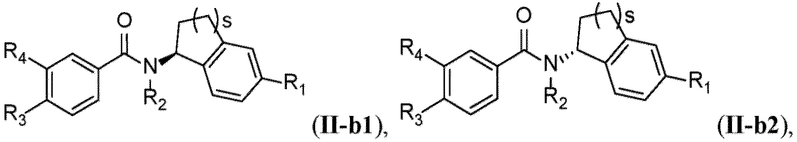 or a pharmaceutically acceptable salt thereof.

In some embodiments, the compound of Formula **II** is a compound of Formula **II-c**, Formula **II-c1**, or Formula **II-c2:** 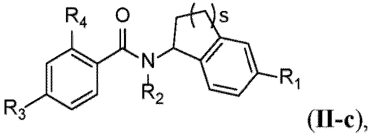 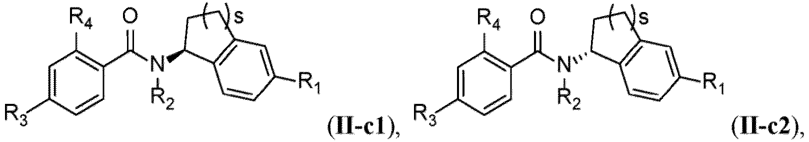 or a pharmaceutically acceptable salt thereof.

In another aspect, the present disclosure features a pharmaceutical composition comprising a compound of Formula **III:** 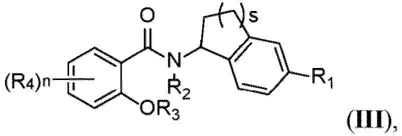
or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is hydrogen;
   R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
n is selected from the group consisting of 0, 1, 2, 3, and 4;
   R₇ and R₈ are each independently hydrogen or C₁₋₆alkyl; and
s is 1 or 2;
and a pharmaceutically acceptable excipient.

In some embodiments, the compound of Formula **III** is a compound of Formula **III-a,** Formula **III-a1,** or Formula **III-a2:** 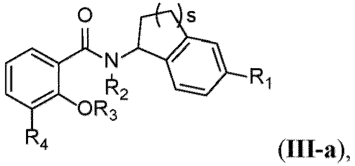 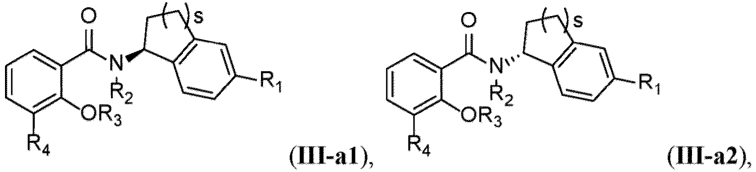 or a pharmaceutically acceptable salt thereof.

In some embodiments, the compound of Formula **III** is a compound of Formula **III-b,** Formula **III-b1**, or Formula **III-b2:** 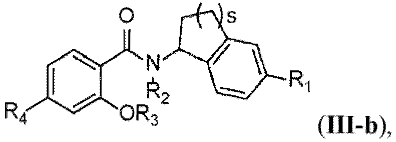 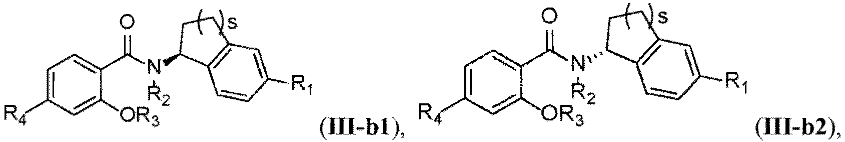 or a pharmaceutically acceptable salt thereof.

In some embodiments, the compound of Formula **III** is a compound of Formula **III-c,** Formula **III-c1,** or Formula III-c2: 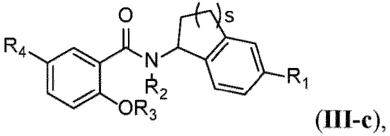 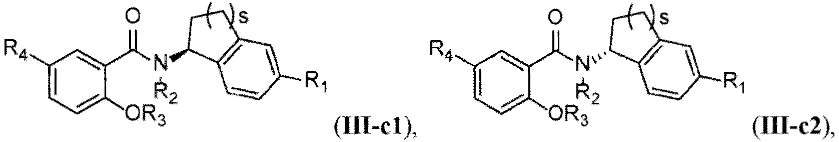 or a pharmaceutically acceptable salt thereof.

In one aspect, the present disclosure provides a method of treating neurological disease or disorder, wherein the method comprises administering to a subject in need thereof a compound disclosed herein (e.g., compound of Formula (I-I) or (1), (e.g., (I-Ia), (I-Ia1), (I-Ib), (I-Ib1), (1-1c), (I-Ic1), (1-a), (I-a1), (1-b), (I-b1), (1-c), (I-c1), (1-d), or (1-d1)), a compound of Formula (II), (e.g., (11-a), (II-a1), (II-a2), (11-b), (II-b1), (11-b2), (II-c), (II-c1), (II-c2)), or a compound of Formula (III), (e.g., (111-a), (III-a1), (III-a2), (III-b), (III-b1), (III-b2), (III-c), (III-c1), (III-c2))) or a pharmaceutical composition disclosed herein (e.g., a pharmaceutical composition comprising a compound of Formula (I-I) or (1), (e.g., (I-Ia), (I-Ia1), (I-Ib), (I-Ib1), (1-1c), (I-Ic1), (1-a), (I-a1), (1-b), (I-b1), (1-c), (I-c1), (1-d), or (1-d1)), a compound of Formula (II), (e.g., (11-a), (II-a1), (II-a2), (11-b), (II-b1), (11-b2), (II-c), (II-c1), (II-c2)), or a compound of Formula (III), (e.g., (111-a), (III-a1), (III-a2), (III-b), (III-b1), (III-b2), (III-c), (III-c1), (III-c2))), or a pharmaceutically acceptable salt thereof, and a pharmaceutically acceptable excipient).

In another aspect, the present disclosure provides a method of treating a disease or condition associated with excessive neuronal excitability, wherein the method comprises administering to a subject in need thereof a compound disclosed herein (e.g., compound of Formula (I-I) or (1), (e.g., (I-Ia), (I-Ia1), (1-1b), (I-Ib1), (I-Ic), (I-Ic1), (1-a), (1-a1), (1-b), (I-b1), (1-c), (I-c1), (1-d), or (I-d1)), a compound of Formula (II), (e.g., (11-a), (11-a1), (11-a2), (11-b), (II-b1), (11-b2), (11-c), (II-c1), (II-c2)), or a compound of Formula (III), (e.g., (IIa-a), (III-a1), (III-a2), (111-b), (III-b1), (111-b2), (111-c), (III-c1), (III-c2))) or a pharmaceutical composition disclosed herein (e.g., a pharmaceutical composition comprising a compound of Formula (I-I) or (1), (e.g., (I-Ia), (I-Ia1), (1-1b), (I-Ib1), (I-Ic), (I-Ic1), (1-a), (1-a1), (1-b), (I-b1), (1-c), (I-c1), (1-d), or (I-d1)), a compound of Formula (II), (e.g., (11-a), (11-a1), (11-a2), (11-b), (II-b1), (11-b2), (11-c), (II-c1), (II-c2)), or a compound of Formula (III), (e.g., (III-a), (III-a1), (III-a2), (111-b), (III-b1), (111-b2), (111-c), (III-c1), (III-c2))), or a pharmaceutically acceptable salt thereof, and a pharmaceutically acceptable excipient).

In another aspect, the present disclosure provides a method of treating a disease or condition associated with a gain-of-function mutation of a gene (e.g. KCNT1), wherein the method comprises administering to a subject in need thereof a compound disclosed herein (e.g., a compound of Formula (1-1) or (1) (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), a compound of Formula (II), (e.g., (11-a), (II-a1), (11-a2), (11-b), (II-b1), (11-b2), (11-c), (II-c1), (II-c2)), or a compound of Formula (III), (e.g., (III-a), (III-a1), (III-a2), (III-b), (III-b1), (III-b2), (111-c), (III-c1), (III-c2))) or a pharmaceutical composition disclosed herein (e.g., a pharmaceutical composition comprising a compound of Formula (I-I) or (1), (e.g., (I-Ia), (I-Ia1), (1-1b), (I-Ib1), (I-Ic), (1-Ic1), (1-a), (I-a1), (1-b), (I-b1), (1-c), (I-c1), (1-d), or (I-d1)), a compound of Formula (II), (e.g., (11-a), (II-a1), (II-a2), (11-b), (II-b1), (II-b2), (11-c), (II-c1), (II-c2)), or a compound of Formula (III), (e.g., (111-a), (III-a1), (III-a2), (III-b), (III-b1), (III-b2), (III-c), (III-c1), (III-c2))), or a pharmaceutically acceptable salt thereof, and a pharmaceutically acceptable excipient).

In some embodiments, the neurological disease or disorder, the disease or condition associated with excessive neuronal excitability, or the disease or condition associated with a gain-of-function mutation of a gene (e.g., KCNT1) is epilepsy, an epilepsy syndrome, or an encephalopathy.

In some embodiments, the neurological disease or disorder, the disease or condition associated with excessive neuronal excitability, or the disease or condition associated with a gain-of-function mutation of a gene (e.g., KCNT1) is a genetic or pediatric epilepsy or a genetic or pediatric epilepsy syndrome.

In some embodiments, the neurological disease or disorder, the disease or condition associated with excessive neuronal excitability, or the disease or condition associated with a gain-of-function mutation of a gene (e.g., KCNT1) is a cardiac dysfunction.

In some embodiments, the neurological disease or disorder, the disease or condition associated with excessive neuronal excitability, or the disease or condition associated with a gain-of-function mutation of a gene (e.g., KCNT1) is selected from epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, Lennox Gastaut syndrome, seizures (e.g., Generalized tonic clonic seizures, Asymmetric Tonic Seizures), leukodystrophy, leukoencephalopathy, intellectual disability, Multifocal Epilepsy, Drug resistant epilepsy, Temporal lobe epilepsy, cerebellar ataxia).

In some embodiments, the neurological disease or disorder, the disease or condition associated with excessive neuronal excitability, or the disease or condition associated with a gain-of-function mutation of a gene (e.g., KCNT1) is selected from the group consisting of cardiac arrhythmia, sudden unexpected death in epilepsy, Brugada syndrome, and myocardial infarction.

In some embodiments, the neurological disease or disorder, the disease or condition associated with excessive neuronal excitability, or the disease or condition associated with a gain-of-function mutation of a gene (e.g., KCNT1) is selected from pain and related conditions (e.g. neuropathic pain, acute/chronic pain, migraine, etc).

In some embodiments, the neurological disease or disorder, the disease or condition associated with excessive neuronal excitability, or the disease or condition associated with a gain-of-function mutation of a gene (e.g., KCNT1) is a muscle disorder (e.g. myotonia, neuromyotonia, cramp muscle spasms, spasticity).

In some embodiments, the neurological disease or disorder, the disease or condition associated with excessive neuronal excitability, or the disease or condition associated with a gain-of-function mutation of a gene (e.g., KCNT1) is selected from itch and pruritis, ataxia and cerebellar ataxias.

In some embodiments, the neurological disease or disorder, the disease or condition associated with excessive neuronal excitability, or the disease or condition associated with a gain-of-function mutation of a gene (e.g., KCNT1) is selected from psychiatric disorders (e.g. major depression, anxiety, bipolar disorder, schizophrenia).

In some embodiments, the neurological disease or disorder or the disease or condition associated with excessive neuronal excitability and/or a gain-of-function mutation in a gene (e.g., KCNT1) is selected from the group consisting of learning disorders, Fragile X, neuronal plasticity, and autism spectrum disorders.

In some embodiments, the neurological disease or disorder, the disease or condition associated with excessive neuronal excitability, or the disease or condition associated with a gain-of-function mutation of a gene (e.g., KCNT1) is selected from the group consisting of epileptic encephalopathy with SCN1A, SCN2A, SCN8A mutations, early infantile epileptic encephalopathy, Dravet syndrome, Dravet syndrome with SCN1A mutation, generalized epilepsy with febrile seizures, intractable childhood epilepsy with generalized tonic-clonic seizures, infantile spasms, benign familial neonatal-infantile seizures, SCN2A epileptic encephalopathy, focal epilepsy with SCN3A mutation, cryptogenic pediatric partial epilepsy with SCN3A mutation, SCN8A epileptic encephalopathy, sudden unexpected death in epilepsy, Rasmussen encephalitis, malignant migrating partial seizures of infancy, autosomal dominant nocturnal frontal lobe epilepsy, sudden expected death in epilepsy (SUDEP), KCNQ2 epileptic encephalopathy, and KCNT1 epileptic encephalopathy.

Other objects and advantages will become apparent to those skilled in the art from a consideration of the ensuing **Detailed Description, Examples,** and **Claims.**

### DETAILED DESCRIPTION OF THE INVENTION

As generally described herein, the present invention provides compounds and compositions useful for preventing and/or treating a disease, disorder, or condition described herein, e.g., a disease, disorder, or condition associated with excessive neuronal excitability, and/or a disease, disorder, or condition associated with gain-of-function mutations in KCNT1. Exemplary diseases, disorders, or conditions include epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, and Lennox Gastaut syndrome, seizures, leukodystrophy, leukoencephalopathy, intellectual disability, Multifocal Epilepsy, Generalized tonic clonic seizures, Drug resistant epilepsy, Temporal lobe epilepsy, cerebellar ataxia, Asymmetric Tonic Seizures) and cardiac dysfunctions (e.g., cardiac arrhythmia, Brugada syndrome, sudden unexpected death in epilepsy, myocardial infarction), pain and related conditions (e.g. neuropathic pain, acute/chronic pain, migraine, etc), muscle disorders (e.g. myotonia, neuromyotonia, cramp muscle spasms, spasticity), itch and pruritis, ataxia and cerebellar ataxias, and psychiatric disorders (e.g. major depression, anxiety, bipolar disorder, schizophrenia).

### Definitions

### Chemical definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

As used herein a pure enantiomeric compound is substantially free from other enantiomers or stereoisomers of the compound (*i.e.,* in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

In the compositions provided herein, an enantiomerically pure compound can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

Compound described herein may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; F may be in any isotopic form, including ¹⁸F and ¹⁹F; and the like.

The following terms are intended to have the meanings presented therewith below and are useful in understanding the description and intended scope of the present invention. When describing the invention, which may include compounds and pharmaceutically acceptable salts thereof, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein. The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example, "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

As used herein, "alkyl" refers to a radical of a straight-chain or branched saturated hydrocarbon group, e.g., having 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). Examples of C₁₋₆ alkyl groups include methyl, ethyl, propyl, isopropyl, butyl, isobutyl, pentyl, hexyl, and the like.

The term "heteroalkyl" as used herein refers to an "alkyl" group in which at least one carbon atom has been replaced with an O or S atom. The heteroalkyl may be, for example, an -O-C₁-C₁₀alkyl group, an -C₁-C₆alkylene-O-C₁-C₆alkyl group, or a C₁-C₆ alkylene-OH group. In certain embodiments, the "heteroalkyl" may be 2-8 membered heteroalkyl, indicating that the heteroalkyl contains from 2 to 8 atoms selected from the group consisting of carbon, oxygen, nitrogen, and sulfur. In yet other embodiments, the heteroalkyl may be a 2-6 membered, 4-8 membered, or a 5-8 membered heteroalkyl group (which may contain for example 1 or 2 heteroatoms selected from the group oxygen and nitrogen). In certain embodiments, the heteroalkyl is an "alkyl" group in which 1-3 carbon atoms have been replaced with oxygen atoms. One type of heteroalkyl group is an "alkoxy" group.

As used herein, "alkenyl" refers to a radical of a straight-chain or branched hydrocarbon group having from 2 to 20 carbon atoms, one or more carbon-carbon double bonds (e.g., 1, 2, 3, or 4 carbon-carbon double bonds), and optionally one or more carbon-carbon triple bonds (e.g., 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like.

As used herein, "alkynyl" refers to a radical of a straight-chain or branched hydrocarbon group having from 2 to 20 carbon atoms, one or more carbon-carbon triple bonds (e.g., 1, 2, 3, or 4 carbon-carbon triple bonds), and optionally one or more carbon-carbon double bonds (e.g., 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like.

As used herein, "alkylene," "alkenylene," and "alkynylene," refer to a divalent radical of an alkyl, alkenyl, and alkynyl group respectively. When a range or number of carbons is provided for a particular "alkylene," "alkenylene," or "alkynylene," group, it is understood that the range or number refers to the range or number of carbons in the linear carbon divalent chain. "Alkylene," "alkenylene," and "alkynylene," groups may be substituted or unsubstituted with one or more substituents as described herein.

As used herein, "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl.

As used herein, "heteroaryl" refers to a radical of a 5-10 membered monocyclic or bicyclic 4n+2 aromatic ring system (e.g., having 6 or 10 electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, i.e., either the ring bearing a heteroatom (e.g., 2-indolyl) or the ring that does not contain a heteroatom (e.g., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 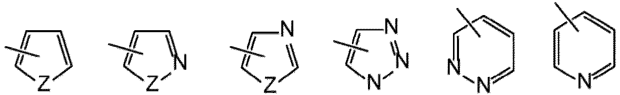 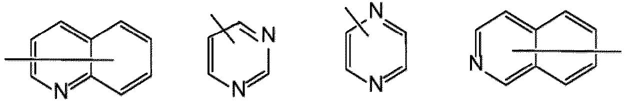 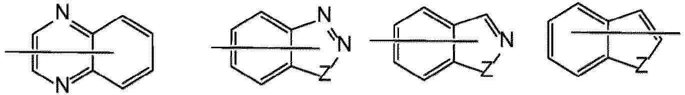 wherein each Z is selected from carbonyl, N, NR⁶⁵, O, and S; and R⁶⁵ is independently hydrogen, C₁-C₈ alkyl, C₃-C₁₀ carbocyclyl, 4-10 membered heterocyclyl, C₆-C₁₀ aryl, and 5-10 membered heteroaryl.

As used herein, "carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃),cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1*H*-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system.

The term "cycloalkyl" refers to a monovalent saturated cyclic, bicyclic, or bridged cyclic (e.g., adamantyl) hydrocarbon group of 3-12, 3-8, 4-8, or 4-6 carbons, referred to herein, e.g., as "C₄₋₈cycloalkyl," derived from a cycloalkane. Exemplary cycloalkyl groups include, but are not limited to, cyclohexanes, cyclopentanes, cyclobutanes and cyclopropanes. Unless specified otherwise, cycloalkyl groups are optionally substituted at one or more ring positions with, for example, alkanoyl, alkoxy, alkyl, haloalkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl or thiocarbonyl. Cycloalkyl groups can be fused to other cycloalkyl, aryl, or heterocyclyl groups. In certain embodiments, the cycloalkyl group is not substituted, i.e., it is unsubstituted.

As used herein, "heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, e.g., heteroalkyl; carbocyclyl, e.g., heterocyclyl; aryl, e.g,. heteroaryl; and the like having from 1 to 5, and particularly from 1 to 3 heteroatoms.

As used herein, "cyano" refers to -CN.

As used herein, "halo" or "halogen" refers to fluoro (F), chloro (Cl), bromo (Br) and iodo (I). In certain embodiments, the halo group is either fluoro or chloro.

As used herein, "haloalkyl" refers to an alkyl group substituted with one or more halogen atoms.

As used herein, "nitro" refers to -NO₂.

As used herein, "oxo" refers to -C=O.

In general, the term "substituted", whether preceded by the term "optionally" or not, means that at least one hydrogen present on a group (e.g., a carbon or nitrogen atom) is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position.

Nitrogen atoms can be substituted or unsubstituted as valency permits, and include primary, secondary, tertiary, and quarternary nitrogen atoms. Exemplary nitrogen atom substitutents include, but are not limited to, hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups attached to a nitrogen atom are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined above.

These and other exemplary substituents are described in more detail in the **Detailed Description, Examples,** and **Claims.** The invention is not intended to be limited in any manner by the above exemplary listing of substituents.

### Other definitions

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describes pharmaceutically acceptable salts in detail in J. Pharmaceutical Sciences (1977) 66:1-19. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

As used herein, a "subject" to which administration is contemplated includes, but is not limited to, humans (i.e., a male or female of any age group, e.g., a pediatric subject (e.g, infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition (also "therapeutic treatment").

In general, the "effective amount" of a compound refers to an amount sufficient to elicit the desired biological response. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

In an alternate embodiment, the present invention contemplates administration of the compounds of the present invention or a pharmaceutically acceptable salt or a pharmaceutically acceptable composition thereof, as a prophylactic before a subject begins to suffer from the specified disease, disorder or condition. As used herein, "prophylactic treatment" contemplates an action that occurs before a subject begins to suffer from the specified disease, disorder or condition. As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

As used herein, a "disease or condition associated with a gain-of-function mutation in KCNT1" refers to a disease or condition that is associated with, is partially or completely caused by, or has one or more symptoms that are partially or completely caused by, a mutation in KCNT1 that results in a gain-of-function phenotype, i.e. an increase in activity of the potassium channel encoded by KCNT1 resulting in an increase in whole cell current.

As used herein, a "gain-of-function mutation" is a mutation in KCNT1 that results in an increase in activity of the potassium channel encoded by KCNT1. Activity can be assessed by, for example, ion flux assay or electrophysiology (e.g. using the whole cell patch clamp technique). Typically, a gain-of-function mutation results in an increase of at least or about 20%, 30%, 40%, 50%, 60%, 70%, 80%, 90%, 100%, 125%, 150%, 175%, 200%, 225%, 250%, 275%, 300%, 325%, 350%, 375%, 400% or more compared to the activity of a potassium channel encoded by a wild-type KCNT1.

### Compounds and compositions

In one aspect, the present disclosure features a compound of Formula **I-I:** 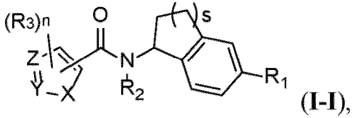
or a pharmaceutically acceptable salt thereof, wherein
   X is selected from the group consisting of NH, O, and S, wherein the hydrogen of NH may be substituted with R₃;
Y is selected from N and CH, wherein the hydrogen of CH may be substituted with R₃;
Z is selected from N and CH, wherein the hydrogen of CH may be substituted with R₃, or Z is C when Z is substituted with the -C(O)N(R₂)- moiety;
   R₁ is selected from the group consisting of -Cl, -F, and C₁₋₆alkyl substituted with one or more substituents independently selected from -Cl and -F;
   R₂ is hydrogen;
   each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆, - C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
n is selected from the group consisting of 0, 1, 2, 3, and 4;
   R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
   each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and s is 1 or 2.

In some embodiments, the present invention features a compound of Formula **I:** 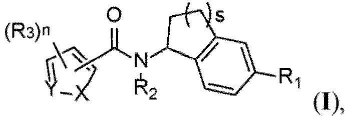 or a pharmaceutically acceptable salt thereof, wherein
X is selected from the group consisting of NH, O, and S, wherein the hydrogen of NH may be substituted with R₃;
Y is selected from N and CH, wherein the hydrogen of CH may be substituted with R₃;
R₁ is selected from the group consisting of -Cl, -F, and C₁₋₆alkyl substituted with one or more substituents independently selected from -Cl and -F;
R₂ is hydrogen;
each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆, - C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
n is selected from the group consisting of 0, 1, 2, 3, and 4;
R₄ and R₅ are each independently hydrogen or C₁₋₆alkyl, or R₄ and R₅ may be taken together with the nitrogen to which R₄ and R₅ are attached to form a 4-7 membered heterocyclyl optionally substituted with one or more halogens;
each R₆ is independently selected from the group consisting of C₁₋₆alkyl, C₂₋₆alkenyl, C₂₋₆alkynyl, C₃₋₇cycloalkyl, phenyl, and benzyl; and
s is 1 or 2.

In another aspect, the present disclosure features a pharmaceutical composition comprising a compound of Formula **I-I:** 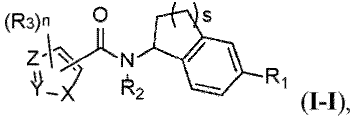 or a pharmaceutically acceptable salt thereof, wherein
X is selected from the group consisting of NH, O, and S, wherein the hydrogen of NH may be substituted with R₃;
Y is selected from N and CH, wherein the hydrogen of CH may be substituted with R₃;
Z is selected from N and CH, wherein the hydrogen of CH may be substituted with R₃, or Z is C when Z is substituted with the -C(O)N(R₂)- moiety;
R₁ is selected from the group consisting of -Cl, -F, and C₁₋₆alkyl substituted with one or more substituents independently selected from -Cl and -F;
R₂ is hydrogen;
each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆, - C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
n is selected from the group consisting of 0, 1, 2, 3, and 4;
R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and
s is 1 or 2;
and a pharmaceutically acceptable excipient.

In some embodiments, the present invention features a pharmaceutical composition of a compound of Formula **I:** 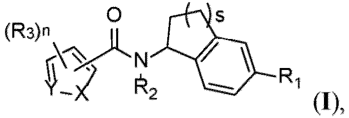
or a pharmaceutically acceptable salt thereof, wherein
   X is selected from the group consisting of NH, O, and S, wherein the hydrogen of NH may be substituted with R₃;
Y is selected from N and CH, wherein the hydrogen of CH may be substituted with R₃;
   R₁ is selected from the group consisting of -Cl, -F, and C₁₋₆alkyl substituted with one or more substituents independently selected from -Cl and -F;
   R₂ is hydrogen;
   each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆, - C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
n is selected from the group consisting of 0, 1, 2, 3, and 4;
   R₄ and R₅ are each independently hydrogen or C₁₋₆alkyl, or R₄ and R₅ may be taken together with the nitrogen to which R₄ and R₅ are attached to form a 4-7 membered heterocyclyl optionally substituted with one or more halogens;
   each R₆ is independently selected from the group consisting of C₁₋₆alkyl, C₂₋₆alkenyl, C₂₋₆alkynyl, C₃₋₇cycloalkyl, phenyl, and benzyl; and
   s is 1 or 2;
   and a pharmaceutically acceptable excipient.

In some embodiments of Formula 1-1, x is NH. In some embodiments of Formula 1-1, x is O. In some embodiments of Formula 1-1, x is S.

In some embodiments of Formula 1-1, Y is N. In some embodiments of Formula 1-1, Y is CH.

In some embodiments of Formula 1-1, Z is N. In some embodiments of Formula 1-1, Z is CH. In some embodiments of Formula 1-1, Z is C when Z is substituted with the -C(O)N(R₂)- moiety.

In some embodiments of Formula I, x is NH. In some embodiments of Formula I, x is O. In some embodiments of Formula I, x is S.

In some embodiments of Formula I, Y is N. In some embodiments of Formula I, Y is CH.

In some embodiments, the compound of Formula 1-1 is a compound of Formula I (e.g., I-a, I-a1, I-b, I-b1, I-c, I-c1, 1-d, or I-d1).

In some embodiments, the compound of Formula **I-I** is a compound of Formula **I-Ia** or Formula **I-Ia1:** 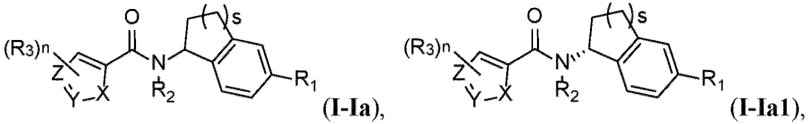 or a pharmaceutically acceptable salt thereof.

In some embodiments, the compound of Formula **I-I** is a compound of Formula **I-Ib** or Formula **I-Ib1:** 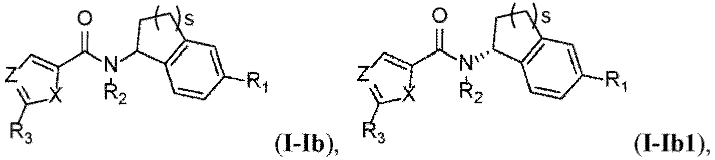 or a pharmaceutically acceptable salt thereof.

In some embodiments, the compound of Formula **I-I** is a compound of Formula **I-Ic** or Formula **I-Ic1**: 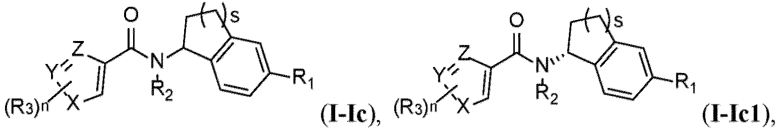 or a pharmaceutically acceptable salt thereof.

In some embodiments, the compound of Formula **1-1** or Formula **I** is a compound of Formula **I-a** or Formula **I-a1**: 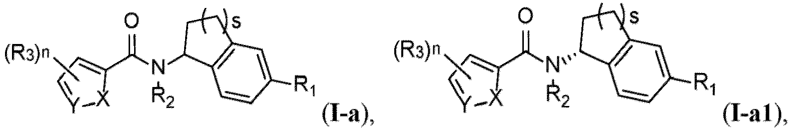 or a pharmaceutically acceptable salt thereof.

In some embodiments, the compound of Formula **1-1** or Formula **I** is a compound of Formula **1-b** or Formula **I-b1**: 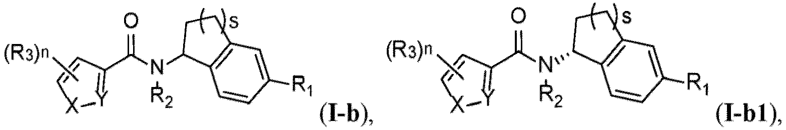 or a pharmaceutically acceptable salt thereof.

In some embodiments, the compound of Formula **1-1** or Formula **I** is a compound of Formula **I-c** or Formula **I-c1**: 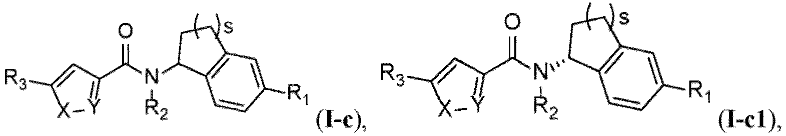 or a pharmaceutically acceptable salt thereof.

In some embodiments, the compound of Formula **1-1** or Formula **I** is a compound of Formula **I-d** or Formula **I-d1**: 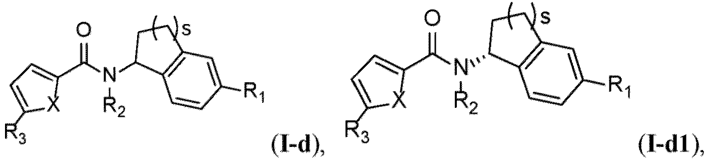 or a pharmaceutically acceptable salt thereof.

In some embodiments, the compound of Formula **I-I** is a compound of Formula **I-Ia1:** 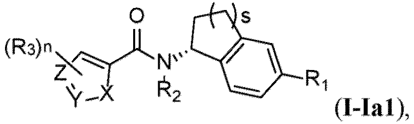 or a pharmaceutically acceptable salt thereof.

In some embodiments, the compound of Formula **I-I** is a compound of Formula **I-Ib1:** 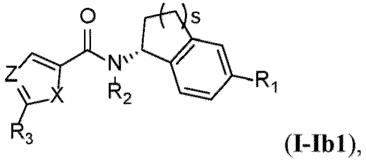 or a pharmaceutically acceptable salt thereof.

In some embodiments, the compound of Formula **I-I** is a compound of Formula **I-Ic1**: 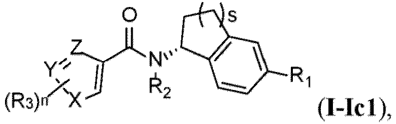 or a pharmaceutically acceptable salt thereof,

In some embodiments, the compound of Formula **1-1** or Formula **I** is a compound of Formula **I-a1**: 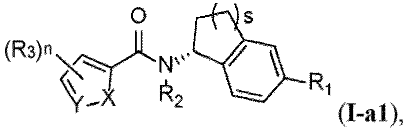 or a pharmaceutically acceptable salt thereof.

In some embodiments, the compound of Formula **1-1** or Formula **I** is a compound of Formula **I-b1**: 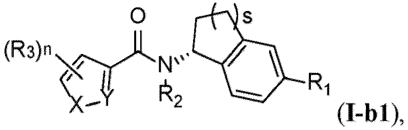 or a pharmaceutically acceptable salt thereof.

In some embodiments, the compound of Formula **1-1** or Formula **I** is a compound of Formula **I-c1**: 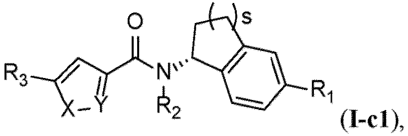 or a pharmaceutically acceptable salt thereof.

In some embodiments, the compound of Formula **1-1** or Formula **I** is a compound of Formula **I-d1**: 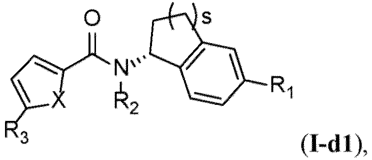 or a pharmaceutically acceptable salt thereof.

In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), R₁ is selected from the group consisting of -Cl, -F, and -CF₃. In some embodiments of Formula I-I (e.g., (I-Ia), (I-Ia1), (I-Ib), (I-Ib1), (I-Ic), (I-Ic1), (1-a), (I-a1), (1-b), (I-b1), (I-c), (I-c1), (1-d), or (I-d1)), R₁ is -Cl. In some embodiments of Formula I-I (e.g., (I-Ia), (I-Ia1), (1-1b), (I-Ib1), (I-Ic), (I-Ic1), (1-a), (1-a1), (1-b), (I-b1), (1-c), (I-c1), (1-d), or (I-d1)), R₁ is -F. In some embodiments of Formula I-I (e.g., (I-Ia), (I-Ia1), (I-Ib), (I-Ib1), (I-Ic), (I-Ic1), (1-a), (I-a1), (1-b), (I-b1), (I-c), (I-c1), (1-d), or (I-d1)), R₁ is -CF₃.

In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₃ is selected from the group consisting of -Cl, methyl, methyl substituted with -NR₄R₅ or -S(O)₂R₆, methoxymethyl, trifluoromethyl, ethyl, cyclopropyl, cyclohexyl, -S(O)₂R₆, -C(O)NR₄R₅, and -S(O)₂NR₄R₅. In some embodiments of Formula I-I (e.g., (I-Ia), (I-Ia1), (1-1b), (I-Ib1), (1-1c), (I-Ic1), (1-a), (I-a1), (1-b), (I-b1), (1-c), (I-c1), (1-d), or (I-d1)), each R₃ is selected from the group consisting of -Cl, methyl, methyl substituted with -NR₄R₅ or -S(O)₂R₆, methoxymethyl, trifluoromethyl, ethyl, cyclopropyl, -S(O)₂R₆, -C(O)NR₄R₅, and - S(O)₂NR₄R₅. In some embodiments of Formula I-I (e.g., (I-Ia), (I-Ia1), (1-1b), (I-Ib1), (1-1c), (I-Ic1), (1-a), (I-a1), (1-b), (I-b1), (1-c), (I-c1), (1-d), or (I-d1)), each R₃ is selected from the group consisting of methyl, ethyl, cyclopropyl, cyclohexyl, and -S(O)₂NR₄R₅. In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₃ is selected from the group consisting of methyl, cyclopropyl, and -S(O)₂NR₄R₅.

In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₃ is methyl. In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₃ is cyclopropyl. In some embodiments of Formula I-I (e.g., (I-Ia), (I-Ia1), (I-Ib), (I-Ib1), (I-Ic), (I-Ic1), (1-a), (I-a1), (1-b), (I-b1), (1-c), (I-c1), (1-d), or (I-d1)), each R₃ is -S(O)₂NR₄R₅. In some embodiments of Formula I-I (e.g., (I-Ia), (I-Ia1), (I-Ib), (I-Ib1), (1-1c), (I-Ic1), (1-a), (I-a1), (1-b), (I-b1), (I-c), (I-c1), (1-d), or (1-d1)), R₃ is C₁₋₆alkyl substituted with -NR₄R₅ or -S(O)₂R₆.

In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), n is 1 or 2. In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), n is 2. In some embodiments of Formula I-I (e.g., (I-Ia), (I-Ia1), (I-Ib), (I-Ib1), (I-Ic), (I-Ic1), (1-a), (I-a1), (1-b), (I-b1), (I-c), (I-c1), (1-d), or (I-d1)), n is 1.

In some embodiments of Formula I-I (e.g., (I-Ia), (I-Ia1), (1-1b), (I-Ib1), (1-1c), (I-Ic1), (1-a), (I-a1), (1-b), (I-b1), (1-c), (I-c1), (1-d), or (I-d1)), each of R₄ and R₅ are independently selected from the group consisting of hydrogen, methyl, ethyl, cyclopropyl, and -C(O)CH₃. In some embodiments of Formula I-I (e.g., (I-Ia), (I-Ia1), (1-1b), (I-Ib1), (1-Ic), (I-Ic1), (1-a), (I-a1), (1-b), (I-b1), (1-c), (I-c1), (1-d), or (I-d1)), each of R₄ and R₅ are independently hydrogen or methyl. In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₄ and R₅ are hydrogen. In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₄ and R₅ are methyl. In some embodiments of Formula I-I (e.g., (I-Ia), (I-Ia1), (1-1b), (I-Ib1), (I-Ic), (1-Ic1), (1-a), (I-a1), (1-b), (I-b1), (1-c), (I-c1), (1-d), or (I-d1)), R₄ is H and R₅ is methyl. In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), R₄ and R₅ are taken together with the nitrogen to which R₄ and R₅ are attached to form a 4-6 membered heterocyclyl optionally substituted with -OH, methyl, or -OCH₃.

In some embodiments of of Formula I-I (e.g., (I-Ia), (I-Ia1), (I-Ib), (I-Ib1), (1-Ic), (I-Ic1), (1-a), (I-a1), (1-b), (I-b1), (1-c), (I-c1), (1-d), or (I-d1)), R₆ is selected from the group consisting of methyl, ethyl, methoxyethyl, and cyclopropyl.

In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), each R₃ is selected from the group consisting of -Cl, methyl, methyl substituted with -NR₄R₅ or -S(O)₂R₆, methoxymethyl, trifluoromethyl, ethyl, cyclopropyl, -S(O)₂R₆, -C(O)NR₄R₅, and - S(O)₂NR₄R₅ wherein each of R₄ and R₅ are independently selected from the group consisting of hydrogen, methyl, and -C(O)CH₃; wherein R₆ is selected from the group consisting of methyl, ethyl, methoxyethyl, and cyclopropyl.

In some embodiments of Formula I-I (e.g., (I-Ia), (I-Ia1), (1-1b), (I-Ib1), (I-Ic), (I-Ic1), (1-a), (I-a1), (1-b), (I-b1), (1-c), (I-c1), (1-d), or (I-d1)), s is 2. In some embodiments of Formula 1-1 (e.g., (I-Ia), (I-Ia1), (I-Ib), (I-Ib1), (I-Ic), (I-Ic1), (1-a), (I-a1), (1-b), (I-b1), (1-c), (I-c1), (1-d), or (I-d1)), s is 1.

In some embodiments of Formula I (e.g., (1-a), (I-a1), (1-b), (I-b1), (1-c), (I-c1), (1-d), or (I-d1)), or a pharmaceutically acceptable salt thereof), R₁ is selected from the group consisting of -Cl, -F, and -CF₃.

In some embodiments of Formula I (e.g., (1-a), (I-a1), (1-b), (I-b1), (1-c), (I-c1), (1-d), or (I-d1)), or a pharmaceutically acceptable salt thereof), R₁ is -Cl. In some embodiments of Formula I (e.g., (1-a), (I-a1), (1-b), (I-b1), (1-c), (I-c1), (1-d), or (I-d1)), or a pharmaceutically acceptable salt thereof), R₁ is -F. In some embodiments of Formula I (e.g., (1-a), (I-a1), (1-b), (I-b1), (1-c), (I-c1), (1-d), or (I-d1)), or a pharmaceutically acceptable salt thereof), R₁ is -CF₃.

In some embodiments of Formula I (e.g., (1-a), (I-a1), (1-b), (I-b1), (1-c), (I-c1), (1-d), or (I-d1)), or a pharmaceutically acceptable salt thereof), R₃ is selected from the group consisting of methyl, ethyl, cyclopropyl, cyclohexyl, and -S(O)₂NR₄R₅. In some embodiments of Formula I (e.g., (1-a), (I-a1), (1-b), (I-b1), (1-c), (I-c1), (1-d), or (I-d1)), or a pharmaceutically acceptable salt thereof), R₃ is selected from the group consisting of methyl, cyclopropyl, and -S(O)₂NR₄R₅. In some embodiments of Formula I (e.g., (1-a), (I-a1), (1-b), (I-b1), (1-c), (I-c1), (1-d), or (I-d1)), or a pharmaceutically acceptable salt thereof), R₃ is methyl. In some embodiments of Formula I (e.g., (1-a), (I-a1), (1-b), (I-b1), (1-c), (I-c1), (1-d), or (I-d1)), or a pharmaceutically acceptable salt thereof), R₃ is cyclopropyl. In some embodiments of Formula I (e.g., (1-a), (I-a1), (1-b), (I-b1), (1-c), (I-c1), (1-d), or (I-d1)), or a pharmaceutically acceptable salt thereof), R₃ is -S(O)₂NR₄R₅.

In some embodiments of Formula I, n is 1 or 2. In some embodiments of Formula I, n is 2. In some embodiments of Formula I, n is 1.

In some embodiments of Formula I (e.g., (1-a), (I-a1), (1-b), (I-b1), (1-c), (I-c1), (1-d), or (I-d1)), or a pharmaceutically acceptable salt thereof), each of R₄ and R₅ are independently hydrogen or methyl. In some embodiments of Formula I (e.g., (1-a), (I-a1), (I-b), (I-b1), (1-c), (I-c1), (1-d), or (I-d1)), or a pharmaceutically acceptable salt thereof), each R₄ and R₅ are hydrogen. In some embodiments of Formula I (e.g., (I-a), (I-a1), (I-b), (I-b1), (I-c), (I-c1), (1-d), or (I-d1)), or a pharmaceutically acceptable salt thereof), each R₄ and R₅ are methyl. In some embodiments of Formula I (e.g., (I-a), (I-a1), (I-b), (I-b1), (I-c), (I-c1), (1-d), or (I-d1)), or a pharmaceutically acceptable salt thereof), R₄ is H and R₅ is methyl.

In some embodiments of Formula I (e.g., (I-a), (I-a1), (I-b), (I-b1), (I-c), (I-c1), (1-d), or (I-d1)), or a pharmaceutically acceptable salt thereof), s is 2. In some embodiments of Formula I (e.g., (I-a), (I-a1), (I-b), (I-b1), (I-c), (I-c1), (I-d), or (I-d1)), or a pharmaceutically acceptable salt thereof), s is 1.

In some embodiments, the compound of Formula 1-1 is selected from the group consisting of: 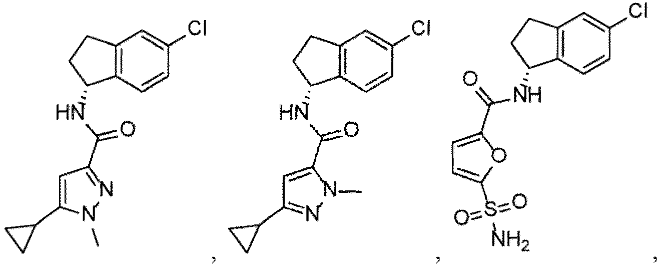 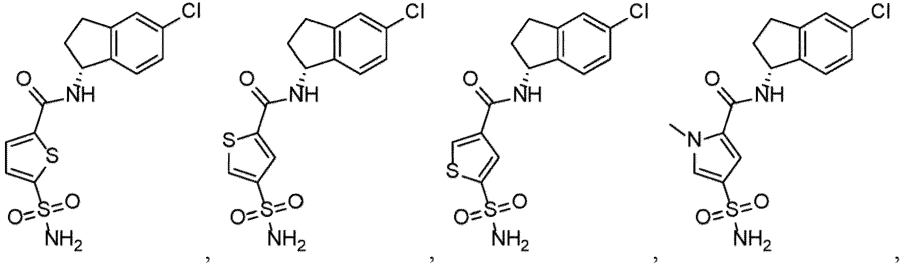 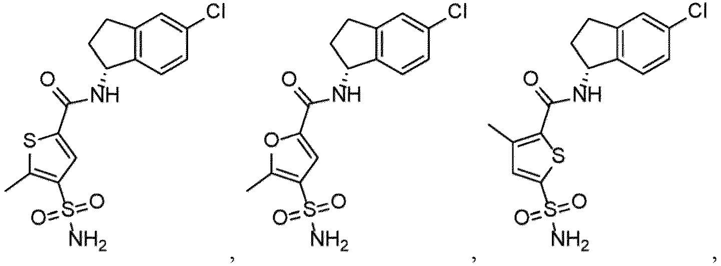 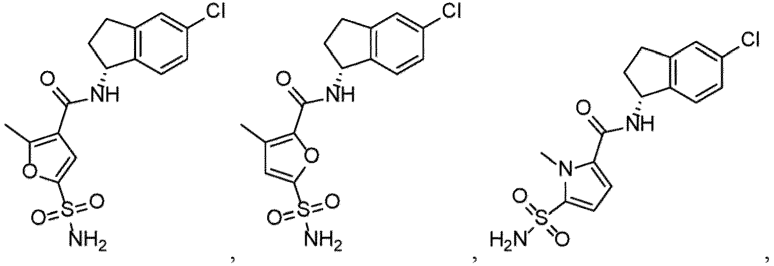 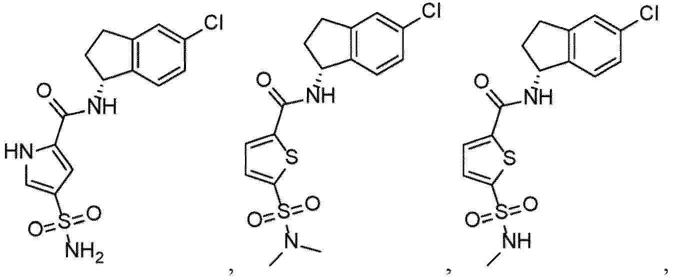 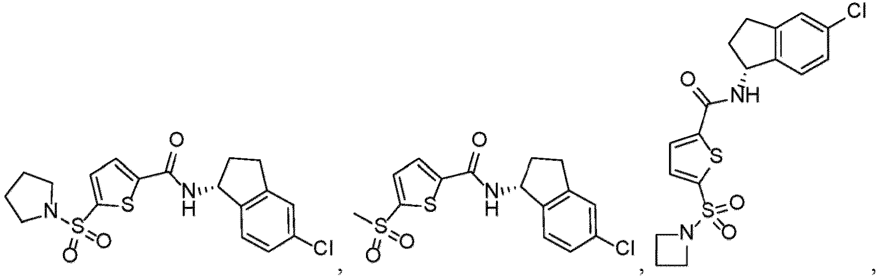 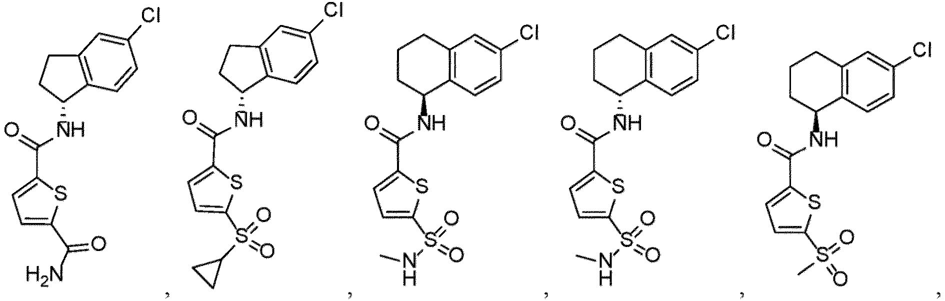 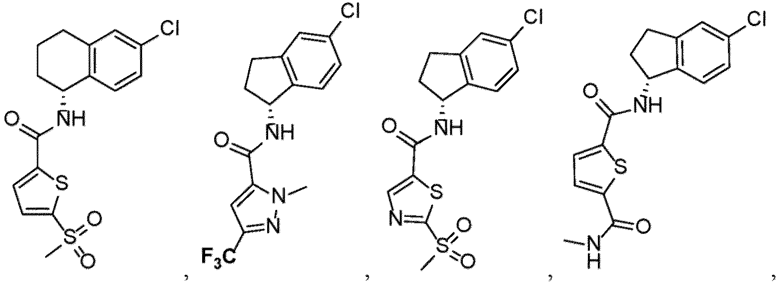 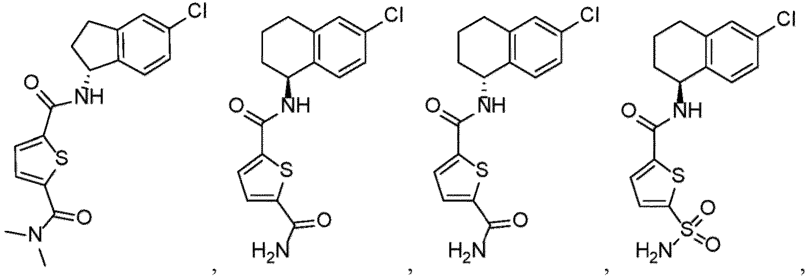 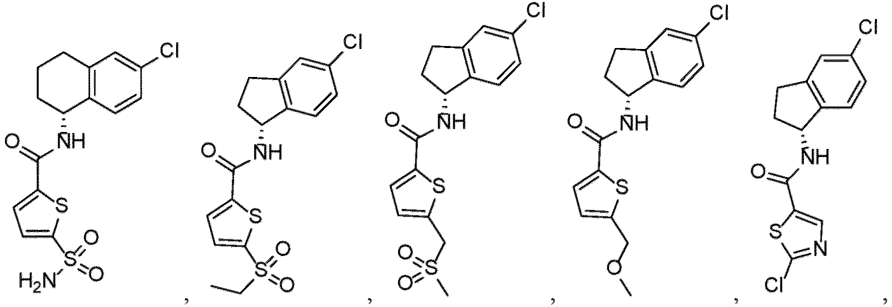 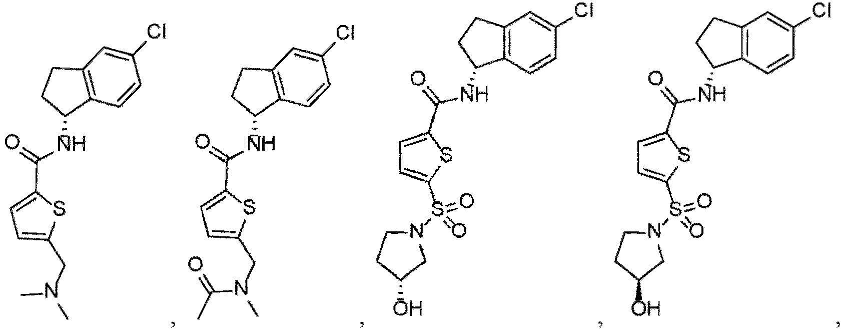 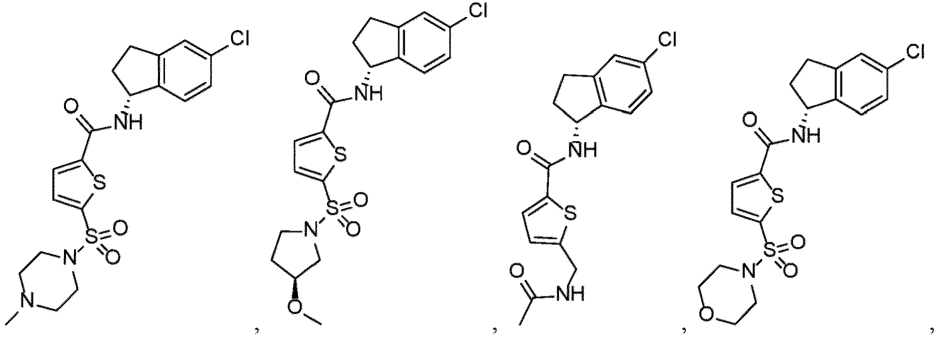 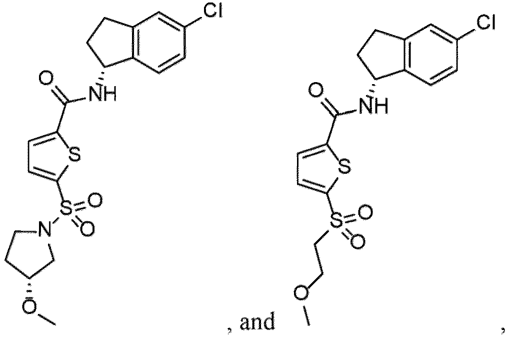 or a pharmaceutically acceptable salt thereof.

In another aspect, the present invention features a compound of Formula **II:** 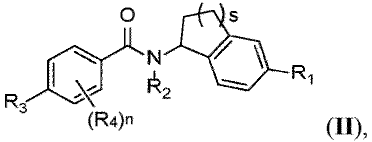 or a pharmaceutically acceptable salt thereof, wherein
R₁ is selected from the group consisting of -Cl, -F, and C₁₋₆alkyl substituted with one or more substituents independently selected from -Cl and -F;
R₂ is hydrogen;
R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl, - NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and - S(O)₂-C₁₋₆alkyl, wherein the C₁₋₆alkyl is optionally substituted with one or more halogen or C₁₋₆alkoxy;
each R₄ is independently selected from the group consisting of C₁₋₆alkyl, halogen, and -OH; wherein R₄ is substituted at the carbon adjacent to R₃ when R₄ is -OH;
n is selected from the group consisting of 0, 1, 2, 3, and 4;
R₅, R₆, R₉, and R₁₀ are each independently hydrogen or C₁₋₆alkyl;
each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
s is 1 or 2.

In another aspect, the present invention features a pharmaceutical composition comprising a compound of Formula **II:** 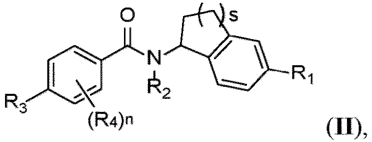 or a pharmaceutically acceptable salt thereof, wherein
R₁ is selected from the group consisting of -Cl, -F, and C₁₋₆alkyl substituted with one or more substituents independently selected from -Cl and -F;
R₂ is hydrogen;
R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl, - NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and - S(O)₂-C₁₋₆alkyl, wherein the C₁₋₆alkyl is optionally substituted with one or more halogen or C₁₋₆alkoxy;
each R₄ is independently selected from the group consisting of C₁₋₆alkyl, halogen, and -OH; wherein R₄ is substituted at the carbon adjacent to R₃ when R₄ is -OH;
n is selected from the group consisting of 0, 1, 2, 3, and 4;
R₅, R₆, R₉, and R₁₀ are each independently hydrogen or C₁₋₆alkyl;
each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
s is 1 or 2;
and a pharmaceutically acceptable excipient.

In some embodiments, the compound of Formula **II** is a compound of Formula **II-a,** Formula **II-a1,** or Formula **II-a2:** 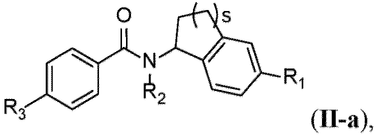 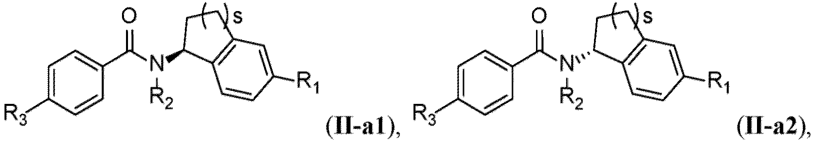 or a pharmaceutically acceptable salt thereof.

In some embodiments, the compound of Formula **II** is a compound of Formula **II-b,** Formula **II-b1**, or Formula **II-b2:** 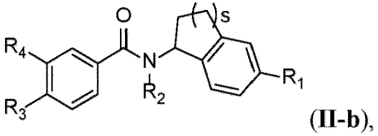 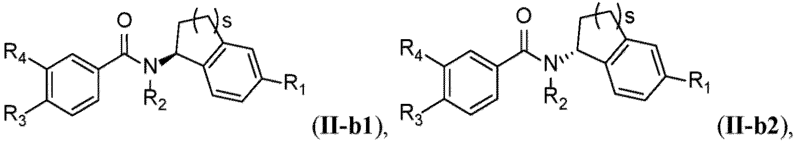 or a pharmaceutically acceptable salt thereof.

In some embodiments, the compound of Formula **II** is a compound of Formula **II-c**, Formula **II-c1**, or Formula **II-c2:** 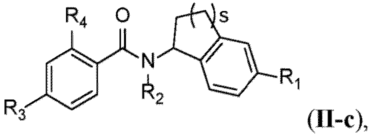 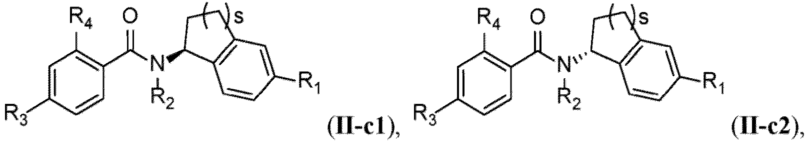 or a pharmaceutically acceptable salt thereof.

In some embodiments, the compound of Formula **II** is a compound of Formula **II-a2:** 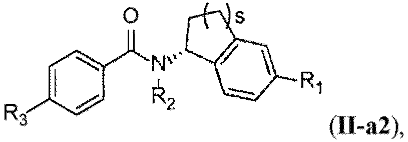 or a pharmaceutically acceptable salt thereof.

In some embodiments, the compound of Formula **II** is a compound of Formula **II-b2:** 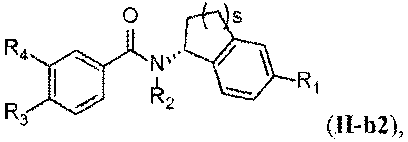 or a pharmaceutically acceptable salt thereof.

In some embodiments, the compound of Formula **II** is a compound of Formula **II-c2:** 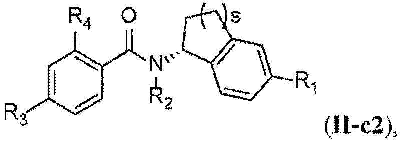 or a pharmaceutically acceptable salt thereof.

In some embodiments of Formula (II) (e.g., (II-a), (II-a1), (II-a2), (II-b), (11-b1), (II-b2), (II-c), (II-c1), (II-c2)), R₁ is selected from the group consisting of -Cl, -F, and - CF₃. In some embodiments of Formula (II) (e.g., (II-a), (II-a1), (II-a2), (II-b), (II-b1), (II-b2), (II-c), (II-c1), (II-c2)), R₁ is -Cl. In some embodiments of Formula (II) (e.g., (II-a), (II-a1), (II-a2), (II-b), (II-b1), (II-b2), (II-c), (II-c1), (II-c2)), R₁ is -F. In some embodiments of Formula (II) (e.g., (II-a), (II-a1), (II-a2), (II-b), (II-b1), (II-b2), (II-c), (II-c1), (II-c2)), R₁ is - CF₃.

In some embodiments of Formula (II) (e.g., (II-a), (II-a1), (II-a2), (II-b), (II-b1), (II-b2), (II-c), (II-c1), (II-c2)), R₃ is selected from the group consisting of -F, methoxy, - NH₂,

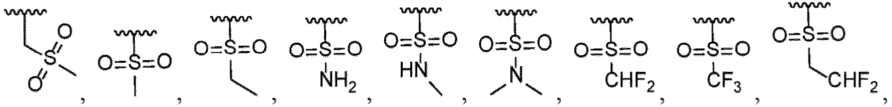

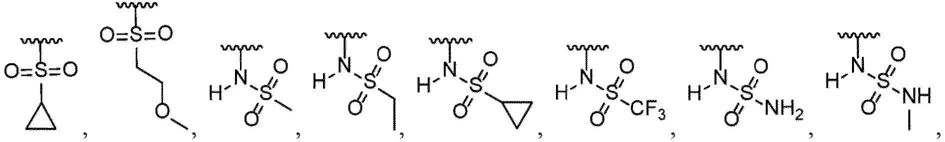

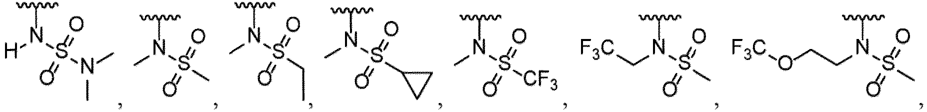

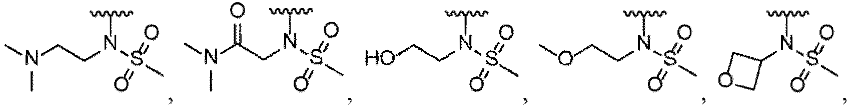

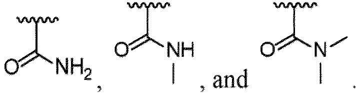

In some embodiments of Formula (II) (e.g., (II-a), (II-a1), (II-a2), (II-b), (II-b1), (II-b2), (II-c), (II-c1), (II-c2)), R₃ is selected from the group consisting of

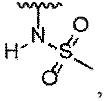

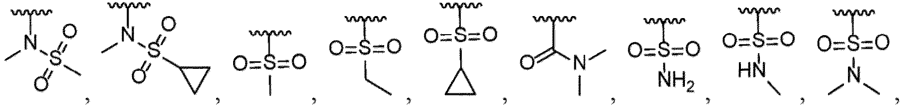

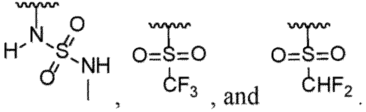

In some embodiments of Formula (II) (e.g., (II-a), (II-a1), (II-a2), (II-b), (II-b1), (II-b2), (II-c), (II-c1), (II-c2)), R₃ is 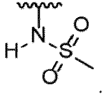 In some embodiments of Formula (II) (e.g., (11-a), (II-a1), (11-a2), (11-b), (II-b1), (11-b2), (11-c), (II-c1), (II-c2)), R₃ is 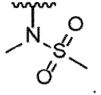 In some embodiments of Formula (II) (e.g., (11-a), (II-a1), (11-a2), (11-b), (II-b1), (11-b2), (II-c), (II-c1), (II-c2)), R₃ is 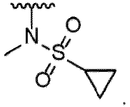 In some embodiments of Formula (II) (e.g., (11-a), (II-a1), (11-a2), (11-b), (II-b1), (11-b2), (11-c), (II-c1), (II-c2)), R₃ is 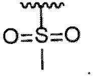 In some embodiments of Formula (II) (e.g., (11-a), (II-a1), (11-a2), (11-b), (II-b1), (11-b2), (11-c), (II-c1), (II-c2)), R₃ is 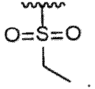 In some embodiments of Formula (II) (e.g., (11-a), (II-a1), (11-a2), (11-b), (II-b1), (11-b2), (11-c), (II-c1), (II-c2)), R₃ is 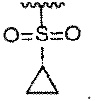 In some embodiments of Formula (II) (e.g., (11-a), (II-a1), (11-a2), (11-b), (II-b1), (11-b2), (11-c), (II-c1), (II-c2)), R₃ is 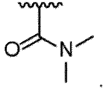 In some embodiments of Formula (II) (e.g., (11-a), (II-a1), (11-a2), (11-b), (II-b1), (11-b2), (II-c), (II-c1), (II-c2)), R₃ is 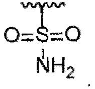 In some embodiments of Formula (II) (e.g., (11-a), (II-a1), (II-a2), (11-b), (II-b1), (11-b2), (11-c), (II-c1), (II-c2)), R₃ is 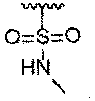 In some embodiments of Formula (II) (e.g., (11-a), (II-a1), (11-a2), (11-b), (II-b1), (11-b2), (11-c), (II-c1), (II-c2)), R₃ is 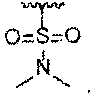 In some embodiments of Formula (II) (e.g., (11-a), (II-a1), (11-a2), (11-b), (II-b1), (11-b2), (11-c), (II-c1), (II-c2)), R₃ is 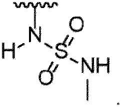 In some embodiments of Formula (II) (e.g., (11-a), (11-a1), (11-a2), (11-b), (II-b1), (11-b2), (11-c), (II-c1), (II-c2)), R₃ is 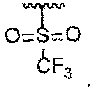 In some embodiments of Formula (II) (e.g., (11-a), (II-a1), (11-a2), (11-b), (II-b1), (11-b2), (II-c), (II-c1), (II-c2)), R₃ is

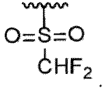

In some embodiments of Formula (II) (e.g., (11-a), (II-a1), (11-a2), (11-b), (11-b1), (11-b2), (11-c), (II-c1), (II-c2)), R₄ is -F or methyl. In some embodiments of Formula (II) (e.g., (11-a), (II-a1), (11-a2), (11-b), (II-b1), (11-b2), (11-c), (II-c1), (II-c2)), R₄ is -F. In some embodiments of Formula (II) (e.g., (11-a), (II-a1), (11-a2), (11-b), (II-b1), (11-b2), (II-c), (II-c1), (II-c2)), R₄ is methyl.

In some embodiments of Formula (II), n is 1.

In some embodiments of Formula (II) (e.g., (11-a), (II-a1), (11-a2), (11-b), (11-b1), (11-b2), (11-c), (II-c1), (II-c2)), each R₅, R₆, R₉, and R₁₀ are hydrogen. In some embodiments of Formula (II) (e.g., (11-a), (II-a1), (11-a2), (11-b), (II-b1), (11-b2), (II-c), (II-c1), (II-c2)), R₅, R₆, R₉, and R₁₀ are methyl.

In some embodiments of Formula (II) (e.g., (11-a), (II-a1), (11-a2), (11-b), (11-b1), (11-b2), (11-c), (II-c1), (II-c2)), R₅ is H and R₆ is methyl.

In some embodiments of Formula (II) (e.g., (11-a), (II-a1), (11-a2), (11-b), (11-b1), (11-b2), (11-c), (II-c1), (II-c2)), R₇ is selected from the group consisting of hydrogen, methyl, ethyl, and 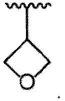 In some embodiments of Formula (II) (e.g., (11-a), (II-a1), (11-a2), (11-b), (II-b1), (11-b2), (II-c), (II-c1), (II-c2)), R₇ is hydrogen. In some embodiments of Formula (II) (e.g., (11-a), (II-a1), (11-a2), (11-b), (II-b1), (11-b2), (11-c), (II-c1), (II-c2)), R₇ is methyl. In some embodiments of Formula (II) (e.g., (11-a), (II-a1), (11-a2), (11-b), (II-b1), (11-b2), (11-c), (II-c1), (II-c2)), R₇ is ethyl. In some embodiments of Formula (II) (e.g., (11-a), (11-a1), (11-a2), (11-b), (II-b1), (11-b2), (11-c), (II-c1), (II-c2)), R₇ is

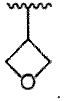

In some embodiments of Formula (II) (e.g., (11-a), (II-a1), (11-a2), (11-b), (11-b1), (11-b2), (11-c), (II-c1), (II-c2)), s is 1. In some embodiments of Formula (II) (e.g., (11-a), (II-a1), (11-a2), (11-b), (II-b1), (11-b2), (11-c), (II-c1), (II-c2)), s is 2.

In some embodiments, the compound of Formula II is selected from the group consisting of: 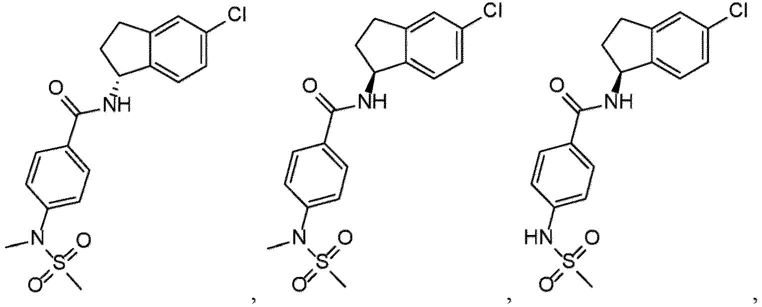 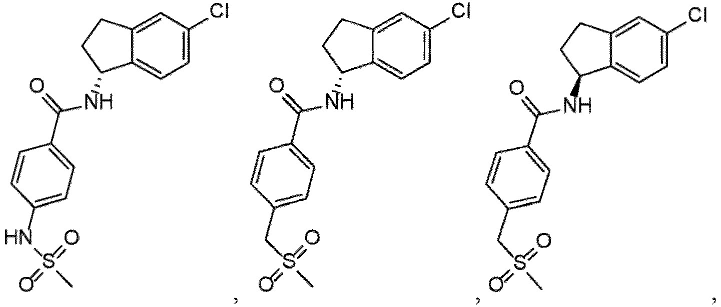 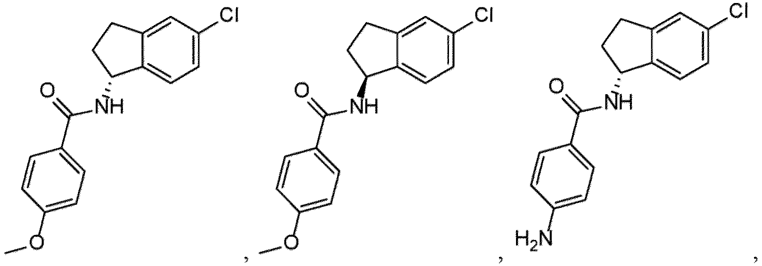 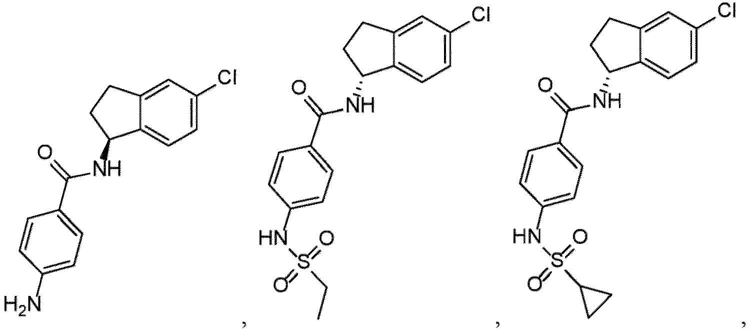 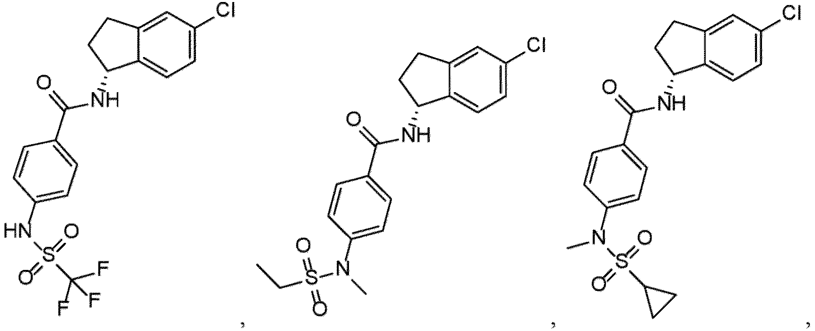 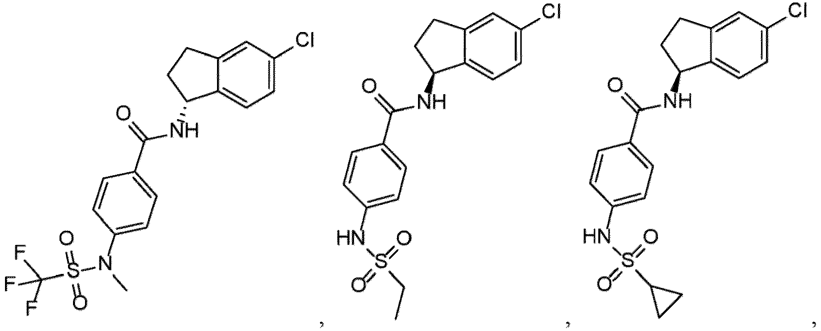 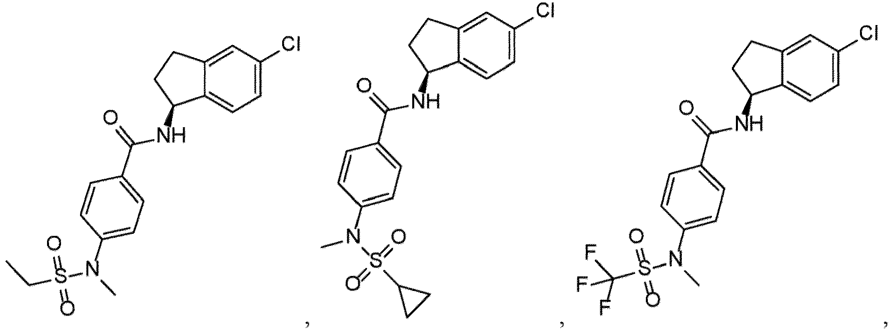 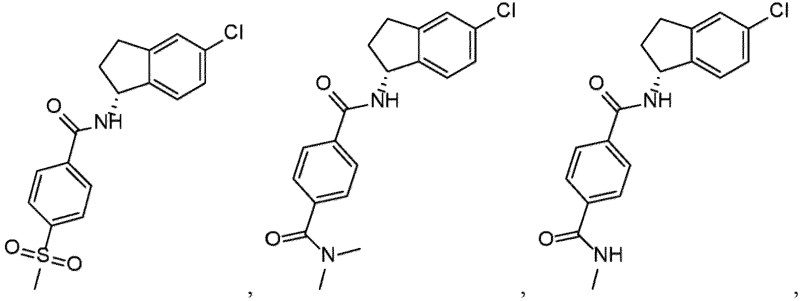 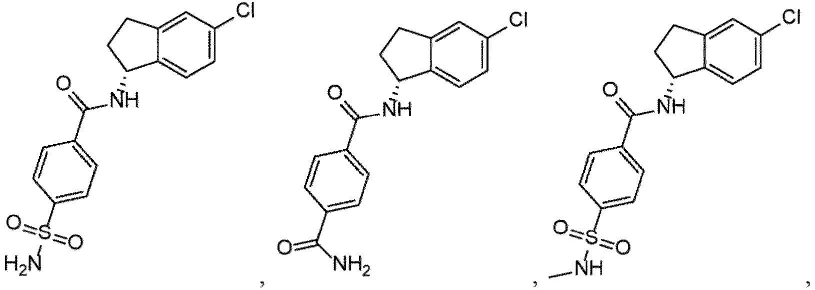 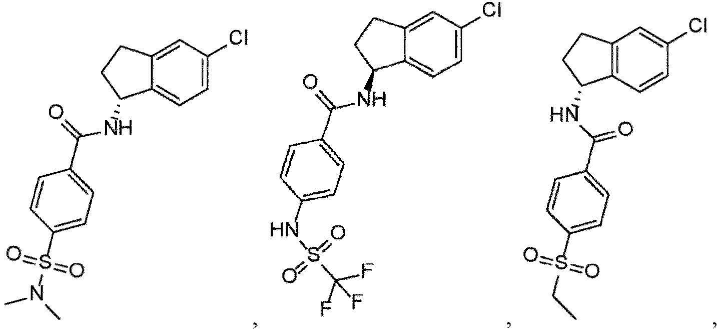 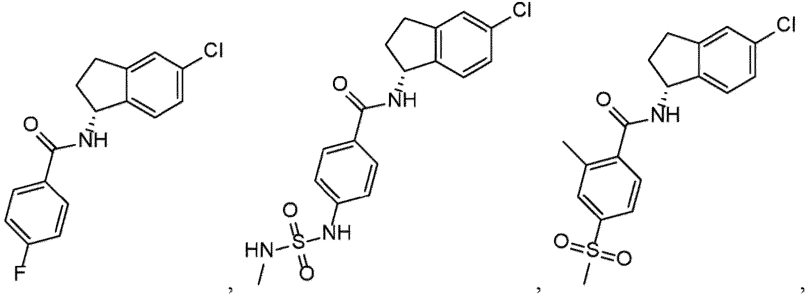 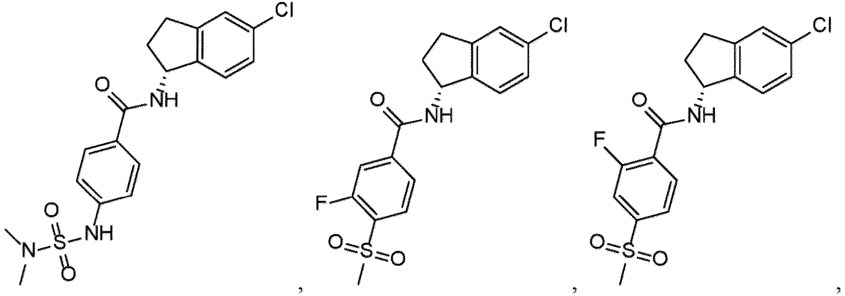 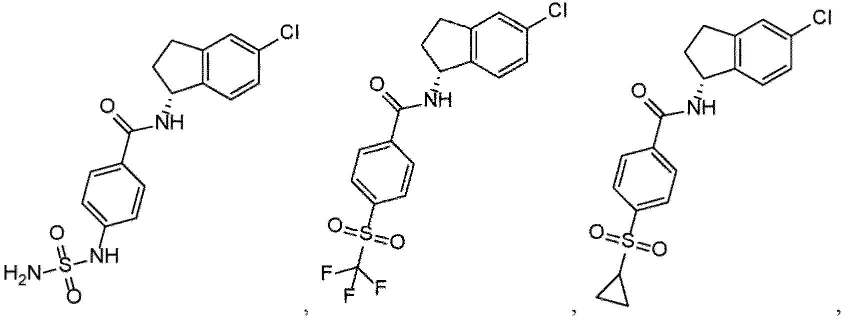 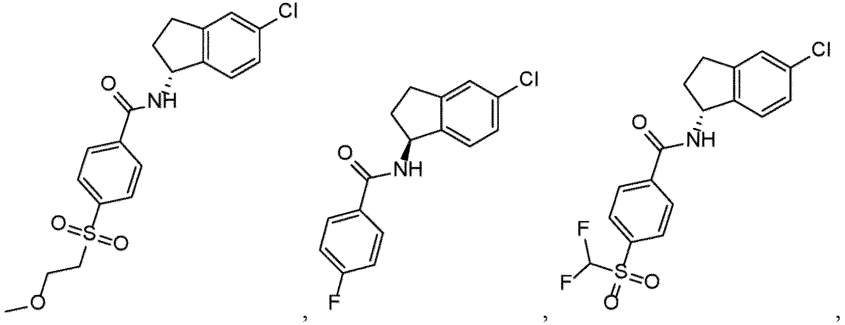 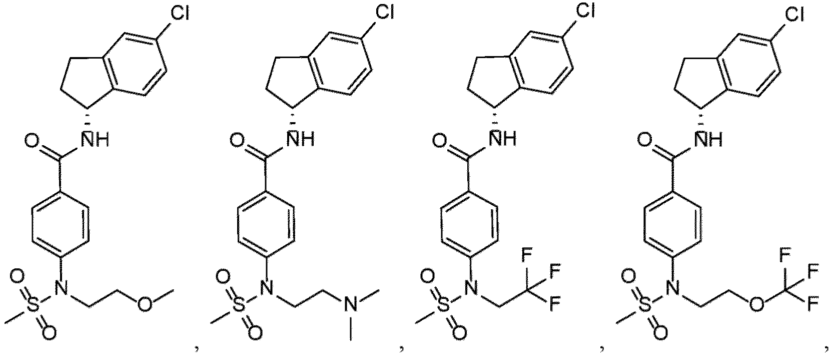 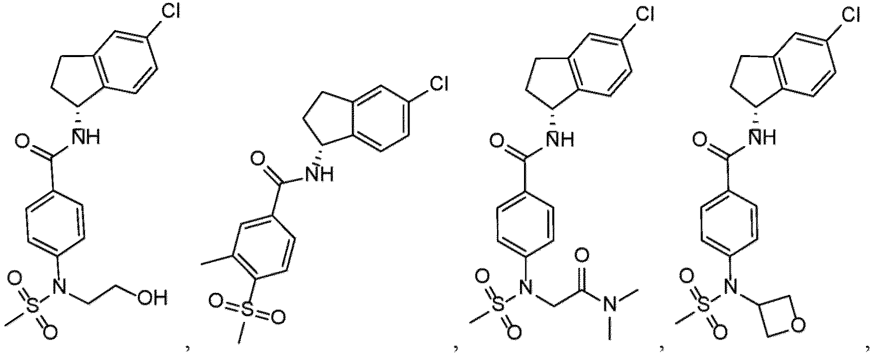 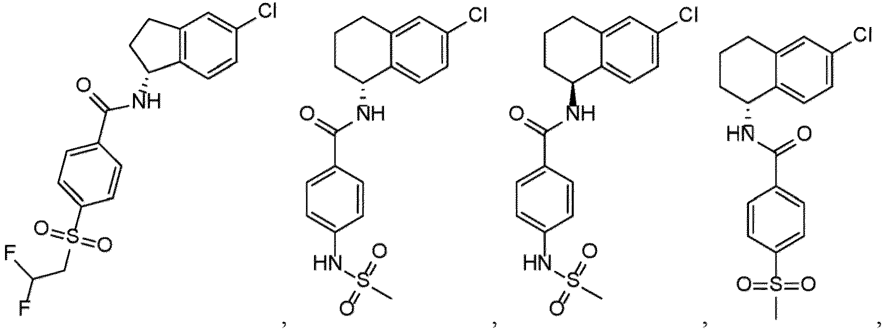 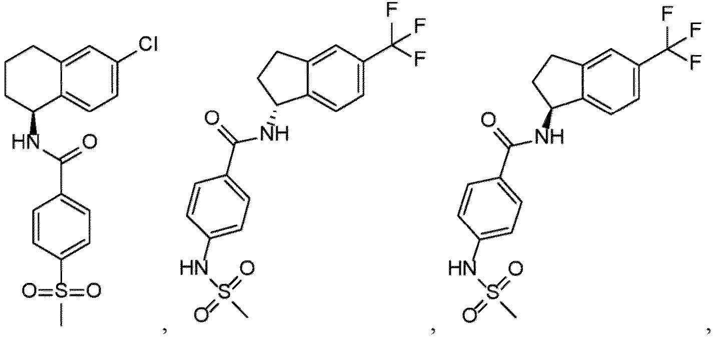 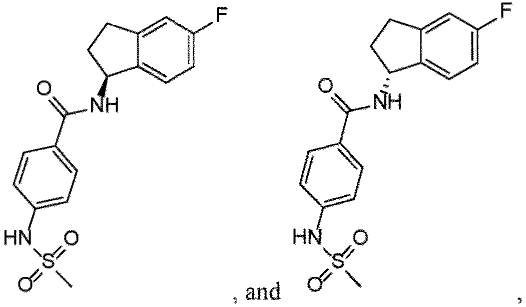 or a pharmaceutically acceptable salt thereof.

In another aspect, the present invention features a compound of Formula **III:** 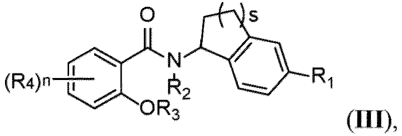 or a pharmaceutically acceptable salt thereof, wherein
R₁ is selected from the group consisting of -Cl, -F, and C₁₋₆alkyl substituted with one or more substituents independently selected from -Cl and -F;
R₂ is hydrogen;
R₃ is hydrogen;
R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
n is selected from the group consisting of 0, 1, 2, 3, and 4;
R₇ and R₈ are each independently hydrogen or C₁₋₆alkyl; and
s is 1 or 2.

In another aspect, the present invention features a pharmaceutical composition comprising a compound of Formula **III:** 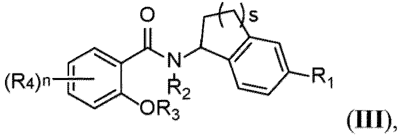
or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is hydrogen;
   R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
n is selected from the group consisting of 0, 1, 2, 3, and 4;
   R₇ and R₈ are each independently hydrogen or C₁₋₆alkyl; and
s is 1 or 2;
and a pharmaceutically acceptable excipient.

In some embodiments, the compound of Formula **III** is a compound of Formula **III-a,** Formula **III-a1**, or Formula **III-a2:** 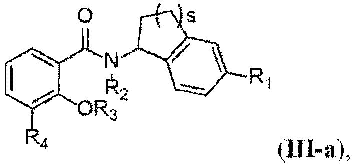 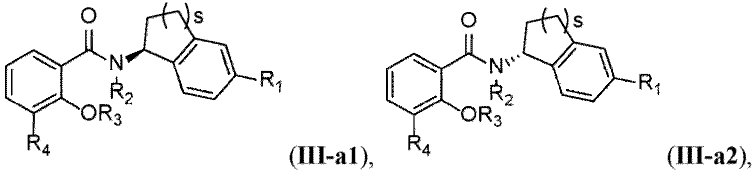 or a pharmaceutically acceptable salt thereof.

In some embodiments, the compound of Formula III is a compound of Formula **III-b,** Formula **III-b1**, or Formula **III-b2:** 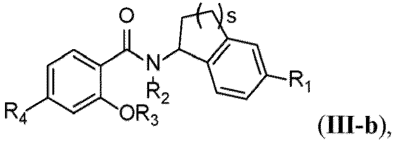 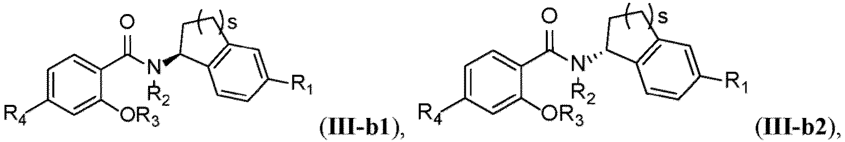 or a pharmaceutically acceptable salt thereof.

In some embodiments, the compound of Formula **III** is a compound of Formula **III-c,** Formula **III-c1,** or Formula **III-c2:** 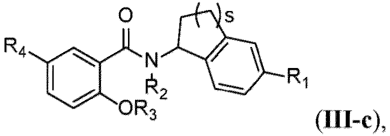 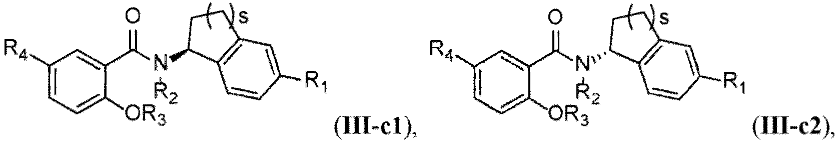 or a pharmaceutically acceptable salt thereof.

In some embodiments, the compound of Formula **III** is a compound of Formula **III-a2:** 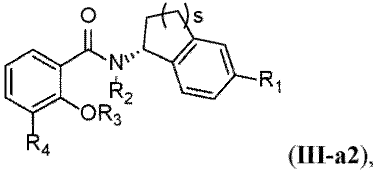 or a pharmaceutically acceptable salt thereof.

In some embodiments, the compound of Formula **III** is a compound of Formula **III-b2:** 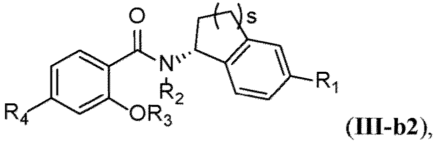 or a pharmaceutically acceptable salt thereof.

In some embodiments, the compound of Formula **III** is a compound of Formula **III-c2:** 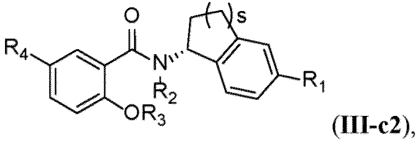 or a pharmaceutically acceptable salt thereof.

In some embodiments of Formula III (e.g., (111-a), (III-a1), (III-a2), (111-b), (III-b1), (III-b2), (III-c), (III-c1), (III-c2)), R₁ is selected from the group consisting of -Cl, -F, and -CF₃. In some embodiments of Formula III (e.g., (111-a), (III-a1), (III-a2), (111-b), (III-b1), (III-b2), (III-c), (III-c1), (III-c2)), R₁ is -Cl. In some embodiments of Formula III (e.g., (III-a), (III-a1), (III-a2), (III-b), (III-b1), (III-b2), (111-c), (III-c1), (III-c2)), R₁ is -F. In some embodiments of Formula III (e.g., (111-a), (III-a1), (III-a2), (111-b), (III-b1), (III-b2), (III-c), (III-c1), (III-c2)), R₁ is -CF₃.

In some embodiments of Formula III (e.g., (111-a), (III-a1), (III-a2), (111-b), (III-b1), (III-b2), (III-c), (III-c1), (III-c2)), R₄ is selected from the group consisting of methyl, methoxy, -F, -Cl, -CF₃, methoxy, 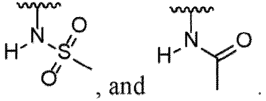 In some embodiments of Formula III (e.g., (III-a), (III-a1), (III-a2), (III-b), (III-b1), (111-b2), (111-c), (III-c1), (III-c2)), R₄ is -F. In some embodiments of Formula III (e.g., (III-a), (III-a1), (III-a2), (111-b), (III-b1), (III-b2), (III-c), (III-c1), (III-c2)), R₄ is -Cl. In some embodiments of Formula III (e.g., (III-a), (III-a1), (III-a2), (III-b), (III-b1), (111-b2), (111-c), (III-c1), (III-c2)), R₄ is -CF₃. In some embodiments of Formula III (e.g., (111-a), (III-a1), (III-a2), (111-b), (III-b1), (III-b2), (III-c), (III-c1), (III-c2)), R₄ is methoxy. In some embodiments of Formula III (e.g., (111-a), (III-a1), (III-a2), (III-b), (III-b1), (III-b2), (III-c), (III-c1), (III-c2)), R₄ is 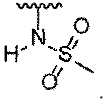 In some embodiments of Formula III (e.g., (III-a), (III-a1), (III-a2), (III-b), (III-b1), (III-b2), (III-c), (III-c1), (III-c2)), R₄ is

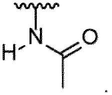

In some embodiments of Formula III, n is selected from the group consisting of 0, 1, and 2. In some embodiments of Formula III, n is 0 or 1. In some embodiments of Formula III, n is 1. In some embodiments of Formula III, n is 0.

In some embodiments of Formula III (e.g., (III-a), (III-a1), (III-a2), (III-b), (III-b1), (III-b2), (III-c), (III-c1), (III-c2)), each R₇ and R₈ are independently hydrogen.

In some embodiments of Formula III (e.g., (III-a), (III-a1), (III-a2), (III-b), (III-b1), (III-b2), (III-c), (III-c1), (III-c2)), s is 2. In some embodiments of Formula III (e.g., (III-a), (III-a1), (III-a2), (III-b), (III-b1), (III-b2), (III-c), (III-c1), (III-c2)), s is 1.

In some embodiments, the compound of Formula III is selected from the group consisting of: 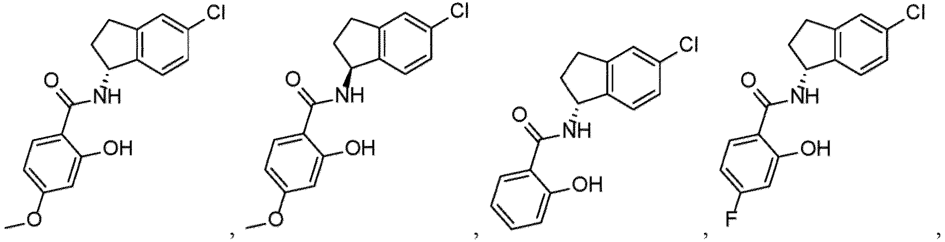 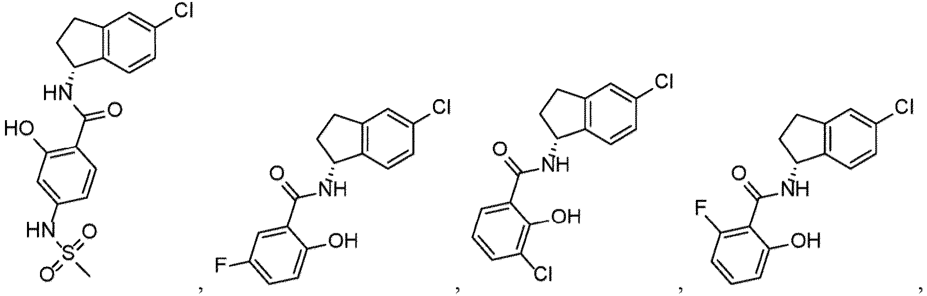 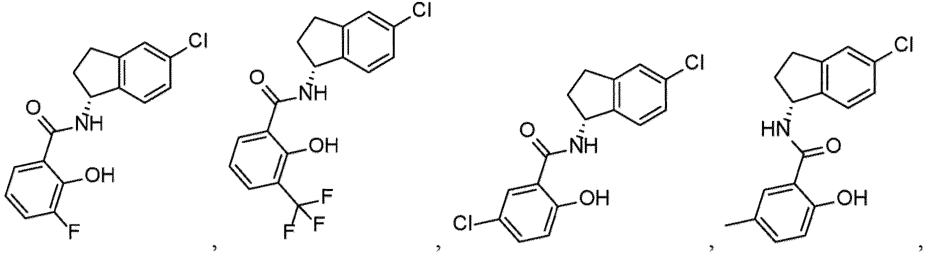 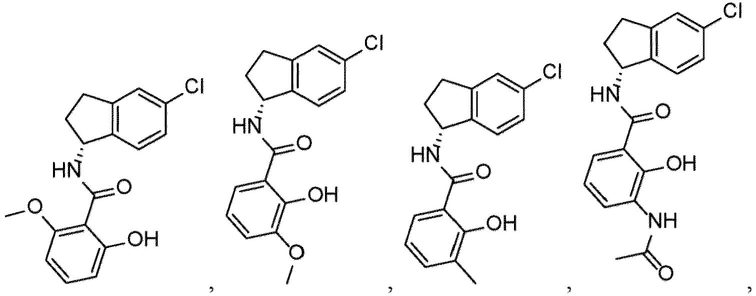 and 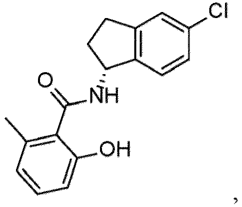 or a pharmaceutically acceptable salt thereof.

### General Synthetic Schemes

Exemplary methods for preparing compounds described herein are illustrated in the following synthetic schemes. These schemes are given for the purpose of illustrating the invention, and should not be regarded in any manner as limiting the scope or the spirit of the invention.

The synthetic route illustrated in Scheme I-1 depicts an exemplary procedure for preparing carboxylic acid **I-C.** In the first step, **I-A** is carbonylated to form ester **I-B** under standard carbonylation reaction conditions (e.g., CO, Pd(dppf)Cl₂ and Et₃N in ROH). Then, ester **I-B** is hydrolyzed to provide carboxylic acid **I-C.**

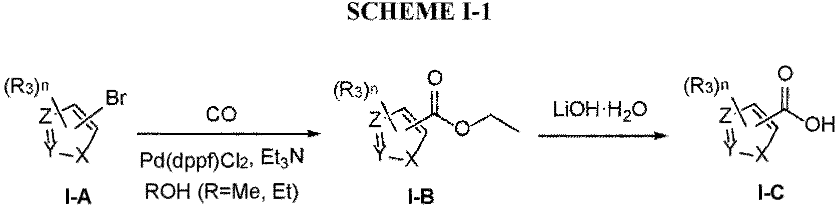

The synthetic route illustrated in Scheme I-2 depicts an exemplary procedure for preparing **I-E** (a compound of Formula I-I). In this route, carboxylic acid **I-C** is coupled with aminoindane **I-D** under standard peptide coupling reaction conditions (e.g., HATU and base) to provide the **I-E** (a compound of Formula I-I).

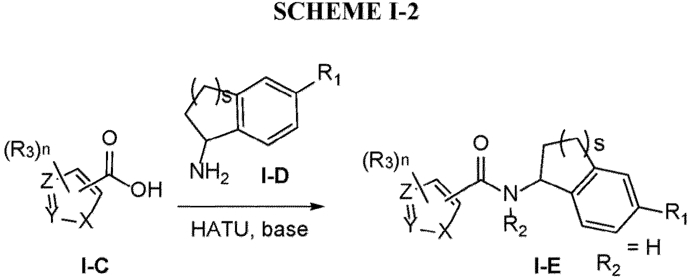

The synthetic route illustrated in Scheme 1 depicts an exemplary procedure for preparing carboxylic acid **C.** In the first step, **A** is carbonylated to form ester **B** under standard carbonylation reaction conditions (e.g., CO, Pd(dppf)Cl₂ and Et₃N in ethanol). Then, ester **B** is hydrolyzed to provide carboxylic acid **C.**

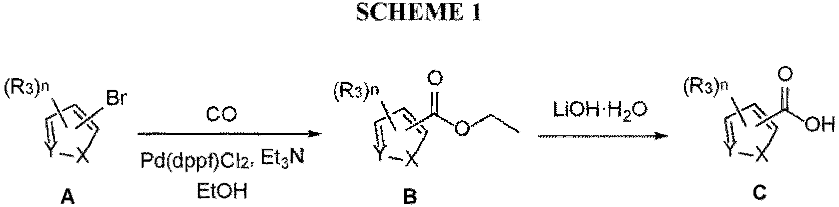

The synthetic route illustrated in Scheme 2 depicts an exemplary procedure for preparing **E** (a compound of Formula I). In this route, carboxylic acid **C** is coupled with aminoindane **D** under standard peptide coupling reaction conditions (e.g., HATU and Et₃N in acetonitrile) to provide the **E** (a compound of Formula I).

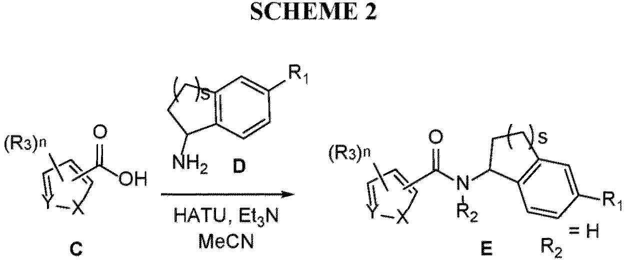

The synthetic route illustrated in Scheme II-1 depicts an exemplary procedure for preparing aminoindane **II-B.** In this route, indanone **II-A** is reacted with ammonium acetate and sodium cyanoborohydride to form aminoindane **II-B.**

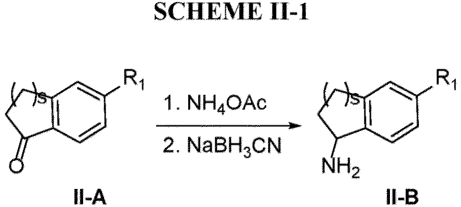

The synthetic route illustrated in Scheme II-2 depicts an exemplary procedure for preparing carboxylic acid **II-D**. In this route, ester **II-C** is hydrolyzed to provide carboxylic acid **11-D.**

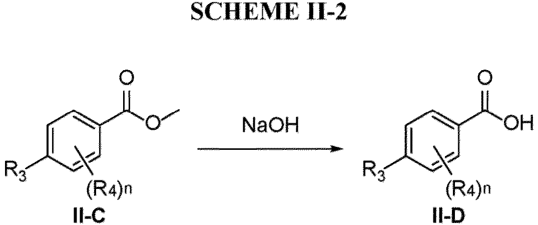

The synthetic route illustrated in Scheme II-3 depicts an exemplary procedure for preparing **II-E** (a compound of Formula II). In this route, carboxylic acid **11-D** is coupled with aminoindane **II-B** under standard peptide coupling reaction conditions (e.g., HOBt and EDCI in dichloromethane in the presence of NEt₃) to provide **II-E** (a compound of Formula II).

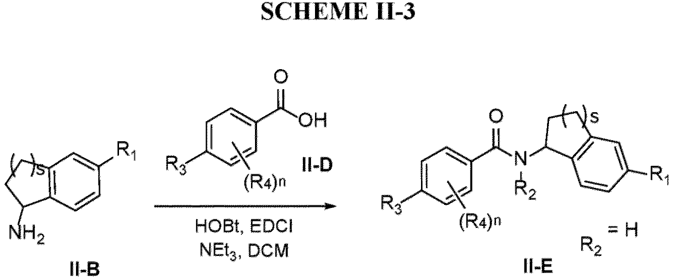

The synthetic route illustrated in Scheme III-1 depicts an exemplary procedure for preparing **III-C** (a compound of Formula III). In this route, carboxylic acid **III-A** is coupled with aminoindane **III-B** under standard peptide coupling reaction conditions (e.g., HOBt and EDCI in dichloromethane in the presence of DMAP) to provide **III-C** (a compound of Formula III).

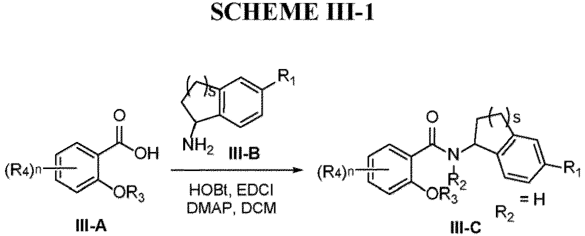

### Methods of Treatment

The compounds and compositions described above and herein can be used to treat a neurological disease or disorder or a disease or condition associated with excessive neuronal excitability and/or a gain-of-function mutation in a gene (e.g., KCNT1). Exemplary diseases, disorders, or conditions include epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, developmental and epileptic encephalopathy (DEE), early infantile epileptic encephalopathy (EIEE), generalized epilepsy, focal epilepsy, multifocal epilepsy, temporal lobe epilepsy, Ohtahara syndrome, early myoclonic encephalopathy and Lennox Gastaut syndrome, drug resistant epilepsy, seizures (e.g., frontal lobe seizures, generalized tonic clonic seizures, asymmetric tonic seizures, focal seizures), leukodystrophy, hypomyelinating leukodystrophy, leukoencephalopathy, and sudden unexpected death in epilepsy, cardiac dysfunctions (e.g., cardiac arrhythmia, Brugada syndrome, , myocardial infarction), pulmonary vasculopathy / hemorrhage, pain and related conditions (e.g. neuropathic pain, acute/chronic pain, migraine, etc), muscle disorders (e.g. myotonia, neuromyotonia, cramp muscle spasms, spasticity), itch and pruritis, movement disorders (e.g., ataxia and cerebellar ataxias), psychiatric disorders (e.g. major depression, anxiety, bipolar disorder, schizophrenia, attention-deficit hyperactivity disorder), neurodevelopmental disorder, learning disorders, intellectual disability, Fragile X, neuronal plasticity, and autism spectrum disorders.

In some embodiments, the neurological disease or disorder or the disease or condition associated with excessive neuronal excitability and/or a gain-of-function mutation in a gene (e.g., KCNT1) is selected from EIMFS, ADNFLE and West syndrome. In some embodiments, the neurological disease or disorder or the disease or condition associated with excessive neuronal excitability and/or a gain-of-function mutation in a gene (e.g., KCNT1) is selected from infantile spasms, epileptic encephalopathy, focal epilepsy, Ohtahara syndrome, developmental and epileptic encephalopathy and Lennox Gastaut syndrome. In some embodiments, the neurological disease or disorder or the disease or condition associated with excessive neuronal excitability and/or a gain-of-function mutation in a gene (e.g., KCNT1) is seizure. In some embodiments, the neurological disease or disorder or the disease or condition associated with excessive neuronal excitability and/or a gain-of-function mutation in a gene (e.g., KCNT1) is selected from cardiac arrhythmia, Brugada syndrome, and myocardial infarction.

In some embodiments, the neurological disease or disorder or the disease or condition associated with excessive neuronal excitability and/or a gain-of-function mutation in a gene (e.g., KCNT1) is selected from the group consisting of the learning disorders, Fragile X, intellectual function, neuronal plasticity, psychiatric disorders, and autism spectrum disorders.

Accordingly, the compounds and compositions thereof can be administered to a subject with a neurological disease or disorder or a disease or condition associated with excessive neuronal excitability and/or a gain-of-function mutation in a gene such as KCNT1 (e.g., EIMFS, ADNFLE, West syndrome, infantile spasms, epileptic encephalopathy, focal epilepsy, Ohtahara syndrome, developmental and epileptic encephalopathy, and Lennox Gastaut syndrome, seizures, cardiac arrhythmia, Brugada syndrome, and myocardial infarction).

EIMFS is a rare and debilitating genetic condition characterized by an early onset (before 6 months of age) of almost continuous heterogeneous focal seizures, where seizures appear to migrate from one brain region and hemisphere to another. Patients with EIMFS are generally intellectually impaired, non-verbal and non-ambulatory. While several genes have been implicated to date, the gene that is most commonly associated with EIMFS is KCNT1. Several de novo mutations in KCNT1 have been identified in patients with EIMFS, including V271F, G288S, R428Q, R474Q, R474H, R474C, I760M, A934T, P924L, G243S, H257D, A259D, R262Q, Q270E, L274I, F346L, C377S, R398Q, P409S, A477T, F502V, M516V, Q550del, K629E, K629N, I760F, E893K, M896K, R933G, R950Q, K1154Q (Barcia et al. (2012) Nat Genet. 44: 1255-1260; Ishii et al. (2013) Gene 531:467-471; McTague et al. (2013) Brain. 136: 1578-1591; Epi4K Consortium & Epilepsy Phenome/Genome Project. (2013) Nature 501:217-221; Lim et al. (2016) Neurogenetics; Ohba et al. (2015) Epilepsia 56:el21-el28; Zhou et al. (2018) Genes Brain Behav. e12456; Moller et al. (2015) Epilepsia. e114-20; Numis et al. (2018) Epilepsia. 1889-1898; Madaan et al. Brain Dev. 40(3):229-232; McTague et al. (2018) Neurology. 90(1):e55-e66; Kawasaki et al. (2017) J Pediatr. 191:270-274; Kim et al. (2014) Cell Rep. 9(5):1661-1672; Ohba et al. (2015) Epilepsia. 56(9):e121-8; Rizzo et al. (2016) Mol Cell Neurosci. 72:54-63; Zhang et al. (2017) Clin Genet. 91(5):717-724; Mikati et al. (2015) Ann Neurol. 78(6):995-9; Baumer et al. (2017) Neurology. 89(21):2212; Dilena et al. (2018) Neurotherapeutics. 15(4):1112-1126). These mutations are gain-of-function, missense mutations that are dominant (i.e. present on only one allele) and result in change in function of the encoded potassium channel that causes a marked increase in whole cell current when tested in Xenopus oocyte or mammalian expression systems (see e.g. Milligan et al. (2015) Ann Neurol. 75(4): 581-590; Barcia et al. (2012) Nat Genet. 44(11): 1255-1259; and Mikati et al. (2015) Ann Neurol. 78(6): 995-999).

ADNFLE has a later onset than EIMFS, generally in mid-childhood, and is generally a less severe condition. It is characterized by nocturnal frontal lobe seizures and can result in psychiatric, behavioural and cognitive disabilities in patients with the condition. While ADNFLE is associated with genes encoding several neuronal nicotinic acetylcholine receptor subunits, mutations in the KCNT1 gene have been implicated in more severe cases of the disease (Heron et al. (2012) Nat Genet. 44: 1188-1190). Functional studies of the mutated KCNT1 genes associated with ADNFLE indicated that the underlying mutations (M896I, R398Q, Y796H and R928C) were dominant, gain-of-function mutations (Milligan et al. (2015) Ann Neurol. 75(4): 581-590; Mikati et al. (2015) Ann Neurol. 78(6): 995-999).

West syndrome is a severe form of epilepsy composed of a triad of infantile spasms, an interictal electroencephalogram (EEG) pattern termed hypsarrhythmia, and mental retardation, although a diagnosis can be made one of these elements is missing. Mutations in KCNT1, including G652V and R474H, have been associated with West syndrome (Fukuoka et al. (2017) Brain Dev 39:80-83 and Ohba et al. (2015) Epilepsia 56:el21-el28). Treatment targeting the KCNT1 channel suggests that these mutations are gain-of-function mutations (Fukuoka et al. (2017) Brain Dev 39:80-83).

In one aspect, the present invention features a method of treating treat a disease or condition associated with excessive neuronal excitability and/or a gain-of-function mutation in a gene such as KCNT1 (for example, epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy (DEE), and Lennox Gastaut syndrome, seizures, leukodystrophy, leukoencephalopathy, intellectual disability, Multifocal Epilepsy, Generalized tonic clonic seizures, Drug resistant epilepsy, Temporal lobe epilepsy, cerebellar ataxia, Asymmetric Tonic Seizures) and cardiac dysfunctions (e.g., cardiac arrhythmia, Brugada syndrome, sudden unexpected death in epilepsy, myocardial infarction), pain and related conditions (e.g. neuropathic pain, acute/chronic pain, migraine, etc), muscle disorders (e.g. myotonia, neuromyotonia, cramp muscle spasms, spasticity), itch and pruritis, ataxia and cerebellar ataxias, psychiatric disorders (e.g. major depression, anxiety, bipolar disorder, schizophrenia), learning disorders, Fragile X, neuronal plasticity, and autism spectrum disorders) comprising administering to a subject in need thereof a compound disclosed herein (e.g., a compound of Formula (I-I) or (I), (e.g., (I-Ia), (I-Ia1), (I-Ib), (I-Ib1), (I-Ic), (I-Ic1), (I-a), (I-a1), (I-b), (I-b1), (I-c), (I-c1), (I-d), or (I-d1)), a compound of Formula (II), (e.g., (IIa), (II-a1), (II-a2), (II-b), (II-b1), (II-b2), (II-c), (II-c1), (II-c2)), or a compound of Formula (III), (e.g., (III-a), (III-a1), (III-a2), (III-b), (III-b1), (III-b2), (III-c), (III-c1), (III-c2)))) or a pharmaceutically acceptable salt thereof) or a pharmaceutical composition disclosed herein (e.g., a pharmaceutical composition comprising a compound disclosed herein (e.g., a compound of Formula (I-I) or (I), (e.g., (I-Ia), (I-Ia1), (I-Ib), (I-Ib1), (I-Ic), (I-Ic1), (I-a), (I-a1), (I-b), (I-b1), (I-c), (I-c1), (I-d), or (I-d1)), a compound of Formula (II), (e.g., (II-a), (II-a1), (II-a2), (II-b), (II-b1), (II-b2), (II-c), (II-c1), (II-c2)), or a compound of Formula (III), (e.g., (III-a), (III-a1), (III-a2), (III-b), (III-b1), (III-b2), (III-c), (III-c1), (III-c2)))) or a pharmaceutically acceptable salt thereof), and a pharmaceutically acceptable excipient).

In some examples, the subject presenting with a disease or condition that may be associated with a gain-of-function mutation in KCNT1 is genotyped to confirm the presence of a known gain-of-function mutation in KCNT1 prior to administration of the compounds and compositions thereof. For example, whole exome sequencing can be performed on the subject. Gain-of-function mutations associated with EIMFS may include, but are not limited to, V271F, G288S, R428Q, R474Q, R474H, R474C, I760M, A934T, P924L, G243S, H257D, A259D, R262Q, Q270E, L274I, F346L, C377S, R398Q, P409S, A477T, F502V, M516V, Q550del, K629E, K629N, I760F, E893K, M896K, R933G, R950Q, and K1154Q. Gain-of-function mutations associated with ADNFLE may include, but are not limited to, M896I, R398Q, Y796H, R928C, and G288S. Gain-of-function mutations associated with West syndrome may include, but are not limited to, G652V and R474H. Gain-of-function mutations associated with temporal lobe epilepsy may include, but are not limited to, R133H and R565H. Gain-of-function mutations associated with Lennox-Gastaut may include, but are not limited to, R209C. Gain-of-function mutations associated with seizures may include, but are not limited to, A259D, G288S, R474C, R474H. Gain-of-function mutations associated with leukodystrophy may include, but are not limited to, G288S and Q906H. Gain-of-function mutations associated with Multifocal Epilepsy may include, but are not limited to, V340M. Gain-of-function mutations associated with EOE may include, but are not limited to, F346L and A934T. Gain-of-function mutations associated with Early-onset epileptic encephalopathies (EOEE) may include, but are not limited to, R428Q. Gain-of-function mutations associated with developmental and epileptic encephalopathies may include, but are not limited to, F346L, R474H, and A934T. Gain-of-function mutations associated with epileptic encephalopathies may include, but are not limited to, L437F, Y796H, P924L, R961H. Gain-of-function mutations associated with Early Infantile Epileptic Encephalopathy (EIEE) may include, but are not limited to, M896K. Gain-of-function mutations associated with drug resistent epilepsy and generalized tonic-clonic seizure may include, but are not limited to, F346L. Gain-of-function mutations associated with migrating partial seizures of infancy may include, but are not limited to, R428Q. Gain-of-function mutations associated with Leukoencephalopathy may include, but are not limited to, F932I. Gain-of-function mutations associated with NFLE may include, but are not limited to, A934T and R950Q. Gain-of-function mutations associated with Ohtahara syndrome may include, but are not limited to, A966T. Gain-of-function mutations associated with infantile spasms may include, but are not limited to, P924L. Gain-of-function mutations associated with Brugada Syndrome may include, but are not limited to, R1106Q. Gain-of-function mutations associated with Brugada Syndrome may include, but are not limited to, R474H.

In other examples, the subject is first genotyped to identify the presence of a mutation in KCNT1 and this mutation is then confirmed to be a gain-of-function mutation using standard in vitro assays, such as those described in Milligan et al. (2015) Ann Neurol. 75(4): 581-590. Typically, the presence of a gain-of-function mutation is confirmed when the expression of the mutated KCNT 1 allele results an increase in whole cell current compared to the whole cell current resulting from expression of wild-type KCNT 1 as assessed using whole-cell electrophysiology (such as described in Milligan et al. (2015) Ann Neurol. 75(4): 581-590; Barcia et al. (2012) Nat Genet. 44(11): 1255-1259; Mikati et al. (2015) Ann Neurol. 78(6): 995-999; or Rizzo et al. Mol Cell Neurosci. (2016) 72:54-63). This increase of whole cell current can be, for example, an increase of at least or about 50%, 100%, 150%, 200%, 250%, 300%, 350%, 400% or more. The subject can then be confirmed to have a disease or condition associated with a gain-of-function mutation in KCNT1.

In particular examples, the subject is confirmed as having a KCNT1 allele containing a gain-of-function mutation (e.g. V271F, G288S, R398Q, R428Q, R474Q, R474H, R474C, G652V, I760M, Y796H, M896I, P924L, R928C or A934T).

The compounds disclosed herein (e.g., a compound of Formula (I-I) or (I), (e.g., (I-Ia), (I-Ia1), (I-Ib), (I-Ib1), (I-Ic), (I-Ic1), (I-a), (I-a1), (I-b), (I-b1), (I-c), (I-c1), (I-d), or (I-d1)), a compound of Formula (II), (e.g., (II-a), (II-a1), (II-a2), (II-b), (II-b1), (II-b2), (II-c), (II-c1), (II-c2)), or a compound of Formula (III), (e.g., (III-a), (III-a1), (III-a2), (III-b), (III-b1), (III-b2), (III-c), (III-c1), (III-c2)))) or a pharmaceutically acceptable salt thereof) or the pharmaceutical composition disclosed herein (e.g., a pharmaceutical composition comprising a compound disclosed herein (e.g., a compound of Formula (I-I) or (I), (e.g., (I-Ia), (I-Ia1), (I-Ib), (I-Ib1), (1-Ic), (I-Ic 1), (I-a), (I-a1), (I-b), (I-b1), (I-c), (I-c1), (I-d), or (I-d1)), a compound of Formula (II), (e.g., (II-a), (II-a1), (II-a2), (II-b), (II-b1), (II-b2), (II-c), (II-c1), (II-c2)), or a compound of Formula (III), (e.g., (III-a), (III-a1), (III-a2), (III-b), (III-b1), (III-b2), (III-c), (III-c1), (III-c2)))) or a pharmaceutically acceptable salt thereof), and a pharmaceutically acceptable excipient) can also be used therapeutically for conditions associated with excessive neuronal excitability where the excessive neuronal excitability is not necessarily the result of a gain-of-function mutation in KCNT1. Even in instances where the disease is not the result of increased KCNT1 expression and/or activity, inhibition of KCNT1 expression and/or activity can nonetheless result in a reduction in neuronal excitability, thereby providing a therapeutic effect. Thus, the compounds disclosed herein (e.g., a compound of Formula (I-I) or (I), (e.g., (I-Ia), (I-Ia1), (I-Ib), (I-IbI), (I-Ic), (I-Ic1), (I-a), (I-a1), (I-b), (I-b1), (I-c), (I-c1), (I-d), or (I-d1)), a compound of Formula (II), (e.g., (II-a), (II-a1), (II-a2), (II-b), (II-b1), (II-b2), (II-c), (II-c1), (II-c2)), or a compound of Formula (III), (e.g., (III-a), (III-a1), (III-a2), (III-b), (III-b1), (III-b2), (III-c), (III-c1), (III-c2)))) or a pharmaceutically acceptable salt thereof) or the pharmaceutical composition disclosed herein (e.g., a pharmaceutical composition comprising a compound disclosed herein (e.g., a compound of Formula (I-I) or (I), (e.g., (I-Ia), (I-Ia1), (I-Ib), (I-Ib1), (I-Ic), (I-Ic1), (I-a), (I-a1), (I-b), (I-b1), (I-c), (I-c1), (I-d), or (I-d1)), a compound of Formula (II), (e.g., (II-a), (II-a1), (II-a2), (II-b), (II-b1), (II-b2), (II-c), (II-c1), (II-c2)), or a compound of Formula (III), (e.g., (III-a), (III-a1), (III-a2), (III-b), (III-b1), (III-b2), (III-c), (III-c1), (III-c2)))) or a pharmaceutically acceptable salt thereof), and a pharmaceutically acceptable excipient) can be used to treat a subject with conditions associated with excessive neuronal excitability, for example, epilepsy and other encephalopathies (e.g., epilepsy of infancy with migrating focal seizures (EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, and Lennox Gastaut syndrome, seizures) or cardiac dysfunctions (e.g., cardiac arrhythmia, Brugada syndrome, myocardial infarction), regardless of whether or not the disease or disorder is associated with a gain-of-function mutation in KCNT1.

### Pharmaceutical Compositions and Routes of Administration

Compounds provided in accordance with the present invention are usually administered in the form of pharmaceutical compositions. This invention therefore provides pharmaceutical compositions that contain, as the active ingredient, one or more of the compounds described, or a pharmaceutically acceptable salt or ester thereof, and one or more pharmaceutically acceptable excipients, carriers, including inert solid diluents and fillers, diluents, including sterile aqueous solution and various organic solvents, permeation enhancers, solubilizers and adjuvants. The pharmaceutical compositions may be administered alone or in combination with other therapeutic agents. Such compositions are prepared in a manner well known in the pharmaceutical art (see, e.g., Remington's Pharmaceutical Sciences, Mace Publishing Co., Philadelphia, Pa. 17th Ed. (1985); and Modern Pharmaceutics, Marcel Dekker, Inc. 3rd Ed. (G. S. Banker & C. T. Rhodes, Eds.)

The pharmaceutical compositions may be administered in either single or multiple doses by any of the accepted modes of administration of agents having similar utilities, for example as described in those patents and patent applications incorporated by reference, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an artery-inserted cylindrical polymer.

One mode for administration is parenteral, particularly by injection. The forms in which the novel compositions of the present invention may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles. Aqueous solutions in saline are also conventionally used for injection, but less preferred in the context of the present invention. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating a compound according to the present invention in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Oral administration is another route for administration of compounds in accordance with the invention. Administration may be via capsule or enteric coated tablets, or the like. In making the pharmaceutical compositions that include at least one compound described herein, the active ingredient is usually diluted by an excipient and/or enclosed within such a carrier that can be in the form of a capsule, sachet, paper or other container. When the excipient serves as a diluent, it can be in the form of a solid, semi-solid, or liquid material (as above), which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl and propylhydroxy-benzoates; sweetening agents; and flavoring agents.

The compositions of the invention can be formulated so as to provide quick, sustained or delayed release of the active ingredient after administration to the patient by employing procedures known in the art. Controlled release drug delivery systems for oral administration include osmotic pump systems and dissolutional systems containing polymer-coated reservoirs or drug-polymer matrix formulations. Examples of controlled release systems are given in U.S. Pat. Nos. 3,845,770; 4,326,525; 4,902,514; and 5,616,345. Another formulation for use in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Pat. Nos. 5,023,252, 4,992,445 and 5,001,139. Such patches may be constructed for continuous, pulsatile, or on demand delivery of pharmaceutical agents.

The compositions are preferably formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient (e.g., a tablet, capsule, ampoule). The compounds are generally administered in a pharmaceutically effective amount. Preferably, for oral administration, each dosage unit contains from 1 mg to 2 g of a compound described herein, and for parenteral administration, preferably from 0.1 to 700 mg of a compound a compound described herein. It will be understood, however, that the amount of the compound actually administered usually will be determined by a physician, in the light of the relevant circumstances, including the condition to be treated, the chosen route of administration, the actual compound administered and its relative activity, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills of the present invention may be coated or otherwise compounded to provide a dosage form affording the advantage of prolonged action, or to protect from the acid conditions of the stomach. For example, the tablet or pill can comprise an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably, the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, preferably orally or nasally, from devices that deliver the formulation in an appropriate manner.

In some embodiments, a pharmaceutical composition comprising a disclosed compound, or pharmaceutically acceptable salt thereof, and a pharmaceutically acceptable carrier.

### EXAMPLES

In order that the invention described herein may be more fully understood, the following examples are set forth. The synthetic and biological examples described in this application are offered to illustrate the compounds, pharmaceutical compositions and methods provided herein and are not to be construed in any way as limiting their scope.

The compounds provided herein can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimal reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include recrystallization, filtration, flash chromatography, trituration, high pressure liquid chromatography (HPLC), or supercritical fluid chromatography (SFC). Note that flash chromatography may either be performed manually or via an automated system. The compounds provided herein may be characterized by known standard procedures, such as nuclear magnetic resonance spectroscopy (NMR) or liquid chromatography mass spectrometry (LCMS). NMR chemical shifts are reported in part per million (ppm) and are generated using methods well known to those of skill in the art.

### List of abbreviations

- MeI: methyliodide
- Pd(dppf)Cl₂: [1,1'-bis(diphenylphosphino)ferrocene]palladium(II) dichloride
- Pd₂(dba)₃: tris(dibenzylideneacetone)dipalladium(0)
- THF: tetrahydrofuran
- TEA or Et₃N: triethylamine
- TFA: trifluoroacetic acid
- FA: formic acid
- DMF: *N,N-*dimethylformamide
- MeOH: methanol
- DCM: dichloromethane
- MeCN or ACN: acetonitrile
- PE: petroleum ether
- EtOAc or EA: ethyl acetate
- EDCI: 1-(3-dimethylaminopropyl)-3-ethylcarbodiimide hydrochloride o-(7-azabenzotriazol-1-yl)-*N,N,N',N'*-tetramethyluronium hexafluorophosphate
- HOBt: 1-hydroxybenzotriazole monohydrate
- DIPEA: *N,N*,-diisopropylethylamine
- DEA: diethylamine
- NBS: N-bromosuccinimide
- NaOMe: sodium methoxide
- mCPBA: meta-chloroperoxybenzoic acid
- PPh₃: triphenylphosphine
- DEA: diethylamine
- NH₄OAc: ammonium acetate
- DIAD: diisopropyl azodicarboxylate
- *i*-PrOH: isopropanol
- Tf₂O: trifluoromethanesulfonic anhydride
- DMAP: 4-dimethylaminopyridine
- oxone: potassium peroxymonosulfate
- t-BuOH: tert-butanol
- CSI: chlorosulfonyl isocyanate
- ODS: octadecylsilane
- HEPES: 4-(2-hydroxyethyl)piperazine-1-ethanesulfonic acid
- DMSO: dimethyl sulfoxide
- EGTA: ethylene glycol-bis(β-aminoethyl ether)*-N,N,N',N*'-tetraacetic acid
- NMDG: *N*-methyl-D-glucamine
- IC₅₀: half maximal inhibitory concentration
- TLC: thin layer chromatography
- LCMS: liquid chromatography-mass spectrometry
- HPLC: high-performance liquid chromatagraphy
- SFC: supercritical fluid chromatography
- MS: mass spectrometry
- ESI: electrospray ionization
- NMR: nuclear magnetic resonance

### Example 1. Synthesis of Compound I-1

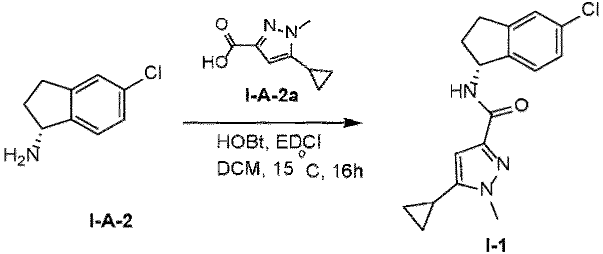

To a mixture of 5-cyclopropyl-1-methyl-pyrazole-3-carboxylic acid (100 mg, 0.60 mmol), HOBt (162.63 mg, 1.2 mmol), EDCI (173.04 mg, 0.90 mmol) and (1R)-5-chloroindan-1-amine (100.88 mg, 0.60 mmol) in DCM (20 mL) was stirred at 15 °C for 16 h. The mixture was extracted with EtOAc (20 mL x 2). The combined organic phase was washed with brine (10 mL), dried over anhydrous Na₂SO₄, filtered and concentrated. The crude product was purified by Prep-HPLC (Xtimate C18 150 x 25 mm, 5µm) A = H₂O (0.05% NH₄OH) and B = CH₃CN; 46-76 %B over 8.5 minutes) to give the product (89.1 mg, 0.28 mmol, 47% yield).
**¹H NMR** (400MHz, DMSO-*d⁶*) δ_{H} = 8.23 (d, 1H), 7.30 (s, 1H), 7.24 - 7.17 (m, 1H), 7.16 - 7.11 (m, 1H), 6.31 (s, 1H), 5.42 (q, 1H), 3.86 (s, 3H), 3.01 - 2.92 (m, 1H), 2.86 - 2.75 (m, 1H), 2.42 - 2.31 (m, 1H), 2.11 - 1.99 (m, 1H), 1.94 - 1.86 (m, 1H), 1.00 - 0.92 (m, 2H), 0.68 - 0.61 (m, 2H)
**LCMS** Rt = 1.28 min in 2.0 min chromatography, 10-80AB, MS ESI calcd. for C₁₇H₁₉ClN₃O [M+H]⁺ 316.1, found 316.0.

### Example 2. Synthesis of Compound 1-2

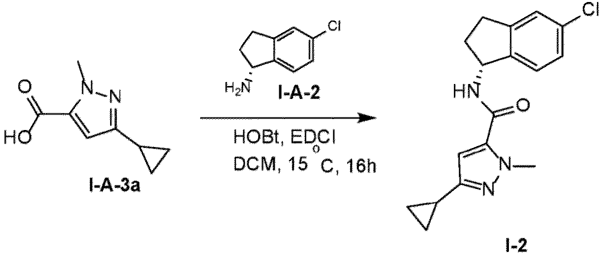

To a mixture of 5-cyclopropyl-2-methyl-pyrazole-3-carboxylic acid (100 mg, 0.60 mmol), HOBt (162.63 mg, 1.2 mmol), EDCI (173.04 mg, 0.90 mmol) and (1R)-5-chloroindan-1-amine (100.88 mg, 0.60 mmol) in DCM (10 mL) was stirred at 15 °C for 16 h. The mixture was extracted with EtOAc (20 mL x 2). The combined organic phase was washed with brine (10 mL), dried over anhydrous Na₂SO₄, filtered and concentrated. The crude product was purified by Prep-HPLC (Boston Prime C18 150 mm x 30 mm, 5µm) A = H₂O (0.05% NH₄OH) and B = CH₃CN; 52-82%B over 8 minutes) to give the product (77.7 mg, 0.25 mmol, 41% yield).
**¹H NMR** (400MHz, DMSO-*d⁶*) δ_{H} = 8.63 (d, 1H), 7.33 (s, 1H), 7.26 - 7.18 (m, 2H), 6.57 (s, 1H), 5.43 (q, 1H), 3.99 (s, 3H), 3.04 - 2.93 (m, 1H), 2.91 - 2.77 (m, 1H), 2.47 - 2.39 (m, 1H), 2.02 - 1.91 (m, 1H), 1.87 - 1.79 (m, 1H), 0.88 - 0.82 (m, 2H), 0.61 - 0.55 (m, 2H). **LCMS** Rt = 1.26 min in 2.0 min chromatography, 10-80AB, MS ESI calcd. for C₁₇H₁₉ClN₃O [M+H]⁺ 316.1, found 316.0.

### Example 3. Synthesis of Compound 1-3

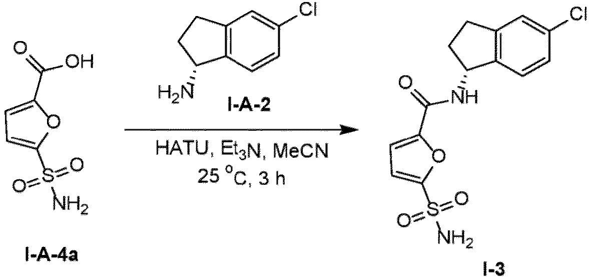

A mixture of 5-sulfamoylfuran-2-carboxylic acid (100 mg, 0.52 mmol), (1*R*)-5-chloroindan-1-amine (131.54 mg, 0.78 mmol) and Et₃N (0.14 mL, 1.05 mmol) and HATU (198.91 mg, 0.52 mmol) in CH₃CN (5 mL) was stirred at 25 °C for 3 hours. The mixture was diluted with H₂O (20 mL) and the mixture was extracted with EtOAc (20 mL x 2). The combined organic phase was washed with brine (30 mL), dried over anhydrous Na₂SO₄, filtered and concentrated. The crude product was purified by Prep-TLC (silica gel, PE: EtOAc = 1:1) to give the product (22.9 mg, 67.3 µmol, 23% yield) as a solid.
**¹H NMR** (400MHz, DMSO-*d⁶*) δ_{H} = 8.90 (d, 1H), 7.91 (s, 2H), 7.35 (s, 1H), 7.29 (d, 1H), 7.26 - 7.21 (m, 2H), 7.06 (d, 1H), 5.47 (q, 1H), 3.05 - 2.95 (m, 1H), 2.91 - 2.79 (m, 1H), 2.48 - 2.41 (m, 1H), 2.05 - 1.94 (m, 1H).
**LCMS** Rₜ = 1.12 min in 2.0 min chromatography, 10-80AB, MS ESI calcd. for C₁₄H₁₄ClN₂O₄S [M+H]⁺ 341.0, found 340.9.

### Example 4. Synthesis of Compound I-4

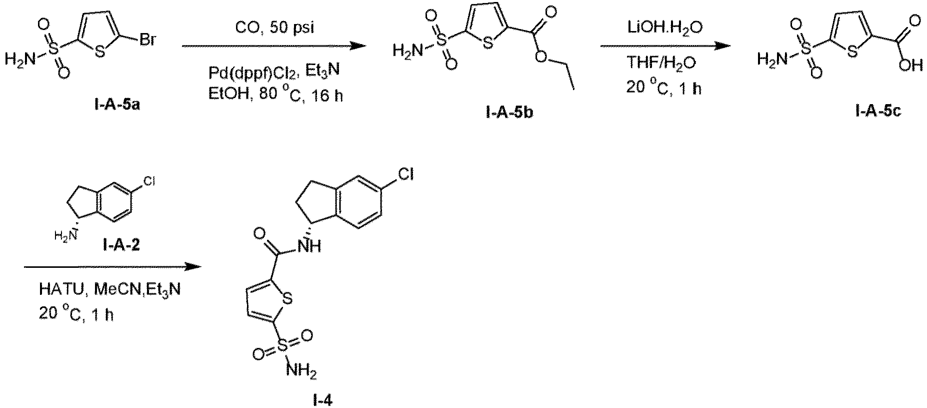

**Synthesis of I-A-5b:** A mixture of 5-bromothiophene-2-sulfonamide (2 g, 8.26 mmol) and Pd(dppf)Cl₂ (906.65 mg, 1.24 mmol) and Et₃N (3.43 mL, 24.78 mmol) in ethanol (20 mL) was stirred under CO (50 psi) at 80 °C for 16 hours. The mixture was filtered through Celite and the filtrate was concentrated. The crude product was purified by flash chromatography on silica gel (EtOAc in PE = 0% to 30% to 50%) to give the product (1 g, 4.25 mmol, 51% yield) as a solid.
**¹H NMR** (400MHz, DMSO-*d⁶*) δ_{H} = 7.95 (s, 2H), 7.77 (d, 1H), 7.58 (d, 1H), 4.32 (q, 2H), 1.30 (t, 3H).

**Synthesis of I-A-5c:** A mixture of ethyl 5-sulfamoylthiophene-2-carboxylate (900 mg, 3.83 mmol) and LiOH·H₂O (481.52 mg, 11.48 mmol) in THF (10 mL) and water (10 mL) was stirred at 20 °C for 1 hour. 1N HCl was added to adjust to pH = 1. The mixture was extracted with EtOAc (30 mL x 2). The combined organic phase was washed with brine (30 mL), dried over anhydrous Na₂SO₄, filtered and concentrated to give the crude product (450 mg, 2.17 mmol) as a solid.
**¹H NMR** (400MHz, DMSO-*d⁶*) δ_{H} = 7.91 (s, 2H), 7.70 (d, 1H), 7.56 (d, 1H).

**Synthesis of I-4:** A mixture of 5-sulfamoylthiophene-2-carboxylic acid (230 mg, 1.11 mmol), (1*R*)-5-chloroindan-1-amine (372.12 mg, 2.22 mmol), Et₃N (0.31 mL, 2.22 mmol) and HATU (422.02 mg, 1.11 mmol) in MeCN (5 mL) was stirred at 20 °C for 1 hour. Water (15 mL) was added and the aqueous layer was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 70%) to give the product (64 mg, 174.7 µmol, 15% yield) as a solid. The product was blended with another batch (36 mg, prepared from 200 mg of 5-sulfamoylthiophene-2-carboxylic acid). The combined solid (100 mg) was triturated from MeOH (3 mL) to give the product (82.9 mg, 232.3 µmol, 85% yield) as a solid.
**¹H NMR** (400MHz, DMSO-*d⁶*) δ_{H} = 9.08 (d, 1H), 7.84 (s, 2H), 7.77 (d, 1H), 7.53 (d, 1H), 7.36 (s, 1H), 7.24 (s, 2H), 5.46 (q, 1H), 3.05 - 2.95 (m, 1H), 2.92 - 2.81 (m, 1H), 2.48 - 2.42 (m, 1H), 2.05 - 1.94 (m, 1H).
**LCMS** Rₜ = 1.20 min in 2.0 min chromatography, 10-80AB, MS ESI calcd. for C₁₄H₁₄ClN₂O₃S₂ [M+H]⁺ 357.0, found 356.8.

### Example 5. Synthesis of Compound 1-5

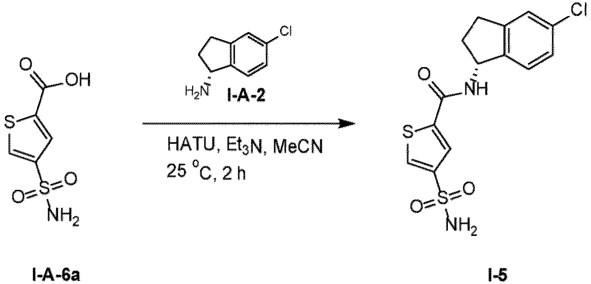

A mixture of 4-sulfamoylthiophene-2-carboxylic acid (150 mg, 0.72 mmol), (1*R*)-5-chloroindan-1-amine (182.02 mg, 1.09 mmol), Et₃N (0.2 mL, 1.45 mmol) and HATU (275.23 mg, 0.72 mmol) in MeCN (5 mL) was stirred at 25 °C for 2 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 70%) and then by Prep-HPLC (Xtimate C18 150 x 25 mm, 5µm, A = H₂O (10 mM NH₄HCO₃) and B = CH₃CN; 30-60% B over 10 minutes) to give the product (53.3 mg, 149.5 µmol, 20% yield) as a solid.
**¹H NMR** (400MHz, DMSO-*d⁶*) δ_{H} = 9.11 (d, 1H), 8.23 (d, 1H), 8.09 (d, 1H), 7.44 (s, 2H), 7.35 (s, 1H), 7.24 (s, 2H), 5.44 (q, 1H), 3.06 - 2.95 (m, 1H), 2.91 - 2.80 (m, 1H), 2.47 - 2.41 (m, 1H), 2.05 - 1.93 (m, 1H).
**LCMS** Rₜ = 1.11 min in 2.0 min chromatography, 10-80AB, MS ESI calcd. for C₁₄H₁₄ClN₂O₃S₂ [M+H]⁺ 357.0, found 356.9.

### Example 6. Synthesis of Compound I-6

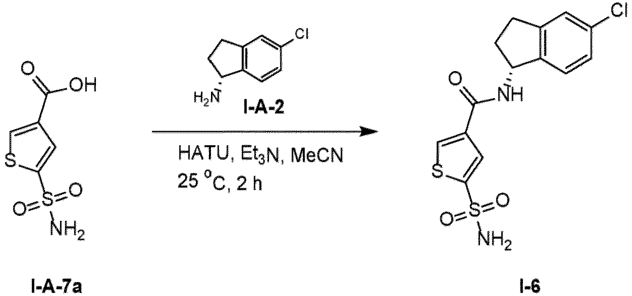

A mixture of (1*R*)-5-chloroindan-1-amine (182.02 mg, 1.09 mmol), 5-sulfamoylthiophene-3-carboxylic acid (150 mg, 0.72 mmol), Et₃N (0.2 mL, 1.45 mmol) and HATU (275.23 mg, 0.72 mmol) in MeCN (0.50 mL) was stirred at 25 °C for 2 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 70%) to give the product (73.09 mg, 204.8 µmol, 28% yield) as a solid.
**¹H NMR** (400MHz, DMSO-*d⁶*) δ_{H} = 8.85 (d, 1H), 8.41 (s, 1H), 8.01 (s, 1H), 7.74 (s, 2H), 7.34 (s, 1H), 7.23 (s, 2H), 5.46 (q, 1H), 3.04 - 2.94 (m, 1H), 2.91 - 2.80 (m, 1H), 2.45 - 2.40 (m, 1H), 2.04 - 1.93 (m, 1H).
**LCMS** Rₜ = 1.09 min in 2.0 min chromatography, 10-80AB, MS ESI calcd. for C₁₄H₁₄ClN₂O₃S₂ [M+H]⁺ 357.0, found 356.9.

### Example 7. Synthesis of Compound I-7

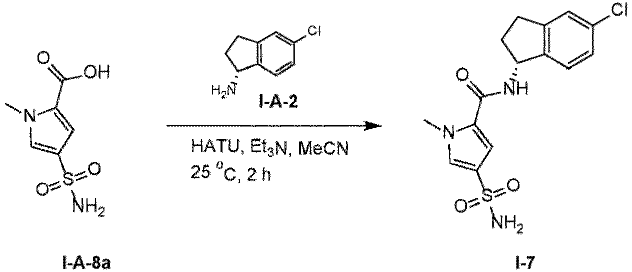

A mixture of 1-methyl-4-sulfamoyl-pyrrole-2-carboxylic acid (150 mg, 0.73 mmol), (1*R*)-5-chloroindan-1-amine (184.72 mg, 1.1 mmol), Et₃N (0.2 mL, 1.47 mmol) and HATU (279.31 mg, 0.73 mmol) in MeCN (5 mL) was stirred at 25 °C for 2 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 70%) to give the product (78.9 mg, 222.9 µmol, 30% yield) as a solid.
**¹H NMR** (400MHz, DMSO-*d⁶*) δ_{H} = 8.62 (d, 1H), 7.41 (d, 1H), 7.32 (s, 1H), 7.26 - 7.17 (m, 2H), 7.14 (d, 1H), 7.03 (s, 2H), 5.43 (q, 1H), 3.90 (s, 3H), 3.03 - 2.93 (m, 1H), 2.88 - 2.77 (m, 1H), 2.45 - 2.37 (m, 1H), 2.05 - 1.92 (m, 1H).
**LCMS** Rₜ = 1.10 min in 2.0 min chromatography, 10-80AB, MS ESI calcd. for C₁₅H₁₇ClN₃O₃S [M+H]⁺ 354.1, found 353.9.

### Example 8. Synthesis of Compound I-8

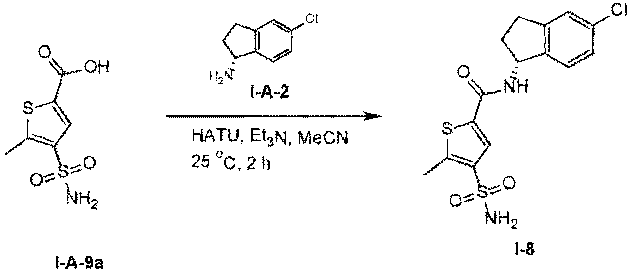

A mixture of 5-methyl-4-sulfamoyl-thiophene-2-carboxylic acid (150 mg, 0.68 mmol), (1*R*)-5-chloroindan-1-amine (170.48 mg, 1.02 mmol), Et₃N (0.19 mL, 1.36 mmol) and HATU (257.79 mg, 0.68 mmol) in MeCN (5 mL) was stirred at 25 °C for 2 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 70%) and then Prep-HPLC (Xtimate C18 150 x 25mm, 5µm, A = H₂O (10 mM NH₄HCO₃) and B = CH₃CN; 30-60% B over 10 minutes) to give the product (120.4 mg, 324.6 µmol, 47% yield) as a solid.
**¹H NMR** (400MHz, DMSO-*d⁶*) δ_{H} = 9.03 (d, 1H), 8.01 (s, 1H), 7.38 (s, 2H), 7.34 (s, 1H), 7.26 - 7.19 (m, 2H), 5.42 (q, 1H), 3.04 - 2.95 (m, 1H), 2.89 - 2.79 (m, 1H), 2.64 (s, 3H), 2.46 - 2.39 (m, 1H), 2.03 - 1.92 (m, 1H).
**LCMS** Rₜ = 1.13 min in 2.0 min chromatography, 10-80AB, MS ESI calcd. for C₁₅H₁₆ClN₂O₃S₂ [M+H]⁺ 371.0, found 370.9.

### Example 9. Synthesis of Compound 1-9

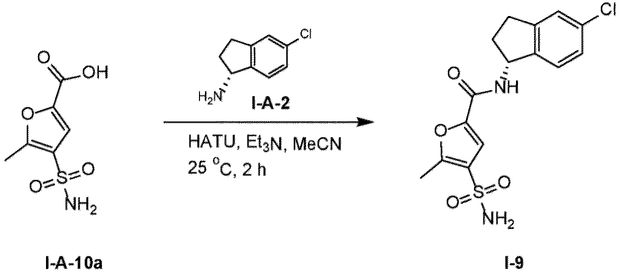

A mixture of 5-methyl-4-sulfamoyl-furan-2-carboxylic acid (150 mg, 0.73 mmol), (1*R*)-5-chloroindan-1-amine (183.82 mg, 1.1 mmol), Et₃N (0.2 mL, 1.46 mmol) and HATU (277.97 mg, 0.73 mmol) in MeCN (5 mL) was stirred at 25 °C for 2 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 70%) and then Prep-HPLC (Xtimate C18 150 x 25mm, 5µm, A = H₂O (10 mM NH₄HCO₃) and B = CH₃CN; 30-60% B over 10 minutes) to give the product (48.2 mg, 135.8 µmol, 18% yield) as a solid.
**¹H NMR** (400MHz, CD₃OD) δ_{H} = 8.84 (d, 1H), 7.47 (s, 2H), 7.36 - 7.29 (m, 2H), 7.26 - 7.14 (m, 2H), 5.43 (q, 1H), 3.04 - 2.93 (m, 1H), 2.89 - 2.78 (m, 1H), 2.52 (s, 3H), 2.45 - 2.38 (m, 1H), 2.05 - 1.93 (m, 1H).
**LCMS** Rₜ = 1.10 min in 2.0 min chromatography, 10-80AB, MS ESI calcd. for C₁₅H₁₆ClN₂O₄S [M+H]⁺ 355.0, found 354.9.

### Example 10. Synthesis of Compound I-10

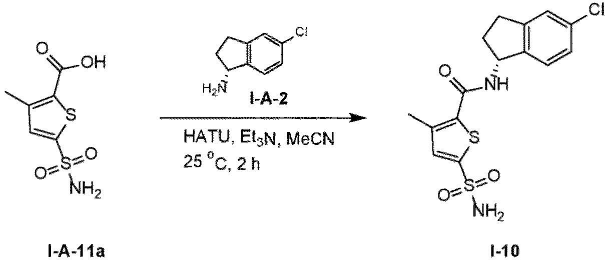

A mixture of 3-methyl-5-sulfamoyl-thiophene-2-carboxylic acid (100 mg, 0.45 mmol), (1*R*)-5-chloroindan-1-amine (113.65 mg, 0.68 mmol), Et₃N (0.13 mL, 0.90 mmol) and HATU (171.86 mg, 0.45 mmol) in MeCN (5 mL) was stirred at 25 °C for 2 hours. The mixture was diluted with H₂O (20 mL) and the mixture was extracted with EtOAc (20 mL x 2). The combined organic phase was washed with brine (30 mL), dried over anhydrous Na₂SO₄, filtered and concentrated. The crude product was purified by Prep-TLC (silica gel, PE: EtOAc = 1:1) and then by Prep-HPLC (Waters Xbridge 150 x 25 mm, 5µm), A = H₂O (10 mM NH₄HCO₃) and B = CH₃CN; 35-65% B over 10 minutes) to give the product (22.8 mg, 61.6 µmol, 76% yield) as a solid.
**¹H NMR** (400MHz, DMSO-*d⁶*) δ_{H} = 8.71 (d, 1H), 8.13 (s, 1H), 7.52 (s, 2H), 7.33 (s, 1H), 7.29 - 7.23 (m, 2H), 5.43 (q, 1H), 3.02 - 2.93 (m, 1H), 2.90 - 2.79 (m, 1H), 2.52 (s, 3H), 2.46 - 2.41 (m, 1H), 2.04 - 1.93 (m, 1H).
**LCMS** Rₜ = 1.13 min in 2.0 min chromatography, 10-80AB, MS ESI calcd. for C₁₅H₁₆ClN₂O₃S₂ [M+H]⁺ 371.0, found 370.9.

### Example 11. Synthesis of Compound I-11

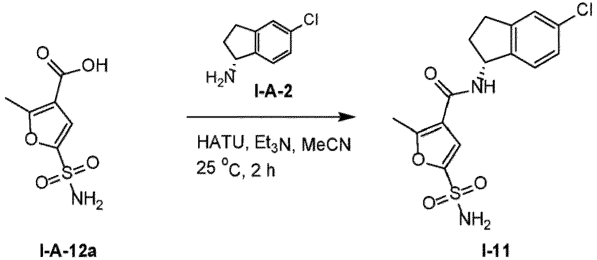

A mixture of 2-methyl-5-sulfamoyl-furan-3-carboxylic acid (150 mg, 0.73 mmol), (1*R*)-5-chloroindan-1-amine (183.82 mg, 1.1 mmol), Et₃N (0.2 mL, 1.46 mmol) and HATU (277.97 mg, 0.73 mmol) in MeCN (5 mL) was stirred at 25 °C for 2 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 30%) to give the product (23.7 mg, 0.07 mmol, 24% yield) as a solid.
**¹H NMR** (400 MHz, DMSO-*d⁶*) δ_{H} = 8.54 (d, 1H), 7.76 (s, 2H), 7.51 (s, 1H), 7.34 (s, 1H), 7.27 - 7.18 (m, 2H), 5.45 (q, 1H), 3.02 - 2.95 (m, 1H), 2.89 - 2.80 (m, 1H), 2.64 (s, 3H), 2.44 - 2.39 (m, 1H), 2.00 - 1.91 (m, 1H).
**LCMS** Rₜ = 1.13 min in 2.0 min chromatography, 10-80AB, MS ESI calcd. For C₁₅H₁₆ClN₂O₄S [M+H]⁺ 355.0, found 355.1.

### Example 12. Synthesis of Compound I-12

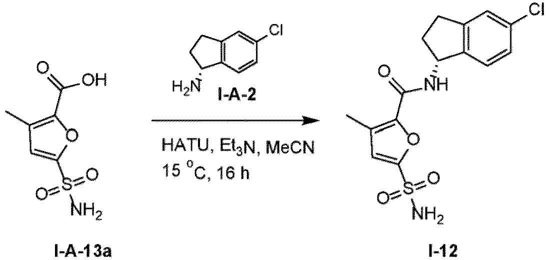

A mixture of 3-methyl-5-sulfamoyl-furan-2-carboxylic acid (150 mg, 0.73 mmol), HATU (277.97 mg, 0.73 mmol), Et₃N (0.2 mL, 1.46 mmol) and (1*R*)-5-chloroindan-1-amine (183.82 mg, 1.1 mmol) in MeCN (5mL) was stirred at 15 °C for 16 hours. Water (15 mL) was added and the mixture was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 30%) to give the product (19.3 mg, 0.05 mmol, 19% yield) as a solid.
**¹H NMR** (400MHz, DMSO-*d⁶*) δ_{H} = 8.53 (d, 1H), 7.76 (s, 2H), 7.35 (s, 1H), 7.29 - 7.19 (m, 2H), 6.98 (s, 1H), 5.48 (q, 1H), 3.07 - 2.94 (m, 1H), 2.90 - 2.81 (m, 1H), 2.47 - 2.40 (m, 1H), 2.33 (s, 3H), 2.09 - 1.97 (m, 1H).
**LCMS** Rₜ = 1.14 min in 2.0 min chromatography, 10-80AB, MS ESI calcd. For C₁₅H₁₆ClN₂O₄S [M+H]⁺ 355.0, found 354.9.

### Example 13. Synthesis of Compound I-13

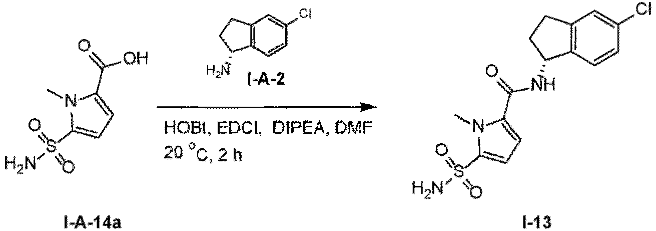

A mixture of 1-methyl-5-sulfamoyl-pyrrole-2-carboxylic acid (150 mg, 0.73 mmol), HOBt (198.53 mg, 1.47 mmol), EDCI (281.64 mg, 1.47 mmol), DIPEA (0.22 mL, 2.2 mmol) and (1*R*)-5-chloroindan-1-amine (184.72 mg, 1.1 mmol) in DMF (5 mL) was stirred at 20 °C for 2 hours. Water (20 mL) was added and the mixture was extracted with EtOAc (20 mL x 2). The combined organic phase was washed with brine (30 mL), dried over anhydrous Na₂SO₄, filtered and concentrated. The crude product was purified by Prep-HPLC (Waters Xbridge 150 x 25 mm, 5 µm, A = H₂O (10 mM NH₄HCO₃) and B = CH₃CN; 35-65%B over 10 minutes) to give the product (79.66 mg, 225.1 µmol, 66% yield) as a solid. **¹H NMR** (400 MHz, DMSO-*d⁶*) δ_{H} = 8.62 (d, 1H), 7.41 (d, 1H), 7.32 (s, 1H), 7.24 - 7.18 (m, 2H), 7.14 (d, 1H), 7.03 (s, 2H), 5.43 (q, 1H), 3.90 (s, 3H), 3.02 - 2.93 (m, 1H), 2.88 - 2.78 (m, 1H), 2.43 - 2.36 (m, 1H), 2.04 - 1.93 (m, 1H).
**LCMS** Rₜ = 1.16 min in 2.0 min chromatography, 10-80AB, MS ESI calcd. for C₁₅H₁₇ClN₃O₃S [M+H]⁺ 354.1, found 353.8.

### Example 14. Synthesis of Compound I-14

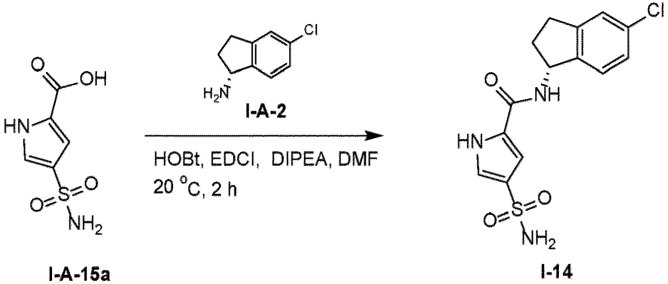

A mixture of 4-sulfamoyl-1*H*-pyrrole-2-carboxylic acid (150 mg, 0.79 mmol), EDCI (302.4 mg, 1.58 mmol), HOBt (213.16 mg, 1.58 mmol), DIPEA (0.24 mL, 2.37 mmol) and (1*R*)-5-chloroindan-1-amine (198.33 mg, 1.18 mmol) in DMF (5 mL)was stirred at 20 °C for 2 hours. Water (20 mL) was added and the mixture was extracted with EtOAc (20 mL x 2). The combined organic phase was washed with brine (30 mL), dried over anhydrous Na₂SO₄, filtered and concentrated. The crude product was purified by Prep-HPLC (Xtimate C18 150 x 25 mm, 5 µm, A = H₂O (10 mM NH₄HCO₃) and B = CH₃CN; 0-60%B over 10 minutes) to give the product (17.0 mg, 50.0 µmol, 14% yield) as a solid.
**¹H NMR** (400 MHz, DMSO-*d⁶*) δ_{H} = 12.03 (s, 1H), 8.62 (d, 1H), 7.34 (s, 1H), 7.24 - 7.18 (m, 3H), 7.15 (d, 1H), 7.02 (s, 2H), 5.46 (q, 1H), 3.02 - 2.94 (m, 1H), 2.89 - 2.79 (m, 1H), 2.46 - 2.39 (m, 1H), 2.02 - 1.92 (m, 1H).
**LCMS** Rₜ = 1.11 min in 2.0 min chromatography, 10-80AB, MS ESI calcd. for C₁₄H₁₅ClN₃O₃S [M+H]⁺ 340.0, found 339.8.

### Example 15. Synthesis of Compound I-15

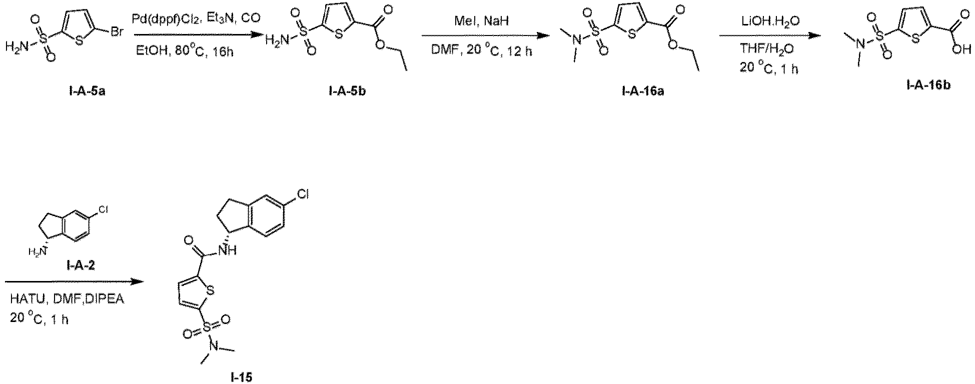

**Synthesis of I-A-5b:** A mixture of 5-bromothiophene-2-sulfonamide (2 g, 8.26 mmol), Pd(dppf)Cl₂ (906.65 mg, 1.24 mmol) and Et₃N (3.43 mL, 24.78 mmol) in ethanol (20 mL) was stirred at 80 °C under CO (50 psi) for 16 hours. The mixture was filtered through Celite and the filtrate was concentrated under reduced pressure. The crude product was purified by flash chromatography on silica gel (EtOAc in PE = 0% to 30% to 50%) to give the product (1 g, 4.25 mmol, 51% yield) as a solid.
**¹H NMR** (400MHz, DMSO-*d₆*) δ_{H} = 7.95 (s, 1H), 7.77 (d, 1H), 7.58 (d, 1H), 4.32 (q, 2H), 1.30 (t, 3H).

**Synthesis of I-A-16a:** To a mixture of ethyl 5-sulfamoylthiophene-2-carboxylate (100 mg, 0.43 mmol) in DMF (5 mL) was added NaH (51 mg, 1.28 mmol, 60% in oil). Then iodomethane (960 mg, 6.76 mmol) was added to the above mixture. The mixture was stirred at 20 °C for 12 hours. Saturated NH₄Cl solution (20 mL) was added and the mixture was extracted with EtOAc (20 mL x 2). The combined organic phase was washed with brine (20 mL), dried over anhydrous Na₂SO₄, filtered and concentrated to give the crude product (100 mg, 379.8 µmol) as a solid.
**LCMS** Rt = 0.82 min in 1.5 min chromatography, 5-95AB, MS ESI calcd. for C₉H₁₄NO₄S₂ [M+H]⁺ 264.0, found 263.8.

**Synthesis of I-A-16b:** A mixture of ethyl 5-(dimethylsulfamoyl)thiophene-2-carboxylate (100 mg, 0.38 mmol) and LiOH·H₂O (47.8 mg, 1.14 mmol) in THF (2 mL) and water (2 mL) was stirred at 20 °C for 1 hour. 1N HCl (10 mL) was added to adjust to pH = 2. The aqueous layer was extracted with EtOAc (20 mL x 2). The combined organic phase was washed with brine (20 mL), dried over anhydrous Na₂SO₄, filtered and concentrated to give the crude product (85 mg, 361.3 µmol) as a solid.
**¹H NMR** (400MHz, DMSO-*d⁶*) δ_{H} = 13.92 (s, 1H), 7.81 (d, 1H), 7.65 (d, 1H), 2.69 (s, 6H).

**Synthesis of I-15:** A mixture of 5-(dimethylsulfamoyl)thiophene-2-carboxylic acid (120 mg, 0.51 mmol), (1*R*)-5-chloroindan-1-amine (102.6 mg, 0.61 mmol), DIPEA (0.15 mL, 1.53 mmol) and HATU (232.72 mg, 0.61 mmol) in DMF (5 mL) was stirred at 20 °C for 1 hour. Water (15 mL) was added and the aqueous layer was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 50%) to give the product (71.6 mg, 186.0 µmol, 36% yield) as a solid.
**¹H NMR** (400MHz, DMSO-*d⁶*) δ_{H} = 9.16 (d, 1H), 7.93 (d, 1H), 7.65 (d, 1H), 7.36 (s, 1H), 7.26 (s, 2H), 5.46 (q, 1H), 3.06 - 2.96 (m, 1H), 2.92 - 2.81 (m, 1H), 2.69 (s, 6H), 2.48 - 2.43 (m, 1H), 2.05 - 1.92 (m, 1H).
**LCMS** Rₜ = 1.26 min in 2.0 min chromatography, 10-80AB, MS ESI calcd. for C₁₆H₁₈ClN₂O₃S₂ [M+H]⁺ 385.0, found 384.9.

### Example 16. Synthesis of Compound I-16

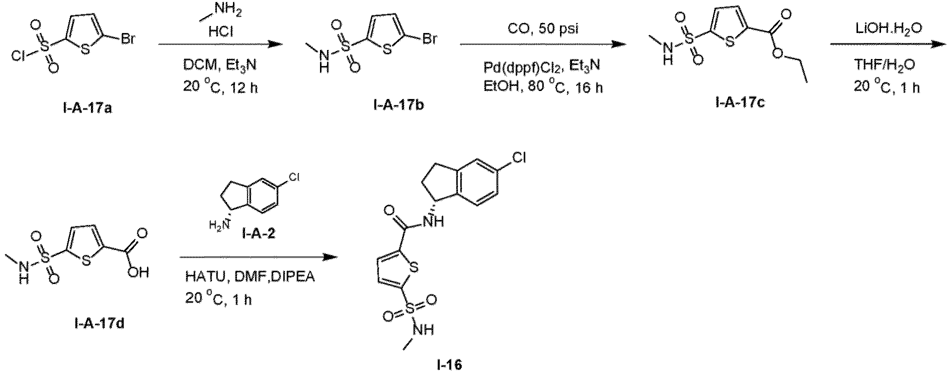

**Synthesis of I-A-17b:** A mixture of 5-bromothiophene-2-sulfonyl chloride (3 g, 11.47 mmol), methanamine hydrochloride (929.39 mg, 13.76 mmol) and Et₃N (4.76 mL, 34.41 mmol) in DCM (30 mL) was stirred at 20 °C for 12 hours. Water (30 mL) was added and the aqueous layer was extracted with EtOAc (30 mL x 2). The combined organic phase was washed with brine (30 mL), dried over anhydrous Na₂SO₄, filtered and concentrated to give the crude product (2.9 g, 11.3 mmol) as an oil.

**Synthesis** of **I-A-17c:** A mixture of 5-bromo-N-methyl-thiophene-2-sulfonamide (2.9 g, 11.32 mmol), Pd(dppf)Cl₂ (1.24 g, 1.7 mmol), Et₃N (4.7 mL, 33.97 mmol) in ethanol (20 mL) was stirred under CO (50 psi) at 80 °C for 16 hours. The mixture was filtered through Celite and the filtrate was concentrated. The crude product was purified by flash chromatography on silica gel (EtOAc in PE = 0% to 30% to 50%) to give the product (2.2 g, 8.82 mmol, 77% yield) as a solid.
**¹H NMR** (400MHz, CDCl₃) δ_{H} = 7.73 (d, 1H), 7.55 (d, 1H), 4.87 - 4.79 (m, 1H), 4.38 (q, 2H), 2.78 (d, 3H), 1.39 (t, 3H).

**Synthesis of I-A-17d:** A mixture of ethyl 5-(methylsulfamoyl)thiophene-2-carboxylate (1 g, 4.01 mmol) and LiOH·H₂O (504.91 mg, 12.03 mmol) in THF (10 mL) and water (10 mL) was stirred at 20 °C for 1 hour. 1N HCl (30 mL) was added to adjust to pH = 2. The aqueous layer was extracted with EtOAc (30 mL x 2). The combined organic phase was washed with brine (30 mL), dried over anhydrous Na₂SO₄, filtered and concentrated to give the crude product (880 mg, 3.97 mmol) as a solid.
**¹H NMR** (400MHz, DMSO-*d⁶*) δ_{H} = 13.78 (brs, 1H), 7.91 (dd, 1H), 7.74 (d, 1H), 7.58 (d, 1H), 2.53 (d, 3H).

**Synthesis of I-16:** A mixture of 5-(methylsulfamoyl)thiophene-2-carboxylic acid (200 mg, 0.90 mmol), (1*R*)-5-chloroindan-1-amine (181.85 mg, 1.08 mmol), DIPEA (0.27 mL, 2.71 mmol) and HATU (412.46 mg, 1.08 mmol) in DMF (10 mL) was stirred at 20 °C for 1 hour. Water (15 mL) was added and the aqueous layer was extracted with EtOAc (20 mL x 2). The combined organic phase was washed with brine (20 mL), dried over anhydrous Na₂SO₄, filtered and concentrated. The crude product was purified by flash chromatography on silica gel (EtOAc in PE = 0% to 35% to 50%) to give the product (201.1 mg, 542.3 µmol, 59% yield) as a solid.
**¹H NMR** (400MHz, DMSO-*d⁶*) δ_{H} = 9.12 (d, 1H), 7.89 - 7.79 (m, 2H), 7.58 (d, 1H), 7.37 (s, 1H), 7.26 (d, 2H), 5.47 (q, 1H), 3.07 - 2.97 (m, 1H), 2.93 - 2.82 (m, 1H), 2.54 - 2.52 (m, 3H), 2.49 - 2.44 (m, 1H), 2.06 - 1.94 (m, 1H).
**LCMS** Rₜ = 1.24 min in 2.0 min chromatography, 10-80AB, MS ESI calcd. for C₁₅H₁₆ClN₂O₃S₂ [M+H]⁺ 371.0, found 371.0.

### Example 17. Synthesis of Compound I-17

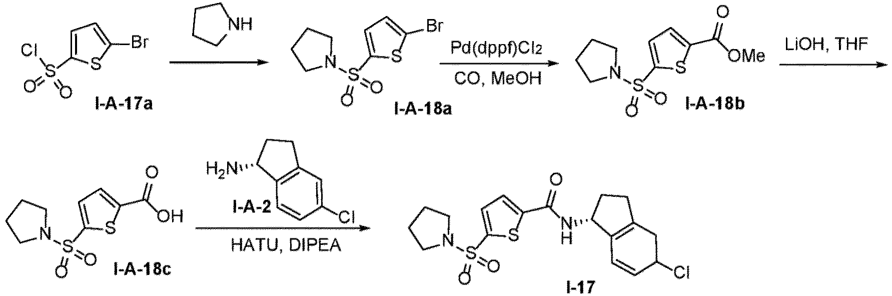

**Synthesis of I-A-18a:** To a stirred solution of **I-A-17a** (1 g, 3.82 mmol) in DCM (30 mL) was added pyrrolidine (0.41 g, 5.74 mmol), K₂CO₃ (1.59 g, 11.47 mmol) and reaction mixture was stirred at RT for 1 hour. The reaction mixture was diluted with DCM (20 mL) and water (15 mL). The organic layer was separated and washed with 2 x 10 mL water and 1 × 10 mL saturated brine solution. Organic layer was dried over MgSO₄ and evaporated to give the crude mixture. The crude mixture was purified by flash column chromatography eluting 50 % EtOAc in hexane to afford desired **I-A-18a** (1 g, 3.33 mmol, 87 % yield) as a solid, which was used for the next step without further purification.

**Synthesis of I-A-18b:** To a stirred solution of **I-A-18a** (1 g, 3.38 mmol) in methanol (20 mL) and DMF (2 mL) was added TEA (0.34 g, 3.38 mmol), Pd(dppf)Cl₂·DCM (0.28 g, 0.34 mmol) in autoclave. Then reaction mass was heated to 80° C under 100 psi CO atm pressure for 6 hours. After completion reaction mass was cooled to RT, filtered on Celite bed and concentrated to dryness. The crude was purified by flash column chromatography eluting 40 % EtOAc in hexane to afford desired **I-A-18b** (0.90 g, 2.57 mmol, 76 % yield) as a solid.

**Synthesis of I-A-18c:** To a stirred solution of **I-A-18b** (0.9 g, 3.27 mmol) in THF (6 mL) were added lithium hydroxide (0.12 g, 4.9 mmol) in water (2 mL) at 0 °C. The reaction mixture was stirred at room temperature for 6 h. The reaction mixture was concentrated and residue was diluted with water and ether. Organic layer was separated. Aqueous layer was acidified with dilute HCl. Solid formed was filtered to afford desired **I-A-18c** (0.60 g, 2.26 mmol, 69 % yield) as a solid that was used for the next step without further purification.

**Synthesis of I-17:** To a stirred solution of **I-A-18c** (0.1 g, 0.38 mmol) in DCM (5 mL) was added **I-A-2** (0.06 g, 0.380 mmol), HATU (0.17 g, 0.46 mmol), DIPEA (0.13 mL, 0.77 mmol) stirred at RT for 6 h. The reaction was diluted with water (10 mL) and DCM (10 mL). The organic layer was separated, washed with 1 × 20 mL saturated brine solution. Organic layer separated, dried over MgSO₄ and concentrated. The crude was purified by flash column chromatography eluting 50 % EtOAc in hexane to give **I-17** (55 mg, 0.13 mmol, 34 % yield) as a solid.
**HPLC:** Rt 9.26 min, 97.6 %; Column: X-select CSH C18 (4.6 x 150) mm, 5 µm; Mobile phase: A: 0.1% FA in water: ACN (95:05), B: ACN; Flow Rate: 1.0 mL/min.
**LCMS:** 411.15 (M+H), Rt 2.05 min, Column: X-select CSH C18 (3 x 50) mm, 2.5 µm
**¹H NMR (400 MHz, DMSO-d6):** δ_{H} = 9.13 (d, 1H), 7.90 (d, 1H), 7.69 (d, 1H), 7.36 (s, 1H), 7.25 (s, 2H), 5.5-5.4 (m,1H), 3.32 - 3.17 (m, 5H), 3.06 - 2.95 (m, 1H), 2.87 (dt, 1H), 2.06 - 1.92 (m, 1H), 1.8-1.60 (m, 4H).

### Example 18. Synthesis of Compound I-18

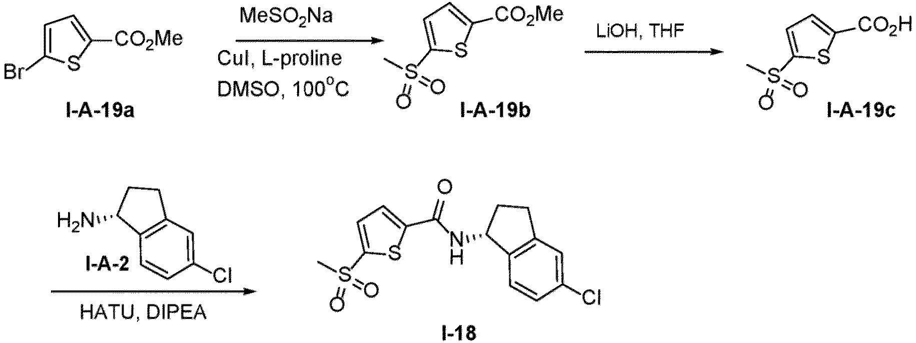

**Synthesis of I-A-19b:** To a stirred solution of **I-A-19a** (1 g, 4.52 mmol) and sodium methanesulfinate (553.67 mg, 5.43 mmol) in DMSO (20 mL) was added copper iodide (85.95 mg, 0.45 mmol) and L-proline (104.16 mg, 0.90 mmol) at RT, followed by addition of sodium hydroxide. The reaction mixture was then allowed to stir for 16 hr at 95 °C. The reaction was quenched by water (50 mL) added EtOAc (50 mL x 2). The organic layer was separated, dried over Na₂SO₄ and concentrated to give the crude mixture. The crude mixture was purified by column chromatography using 100-200 silica and 20-40 % EtOAc/Hexane to afford the desired **I-A-19b** (270 mg, 1.16 mmol, 26 % yield) as a solid, which was used for the next step without further purification.

**Synthesis of I-A-19c:** To a stirred solution of **I-A-19b** (0.25 g, 1.13 mmol) in THF: water (8:2 mL) was added LiOH.HzO (57.15 mg, 1.36 mmol) at RT. Then reaction mixture was stirred for 2 hr at RT. The reaction was quenched by water (100 mL) and was diluted with EtOAc (50 mL x 2). The organic layer was separated and the water layer was acidified with 1*N* HCl. The precipitate formed was filtered, separated, dried on high vacuum to afford the desired **I-A-19c** as a solid (0.15 g, 0.69 mmol, 61 %). The **I-A-19c** was used for the next step without further purification.

**Synthesis of I-18:** To a stirred solution of **I-A-19c** (0.1 g, 0.48 mmol) and **I-A-2** (97.54 mg, 0.58 mmol) in DCM (10 mL) were added DIPEA (0.17 mL, 0.97 mmol) and HATU (276.54 mg, 0.73 mmol) at RT. After that reaction mixture was stirred for 2 hr at RT. The reaction was quenched using water (100 mL) and DCM (2 x 100 mL). The organic layer was separated, dried with Na₂SO₄ and then filtered and concentrated. The crude was purified by column chromatography in 100-200 silica at 30-80 % EtOAc/Hexane eluent to afford I**-18** as a solid (80 mg, 0.22 mmol, 46 % yield).
**HPLC:** Rt 8.31 min, 99.7%; Column: X-select CSH C18 (4.6 x 150) mm, 3.5 µm; Mobile phase: A: 0.1% FA in water: ACN (95:05), B: ACN; Flow Rate: 1.0 mL/min
**LCMS:** 355.95 (M+H), Rt 1.88 min, Column: X-select CSH C18 (3 x 50) mm, 2.5 µm
**¹H NMR (400 MHz, DMSO-d6):** δ_{H} = 9.17 (d, 1H), 7.88 (d, 1H), 7.81 (d, 1H), 7.36 (s, 1H), 7.25 (bs, 2H), 5.47 (q, 1H), 3.38 (s, 3H), 3.04-2.98 (m, 1H), 2.91-2.83 (m, 1H), 2.47-2.44 (m, 1H), 2.00-1.95 (m, 1H).

### Example 19. Synthesis of Compound I-19

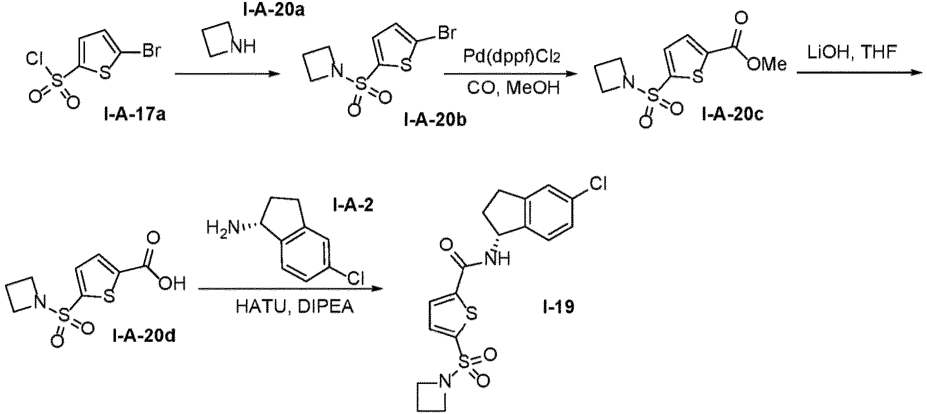

**Synthesis of I-A-20b:** To a stirred solution of **I-A-17a** (1 g, 3.82 mmol) in DCM (20 mL) was added **I-A-20a** (436.57 mg, 7.65 mmol) and stirred at RT for 30 min. The reaction was quenched with water (50 mL) and diluted with DCM (50 mL x 2). The organic layer was separated, dried over Na₂SO₄ and concentrated to give crude mixture. The crude mixture was then purified by column chromatography using 100-200 silica and 5-10 % EtOAc/Hexane as an eluent to give **I-A-20b** (900 mg, 3.16 mmol, 83 % yield) as a solid.

**Synthesis of I-A-20c:** To a stirred solution of **I-A-20b** (0.5 g, 1.77 mmol) in methanol: DMF (20:2 mL) was added TEA (179.08 mg, 1.77 mmol) at RT. The reaction mixture was degassed for 30 min using argon gas at RT and treated with Pd(dppf)Cl₂·DCM (144.68 mg, 0.180 mmol). CO gas was purged into the reaction mixture (100 psi) and the reaction mixture was heated at 80 °C for 16 hr. The reaction mixture was evaporated under reduced pressure. The crude was purified by column chromatography using 100-200 silica at 10-20% EtOAc/Hexane as an eluent to afford desired **I-A-20c** (250 mg, 0.90 mmol, 51.25 % yield) as a solid that was used for the next step without further purification.

**Synthesis of I-A-20d:** To a stirred solution of **I-A-20c** (0.25 g, 0.95 mmol) in THF: water (8:2 mL) was added lithium hydroxide (0.06 mg, 0.0014 mmol) at RT and stirred for 2 h. The reaction was quenched with water (100 mL) and diluted with EtOAc (50 mL x 2). The organic layer was separated and the aqueous layer was acidified with 1N HCl leading to precipitation. The precipitate formed was filtered, separated and dried over high vacuum to afford desired **I-A-20d** (120 mg, 0.47 mmol, 49 % yield) as a solid that was used for the next step without further purification.

**Synthesis of I-19:** To a stirred solution of **I-A-20d** (0.1g, 0.40 mmol) and **I-A-2** (81.35 mg, 0.49 mmol) in DCM (10 mL) were added DIPEA (0.14 mL, 0.81 mmol) and HATU (230.64 mg, 0.610 mmol) at RT. The reaction mixture was stirred at RT for 2 h. The reaction was quenched with water (100 mL) and diluted with DCM (100 mL x 2). The organic layer was separated, dried over Na₂SO₄ and concentrated to obtain crude product. The crude was purified by column chromatography using 100-200 silica at 30-80% EtOAc/Hexane as an eluent to give I**-19** (60 mg, 0.14 mmol, 37 % yield) as a solid.
**HPLC:** Rt 8.99 min, 99.6 %; Column: X-select CSH C18 (4.6 x 150) mm, 5 µm; Mobile phase: A: 0.1% FA in water: ACN (95:05), B: ACN; Flow Rate: 1.0 mL/min
**LCMS:** 397 (M+H), Rt 2.01 min, Column: X-select CSH C18 (3.0 x 50) mm, 2.5 µm
**¹H NMR (400 MHz, DMSO-d6)** δ_{H} = 9.19 (d, 1H), 7.99 (d, 1H), 7.74 (d, 1H), 7.37 (s, 1H), 7.29-7.24 (m, 2H), 5.48 (q, 1H), 3.77 (t, 4H), 3.02 (ddd, 1H), 2.88 (dt, 1H), 2.51 - 2.41 (m, 1H), 2.12 - 1.93 (m, 3H).

### Example 20. Synthesis of Compound I-20

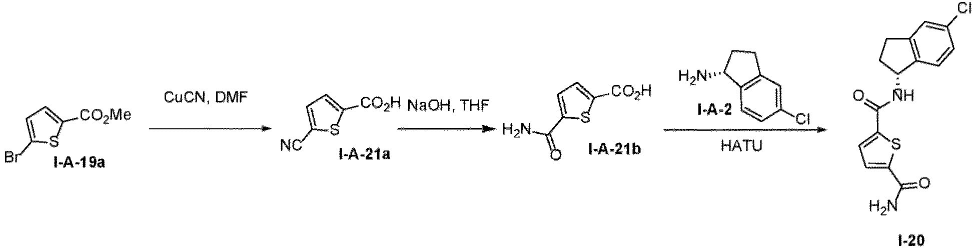

**Synthesis of I-A-21a:** To a stirred solution of **I-A-19a** (1 g, 4.52 mmol) and copper(I) cyanide (0.61 g, 6.79 mmol) in DMF (10 mL) was heated at 120° C for 12 h. After completion, ice cold water was added to the reaction mixture. The solid was filtered and purified by column chromatography using silica gel (100-200) and 20 % ethyl acetate in hexane as eluent to give **I-A-21a** (500 mg, 2.66 mmol, 59 % yield) as a solid.

**Synthesis of I-A-21b:** To a stirred solution of **I-A-21a** (0.5 g, 2.99 mmol) in THF (5 mL) was added NaOH (0.36 g, 8.97 mmol) in water (5 mL) and reaction was then heated at 90° C for 12 h. The solvent was removed, and the aqueous layer was cooled to 0 °C and acidified with 2N HCl. The solid was filtered and dried to give **I-A-21b** (0.35 g, 1.02 mmol, 34 % yield) as a solid that was used for the next step without further purification.

**Synthesis of I-20:** To a stirred solution of **I-A-21b** (0.1 g, 0.58 mmol) in DCM (3 mL) were added DIPEA (0.2 mL, 1.17 mmol) and HATU (0.3 g, 0.88 mmol) at 0 °C and stirred for 10 min. To the resulting reaction mixture **I-A-2** (0.11 g, 0.64 mmol) was added and reaction mixture was stirred at RT for 6 h. The reaction mixture was quenched with water, extracted with DCM (10 mL x 3) and organic layer separated. The combined organic layer was dried over sodium sulphate and solvent was removed under reduced pressure to give crude product which was purified by Prep HPLC to give I**-20** (20.61 mg, 11% yield) as a solid.
**HPLC:** Rt 7.64 min, 97.8 %; Column: X-select CSH C18 (4.6 x 150) mm, 5 µm; Mobile phase: A: 0.1% FA in water: ACN (95:05), B: ACN; Flow Rate: 1.0 mL/min
**LCMS:** 321.05 (M+H), Rt 1.74 min, Column: X-select CSH C18 (3 x 50) mm, 2.5 µm
**¹H NMR (400 MHz, DMSO-d6)** δ_{H} = 8.92 (d, 1H), 8.07 (bs, 1H), 7.76 (d, 1H), 7.68 (d, 1H), 7.57 (bs, 1H), 7.35 (s, 1H), 7.24 (d, 2H), 5.48-5.41 (m, 1H), 3.02-2.96 (m, 1H), 2.90-2.84 (m, 1H), 2.50-2.42 (m, 1H), 2.01-1.96 (m, 1H).

### Example 21. Synthesis of Compound I-21

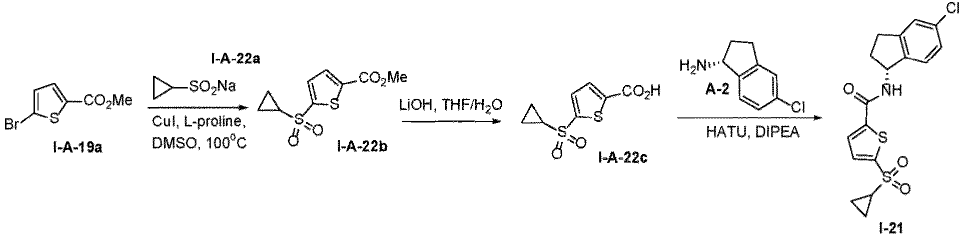

**Synthesis of I-A-22b:** To a stirred solution of **I-A-19a** (1.5 g, 6.79 mmol) and **I-A-22a** (1.3 g, 10.18 mmol) in DMSO (20 mL) were added copper iodide (0.13 g, 0.68 mmol), L-proline (0.16 g, 1.36 mmol) and sodium hydroxide (0.05 g, 1.35 mmol) at RT. The reaction mixture was stirred at 95 °C for 16 h. The reaction was quenched using water (50 mL) and diluted with EtOAc (50 mL x 2). The organic layer was separated, dried over Na₂SO₄, filtered and evaporated under reduced pressure to give the crude product. The crude was purified by column chromatography using 100-200 silica and 20-40 % EtOAc/Hexane eluent to give **I-A-22b** (0.4 g, 1.54 mmol, 23 % yield) as a solid that was used for the next step without further purification.

**Synthesis of I-A-22c:** To a stirred solution of **I-A-22b** (0.4 g, 1.62 mmol) in THF: water (10:3 mL) was added LiOH.HzO (0.1 g, 2.44 mmol) at RT. The reaction mixture was stirred at RT for 2 h. The reaction was quenched using water (100 mL) and diluted with EtOAc (50 mL x 2). The organic layer was separated and resulting aqueous layer was acidified with 1N HCl, leading to precipitation. The solid formed was filtered, separated and dried to give **I-A-22c** (0.23 g, 0.95 mmol, 58 % yield) as a solid that was used for the next step without further purification.

**Synthesis of I-21:** To a stirred solution of **I-A-22c** (0.1 g, 0.43 mmol) and **I-A-2** (86.61 mg, 0.52 mmol) in DCM (10 mL) was added. To the resulting reaction mixture HATU (245.54 mg, 0.65 mmol) and DIPEA (0.15 mL, 0.86 mmol) were added at RT. The reaction mixture was stirred at RT for 2 h. The reaction was quenched by water (100 mL), diluted with DCM (100 mL x 2). The organic layer was then separated, dried by Na₂SO₄, filtered, evaporated under reduced pressure to give crude product. The crude mixture was purified by column chromatography using 100-200 silica and 30-80% EtOAc/Hexane eluent to give I**-21** (0.092 g, 0.24 mmol, 56 % yield) as a solid.
**HPLC:** Rt 8.90 min, 99.9 %; Column: X-select CSH C18 (4.6 x 150) mm, 5 µm; Mobile phase: A: 0.1% FA in water: ACN (95:05), B: ACN; Flow Rate: 1.0 mL/min
**LCMS:** 382.00 (M+H), Rt 2.00 min, Column: X-select CSH C18 (3 x 50) mm, 2.5 µm
**¹H NMR (400 MHz, DMSO-d6)** δ_{H} = 9.18 (d, 1H), 7.89 (d, 1H), 7.79 (d, 1H), 7.36 (s, 1H), 7.25 (s, 2H), 5.47 (q, 1H), 3.04-2.97 (m, 2H), 2.91-2.83 (m, 1H), 2.46-2.44 (m, 1H), 2.04-1.92 (m, 1H), 1.28 - 1.08 (m, 4H).

### Example 22. Syntheses of Compound 1-22 and Compound I-23

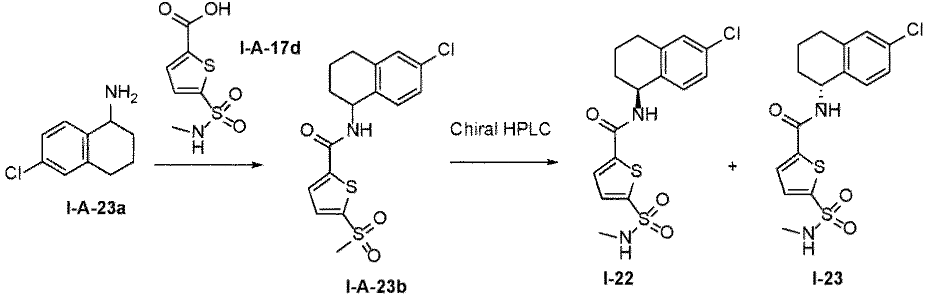

To a stirred solution of **I-A-23a** (160 mg, 0.90 mmol) and 5-(methylsulfamoyl)thiophene-2-carboxylic acid (210 mg, 0.90 mmol) in DCM (20 mL) were added DIPEA (0.31 mL, 1.81 mmol)) and HATU (380 mg, 0.99 mmol) at 0 °C and stirred at RT for 6 h. The reaction mixture was quenched with water (10 mL) and diluted with DCM (10 mL). The organic layer was washed with brine solution (10 mL), dried over MgSO₄ and evaporated to give the crude product. The crude product **I-A-23b** was purified by chiral SFC chromatography with mobile phase: A)CO₂ B)MeOH+NH₃,Gradient:25-50%B in 5 min, hold 50%B till 9 min, 50-25%B at 10 min, hold 25%B till 12 min. Column: DAICEL CHIRALPAK-IG (250 x 4.6 mm, 5 um). Wavelength: 263nm. Flow: 3 mL/min to give I**-22** (53mg, 0.13 mmol, 15% yield) and I**-23** (53 mg, 0.13 mmol, 15% yield) as solids. Stereochemistry is randomly assigned.
**I-22: HPLC:** Rt 7.25 min, 98.5%; Column: X-Bridge C18 (4.6 x 150) mm, 5 µm; Mobile phase: A: 0.1% NH₃ in water B: ACN; Flow Rate: 1.2 mL/min.
**LCMS** : 384.98 (M+H), Rt 1.98 min; Column: X-select CSH C18 (3 x 50) mm, 2.5 µm.
**¹H NMR (400 MHz, DMSO-d6)** δ_{H} = 9.11 (d, 1H), 7.88 - 7.80 (m, 2H), 7.56 (d, 1H), 7.26 - 7.16 (m, 3H), 5.16-5.12 (m, 1H), 2.80-2.70 (m, 2H), 2.52-2.48 (m, 3H), 2.00-1.90 (m, 2H), 1.86 - 1.68 (m, 2H).
**Chiral HPLC:** Rt: 3.62 min, 100%, Column: DIACEL CHIRALPAK-IG (250 x 4.6 mm, 5 um); Mobile phase: A) CO₂ B) MeOH + 0.1% NH₃, Gradient: 35-50% B in 5 min, hold 50% Bt ill 9 min, 50-35% B in 10 min, hold 35% B till 12 min. Wavelength: 265 nm, Flow Rate: 3 mL/min.
**I-23: HPLC:** Rt 7.25 min, 99.4%; Column: X-Bridge C18 (4.6 x 150) mm, 5 µm; Mobile phase: A: 0.1% NH₃ in water B: ACN; Flow Rate: 1.2 mL/min.
**LCMS** : 384.91 (M+H), Rt 1.97 min; Column: X-select CSH C18 (3 x 50) mm, 2.5 µm.
**¹H NMR (400 MHz, DMSO-d6)** δ_{H} = 9.11 (d, 1H), 7.86-7.78 (m, 2H), 7.56 (d, 1H), 7.26 - 7.12 (m, 3H), 5.16-5.10 (m, 1H), 2.82-2.72 (m, 2H), 2.52-2.44 (m, 3H), 2.00 - 1.90 (m, 2H), 1.85-1.74 (m, 2H).
**Chiral HPLC:** Rt: 4.68 min, 100%, Column: DIACEL CHIRALPAK-IG (250 x4.6 mm, 5 µm) Mobile phase: A) CO₂ B) MeOH + 0.1% NH₃, Gradient: 35-50% B in 5 min, hold 50% B till 9 min, 50-35% B in 10 min, hold 35% B till 12 min. Wavelength: 265 nm, Flow Rate: 3 mL/min.

### Example 23. Syntheses of Compound 1-24 and Compound I-25

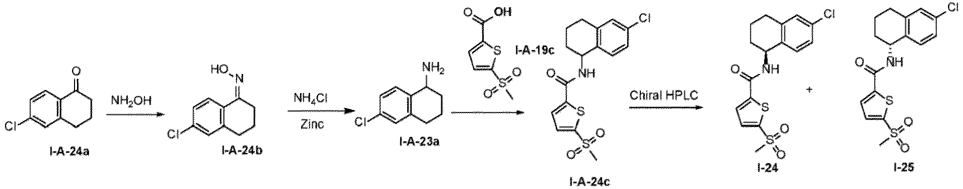

**Synthesis of I-A-24b:** To a stirred solution of **I-A-24a** (2.3 g, 12.73 mmol) in water: THF (1:5, 60 mL) was added NH₂OH (8.85 g, 127.33 mmol), sodium acetate (10.45 g, 127.33 mmol) and heated at 70 °C for 4 h. The reaction mixture was cooled, concentrated and then washed with water. The organic layer was dried over MgSO₄ and evaporated to give **I-A-24b** (2.4 g, 11.81 mmol, 92 % yield) as a solid.

**Synthesis of I-A-23a:** To a stirred solution of **I-A-24b** (2.4 g, 12.27 mmol) in acetic acid (50 mL) was added ammonium chloride (6.56 g, 122.67 mmol), zinc powder (8.02 g, 122.67 mmol) and stirred at RT for 24 h. The reaction mixture was evaporated to dryness; the residue was then washed with EtOAc (100 mL) and saturated NaHCO₃. The organic layer was washed with (2 x 30 mL) water and then with saturated brine solution (30 mL) and dried over MgSO₄ to give **I-A-23a** (1.4 g, 3.94 mmol, 32 % yield) as a liquid.

**Synthesis of I-24 & I-25:** To a stirred solution of **I-A-23a** (132.12 mg, 0.73 mmol) and 5-(methylsulfonyl)thiophene-2-carboxylic acid (150 mg, 0.73 mmol) in DCM (10 mL) were added DIPEA (0.25 mL, 1.45 mmol) and HATU (414.82 mg, 1.09 mmol) at RT and stirred at RT for 2 h. The reaction was quenched using water (100 mL) and diluted with DCM (100 mL x 2). The combined organic layer was dried over sodium sulphate and evaporated to obtain crude product which was purified by column chromatography in 100-200 silica at 30 to 80 % of EtOAc in hexane to afford racemic mixture **I-A-24c** which was then purified by chiral SFC chromatography with mobile phase:
A)CO₂ B)MeOH+NH₃,Gradient:25-50%B in 5 min, hold 50%B till 9 min, 50-25%B at 10 min, hold 25%B till 12 min. Column: DAICEL CHIRALPAK-IG (250 x 4.6 mm, 5 um). Wavelength: 263nm. Flow: 3 mL/min. to give I**-24** (56.1 mg, 0.15 mmol, 21 % yield) and **I-25** (62.5 mg, 0.16 mmol, 23 % yield) as solids. Stereochemistry is randomly assigned.
**I-24: HPLC:** Rt 8.79 min, 99.2%; Column: X-Select CSH C18 (4.6 x 150) mm, 5 µm; Mobile phase: A: 0.1% FA in water: ACN (95:05), B: ACN; Flow Rate: 1.0 mL/min.
**LCMS** : 369.95 (M+H), Rt 2.07 min, Column: X-select CSH C18 (3 x 50) mm, 2.5 µm.
**¹H NMR (400 MHz, DMSO-d6)** δ_{H} = 9.18 (d, 1H), 7.89 (d, 1H), 7.80 (d, 1H), 7.26 - 7.16 (m, 3H), 5.16-5.12 (m, 1H), 3.38-3.30 (m, 3H), 2.84-2.70 (m, 2H), 2.01-1.92 (m, 2H), 1.90 - 1.76 (m, 2H).
**1-25: HPLC:** Rt 8.52 min, 98.2%; Column: X-Select CSH C18 (4.6 x 150) mm, 5 µm; Mobile phase: A: 0.1% FA in water: ACN (95:05), B: ACN; Flow Rate: 1.0 mL/min.
**LCMS** : 369.95 (M+H), Rt 2.05 min, Column: X-select CSH C18 (3 x 50) mm, 2.5 µm.
**¹H NMR (400 MHz, DMSO-d6)** δ_{H} = 9.15 (d, 1H), 7.88 (d, 1H), 7.78 (d, 1H), 7.24 - 7.14 (m, 3H), 5.16-5.10 (m, 1H), 3.36 (d, 3H), 2.78-2.72 (m, 2H), 1.96-1.88 (m, 2H), 1.84-1.55 (m, 2H).

### Example 24. Synthesis of Compound I-26

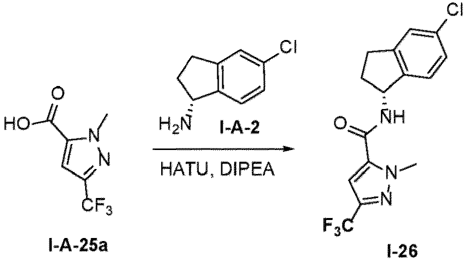

To a stirred solution of **I-A-25a** (115.8 mg, 0.6 mmol) and **I-A-2** (100 mg, 0.6 mmol) in DCM (2 mL) were added DIPEA (0.26 mL, 1.49 mmol) and HATU (340.2 mg, 0.89 mmol) at RT and stirred at RT for 5 h. The reaction mixture was quenched using water (10 mL) and diluted with DCM (10 mL). The organic layer was washed with brine solution (15 mL), dried over (Na₂SO₄) and evaporated to give the crude product. The crude product was purified by prep HPLC to give **1-26** (75 mg, 0.25 mmol, 36 % yield) as a solid.
**HPLC:** Rt 9.40 min, 98.4%; Column: X-Select CSH C18 (4.6 x 150) mm, 5 µm; Mobile phase: A: 0.1% FA in water: ACN (95:05), B: ACN; Flow Rate: 1.0 mL/min.
**LCMS** : 344 (M+H), Rt 2.17 min, Column: X-select CSH C18 (3 x 50) mm, 2.5 µm.
**¹H NMR (400 MHz, DMSO-d6)** δ_{H} = 8.97 (d, 1H), 7.36 (d, 2H), 7.32-7.21 (m, 2H), 5.46 (q, 1H), 4.18 (s, 3H), 3.04-2.96 (m, 1H), 2.92-2.82 (m, 1H), 2.51-2.41 (m, 1H), 2.02-1.92 (m, 1H).
**Chiral method:** Rt 3.20 min, 95%; SFC column: DIACEL CHIRALPAK-IG (250 x 4.6 mm, 5 um), - Mobile Phase: A) CO₂ B) MeOH + 0.1% NH₃, Gradient: 10-40% B in 5 min, hold 40% B till 9 min, 40-10% B in 10 min hold, hold 10% B till 12 min. Wavelength: 280 nm, Flow Rate: 3 mL/min.

### Example 25. Synthesis of Compound 1-27

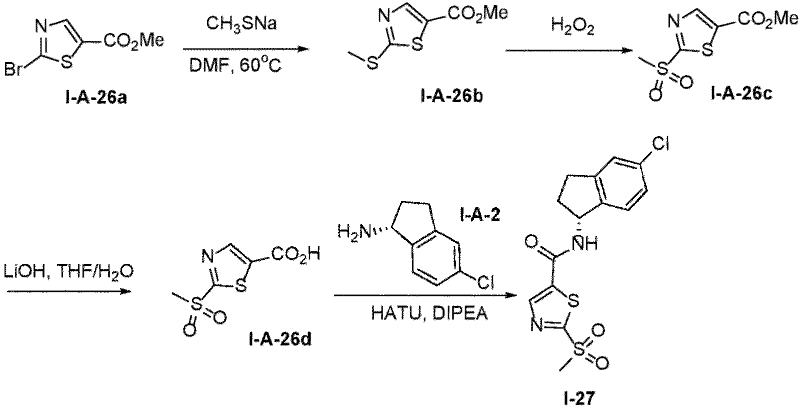

**Synthesis of I-A-26b:** To **I-A-26a** (2 g, 9.01 mmol), sodium methanethiolate (1.26 g, 18.01 mmol) in DMF (10 mL) was added at RT and reaction mixture was stirred at 60 °C for 2 h. The reaction was quenched using water (100 mL) and diluted with EtOAc (100 mL x 2). The organic layer was separated, dried over Na₂SO₄, filtered and evaporated to obtain crude product. The crude product was purified by column chromatography using 100-200 silica and 10-40% EtOAc/Hexane as an eluent to give **I-A-26b** (0.70 g, 3.66 mmol, 40 % yield) as a solid.

**Synthesis of I-A-26c:** To a stirred solution of **I-A-26b** (0.5 g, 2.7 mmol) and sodium tungstate dihydrate (0.09 g, 0.0003 mmol) in acetic acid (10 mL) was added H₂O₂ 30% in water (0.6 mL, 0.01 mmol) at RT and reaction mixture was stirred for 10 min. The reaction was quenched using water (100 mL) and diluted with EtOAc (100 mL x 2). The organic layer was separated, dried over Na₂SO₄, filtered and evaporated to give crude product. The crude product was purified by column chromatography using 100-200 silica and 10-40% EtOAc/Hexane as an eluent to give **I-A-26c** (0.50 g, 2.24 mmol, 84 % yield) as a solid.

**Synthesis of I-A-26d:** To a stirred solution of **I-A-26c** (0.5 g, 2.27 mmol) in THF: water (10: 5 mL) was added LiOH.H₂O (114.3 mg, 2.72 mmol) at RT and stirred at RT for 2 h. The reaction mixture was quenched using water (100 mL) and diluted with EtOAc (50 mL x 2). The aqueous layer was separated. The aqueous layer was acidified with 1*N* HCl. The precipitate thus formed was filtered and dried using high vacuum to give **I-A-26d** (0.30 g, 1.36 mmol, 60 % yield) as a solid.

**Synthesis of 1-27:** To a stirred solution of **I-A-26d** (0.1 g, 0.48 mmol) and **I-A-2** (97.07 mg, 0.58 mmol) in DCM (10 mL) were added HATU (275.22 mg, 0.72 mmol) followed by DIPEA (0.17 mL, 0.97 mmol) at 0 °C and stirred at RT for 2 h. The reaction mixture was diluted with water (100 mL) and DCM (100 mL x 2). The organic layer was separated, dried over Na₂SO₄, filtered and concentrated to give the crude product. The crude product was then purified by column chromatography using 100-200 silica and 30-80 % EtOAc in hexane as an eluent to give **1-27** (85.1 mg, 0.23 mmol, 49 % yield) as a solid.
**HPLC:** Rt 8.34 min, 99.7%; Column: X-select CSH C18 (4.6 x 150) mm, 3.5 µm; Mobile phase: A- 0.1 % FA in water: Acetonitrile (95:05), B: Acetonitrile; Flow Rate: 1.0 mL/min.
**LCMS:** 356.85 (M+H), Rt 2.01 min, Column: X-select CSH C18 (3.0 x 50) mm, 2.5 µm.
**¹H NMR (400 MHz, DMSO-d6):** δ_{H} = 9.35 (d, 1H), 8.68 (d, 1H), 7.37 (s, 1H), 7.33 - 7.22 (m, 2H), 5.52-5.42 (m,1H), 3.50 (d, 3H), 3.05-2.98 (m, 1H), 2.93-2.84 (m, 1H), 2.06-1.92 (m, 1H). Note: 1H not observed

### Example 26. Syntheses of Compound 1-28 and Compound 1-29

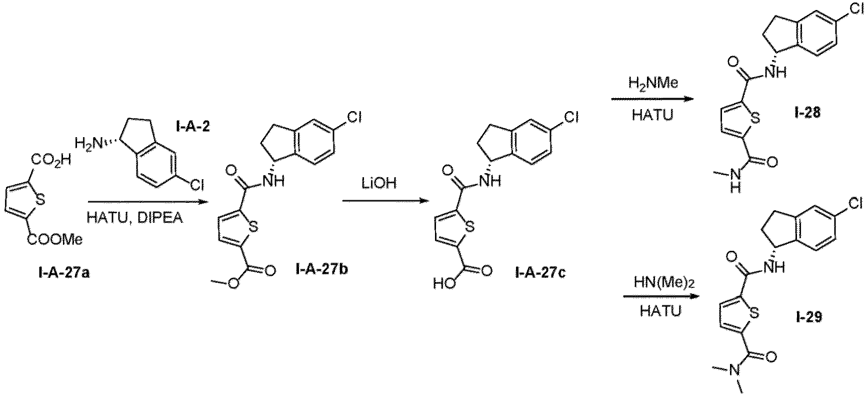

**Synthesis of I-A-27b:** To a stirred solution of **I-A-27a** (400 mg, 2.15 mmol) and **I-A-2** (432.18 mg, 2.58 mmol) in DCM (10 mL) were added HATU (1225.3 mg, 3.22 mmol) and DIPEA (0.75 mL, 4.3 mmol) at RT. The reaction was then stirred at RT for 2 h. The reaction was quenched using water (100 mL) and diluted with DCM (100 mL x 2). The combined organic layer was separated, dried over Na₂SO₄, filtered and evaporated to obtain crude product which was purified by column chromatography using 100-200 silica and 30-80 % of EtOAc/Hexane eluent to give **I-A-27b** (600 mg, 1.71 mmol, 80 % yield) as a solid.

**Synthesis of I-A-27c:** To a solution of **I-A-27b** (500 mg, 1.49 mmol) in THF: water (10:5 mL) was added LiOH.H₂O (74.97 mg, 1.79 mmol) at RT and stirred for 2 h. The reaction was quenched using water (100 mL) and diluted with EtOAc (50 mL x 2). The combined organic layer was separated and acidified with 1N HCl leading to a precipitate, which was filtered, separated and dried to give **I-A-27c** (0.40 g, 1.21 mmol, 81 % yield) as a solid.

**Synthesis of 1-28:** To a stirred solution of **I-A-27c** (100 mg, 0.31 mmol) in DCM (10 mL) and methyl amine in THF (2M) (0.2 mL, 0.37 mmol) was added HATU (177.25 mg, 0.47 mmol) and DIPEA (0.11 mL, 0.62 mmol) at RT and stirred for 2 h. The reaction was quenched with water (100 mL) and diluted with DCM (100 mL x 2). The combined organic layer was separated, dried by Na₂SO₄, filtered and evaporated under reduced pressure to give crude product which was purified by column chromatography using 100-200 silica and 30-80% EtOAc/Hexane eluent to give **1-28** (44.1 mg, 0.13 mmol, 42 % yield) as a solid.
**HPLC:** Rt 7.84 min, 99.5 %; Column: X-select CSH C18 (4.6 x 150) mm, 5 µm; Mobile phase: A: 0.1% FA in water: ACN (95:05), B: ACN; Flow Rate: 1.0 mL/min
**LCMS:** 335.15 (M+H), Rt 1.95 min, Column: X-select CSH C18 (3 x 50) mm, 2.5 µm
**¹H NMR (400 MHz, DMSO-d6)** δ_{H} = 8.92 (d, 1H), 8.57 (d, 1H), 7.76 (d, 1H), 7.64 (d, 1H), 7.35 (s, 1H), 7.24 (s, 2H), 5.50-5.40 (m, 1H), 3.02-2.97 (m, 1H), 2.92 - 2.73 (m, 4H), 2.48-2.42 (m, 1H), 2.03-1.96 (m, 1H).
**Chiral method:** Rt 7.28 min, 98.7%; SFC column: DIACEL CHIRALPAK-IG (250 x 4.6 mm, 5 um), - Mobile Phase: A) CO₂ B) MeOH+0.1% NH₃, Gradient: 20-40% B in 5 min, hold 40% B till 9 min, hold 20% B till 12 min. Wavelength: 280 nm, Flow Rate: 3 mL/min.

**Synthesis of 1-29:** To a stirred solution of **I-A-27c** (100 mg, 0.31 mmol) and dimethyl amine in THF (2M) (0.2 mL, 0.37 mmol) in DCM (10 mL) were added HATU (177.25 mg, 0.47 mmol) and DIPEA (0.11 mL, 0.62 mmol) at RT and stirred at RT for 2 h. Thereaction was quenched with water (100 mL) and diluted with DCM (100 mL x 2). The combined organic layer was separated, dried by Na₂SO₄, filtered and evaporated under reduced pressure. The crude product was purified by column chromatography using 100-200 silica and 30-80% EtOAc/Hexane eluent to give **I-29** (65.2 mg, 0.18 mmol, 59 % yield) as a solid.
**HPLC:** Rt 7.98 min, 98.7 %; Column: X-select CSH C18 (4.6 x 150) mm, 3.5 µm; Mobile phase: A: 0.1% FA in water: ACN (95:05), B: ACN; Flow Rate: 1.0 mL/min
**LCMS:** 348.95 (M+H), Rt 2.01 min, Column: X-select CSH C18 (3 x 50) mm, 2.5 µm
**¹H NMR (400 MHz, DMSO-d6)** δ_{H} = 8.94 (d, 1H), 7.76 (d, 1H), 7.45 (d, 1H), 7.35 (s, 1H), 7.24 (s, 2H), 5.52-5.41 (m, 1H), 3.18 - 2.82 (m, 8H), 2.51 - 2.41 (m, 1H), 2.02-1.96 (m, 1H).
**Chiral method:** Rt 6.35 min, 98.4%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 10% B till 12 min. Wavelength: 280 nm, Flow Rate: 3 mL/min.

### Example 27. Syntheses of Compound 1-30 and Compound 1-31

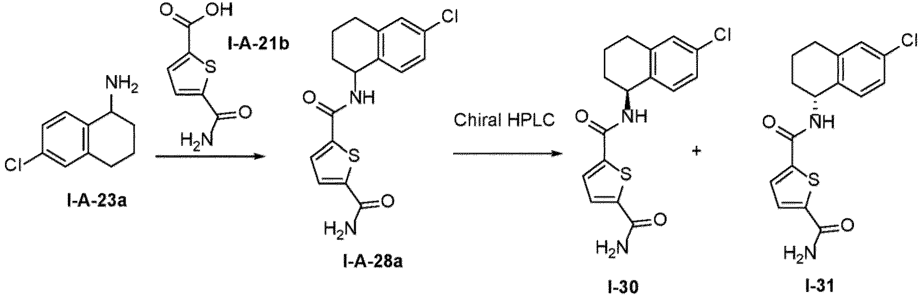

To a stirred solution of **I-A-23a** (151.95 mg, 0.84 mmol) and 5-carbamoylthiophene-2-carboxylic acid (119.31 mg, 0.70 mmol) in DCM (10 mL) were added DIPEA (0.24 mL, 1.39 mmol) and HATU (318.04 mg, 0.84 mmol) at 0 °C and stirred at RT for 3 h. The reaction was quenched using water (15 mL) and diluted with DCM (30 mL). The aqueous layer was washed with (2 x 30 mL) DCM. The organic layer was washed with brine solution (20 mL) and dried over Na₂SO₄ and evaporated to give crude product. The crude product was purified by flash column chromatography using 100-200 silica and 25 % of EtOAc in hexane as an eluent. The desired fractions were evaporated and the racemic compound **I-A-28a** was purified by chiral SFC chromatography with a mobile phase: A)CO₂ B)MeOH+NH₃,Gradient:25-50%B in 5 min, hold 50%B till 9 min, 50-25%B at 10 min, hold 25%B till 12 min. Column: DAICEL CHIRALPAK-IG (250 x 4.6 mm, 5 um). Wavelength: 263nm. Flow: 3 mL/min. to give **1-30** (4.85 mg, 0.01 mmol, 2 % yield) and **1-31** (2.03 mg, 0.0061 mmol, 1 % yield) as solids. Stereochemistry is randomly assigned.
**1-30: HPLC:** Rt 7.74 min, 99.5% Column: X-Select CSH C18 (4.6 x 150) mm, 3.5 µm; Mobile phase: A: 0.1% FA in water: Acetonitrile (95:05), B: ACN; Flow Rate: 1.0 mL/min **LCMS** : 335.10 (M+H), Rt 1.95 min, Column: X-select CSH (3 x 50) mm, 2.5 µm
**¹H NMR (400 MHz, DMSO-d6):** δ_{H} = 8.91 (d, 1H), 8.08 - 8.03 (m, 1H), 7.75 (d, 1H), 7.66 (d, 1H), 7.55 (s, 1H), 7.24 - 7.14 (m, 3H), 5.16-5.08 (m, 1H), 2.85 - 2.68 (m, 2H), 1.98-1.88 (m, 2H), 1.84-1.70 (m, 2H).
**1-31: HPLC:** Rt 7.75 min, 99.8% Column: X-select CSH C18 (4.6 x 150) mm, 3.5 µm; Mobile phase: A: 0.1% FA in water: Acetonitrile (95:05), B: ACN; Flow Rate: 1.0 mL/min **LCMS** : 335.10 (M+H), Rt 1.97 min, Column: X-select CSH C18 (3 x 50) mm, 2.5 µm
**¹H NMR (400 MHz, DMSO-d6)** δ_{H} = 8.91 (d, 1H), 8.05 (s, 1H), 7.76 (d, 1H), 7.66 (d, 1H), 7.55 (s, 1H), 7.24 - 7.14 (m, 3H), 5.16-5.08 (m, 1H), 2.84 - 2.69 (m, 2H), 2.00 - 1.88 (m, 2H), 1.85 - 1.66 (m, 2H).

### Example 28. Syntheses of Compound 1-32 and Compound 1-33

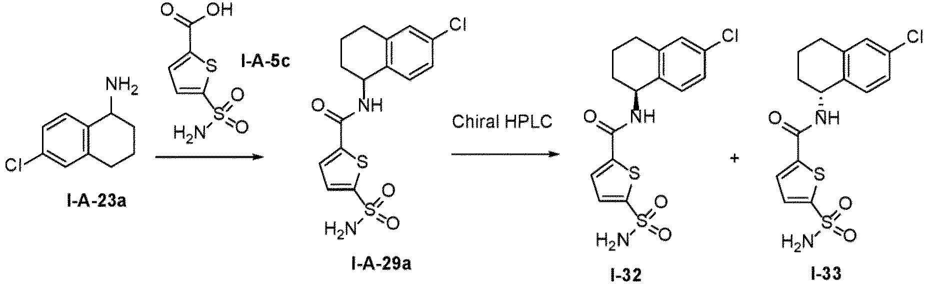

To a stirred solution of **I-A-23a** (260 mg, 1.45 mmol) and 5-sulfamoylthiophene-2-carboxylic acid (300 mg, 1.45 mmol) in DCM (20 mL) were added DIPEA (0.5 mL, 2.9 mmol)) and HATU (660 mg, 1.74 mmol) at RT and stirred at RT for 6 h. The reaction mixture was quenched using water (10 mL) and diluted with DCM (10 mL). The organic layer was washed with brine solution (15 mL), dried over MgSO₄ and evaporated to give the crude product. The crude product was purified by flash column chromatography using 100-200 silica and 50 % of EtOAc in hexane as an eluent. The desired fractions were evaporated and the racemic compound **I-A-29a** was purified by chiral SFC chromatography with mobile phase: A)COz B)MeOH+NH₃,Gradient:25-50%B in 5 min, hold 50%B till 9 min, 50-25%B at 10 min, hold 25%B till 12 min. Column: DAICEL CHIRALPAK-IG (250 x 4.6 mm, 5 um). Wavelength: 263nm. Flow: 3 mL/min to give **1-32** (18 mg, 0.04 mmol, 3 % yield) and **1-33** (23 mg, 0.06 mmol, 4 % yield) as solids. Stereochemistry is randomly assigned.
**1-32: HPLC:** Rt 8.15 min, 96.3%; Column: X-Select CSH C18 (4.6 x 150) mm, 3.5 µm; Mobile phase: A: 0.1% FA in water: Acetonitrile (95:05), B: ACN; Flow Rate: 1.0 mL/min
**LCMS** : 371.05 (M+H), Rt 2.05 min, Column: X-select CSH C18 (3 x 50) mm, 2.5 µm
**¹H NMR (400 MHz, DMSO-d6)** δ_{H} = 9.07 (d, 1H), 7.84-7.75 (m, 3H), 7.52 (d, 1H), 7.25 - 7.10 (m, 3H), 5.16-5.08 (m, 1H), 2.85 - 2.68 (m, 2H), 2.00-1.92 (m, 2H), 1.86 - 1.70 (m, 2H).
**Chiral method:** Rt 9.36 min, 100%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 270 nm, Flow Rate: 3 mL/min.
**1-33: HPLC:** Rt 8.27 min, 99.6%; Column: X-select CSH C18 (4.6 x 150) mm, 5 µm; Mobile phase: A: 0.1% FA in water: Acetonitrile (95:05), B: ACN; Flow Rate: 1.0 mL/min
**LCMS** : 371.05 (M+H), Rt 2.05 min, Column: X-select CSH C18 (3 x 50) mm, 2.5 µm
**¹H NMR (400 MHz, DMSO-d6):** δ_{H} = 9.07 (d, 1H), 7.86 - 7.70 (m, 2H), 7.54-7.50 (m, 1H), 7.25 - 7.15 (m, 3H), 5.16-5.09 (m, 1H), 2.84-2.65 (m, 2H), 2.00-1.90 (m, 2H), 1.86 - 1.68 (m, 2H).
**Chiral method:** Rt 10.64 min, 99.5 %; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50 % B till 9 min, 50-35% B at 10 min, hold 35% B till 12 min. Wavelength: 270 nm, Flow Rate: 3 mL/min.

### Example 29. Synthesis of Compound 1-34

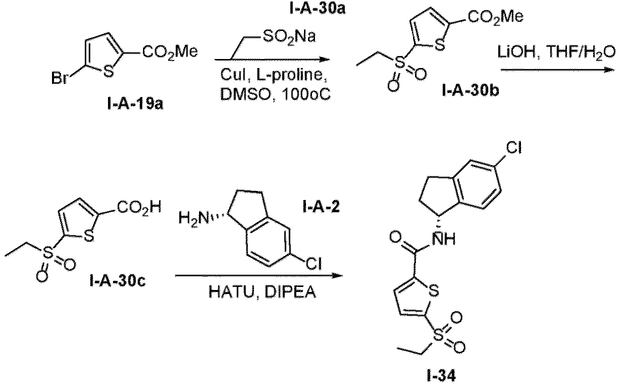

**Synthesis of I-A-30b:** To a stirred solution of **I-A-19a** (1 g, 4.52 mmol) in DMSO (10 mL) was added **I-A-30a** (630.26 mg, 5.43 mmol), copper iodide (85.95 mg, 0.45 mmol), sodium hydroxide (36.19 mg, 0.90 mmol) and L-proline (104.16 mg, 0.90 mmol) at RT and stirred at 95 °C for 16 h. The reaction mixture was diluted with water (100 mL) and extracted with EtOAc (5 x 25 mL). The combined organic layer was dried over Na₂SO₄ and evaporated to obtain crude compound. The crude was purified by column chromatography in 100-200 silica at 8-10 % EtOAc/Hexane eluent to give **I-A-30b** (300 mg, 1.21 mmol, 26 % yield) as a solid.

**Synthesis of I-A-30c:** To a stirred solution of **I-A-30b** (300 mg, 1.28 mmol) in THF (5 mL) was added lithium hydroxide (46 mg, 1.92 mmol) in water (1 mL) at 0 °C and stirred at RT for 2 h. The reaction mixture was concentrated to give the crude product. The crude product was diluted with cold water (10 mL), acidified with 2 N HCl aqueous solution up to pH 4 and extracted with DCM (3 x 15 mL). The Combined organic layer was separated and dried over Na₂SO₄ to give **I-A-30c** (220 mg, 0.60 mmol, 46 % yield) as a solid.

**Synthesis of 1-34:** To a stirred solution of **I-A-30c** (100 mg, 0.45 mmol) and **I-A-2** (76.11 mg, 0.45 mmol) in DCM (10 mL) were added HATU (207.14 mg, 0.54 mmol) followed by DIPEA (0.16 mL, 0.91 mmol) at 0 °C and stirred at RT for 3 h. The reaction mixture was diluted with water (100 mL) and extracted with EtOAc (5 x 25 mL). The combined organic layer was separated, dried over Na₂SO₄ and evaporated to give crude product. The crude product was purified by column chromatography using 100-200 mesh silica, and 20-22 % of EtOAc in hexane as an eluent to give **I-34** (130 mg, 0.34 mmol, 77 % yield) as a solid.
**HPLC:** Rt 9.01 min, 99.5 %; Column: X-select CSH C18 (4.6 x 150) mm, 5 µm; Mobile phase: A: 10 mM ammonium bicarbonate in water, B: ACN; Flow Rate: 1.0 mL/min
**LCMS:** 369.80 (M+H), Rt 1.97 min, Column: X-select CSH C18 (3 x 50) mm, 2.5 µm
**¹H NMR (400 MHz, DMSO-d6):** δ_{H} = 9.17 (d, 1H), 7.91 (d, 1H), 7.78 (d, 1H), 7.36 (s, 1H), 7.25 (s, 2H), 5.52-5.41 (m, 1H), 3.49 - 3.35 (m, 2H), 3.29 (d, 1H), 2.98-2.82 (m, 1H), 2.5-2.45 (m, 1H), 2.02-1.96 (m, 1H), 1.18 (t, 3H).

**Chiral method:** Rt 6.00 min, 98.9%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 261 nm, Flow Rate: 3 mL/min.

### Example 30. Synthesis of Compound I-35

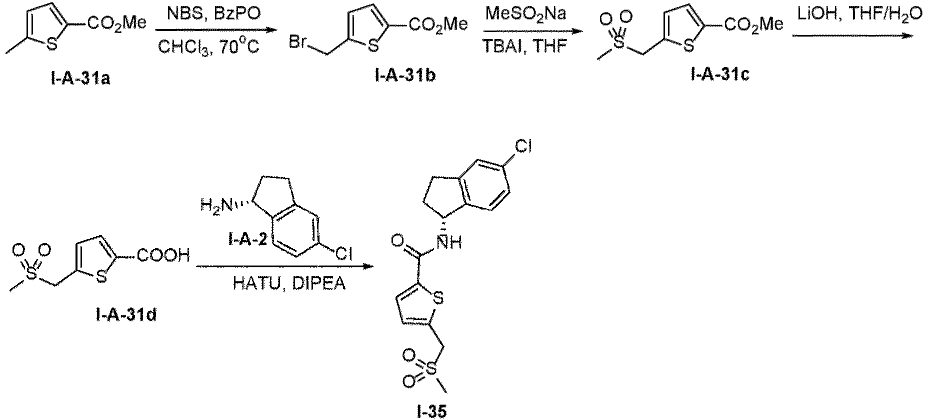

**Synthesis of I-A-31b:** To a stirred solution of **I-A-31a** (1 g, 6.4 mmol) in chloroform (10 mL) was added NBS (1.25 g, 7.04 mmol) and benzyl peroxide (0.16 g, 0.64 mmol) at RT and stirred at 70 °C for 7 h. The reaction mixture was then cooled to RT and diluted with DCM (20 mL), washed with water (2 x 20 mL) and brine solution (20 mL). The organic layer was dried over Na₂SO₄ and evaporated to give the crude product. The crude product was purified by column chromatography using 100-200 silica and 1-2 % EtOAc/Hexane eluent to give **I-A-31b** (0.50 g, 1.94 mmol, 30 % yield) as a solid.

**Synthesis of I-A-31c:** To a stirred solution of **I-A-31b** (300 mg, 1.28 mmol) in DMF (10 mL) was added sodium methanesulfinate (156.33 mg, 1.53 mmol) and tetrabutylammonium iodide (94.27 mg, 0.26 mmol) at RT and stirred for 16 h. After completion reaction was quenched using ice cold water and extracted with ethyl acetate (10 mL x 2). The combined organic layer was washed with ice cold water (20 mL x 2) and dried over Na₂SO₄ and evaporated to give crude product. The crude product was purified by column chromatography using 100-200 silica and 4-5 % EtOAc/Hexane eluent to give **I-A-31c** (250 mg, 1.06 mmol, 83 % yield) as a solid.

**Synthesis of I-A-31d:** To a stirred solution of **I-A-31c** (300 mg, 1.28 mmol) in THF (9 mL) was added LiOH.H₂O (161.18 mg, 3.84 mmol) in water (1 mL) at RT and stirred at RT for 16 h. The reaction mixture was diluted with water (1 mL) and washed with ether (2 x 5 mL) and separated. Aqueous layer was cooled to 0-5 °C and acidified using 6M HCl then stirred at RT for 30 min leading to precipitation. The solid obtained was filtered and dried to give **I-A-31d** (250 mg, 1.06 mmol, 82 % yield) as a solid.

**Synthesis of 1-35:** To a stirred solution of **I-A-31d** (100 mg, 0.45 mmol) in DCM (2 mL) were added **I-A-2** (91.33 mg, 0.54 mmol), HATU (258.93 mg, 0.68 mmol) and DIPEA (0.16 mL, 0.91 mmol) at RT and stirred at RT for 2 h. The reaction mixture was diluted with DCM and washed with water (10 mL x 2). The organic layer was separated, dried by Na₂SO₄ and evaporated to obtain the crude product. The crude product was purified by column chromatography using 100-200 silica and 25 % EtOAc/Hexane eluent to give **I-35** (55 mg, 0.14 mmol, 31 % yield) as a solid.
**HPLC:** Rt 8.07 min, 99.7 %; Column: X-select CSH C18 (4.6 x 150) mm, 5 µm; Mobile phase: A: 0.1% FA in water: ACN (95:05), B: ACN; Flow Rate: 1.0 mL/min
**LCMS:** 370.05 (M+H), Rt 1.87 min, Column: X-select CSH C18 (3.0 x 50) mm, 2.5 µm
**¹H NMR (400 MHz, DMSO-d6)** δ_{H} = 8.86 (d, 1H), 7.74 (dd, 1H), 7.35 (d, 1H), 7.28 - 7.17 (m, 2H), 7.15 (dd, 1H), 5.48-5.42 (m, 1H), 4.80 (s, 2H), 3.05 - 2.94 (m, 3H), 2.89-2.82 (m, 1H), 2.69 (d, 1H), 2.51 - 2.46 (m, 1H), 2.01-1.94 (m, 1H).
**Chiral method: Rt** 5.19 min, 97.3%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 272 nm, Flow Rate: 3 mL/min.

### Example 31. Synthesis of Compound 1-36

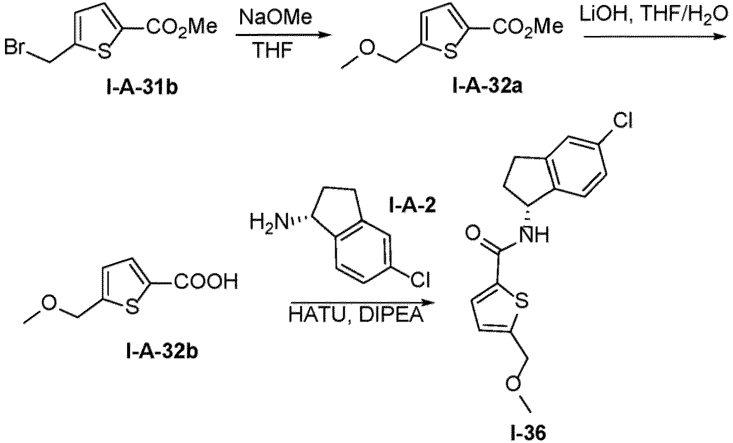

**Synthesis of I-A-32a:** To a stirred solution of **I-A-31b** (400 mg, 1.7 mmol) in methanol (5 mL) was added NaOMe (183.82 mg, 3.4 mmol) at RT and stirred for 16 h. The reaction mixture was diluted with ethyl acetate (20 mL) and washed with water (2 x 10 mL) and brine (10 mL). The organic layer was dried over Na₂SO₄ and evaporated to obtain the crude product. The crude product was purified by column chromatography using 100-200 silica and 2-3 % EtOAc/Hexane eluent to give **I-A-32a** (120 mg, 0.59 mmol, 35 % yield) as a liquid.

**Synthesis of I-A-32b:** To a stirred solution of **I-A-32a** (120 mg, 0.64 mmol) in THF (5 mL) was added LiOH.HzO (81.11 mg, 1.93 mmol) in water (0.50 mL) at RT and stirred for 16 h. The reaction mixture was evaporated and diluted with water (0.5 mL). The aqueous layer was cooled to 0-5 °C and acidified with 6M HCl and stirred at RT for 30 min. Solid was filtered and dried to give **I-A-32b** (65 mg, 0.36 mmol, 57 % yield).

**Synthesis of I-36:** To a stirred solution of **I-A-32b** (60 mg, 0.35 mmol) in DCM (2 mL) were added **I-A-2** (70.09 mg, 0.42 mmol) HATU (198.73 mg, 0.52 mmol) and DIPEA (0.12 mL, 0.70 mmol) at RT and stirred at RT for 2 h. The reaction mixture was diluted with DCM and was washed with water (10 mL x 2). The organic layer was separated, dried using Na₂SO₄ and evaporated to obtain crude product. The crude product was purified by column chromatography using 100-200 silica and 25 % EtOAc/Hexane eluent to give **I-36** (30 mg, 0.09 mmol, 25 % yield) as a solid.
**HPLC:** Rt 8.85 min, 96.0%; Column: X-select CSH C18 (4.6 x 150) mm, 5 µm; Mobile phase: A: 0.1% FA in water: ACN (95:05), B: ACN; Flow Rate: 1.0 mL/min
**LCMS:** 321.9 (M+H), Rt 1.94 min, Column: X-select CSH C18 (3.0 x 50) mm, 2.5 µm
**¹H NMR (400 MHz, DMSO-d6)** δ_{H} = 8.79 (d, 1H), 7.70 (d, 1H), 7.36 (s, 1H), 7.27-7.22 (m, 2H), 7.06 (d, 1H), 5.51-5.41 (m, 1H), 4.59 (s, 2H), 3.31 (d, 3H), 3.04-2.98 (m, 1H), 2.91-2.82 (m, 1H), 2.49-2.43 (m, 1H), 2.05-1.95 (m, 1H).
**Chiral method:** Rt 7.03 min, 96.1%; SFC column: DIACEL CHIRALPAK-IG (250 x 4.6 mm, 5 um), - Mobile Phase: A) CO₂ B) MeOH+0.1% NH₃, Gradient: 10-40% B in 5 min, hold 40% B till 9 min, 40-10% B, at 10 min, hold 10 % B till 12 min. Wavelength: 264 nm, Flow Rate: 3 mL/min.

### Example 32. Synthesis of Compound 1-37

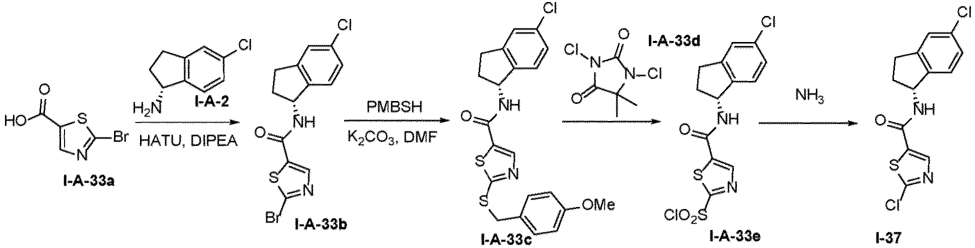

**Synthesis of I-A-33b:** To cooled solution of **I-A-33a** (500 mg, 2.4 mmol) in DCM (10 mL) was added DIPEA (0.84 mL, 4.81 mmol), HATU (1.37 g, 3.61 mmol) and (1R)-5-chloroindan-1-amine (443.21 mg, 2.64 mmol) and the reaction was stirred at RT for 3 h. The reaction was diluted with water and extracted with DCM (10 mL x 3 times) and combined organic layer was dried over sodium sulphate and evaporated to give crude product which was purified by column chromatography using silica gel (100-200) and MeOH:DCM (1:99) as an eluent to give **I-A-33b** (380 mg, 1.06 mmol, 44% yield) as a solid.

**Synthesis of I-A-33c:** To a solution of **I-A-33b** (380.mg, 1.06 mmol) in DMF (5 mL) was added K₂CO₃ (440.45 mg, 3.19 mmol) and (4-methoxyphenyl)methanethiol (163.87 mg, 1.06 mmol) at RT and stirred at RT for 16 h. The reaction mixture was diluted with water and extracted with ethyl acetate (10 mL x 3), combined organic layer was washed with water and brine. The organic layer was dried over sodium sulphate and evaporated to give the crude product which was purified by combi-flash chromatography using ethyl acetate:hexane as an eluent to give **I-A-33c** as (210 mg, 0.45 mmol, 43% yield) as a solid.

**Synthesis of I-A-33e:** To a cooled solution of **I-A-33c** (210.mg, 0.4900 mmol) in MeCN (2 mL), water (0.5 mL) and acetic acid (0.5 mL) was added 1,3-dichloro-5,5-dimethylhydantoin (191.99 mg, 0.97 mmol) at 0 ° C and stirred at 0 ° C for 2 h. Then reaction mixture was stirred at room temp for 2 h. The reaction mixture was quenched with water and extracted with DCM (10 mL x 3). The combined organic layer was dried over sodium sulphate, evaporated to give the crude product which was purified on combi-flash chromatography using ethyl acetate: hexane as an eluent to give **I-A-33e** (150 mg, 0.39 mmol, 81 % yield) as a solid.

**Synthesis of I-37:** To a solution of **I-A-33e** (200 mg, 0.530 mmol) in DCM (10 mL), was added saturated ammonia in DCM at 0 °C and then reaction was stirred at the same temp for 30 min and then reaction was stirred for 2 h. The reaction mixture was diluted with water and extracted with DCM (10 mL x 3), combined organic layer were dried over sodium sulphate and evaporated to give crude product which was purified by combi-flash chromatography to give **I-37** (65.1 mg, 0.21 mmol, 39% yield) as a solid.
**HPLC:** Rt 9.26 min, 99.3%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 312.95 (M+H), Rt 2.08 min, Column: X-select CSH C18 (3 x 50) mm, 2.5 µm
**¹H NMR (400 MHz, DMSO-d6)** δ_{H} = 9.12 (d, 1H), 8.28 (s, 1H), 7.36 (s, 1H), 7.28-7.24 (m, 2H), 5.43 (q, 1H), 3.03-2.96 (m, 1H), 2.90-2.82 (m, 1H), 2.52-2.41 (m, 1H), 2.02-1.92 (m, 1H).
**Chiral method:** Rt 6.90 min, 98.0%; SFC column: DIACEL CHIRALPAK-IG (250 x 4.6 mm, 5 um), - Mobile Phase: A) CO₂ B) MeOH+0.1% NH₃, Gradient: 10-40% B in 5 min, hold 40% B till 9 min, 40-10% B in 10 min, hold 10% B till 12 min. Wavelength: 263 nm, Flow Rate: 3 mL/min.

### Example 33. Synthesis of Compound I-38

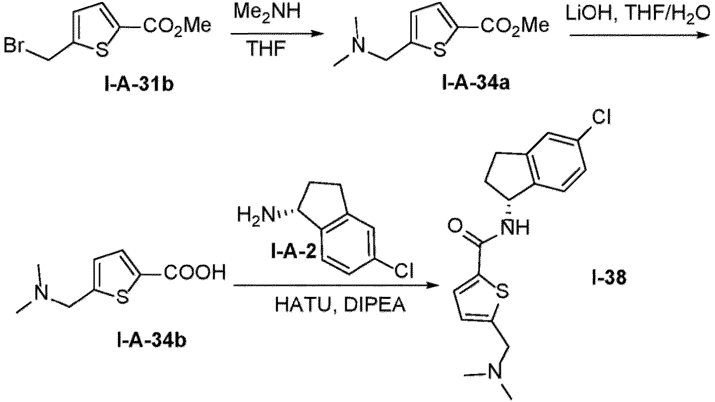

**Synthesis of I-A-34a:** To a stirred solution of **I-A-31b** (0.5 g, 2.13 mmol) and dimethylamine (0.16 mL, 3.19 mmol) in DMF (10 mL) was added K₂CO₃ (587.75 mg, 4.25 mmol) at RT and stirred for 2 hr. The reaction was quenched with water (100 mL) and extracted with EtOAc (100 mL x 2). The combined organic layer was separated, dried over Na₂SO₄, filtered and evaporated under reduced pressure to give crude product which was purified by column chromatography using 100-200 silica and 30-80% EtOAc/Hexane as an eluent to give **I-A-34a** (0.3 g, 1.2 mmol, 57 % yield) as a solid.

**Synthesis of I-A-34b:** To a stirred solution of **I-A-34a** (0.3g, 1.5 mmol) in THF:water (10:5mL) was added LIOH.H₂O (63.17 mg, 1.51 mmol) at RT and stirred for 2 hr. Thereaction was quenched with water (100 mL) and extracted with EtOAc (50 mL x 2). The organic layer was separated, and remaining aqueous layer was acidified with 1N HCl leading to precipitation. The precipitated solid was filtered and dried to give **I-A-34b** (0.1 g, 0.48 mmol, 32 % yield).

**Synthesis of I-38:** To a stirred solution of **I-A-34b** (0.2g, 1.08 mmol) and (1R)-5-chloroindan-1-amine (217.2 mg, 1.3 mmol) in DCM(10 mL) were added HATU (615.79 mg, 1.62 mmol) and DIPEA (0.38 mL, 2.16 mmol) at RT and stirred for 2 hr at RT. The reaction was quenched with water (100 mL) and extracted with DCM (100 mL x 2). The combined organic layer was separated, dried by Na₂SO₄, filtered and evaporated under reduced pressure to give crude product. The crude product was purified by column chromatography using 100-200 silica and 30-80% EtOAc/Hexane as an eluent to give **I-38** (20 mg, 0.06 mmol, 5% yield).
**HPLC:** Rt 5.79 min, 99.7%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 334.95 (M+H), Rt 1.46 min, Column: X-select CSH C18 (3 x 50) mm, 2.5 µm
**¹H NMR (400 MHz, DMSO-d6)** δ_{H} = 8.70 (d, 1H), 7.63 (d, 1H), 7.33 (s, 1H), 7.27-7.17 (m, 2H), 6.94 (d, 1H), 5.43 (q, 1H), 3.58 (s, 2H), 3.00-2.94 (m, 1H), 2.88-2.78 (m, 1H), 2.46-2.40 (m,1H), 2.17 (s, 6H), 2.02-1.92 (m, 1H).
**Chiral method:** Rt 5.98 min, 100%; SFC column: DIACEL CHIRALPAK-IG (250 x 4.6 mm, 5 um), - Mobile Phase: A) CO₂ B) MeOH+0.1% NH₃, Gradient: 10-40% B in 5 min, hold 40% B till 9 min, 40-10% B in 10 min, hold 10% B till 12 min. Wavelength: 280 nm, Flow Rate: 3 mL/min.

### Example 34. Synthesis of Compound I-39

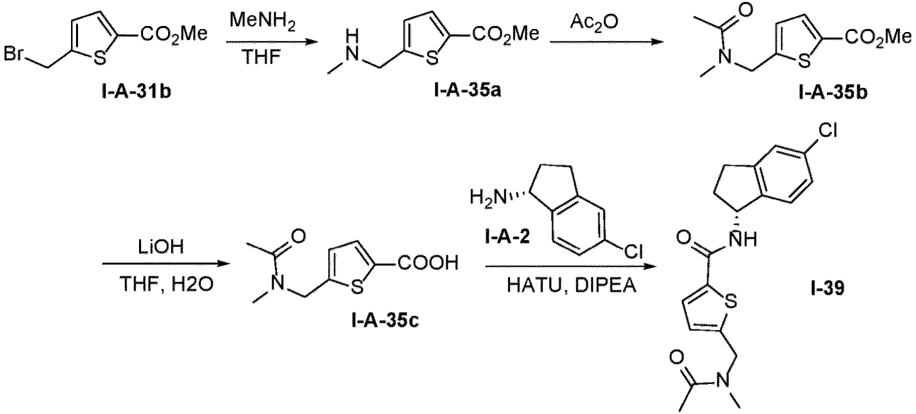

**Synthesis of I-A-35a:** To a stirred solution of **I-A-31b** (0.5 g, 2.13 mmol) in DMF (10 mL), 1M methanamine in THF (132.11 mg, 4.25 mmol) and K₂CO₃ (587.75 mg, 4.25 mmol) was added at RT and stirred for 2 hr. The reaction mixture was quenched with water (100 mL) and extracted with EtOAc (100 mL x 2). The organic layer was separated, dried over Na₂SO₄, filtered and evaporated under reduced pressure. The crude product was purified by column chromatography using 100-200 silica and 30-80% EtOAc/Hexane eluent to give **I-A-35a** (0.2 g, 0.86 mmol, 40 % yield)

**Synthesis of I-A-35b:** To a stirred solution of **I-A-35a** (0.2 g, 1.08 mmol) in acetic anhydride (10 mL) was added sodium acetate (177.13 mg, 2.16 mmol) at RT and stirred for 12 hr at 50 °C. The reaction was quenched with water (100 mL) diluted with EtOAc (50 mL x 2). The organic layer was separated, dried over sodium sulphate than evaporated. The crude material was purified by column chromatography using 100-200 silica and 30-50% EtOAc/Hexane as an eluent to give **I-A-35b** (0.15 g, 0.35 mmol, 33% yield) as a solid.

**Synthesis of I-A-35c:** To a stirred solution of **I-A-35b** (0.3 g, 1.51 mmol) in THF:water (10:5 mL) was added LiOH.H₂O (63.17 mg, 1.51 mmol) at RT and stirred for2 hr. The reaction mixture was quenched with water (100 mL) andEtOAc (50 mL x 2) added. The organic layer was separated and the water layer acidified with 1N HCl leading to precipitation. The precipitate thus formed was then filtered. The solid was separated, dried on high vacuum to give **I-A-35c** (0.1 g, 0.48 mmol, 32 % yield).

**Synthesis of I-39:** To a stirred solution of **I-A-35c** (0.1 g, 0.48 mmol) and (1R)-5-chloroindan-1-amine (96.13 mg, 0.57 mmol) in DCM (10 mL) were added HATU (272.54 mg, 0.72 mmol) and DIPEA (0.17 mL, 0.96 mmol) at RT and stirred for 2 hr. The reaction was quenched with water (100 mL) andDCM (100 mL x 2). The organic layer was separated, dried with Na₂SO₄, then filtered. The organic layer was evaporated, and the crude product was purified by column chromatography using 100-200 silica and 30-80% EtOAc/Hexane as eluent to give **I-39** as a solid (75 mg, 0.2 mmol, 43 % yield).
**HPLC:** Rt 8.14 min, 99.5%; Column: X-Select CSH C18 (4.6 x 150) mm, 3.5 µm; Mobile phase: A: 0.1% FA in water: ACN (95:05), B: ACN; Flow Rate: 1.0 mL/min
**LCMS** : 362.95 (M+H), Rt 1.87 min, Column: X-select CSH C18 (3 x 50) mm, 2.5 µm
**¹H NMR (400 MHz, DMSO-d6) (VT at 80 °C):** δ_{H} = 8.48 (d, 1H), 7.63 (s, 1H), 7.30 (s, 1H), 7.29 - 7.17 (m, 2H), 6.98 (d, 1H), 5.44 (q, 1H), 4.80-4.60 (m, 2H), 3.08-2.76 (m, 5H), 2.49-2.44 (m,1H), 2.05-2.00 (m, 4H).
**Chiral method:** Rt 5.08 min, 99.7%; SFC column: DIACEL CHIRALPAK-IG (250 x 4.6 mm, 5 um), - Mobile Phase: A) CO₂ B) MeOH+0.1% NH₃, Gradient: 10-40% B in 5 min, hold 40% B till 9 min, 40-10% B in 10 min, hold 10% B till 12 min. Wavelength: 265 nm, Flow Rate: 3 mL/min.

### Example 35. Synthesis of Compound I-40

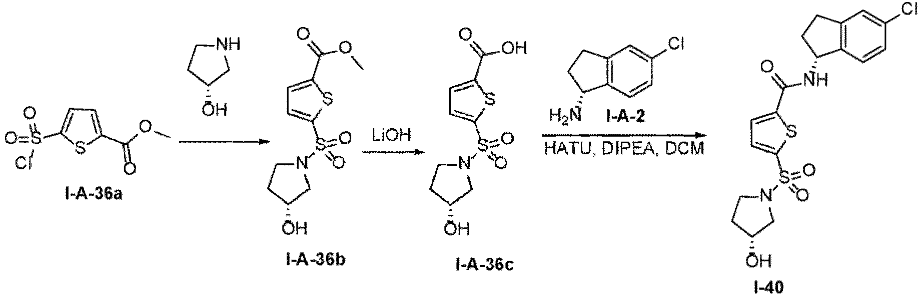

**Synthesis of I-A-36b:** To a stirred solution of **I-A-36a** (0.5 g, 2.08 mmol) in DCM (5 mL) was added DIPEA 1.08 g, 6.23 mmol) and R-(+)-3-pyrrolidinol (0.34 mL, 4.15 mmol) at RT and stirred for 1 h. The reaction mixture was diluted with water, extracted with DCM. The organic layer was dried over Na₂SO₄ filtered and evaporated to give I-A-36b (250 mg, 0.86 mmol, 41 % yield) as a liquid.

**Synthesis of I-A-36c:** To a stirred solution of **I-A-36b** (0.4 g, 1.32 mmol) in THF:water (1:1, 3 mL) were added NaOH (158 mg, 3.96 mmol) at RT and stirred at RT for 2 h. The reaction mixture was then evaporated, diluted with water (10 mL) and extracted with ethyl acetate (15 mL). The aqueous layer thus separated was acidified with 3 N HCl, leading to precipitation. The precipitate was filtered, washed with water and dried to give **I-A-36c** (220 mg, 0.64 mmol, 49 % yield) as a solid.

**Synthesis of I-40:** To a stirred solution of **I-A-36c** (200 mg, 0.72 mmol) and **I-A-2** (145 mg, 0.87 mmol) in DCM (5 mL) were added DIPEA (0.38 mL, 2.16 mmol)) and HATU (411.3 mg, 1.08 mmol) at RT and stirred for 16 h. The reaction mixture was quenched with water (10 mL) and diluted with EtOAc (20 mL). The organic layer was washed with brine solution (15 mL), dried over (Na₂SO₄) and evaporated to give the crude product. The crude product was purified by prep HPLC to give **I-40** (40 mg, 0.09 mmol, 13 % yield) as a solid.
**HPLC:** Rt 8.30 min, 96.6%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 427.15 (M+H), Rt 1.89 min, Column: X-select CSH C18 (3 x 50) mm, 2.5 µm
**¹H NMR (400 MHz, DMSO-d6)** δ_{H} = 9.12 (d, 1H), 7.89 (d, 1H), 7.66 (d, 1H), 7.36 (s, 1H), 7.26-7.24 (m, 2H), 5.46 (q, 1H), 4.20-4.18 (m, 1H), 3.38- 3.22 (m, 4H), 3.12-2.95 (m, 2H), 2.90-2.84 (m, 1H), 2.49-2.46 (m, 1H), 2.02-1.96 (m, 1H), 1.86- 1.63 (m, 2H).
**Chiral method:** Rt 5.80 min, 99.5%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 273 nm, Flow Rate: 3 mL/min.

### Example 36. Synthesis of Compound I-41

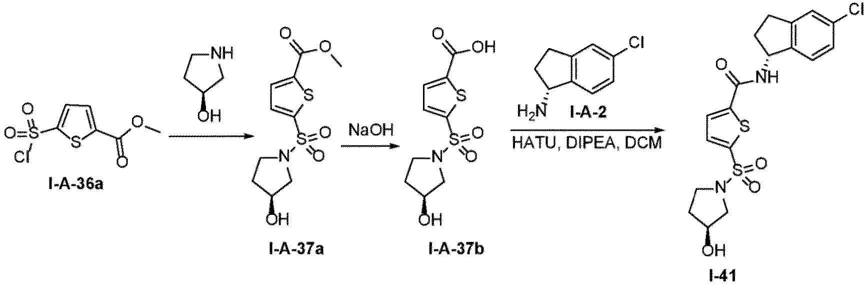

**Synthesis of I-A-37a:** To a stirred solution of **I-A-36a** (0.5 g, 2.08 mmol) in DCM (10 mL) was added DIPEA 1.08 g, 6.23 mmol) and S-(+)-3-pyrrolidinol (0.34 mL, 4.15 mmol) at RT and stirred for 1 h. The reaction mixture was diluted with water, extracted with DCM. The organic layer was dried over Na₂SO₄ filtered and evaporated to give **I-A-37a** (300 mg, 1.03 mmol, 49 % yield) as a liquid.

**Synthesis of I-A-37b:** To a stirred solution of **I-A-37a** (0.3 g, 1.05 mmol) in THF:water (1:1, 6 mL) was added NaOH (126 mg, 3.15 mmol) at RT and stirred for 2 h. The reaction mixture was then evaporated, diluted with water (10 mL) and extracted with ethyl acetate (15 mL). The aqueous layer thus separated was acidified with 3 N HCl leading to precipitation. The precipitate was filtered, washed with water and dried to give **I-A-37b** (200 mg, 0.52 mmol, 49 % yield) as a solid.

**Synthesis of I-41:** To a stirred solution of **I-A-37b** (200 mg, 0.72 mmol) and **I-A-2** (181.3 mg, 1.08 mmol) in DCM (5 mL) was added DIPEA (0.38 mL, 2.16 mmol)) and HATU (411.3 mg, 1.08 mmol) at RT and stirred for 16 h. The reaction mixture was quenched with water (10 mL) and diluted with EtOAc (20 mL). The organic layer was washed with brine solution (15 mL), dried over (Na₂SO₄) and evaporated to give the crude product. The crude product was purified by prep HPLC to give **I-41** (32 mg, 0.07 mmol, 10 % yield) as a solid.
**HPLC:** Rt 8.29 min, 98.7%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 427 (M+H), Rt 1.89 min, Column: X-select CSH C18 (3 x 50) mm, 2.5 µm
**¹H NMR (400 MHz, DMSO-d6)** δ_{H} = 9.12 (d, 1H), 7.89 (d, 1H), 7.66 (d, 1H), 7.36 (s, 1H), 7.26-7.18 (m, 2H), 5.46 (q, 1H), 4.96-4.94 (m, 1H), 4.20-4.18 (m, 1H), 3.38-3.24 (m, 3H), 3.10-2.96 (m, 2H), 2.88-2.84 (m, 1H), 2.49-2.46 (m, 1H), 2.10-1.92 (m, 1H), 1.86-1.68 (m, 2H).
**Chiral method:** Rt 5.86 min, 99.6%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 273 nm, Flow Rate: 3 mL/min.

### Example 37. Synthesis of Compound I-42

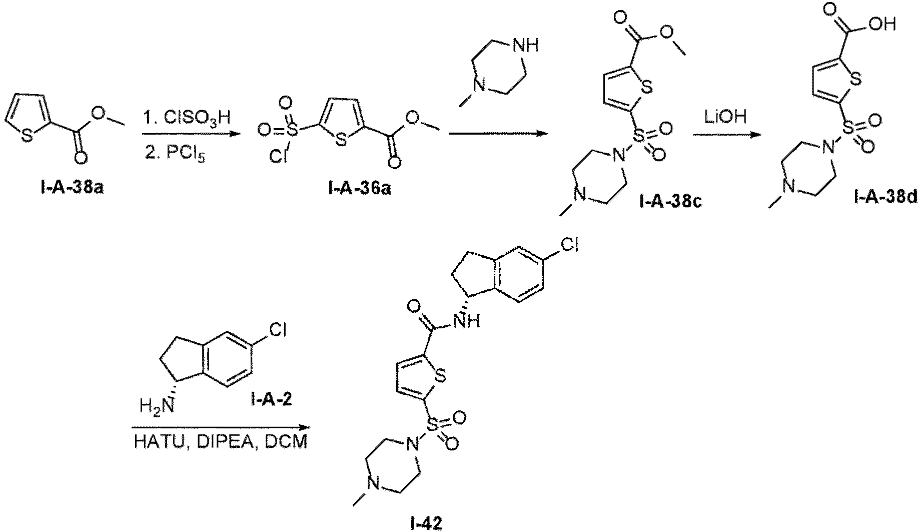

**Synthesis of I-A-36a:** To a stirred solution of **I-A-38a** (10 g, 70.3 mmol) in chloroform (100 mL) was added dropwise chlorosulphonic acid (12.24 g, 105.5 mmol) at -10 °C and the reaction mixture was heated at 70 °C for 16 h. The reaction mixture was cooled to -10 °C and treated with pyridine (18.73 mL, 232.1 mmol) in dropwise addition manner, followed by the addition of phosphorus pentachloride (17.58 g, 84.4 mmol) in portions over 30 mins. The resulting reaction mixture was stirred at RT for 16 h. The reaction mixture was poured on to ice-cold water (300 mL) and stirred for 1h. The reaction was extracted with DCM (3 x 250 mL). The combined organic phase was washed with brine (3 x 200 mL), dried over anhydrous sodium sulphate, filtered and evaporated under reduced pressure. Crude product was purified by column chromatography (100-200 mesh silica, 2-5 % of EtOAc in hexane as an eluent) to givet **I-A-36a** (12 g, 48.86 mmol, 69 % yield) as an oil.

**Synthesis of I-A-38c:** To a stirred solution of **I-A-36a** (0.5 g, 2.08 mmol) in DCM (5 mL), DIPEA (0.72 mL, 4.15 mmol) and 1-methylpiperazine (0.28 mL, 2.49 mmol) were added and the reaction mixture was stirred at RT for 16 h. The reaction mixture was diluted with water and extracted with DCM (10 mL x 3). The combined organic layer was dried over sodium sulphate and evaporated to give the crude product, which was purified by combi-flash chromatography using ethyl acetate: hexane as an eluent to give **I-A-38c** (300 mg, 0.88 mmol, 42 % yield) as an oil.

**Synthesis of I-A-38d:** To a stirred solution of **I-A-38c** (300 mg, 0.99 mmol) in THF (5 mL) was added a solution of LiOH.H₂O (82.71 mg, 1.97 mmol) in water (1 mL) at 0 ° C and stirred at room temp for 2 h. The reaction mixture was evaporated and residue was diluted with water and acidified with 2N HCl. Then aqueous layer was evaporated to give **I-A-38d** (180 mg, 0.57 mmol, 58% yield) as a solid which was used directly for next step.

**Synthesis of I-42:** To a stirred solution of **I-A-38d** (100 mg, 0.6 mmol) and **I-A-2** (207.8 mg, 0.72 mmol) in DCM (10 mL) was added DIPEA (0.21 mL, 1.19 mmol) and HATU (272.8 mg, 0.72 mmol) at 0 °C and stirred at RT for 3 h. The reaction mixture was quenched with water (10 mL) and diluted with DCM (20 mL). The separated aqueous layer was washed with DCM (20 mL). The combined organic layer was washed with brine solution, dried over Na₂SO₄ and evaporated to give the crude product. The crude product was purified by column chromatography using 30% EA in Hexane as an eluent to give **I-42** (10 mg, 0.02 mmol, 3.6 % yield) as a solid.
**HPLC:** Rt 6.27 min, 95.9%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 440 (M+H), Rt 1.43 min, Column: X-select CSH C18 (3 x 50) mm, 2.5 µm
**¹H NMR (400 MHz, DMSO-d6)** δ_{H} = 9.16 (d, 1H), 7.92 (d, 1H), 7.64 (d, 1H), 7.36 (s, 1H), 7.26-7.24 (m, 2H), 5.46 (q, 1H), 3.07-2.80 (m, 6H), 2.50-2.36 (m, 5H), 2.16 (s, 3H), 2.05-1.95 (m, 1H).
**Chiral method:** Rt 7.87 min, 97.5 %; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 272 nm, Flow Rate: 3 mL/min.

### Example 38. Synthesis of Compound I-43

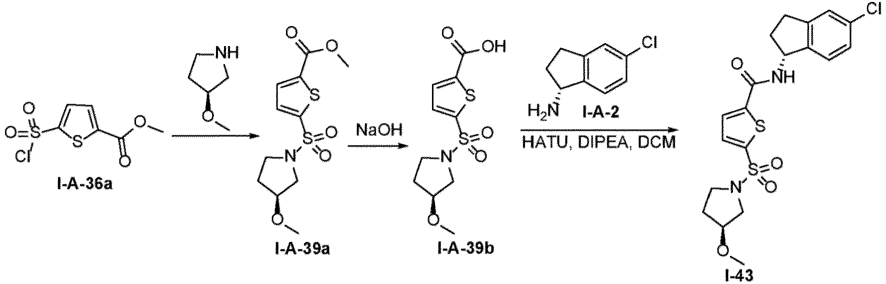

**Synthesis of I-A-39a:** To a stirred solution of **I-A-36a** (0.5 g, 2.08 mmol) in DCM (5 mL) was added DIPEA (0.72 mL, 4.15 mmol) and (3S)-3-methoxypyrrolidine (0.25 g, 2.49 mmol) at 0 °C and stirred at RT for 10 h. The reaction mixture was diluted with water, extracted with DCM (10 mL x 3). The combined organic layer was dried over Na₂SO₄, filtered and evaporated to give crude product which was purified by combi-flash chromatography using ethyl acetate: hexane as an eluent to give **I-A-39a** (300 mg, 1..88 mmol, 42 % yield).

**Synthesis of I-A39b:** To a stirred solution of **I-A-39a** (0.3 g, 0.98 mmol) in THF (3 mL) were added LiOH.H2O (82.44 mg, 1.96 mmol) in water (1 mL) at 0 ° C and stirred at RT for 3 h. The reaction mixture was then evaporated to give a residue, which was dissolved in water and acidified with 2N HCl. The solid obtained was filtered and dried to give **I-A-39b** (250 mg, 0.7870 mmol, 80% yield) as a solid which was used for the next step.

**Synthesis of I-43:** To a stirred solution of **I-A-39b** (208 mg, 0.72 mmol) and **I-A-2** (100 mg, 0.6 mmol) in DCM (10 mL) were added DIPEA (0.21 mL, 1.19 mmol) and HATU (272 mg, 0.72 mmol) at 0 °C RT and stirred at RT for 3 h. The reaction mixture was quenched using water (20 mL) and diluted with DCM (20 mL). The organic layer was washed with brine solution, dried over Na₂SO₄ and evaporated to give the crude product. The crude product was purified column chromatography using silica gel 100-200 mesh and 40% EA in Hexane as an eluent to give **I-43** (66 mg, 0.15 mmol, 25 % yield) as a solid.
**HPLC:** Rt 8.95 min, 99.8%; Column: X-Select CSH C18 (4.6 x 150) mm, 3.5 µm; Mobile phase: A: 0.1% FA in water: ACN (95:05), B: ACN; Flow Rate: 1.0 mL/min
**LCMS** : 441.05 (M+H), Rt 2.01 min, Column: X-select CSH C18 (3 x 50) mm, 2.5 µm
**¹H NMR (400 MHz, DMSO-d6)** δ_{H} = 9.12 (d, 1H), 7.89 (d, 1H), 7.69 (d, 1H), 7.36 (s, 1H), 7.26-7.24 (m, 2H), 5.46 (q, 1H), 3.90-3.84 (m, 1H), 3.30-3.16 (m, 4H), 3.07 (s, 3H), 3.04-2.98 (m, 1H), 2.90-2.84 (m, 1H), 2.49-2.44 (m, 1H), 2.04-1.94 (m, 1H), 1.90-1.80 (m, 2H).
**Chiral method:** Rt 5.82 min, 98.6%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 273 nm, Flow Rate: 3 mL/min.

### Example 39. Synthesis of Compound I-44

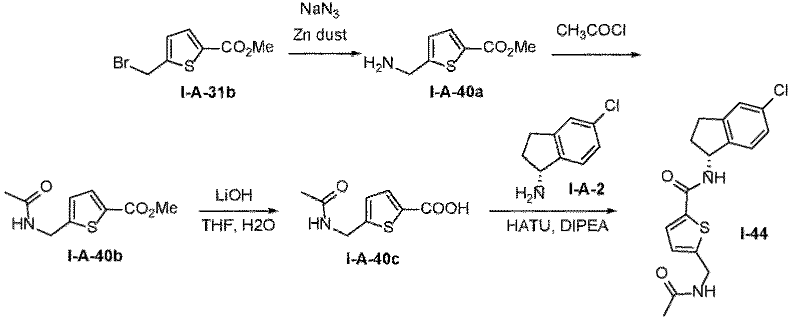

**Synthesis of I-A-40a:** To a stirred solution of **I-A-31b** (1 g, 4.25 mmol) in ACN (10 mL) was added NaN₃ (0.55 g, 8.51 mmol) at RT and reaction mixture was stirred at 90 °C for 1 h. The reaction mixture evaporated, treated with acetic acid (5 mL), Zn dust (556.27 mg, 8.51 mmol) in portion and stirred at RT for 3 h. The reaction mixture was filtered over Celite and diluted with 1*N* HCl and aqueous layer treated with ethyl acetate (30 mL). The aqueous layer was basified with NaHCO₃ and extracted with ethyl acetate. The organic layer was dried over Na₂SO₄ filtered and evaporated to give **I-A-40a** (400 mg, 2.3 mmol, 54% yield) as an oil.

**Synthesis of I-A-40b:** To a stirred solution of **I-A-40a** (0.5 g, 2.41 mmol) in DCM (10 mL) were added acetyl chloride (0.25 mL, 3.5 mmol), DIPEA (0.92 mL, 5.26 mmol) and stirred at RT for 2 h. The reaction mixture was diluted in water and extracted with DCM. The organic layer was dried over Na₂SO₄ filtered and concentrated to give **I-A-40b** (300 mg, 1.4 mmol, 80% yield) as a solid.

**Synthesis of I-A-40c:** To a stirred solution of **I-A-40b** (0.3 g, 1.41 mmol) in THF : water (1:1) was added NaOH (56.3, 1.41 mmol) and stirred at RT for 1 h. The reaction mixture was evaporated and crude product diluted with water and treated with ethyl acetate. The aqueous layer was acidified with 2 N HCl until pH = 2 and was obtained, extracted with ethyl acetate, dried over Na₂SO₄, filtered and evaporated to give **I-A-40c** (160 mg, 0.8 mmol, 57% yield) as a solid.

**Synthesis of I-44:** To a stirred solution of **I-A-2** (100 mg, 0.6 mmol) and **I-A-40c** (119 mg, 0.6 mmol) in DCM (5 mL) was added DIPEA (0.31 mL, 1.79 mmol) and HATU (340.22 mg, 0.89 mmol) at RT and stirred at RT for 16 h. The mixture was diluted in water and extracted with DCM organic layer dried over Na₂SO₄ filtered off and concentrated to give the crude product. The crude product was purified by column chromatography using 5% MeOH/DCM as an eluent to give **I-44** (44 mg, 0.12 mmol, 20.5% yield) as a solid.
**HPLC:** Rt 7.49 min, 97%; Column: X-Select CSH C18 (4.6 x150) mm, 5 µm; Mobile phase: A: 0.1% FA in water: ACN (95:05), B: ACN; Flow Rate: 1.0 mL/min
**LCMS** : 349 (M+H), Rt 1.82 min, Column: X-select CSH C18 (3 x 50) mm, 2.5 µm
**¹H NMR (400 MHz, DMSO-d6)** δ_{H} = 8.72 (d, 1H), 8.51 (t, 1H), 7.62 (d, 1H), 7.34 (s, 1H), 7.26-7.18 (m, 2H), 6.94 (d, 1H), 5.44 (q, 1H), 4.39 (d, 2H), 3.04-2.92 (m, 1H), 2.88-2.82 (m, 1H), 2.46-2.40 (m, 1H), 2.00-1.94 (m, 1H), 1.85 (s, 3H).
**Chiral method:** Rt 6.84 min, 99.3%; SFC column: DIACEL CHIRALPAK-IG (250 x 4.6 mm, 5 um), - Mobile Phase: A) CO₂ B) MeOH+0.1% NH₃, Gradient: 10-40% B in 5 min, hold 40% B till 9 min, 40-10% B in 10 min, hold 20% B till 12 min. Wavelength: 272 nm, Flow Rate: 3 mL/min.

### Example 40. Synthesis of Compound I-45

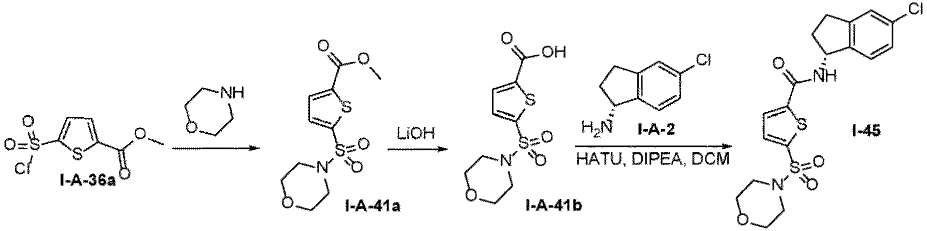

**Synthesis of I-A-41a:** To a stirred solution of **I-A-36a** (0.5 g, 2.08 mmol) in DCM (5 mL), DIPEA (0.72 mL, 4.15 mmol) and morpholine (217.2 mg, 2.49 mmol) were added at 0 °C and the reaction mixture was stirred at RT for 6 h. The reaction mixture was diluted with water (15 mL) and extracted with DCM (20 mL). The combined organic layer was dried over sodium sulphate and evaporated to give the crude product, which was purified by combi-flash chromatography using ethyl acetate : hexane as eluent to give **I-A-41a** (400 mg, 1.15 mmol, 55 % yield).

**Synthesis of I-A-41b:** To a stirred solution of **I-A-41a** (300 mg, 0.99 mmol) in THF: water (1:1, 10 mL) was added a solution of LiOH.HzO (144 mg, 3.43 mmol) at 0 ° C and gradually warmed to RT. The reaction was concentrated to dryness and the residue was taken up in EtOAc and the organic layer washed with 2 x mL water then 1 x mL saturated brine solution. The organics were then separated and dried over MgSO₄ before concentration to dryness. The crude product was then purified by flash column chromatography using EtOAc in Isohexane as an eluent. The desired fractions were concentrated to dryness in vacuo to give **I-A-41b** (320 mg, 67%) as an oil.

**Synthesis of I-45:** To a stirred solution of **I-A-41b** (90 mg, 0.32 mmol) and **I-A-2** (90 mg, 0.32 mmol) in DCM (10 mL) were added DIPEA (0.11 mL, 0.65 mmol) and HATU (148 mg, 0.39 mmol) at 0 ^{O}C and stirred at RT for 3 h. The reaction mixture was quenched using water (10 mL) and diluted with DCM (25 mL). The organic layer was washed with brine solution, dried over Na₂SO₄ and evaporated to give the crude product. The crude product was purified by column chromatography using 35% EA in Hexane as an eluent to give **I-45** (10 mg, 0.023 mmol, 7.13% yield) as a solid.
**HPLC:** Rt 8.93 min, 98.8%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 427.20 (M+H), Rt 2.18 min, Column: X-select CSH C18 (3 x 50) mm, 2.5 µm
**¹H NMR (400 MHz, DMSO-d6)** δ_{H} = 9.16 (d, 1H), 7.94 (d, 1H), 7.66 (d, 1H), 7.36 (s, 1H), 7.26 -7.24 (m, 2H), 5.46 (q, 1H), 3.67 (t, 4H), 3.07 -2.80 (m, 7H), 2.07-1.92 (m, 1H).
**Chiral method:** Rt 9.00 min, 100%; SFC column: DIACEL CHIRALPAK-IG (250 x 4.6 mm, 5 um), - Mobile Phase: A) CO₂ B) MeOH+0.1% NH₃, Gradient: 35-50% B in 5 min, hold 50% B till 9 min, 50-35% B in 10 min, hold 35% B till 12 min. Wavelength: 272 nm, Flow Rate: 3 mL/min.

### Example 41. Synthesis of Compound I-46

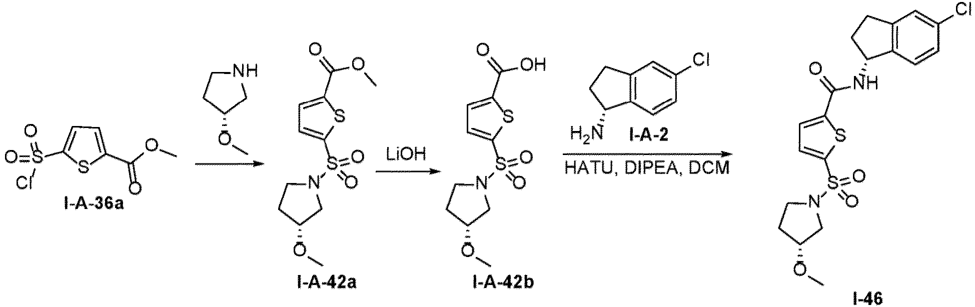

**Synthesis of I-A-42a:** To a stirred solution of **I-A-36a** (0.5 g, 2.25 mmol) in DCM (10 mL) were added DIPEA (0.78 mL, 4.49 mmol) and (3R)-3-methoxypyrrolidine (0.27 g, 2.69 mmol) at 0 °C and stirred at RT for 3 h. The reaction mixture was diluted with water (10 mL), extracted with DCM (10 mL). The organic layer was washed using brine, dried over Na₂SO₄ filtered and evaporated to give **I-A-42a** (500 mg, 1. 64 mmol, 50 % yield) as an oil.

**Synthesis of I-A-42b:** To a stirred solution of **I-A-42a** (0.5 g, 1.64 mmol) in THF: water (1:1, 10 mL) was added lithium hydroxide (78.43 mg, 3.27 mmol) at 0 ° C and stirred at RT for 3 h. The reaction mixture was then evaporated to give a residue, which was dissolved in water (5 mL) and acidified with 5N HCl (4 mL) leading to precipitate which was filtered and dried to give **I-A-42b** (300 mg, 1.0 mmol, 61% yield) as a solid which was used for the next step without further purification.

**Synthesis of I-46:** To a stirred solution of **I-A-42b** (100 mg, 0.34 mmol) and **I-A-2** (69.5 mg, 0.41 mmol) in DCM (5 mL) were added DIPEA (0.12 mL, 0.69 mmol) and HATU (157 mg, 0.41 mmol) at 0 °C and stirred at RT for 3 h. The reaction mixture was quenched using water (10 mL) and diluted with DCM (15 mL x 2). The combined organic layer was washed with brine solution (10 mL), dried over Na₂SO₄ and evaporated to give the crude product. The crude product was purified column chromatography using silica gel 100-200 mesh and 40% EA in Hexane as an eluent to give **I-46** (30.3 mg, 0.69 mmol, 20 % yield) as a solid.
**HPLC:** Rt 8.99 min, 99.9%; Column: X-Select CSH C18 (4.6 x 150) mm, 3.5 µm; Mobile phase: A: 0.1% FA in water: ACN (95:05), B: ACN; Flow Rate: 1.0 mL/min
**LCMS** : 441.09 (M+H), Rt 2.02 min, Column: X-select CSH C18 (3 x 50) mm, 2.5 µm
**¹H NMR (400 MHz, DMSO-d6)** δ_{H} = 9.13 (d, 1H), 7.89 (d, 1H), 7.69 (d, 1H), 7.36 (s, 1H), 7.26-7.24 (m, 2H), 5.46 (q, 1H), 3.90-3.85 (m, 1H), 3.33 - 3.18 (m, 3H), 3.07 (s, 3H), 3.00-2.96 (m, 1H), 2.89-2.84 (m, 1H), 2.49-2.44 (m, 2H), 2.02-1.96 (m, 1H), 1.90 -1.78 (m, 2H).
**Chiral method:** Rt 5.33 min, 100%; column: DIACEL CHIRALPAK-IG (250 x 4.6 mm, 5 um), - Mobile Phase: A) n-Hexane+0.1%Isopropylamine B) DCM:MeOH (1:1), Isocratic: 50% B; Wavelength: 273 nm, Flow Rate: 1.0 mL/min.

### Example 42. Synthesis of Compound I-47

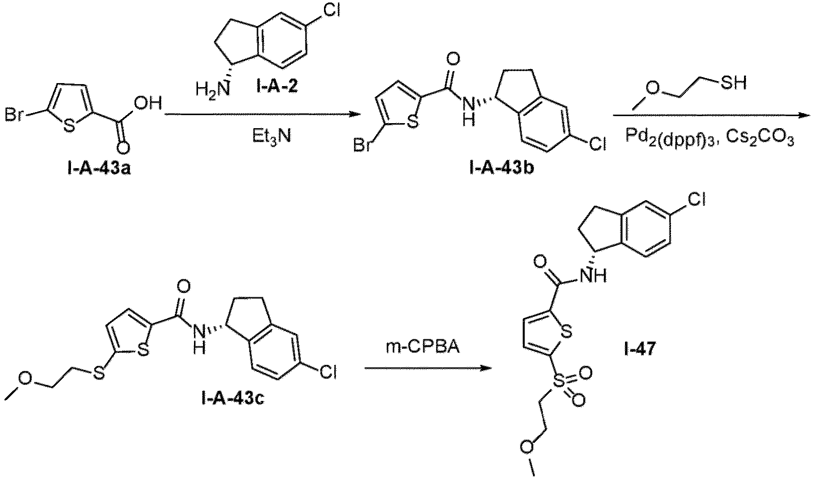

**Synthesis of I-A-43b:** To a stirred solution of **I-A-43a** (0.5 g, 2.41 mmol) in DCM (5 mL) was added TEA (1.01 g, 7.24 mmol) at RT and reaction mixture was stirred at RT for 10 min. The reaction mixture was then treated with (1R)-5-chloroindan-1-amine (404.8 mg, 2.4 mmol) and stirred at RT for 2 h. The reaction mixture was diluted with water, extracted with DCM. The organic layer was dried over Na₂SO₄ filtered and evaporated to give the crude product which was purified using flash column chromatography and 10% EtOAc/hexane as eluent to give **I-A-43b** (310 mg, 0.78 mmol, 33% yield) as a solid.

**Synthesis of I-A-43c:** To a stirred solution of **I-A-43b** (0.3 g, 0.84 mmol) in 1,4-dioxane (4 mL), were added 2-methoxyethanethiol (155.03 mg, 1.68 mmol) and DIPEA (0.44 mL, 2.52 mmol). The reaction mixture was purged with N₂ gas for 20 min and charged with Pd₂(dba)₃ (77.05 mg, 0.08 mmol) and DPPF (46.63 mg, 0.08 mmol) at RT and stirred for 6 h at 100 °C. The reaction mixture was diluted in water (10 mL) and extracted with EtOAc (20 mL). The organic layer was dried over Na₂SO₄ filtered and evaporated to give crude product which was purified by combi-flash and 7% EtOAc /hexane as an eluent to give **I-A-43c** ((210 mg, 0.55 mmol, 66% yield) as a oil.

**Synthesis of I-47:** To a stirred solution of **I-A-2** (150 mg, 0.41 mmol) in DCM (5 mL), mCPBA (211 mg, 1.22 mmol) was added portion wise at RT and stirred at RT for 2 h. The reaction mixture was diluted using water (10 mL) and extracted with DCM (20 mL). The organic layer was washed with NaHCOs (15 mL), dried over Na₂SO₄, filtered and evaporated to give the crude product. The crude product was purified by prep HPLC to give **I-47** (11 mg, 0.03 mmol, 7 % yield) as a solid.
**HPLC:** Rt 8.49 min, 98.2%; Column: X-Select CSH C18 (4.6 x 150) mm, 5 µm; Mobile phase: A: 0.1% FA in water: ACN (95:05), B: ACN; Flow Rate: 1.0 mL/min
**LCMS** : 399.8 (M+H), Rt 2.06 min, Column: X-select CSH C18 (3 x 50) mm, 2.5 µm
**¹H NMR (400 MHz, DMSO-d6)** δ_{H} = 9.17 (d, 1H), 7.87 (d, 1H), 7.77 (d, 1H), 7.36 (s, 1H), 7.26-7.24 (m, 2H), 5.46 (q, 1H), 3.79-3.60 (m, 4H), 3.16 (s, 3H), 3.05-2.80 (m, 2H), 2.10-1.95 (m, 1H), 1H merged in solvent peak
**Chiral method:** Rt 5.29 min, 99%; column: DIACEL CHIRALPAK-IG (250 x 4.6 mm, 5 um), - Mobile Phase: A) n-Hexane+0.1% Isopropylamine B) DCM:MeOH (1:1), Isocratic: 50% B; Wavelength: 265 nm, Flow Rate: 1.0 mL/min

### Example 43. Synthesis of Compounds II-1 and II-2

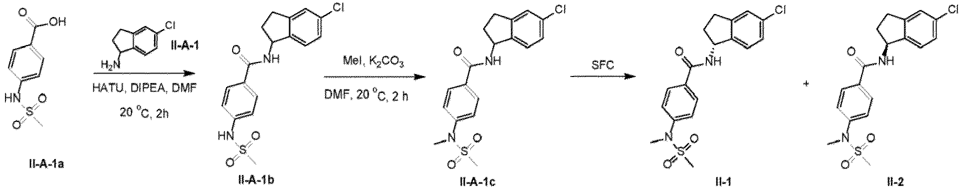

**Synthesis of II-A-1b:** To a mixture of 4-(methanesulfonamido)benzoic acid (300 mg, 1.39 mmol), HATU (795 mg, 2.09 mmol) and DIPEA (0.73 mL, 4.18 mmol) in DMF (10 mL) was added 5-chloroindan-1-amine (280 mg, 1.67 mmol). The resulting mixture was stirred at 20 °C for 2 hours. Saturated NH₄Cl solution (50 mL) was added to the solution and the aqueous layer was extracted with EtOAc (50 mL x 2). The combined organic phase was washed with water (50 mL x 2) and brine (50 mL x 2), dried over anhydrous Na₂SO₄, filtered and concentrated. The crude product was purified by flash chromatography on silica gel (EtOAc in PE = 0% to 30% to 50%) to give the product (400 mg, 1.10 mmol, 79% yield) as a solid.
**LCMS** Rₜ = 0.78 min in 1.5 min chromatography, 5-95AB, MS ESI calcd. for C₁₇H₁₈ClN₂O₃S [M+H]⁺ 365.1, found 365.0.

**Synthesis of II-A-1c:** To a mixture of*N*-(5-chloroindan-1-yl)-4-(methanesulfonamido)benzamide (150 mg, 0.41 mmol) and K₂CO₃ (170.47 mg, 1.23 mmol) in DMF (7 mL) was added Mel (116.71 mg, 0.82 mmol). The resulting mixture was stirred at 20 °C for 2 hours. Saturated NH₄Cl solution (20 mL) was added and the aqueous layer was extracted with EtOAc (20 mL x 2). The combined organic phase was washed with water (20 mL x 2) and brine (20 mL x 2), dried over anhydrous Na₂SO₄, filtered and concentrated to give the product (128 mg, 0.34 mmol, 82% yield) as a solid.
**¹H NMR** (400MHz, CDCl₃) δ_{H} = 7.89 - 7.76 (m, 2H), 7.51 - 7.40 (m, 2H), 7.27 - 7.24 (m, 2H), 7.23 - 7.17 (m, 1H), 6.29 (d, 1H), 5.66 (q, 1H), 3.36 (s, 3H), 3.08 - 2.98 (m, 1H), 2.97 - 2.88 (m, 1H), 2.85 (s, 3H), 2.76 - 2.66 (m, 1H), 2.02 - 1.88 (m, 1H).
**LCMS** Rₜ = 0.80 min in 1.5 min chromatography, 5-95AB, MS ESI calcd. for C₁₈H₂₀ClN₂O₃S [M+H]⁺ 379.1, found 379.0.
**Analytical SFC:** Chiralcel OJ-3 100 mm x 4.6 mm I.D, 3 µm, Mobile phase: A: CO₂ B:ethanol (0.05% DEA), Gradient: from 5% to 40% of B in 5 min and hold 40% of B for 2.5 min, then 5% of B for 2.5 min. Flow rate: 2.8 mL/min, Column temp.: 35 °C) showed two peaks at 1.12 min (50%) and 3.02 min (50%).

**Synthesis of Compounds II-1 and II-2:** *N*-(5-chloroindan-1-yl)-4-[methyl(methylsulfonyl)amino]benzamide (100 mg, 0.26 mmol) was purified by SFC (YMC CHIRAL Amylose-C (250 mm x 30 mm I.D., 10µm); A = CO₂ and B = EtOH (0.1% NH₃H₂O); 38 °C; 80 mL/min; 55% B; 8 min run; 7 injections,) to give the enantiomer 1, randomly assigned as Compound **II-1** (38.4 mg, 0.1 mmol) (Rt of peak 1 = 1.12 min) as a solid and the enantiomer 2, randomly assigned as Compound **II-2** (53.3 mg, 0.14 mmol) (Rt of peak 2 = 3.02 min) as a solid.

### Compound II-1

**¹H NMR** (400MHz, CD₃CN) δ_{H} = 7.91 - 7.78 (m, 2H), 7.46 (d, 2H), 7.35-7.25 (m, 3H), 7.23 - 7.15 (m, 1H), 5.56 (q, 1H), 3.29 (s, 3H), 3.09 - 2.97 (m, 1H), 2.95 - 2.81 (m, 4H), 2.62-2.50 (m, 1H), 2.07 - 1.97 (m, 1H).
**LCMS** Rt = 1.08 min in 2 min chromatography, 10-80AB, MS ESI calcd. for C₁₈H₂₀ClN₂O₃S [M+H]⁺ 379.1, found 379.0.

### Compound II-2

**¹H NMR** (400MHz, CD₃CN) δ_{H} = 7.89 - 7.80 (m, 2H), 7.46 (d, 2H), 7.35-7.25 (m, 3H), 7.24 - 7.17 (m, 1H), 5.56 (q, 1H), 3.30 (s, 3H), 3.09 - 2.97 (m, 1H), 2.94 - 2.84 (m, 4H), 2.62 - 2.50 (m, 1H), 2.08 - 1.98 (m, 1H).
**LCMS** Rt = 1.06 min in 2 min chromatography, 10-80AB, MS ESI calcd. for C₁₈H₂₀ClN₂O₃S [M+H]⁺ 379.1, found 379.0.

### Example 44. Synthesis of Compound II-3

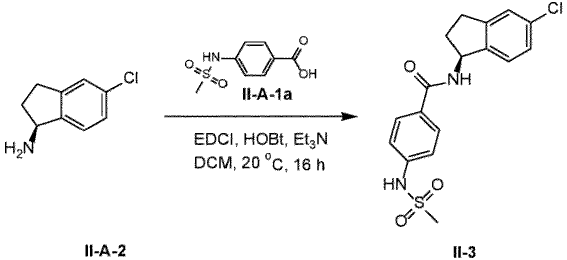

To a mixture of 4-(methanesulfonamido)benzoic acid (128.39 mg, 0.60 mmol), EDCI (171.53 mg, 0.89 mmol) and HOBt (161.21 mg, 1.19 mmol) in DCM (5 mL) were added Et₃N (0.25 mL, 1.79 mmol) and (1*S*)-5-chloroindan-1-amine (100 mg, 0.60 mmol). The resulting mixture was stirred at 20 °C for 16 hours. The mixture was diluted with H₂O (10 mL) and the mixture was extracted with EtOAc (30 mL x 2). The combined organic phase was washed with water (20 mL x 2) and brine (20 mL), dried over anhydrous Na₂SO₄, filtered and concentrated to give the crude product (220 mg) as a solid. The crude product (40 mg) was purified by Prep-TLC (silica gel, PE: EtOAc = 1:2) to give the product (12.2 mg, 33.5 µmol, 31% yield) as a solid.
**¹H NMR** (400MHz, CD₃CN) δ_{H} = 7.89 - 7.77 (m, 3H), 7.33 - 7.17 (m, 6H), 5.56 (q, 1H), 3.09 - 2.96 (m, 4H), 2.95 - 2.83 (m, 1H), 2.61 - 2.49 (m, 1H), 2.05 - 1.97 (m, 1H).
**LCMS** Rₜ = 1.11 min in 2.0 min chromatography, 10-80AB, MS ESI calcd. for C₁₇H₁₈ClN₂O₃S [M+H]⁺ 365.1, found 364.9.

### Example 45. Synthesis of Compound II-4

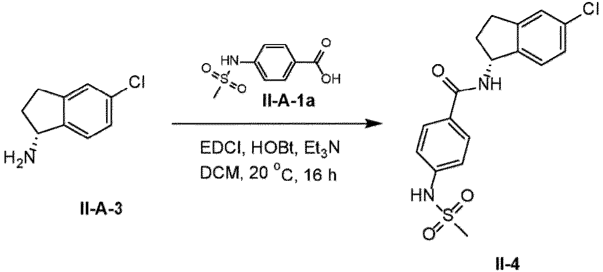

To a mixture of 4-(methanesulfonamido)benzoic acid (89.87 mg, 0.42 mmol), EDCI (120.07 mg, 0.63 mmol) and HOBt (112.85 mg, 0.84 mmol) in DCM (5 mL) were added Et₃N (0.17 mL, 1.25 mmol) and (1*R*)-5-chloroindan-1-amine (70 mg, 0.42 mmol). The resulting mixture was stirred at 20 °C for 16 hours. The mixture was diluted with H₂O (10 mL) and the mixture was extracted with EtOAc (30 mL x 2). The combined organic phase was washed with water (20 mL x 2) and brine (20 mL), dried over anhydrous Na₂SO₄, filtered and concentrated. The crude product was purified by flash chromatography on silica gel (EtOAc in PE = 0% to 50% to 100%) to give the product (109.6 mg, 297.3 µmol, 71% yield) as a solid.
**¹H NMR** (400MHz, CD₃CN) δ_{H} = 7.90 - 7.79 (m, 3H), 7.37 - 7.19 (m, 6H), 5.59 (q, 1H), 3.11 - 2.99 (m, 4H), 2.98 - 2.86 (m, 1H), 2.64 - 2.52 (m, 1H), 2.08 - 2.01 (m, 1H).
**LCMS** Rₜ = 1.10 min in 2.0 min chromatography, 10-80AB, MS ESI calcd. for C₁₇H₁₈ClN₂O₃S [M+H]⁺ 365.1, found 364.9.

### Example 46. Synthesis of Compounds II-5 and II-6

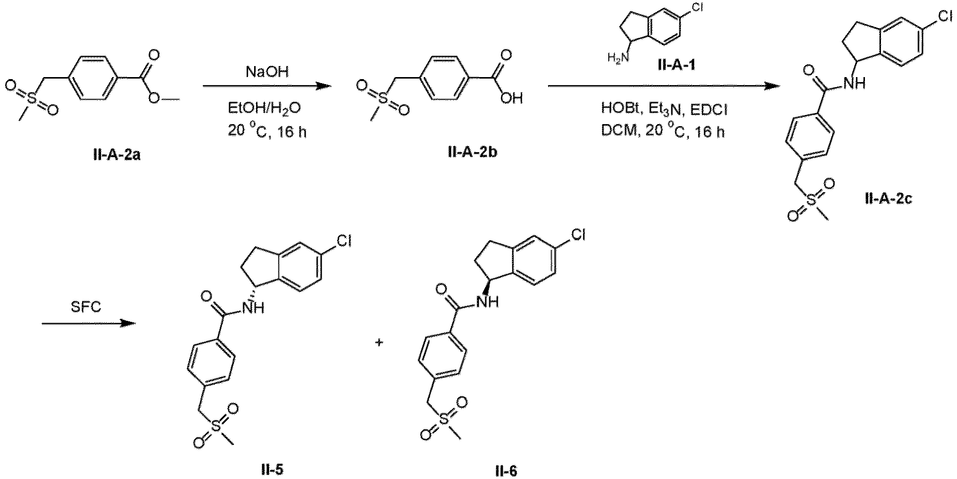

**Synthesis of II-A-2b:** A mixture of methyl 4-(methylsulfonylmethyl)benzoate (300 mg, 1.31 mmol) and NaOH (105.14 mg, 2.63 mmol) in water (4 mL) and ethanol (4 mL) was stirred at 20 °C for 16 hours. Ethanol was concentrated and the mixture was quenched with 1 N HCl to adjust to pH 2. The mixture was extracted with EtOAc (20 mL x 2). The combined organic phase was washed with brine (10 mL), dried over anhydrous Na₂SO₄, filtered and concentrated to give the crude product (270 mg, 1.26 mmol) as a solid.
**¹H NMR** (400MHz, DMSO-*d⁶*) δ_{H} = 13.03 (br s, 1H), 7.96 (d, 2H), 7.53 (d, 2H), 4.59 (s, 2H), 2.93 (s, 3H).

**Synthesis of II-A-2c:** A mixture of 4-(methylsulfonylmethyl)benzoic acid (138.02 mg, 0.64 mmol) and HOBt (193.46 mg, 1.43 mmol) and EDCI (205.83 mg, 1.07 mmol) and Et₃N (0.3 mL, 2.15 mmol) and 5-chloroindan-1-amine (120 mg, 0.72 mmol) in DCM (5 mL) was stirred at 20 °C for 16 hours under N₂. The reaction was quenched with sat. NH₄Cl (10 mL) and the mixture was extracted with DCM (20 mL x 2). The combined organic phase was washed with brine (10 mL), dried over anhydrous Na₂SO₄, filtered and concentrated to give the product (150 mg, 0.41 mmol) as a solid.
**LCMS** Rt = 0.79 min in 1.5 min chromatography, 5-95AB, MS ESI calcd. for C₁₈H₁₉ClNO₃S [M+H]⁺ 364.1, found 363.9.
**Analytical SFC:** (Chiralpak OJ-3 150 mm x 4.6 mm I.D, 3 µm, Mobile phase: A: CO₂ B: ethanol (0.05% DEA), Gradient: from 5% to 40% of B in 5 min and hold 40% of B for 2.5 min, then 5% of B for 2.5 min. Flow rate: 2.8 mL/min, Column temp.: 35 °C) showed two peaks at 1.23 min and 1.92 min.

**Synthesis of Compounds II-5 and II-6:** *N*-(5-chloroindan-1-yl)-4-(methylsulfonylmethyl)benzamide (150 mg, 0.41 mmol) was purified by SFC [DAICEL CHIRALCEL OJ-H(250 mm x 30 mm I.D., 5 µm); A = CO₂ and B = EtOH (0.1% NH₃H₂O); 38 °C; 60 mL/min; 35% B; 8 min run; 12 injections] to give the enantiomer 1, randomly assigned as Compound 5 (22.26 mg, 61.2 µmol) (Rₜ of Peak 1= 1.23 min) as a solid and the enantiomer 2, randomly assigned as Compound 6 (27.97 mg, 76.9 µmol) (Rₜ of Peak 2= 1.92 min) as a solid.

### Compound II-5

**¹H NMR** (400MHz, CD₃CN) δ_{H} = 7.85 (d, 2H), 7.49 (d, 2H), 7.37-7.24 (m, 3H), 7.23-7.14 (m, 1H), 5.61-5.51 (m, 1H), 4.37 (s, 2H), 3.09-2.97 (m, 1H), 2.95-2.85 (m, 1H), 2.82 (s, 3H), 2.61-2.50 (m, 1H), 2.08-1.98 (m, 1H).
**LCMS** Rₜ = 1.03 min in 2 min chromatography, 10-80AB, MS ESI calcd. for C₁₈H₁₉ClNO₃S [M+H]⁺ 364.1, found 364.0.

### Compound II-6

**¹H NMR** (400MHz, CD₃CN) δ_{H} = 7.85 (d, 2H), 7.49 (d, 2H), 7.37-7.23 (m, 3H), 7.23-7.16 (m, 1H), 5.61-5.51 (m, 1H), 4.37 (s, 2H), 3.09-2.98 (m, 1H), 2.96-2.84 (m, 1H), 2.82 (s, 3H), 2.61-2.51 (m, 1H), 2.07-1.98 (m, 1H).
**LCMS** Rₜ = 1.01 min in 2 min chromatography, 10-80AB, MS ESI calcd. for C₁₈H₁₉ClNO₃S [M+H]⁺ 364.1, found 364.0.

### Example 47. Synthesis of Compounds II-7 and II-8

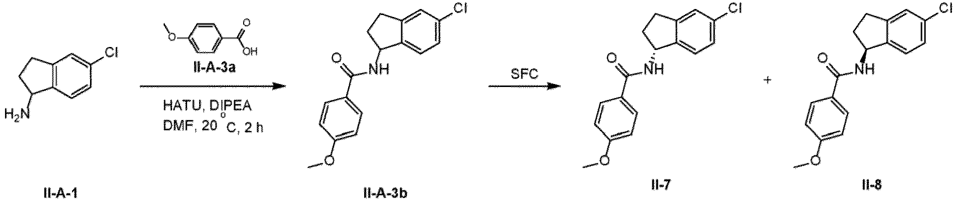

Synthesis of II-A-3b: To a mixture of 4-methoxybenzoic acid (150 mg, 0.99 mmol) and HATU (562.29 mg, 1.48 mmol) and DIPEA (0.34 mL, 1.97 mmol) in DMF (5 mL) was added 5-chloroindan-1-amine (198.33 mg, 1.18 mmol). The resulting mixture was stirred at 20 oC for 2 hours. Saturated NH4Cl aqueous (30 mL) was added and the aqueous layer was extracted with EtOAc (30 mL x 2). The combined organic phase was washed with water (20 mL x 2) and brine (20 mL x 2), dried over anhydrous Na2SO4, filtered and concentrated. The crude product was purified by Prep-HPLC [Xtimate C18 (150 mm x 25 mm, 5 µm) A = H2O (0.04% NH3H2O+10 mM NH4HCO3) and B = CH3CN; 47-77% B over 7 minutes] to give the product (250 mg, 819.3 µmol, 83% yield) as a solid.
**LCMS** Rt = 0.84 min in 1.5 min chromatography, 5-95AB, MS ESI calcd. for C₁₇H₁₇ClNO₂ [M+H]⁺ 302.1, found 301.9.
**Analytical SFC:** Chiralcel OJ-3 100 mm x 4.6 mm I.D., 3µm, Mobile phase: A: CO₂, B: 40% of ethanol (0.05% DEA), Gradient: from 5% to 40% of B in 5 min and hold 40% of B for 2.5 min, then 5% ofB for 2.5 min. Flow rate: 2.8 mL/min, Column temperature: 40 °C) showed two peaks at 0.94 min and 2.18 min.

Synthesis of Compounds II-7 and II-8: N-(5-chloroindan-1-yl)-4-methoxybenzamide (250 mg, 819.3 µmol) was purified by SFC [DAICEL CHIRALPAK OJ-H(250 mm x 30 mm, 5 µm); A = CO2 and B = EtOH (0.1% NH3.H2O); 35 oC; 60 mL/min; 40% B; 13 min run; 9 injections] to give the enantiomer 1, randomly assigned as Compound II-7 (92.5 mg, 306.4 µmol) ((Rt of Peak 1= 0.94 min) as a solid and the enantiomer 2, randomly assigned as Compound II-8 (87.4 mg, 289.6 µmol) (Rt of Peak 2= 2.18 min,) as a solid.

### Compound II-7

**¹H NMR** (400MHz, CD₃OD) δ_{H} = 7.84 (dd, 2H), 7.29-7.21 (m, 2H), 7.21-7.16 (m, 1H), 7.02-6.96 (m, 2H), 5.60 (t, 1H), 3.85 (s, 3H), 3.10-3.01 (m, 1H), 2.96-2.85 (m, 1H), 2.64-2.54 (m, 1H), 2.09-1.98 (m, 1H).
**LCMS** Rt = 1.13 min in 2.0 min chromatography, 10-80AB, MS ESI calcd. for C₁₇H₁₇ClNO₂ [M+H]⁺ 302.1, found 301.9.

### Compound II-8

**¹H NMR** (400MHz, CD₃OD) δ_{H} = 7.84 (d, 2H), 7.28-7.22 (m, 2H), 7.21-7.17 (m, 1H), 6.98 (d, 2H), 5.60 (t, 1H), 3.87 (s, 3H), 3.10-3.00 (m, 1H), 2.96-2.85 (m, 1H), 2.64-2.54 (m, 1H), 2.10-1.97 (m, 1H).
**LCMS** Rt = 1.11 min in 2.0 min chromatography, 10-80AB, MS ESI calcd. for C₁₇H₁₇ClNO₂ [M+H]⁺ 302.1, found 301.9.

### Example 48. Synthesis of Compounds II-9 and II-16

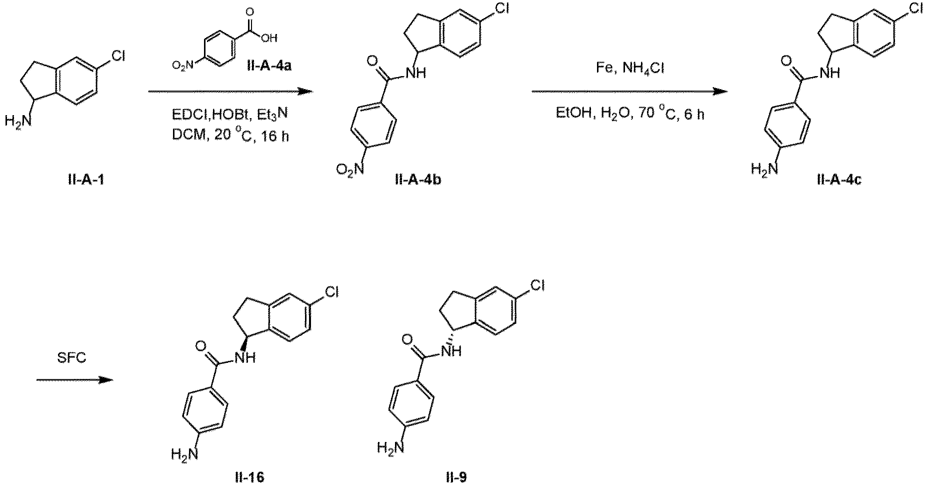

Synthesis of II-A-4b: A mixture of 4-nitrobenzoic acid (3.99 g, 23.86 mmol) and HOBt (6.45 g, 47.72 mmol) and Et₃N (9.9 mL, 71.58 mmol) and EDCI (6.86 g, 35.79 mmol) and 5-chloroindan-1-amine (4 g, 23.86 mmol) in DCM (50 mL) was stirred at 20 °C for 16 hours under N₂. The reaction was quenched with sat. NH₄Cl (50 mL) and the mixture was extracted with DCM (50 mL x 2). The combined organic phase was washed with brine (40 mL), dried over anhydrous Na₂SO₄, filtered and concentrated to give the product (6941 mg, 19.45 mmol) as a solid.
**LCMS** Rt = 0.84 min in 1.5 min chromatography, 5-95AB, MS ESI calcd. for C₁₆H₁₄ClN₂O₃ [M+H]⁺ 317.1, found 316.9.

Synthesis of II-A-4c: A mixture of*N*-(5-chloroindan-1-yl)-4-nitro-benzamide (3 g, 9.47 mmol) and NH₄Cl (5.07 g, 94.71 mmol) and Fe (5.29 g, 94.71 mmol) in ethanol (30 mL) and water (30 mL) was stirred at 70 °C for 6 hours under N₂. The mixture was filtered through Celite and the filter cake was eluted with EtOAc (50 mL x 2). The filtrate was concentrated under reduced pressure. The crude product was diluted with H₂O (50 mL) and the aqueous layer was extracted with EtOAc (50 mL x 2). The combined organic phase was washed with brine (50 mL), dried over anhydrous Na₂SO₄, filtered and concentrated to give the crude product (1260 mg, 4.3 mmol) as a solid.
**LCMS** Rt = 0.98 min in 2 min chromatography, 10-80AB, MS ESI calcd. for C₁₆H₁₆ClN₂O [M+H]⁺ 287.1, found 286.9.
**Analytical SFC:** (Chiralcel OJ-3 150 mm x 4.6 mm I.D, 3 µm, Mobile phase: A: CO₂ B: ethanol (0.05% DEA), Gradient: from 5% to 40% of B in 5 min and hold 40% of B for 2.5 min, then 5% ofB for 2.5 min. Flow rate: 2.5 mL/min, Column temp.: 35 °C) showed two peaks at 2.24 min and 4.83 min.

Synthesis of Compounds II-16 and II-9: 4-amino-N-(5-chloroindan-1-yl)benzamide (4 g, 13.95 mmol) was purified by SFC (DAICEL CHIRALCEL OJ (250 mm x 30 mm I.D., 5 µm); A = CO₂ and B = EtOH (0.1% NH₃H₂O); 38 °C; 50 mL/min; 45% B; 13 min run; 10 injections) to give enantiomer 1, assigned as Compound II-16 (Rt of Peak 1= 2.26 min, 46.6 mg) as a solid and enantiomer 2, assigned as Compound II-9 (Rt of Peak 2= 4.83 min) as a solid. Stereochemistry is randomly assigned.

### Compound II-16

**¹H NMR** (400MHz, DMSO-*d⁶*) δ_{H} = 8.26 (d, 1H), 7.62 (d, 2H), 7.31 (s, 1H), 7.24 - 7.14 (m, 2H), 6.53 (d, 2H), 5.61 (s, 2H), 5.47 (q, 1H), 3.03 - 2.91 (m, 1H), 2.90 - 2.75 (m, 1H), 2.52 - 2.48 (m, 1H) 2.06 - 1.92 (m, 1H).
**LCMS** Rt = 1.04 min in 2 min chromatography, 10-80AB, MS ESI calcd. for C₁₆H₁₆ClN₂O [M+H]⁺ 287.1, found 286.9.

4-amino-*N-*[(1*R*)-5-chloroindan-1-yl]benzamide (50 mg, 0.17 mmol) (Rt of Peak 2= 4.83 min) was triturated from CH₂Cl₂/*n*-hexane (5 mL, 1:5) to give the product (45.4 mg, 0.16 mmol) as a solid. The absolute stereochemistry of Compound II-9 was confirmed by X-ray crystallography.
**¹H NMR** (400MHz, DMSO-*d⁶*) δ_{H} = 8.27 (d, 1H), 7.62 (d, 2H), 7.32 (s, 1H), 7.23 - 7.16 (m, 2H), 6.53 (d, 2H), 5.62 (s, 2H), 5.47 (q, 1H), 3.01 - 2.93 (m, 1H), 2.87 - 2.78 (m, 1H), 2.45 - 2.37 (m, 1H), 2.04 - 1.93 (m, 1H).
**LCMS** Rₜ = 1.03 min in 2.0 min chromatography, 10-80AB, MS ESI calcd. for C₁₆H₁₆ClN₂O [M+H]⁺ 287.1, found 286.9.

### Example 49. Synthesis of Compound II-10

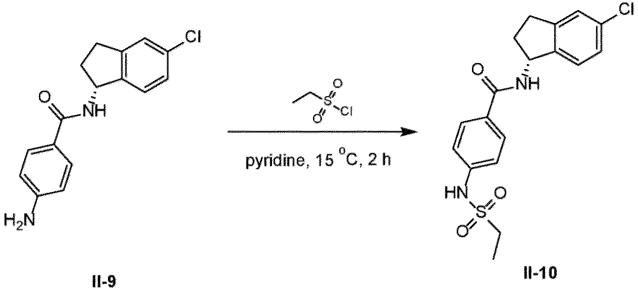

A mixture of 4-amino-*N-*[(1*R*)-5-chloroindan-1-yl]benzamide (100 mg, 0.35 mmol) and ethanesulfonyl chloride (58.29 mg, 0.45 mmol) in pyridine (4 mL) was stirred at 15 °C for 2 hours. The reaction was quenched with 1 N HCl (5 mL) and the mixture was extracted with EtOAc (5 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 32-62% B over 6 minutes) to give the product (64.8 mg, 0.17 mmol, 49% yield) as a solid.
**¹H NMR** (400MHz, DMSO-*d⁶*) δ_{H} = 10.13 (br s, 1H), 8.67 (d, 1H), 7.86 (d, 2H), 7.33 (s, 1H), 7.27 - 7.18 (m, 4H), 5.50 (q, 1H), 3.15 (q, 2H), 3.03 - 2.94 (m, 1H), 2.90 - 2.80 (m, 1H), 2.47 - 2.40 (m, 1H), 2.06 - 1.95 (m, 1H), 1.18 (t, 3H).
**LCMS** Rₜ = 1.12 min in 2.0 min chromatography, 10-80AB, MS ESI calcd. for C₁₈H₂₀ClN₂O₃S [M+H]⁺ 379.1, found 378.9.

### Example 50. Synthesis of Compound II-11

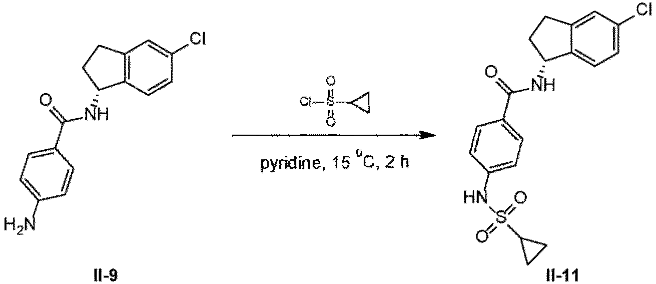

A mixture of 4-amino-*N-*[(1*R*)-5-chloroindan-1-yl]benzamide (100 mg, 0.35 mmol) and cyclopropanesulfonyl chloride (63.74 mg, 0.45 mmol) in pyridine (4 mL) was stirred at 15 °C for 2 hours. The reaction was quenched with 1M HCl (5 mL) and the mixture was extracted with EtOAc (5 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 32-62% B over 6.5 minutes) to give the product (71.4 mg, 0.18 mmol, 52% yield) as a solid.
**¹H NMR** (400MHz, DMSO-*d⁶*) δ_{H} = 10.08 (s, 1H), 8.68 (d, 1H), 7.87 (d, 2H), 7.34 (s, 1H), 7.27 (d, 2H), 7.25 - 7.19 (m, 2H), 5.50 (q, 1H), 3.03 - 2.95 (m, 1H), 2.90 - 2.80 (m, 1H), 2.73 - 2.66 (m, 1H), 2.48 - 2.40 (m, 1H), 2.06 - 1.95 (m, 1H), 1.00 - 0.91 (m, 4H).
**LCMS** Rₜ = 1.14 min in 2.0 min chromatography, 10-80AB, MS ESI calcd. for C₁₉H₂₀ClN₂O₃S [M+H]⁺ 391.1, found 390.9.

### Example 51. Synthesis of Compound II-12

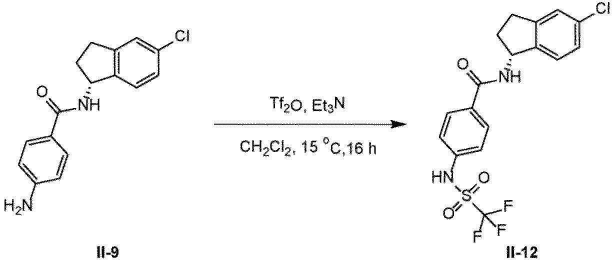

A mixture of trifluoromethylsulfonyl trifluoromethanesulfonate (177.1 mg, 0.63 mmol), Et₃N (0.11 mL, 0.78 mmol) and 4-amino-*N-*[(1*R*)-5-chloroindan-1-yl]benzamide (150 mg, 0.52 mmol) in CH₂Cl₂ (10 mL) was stirred at 15 °C for 16 hours. The reaction was quenched with sat. NH₄Cl solution (10 mL). The mixture was extracted with CH₂Cl₂ (10 mL x 2). The combined organic phase was washed with brine (10 mL), dried over anhydrous Na₂SO₄, filtered and concentrated. The crude product was purified by Prep-HPLC (Xbridge BEH C18 (250 mm x 50 mm, 10 µm) A = H₂O (10 mM NH₄HCO₃) and B = CH₃CN; 25-45% B over 9 minutes) to give the product (17.9 mg, 42.7 µmol, 8% yield) as a solid.
**¹H NMR** (400MHz, CD₃CN) δ_{H} = 7.82 (d, 2H), 7.36 - 7.23 (m, 5H), 7.21 - 7.18 (m, 1H), 5.56 (q, 1H), 3.06 - 2.98 (m, 1H), 2.93 - 2.84 (m, 1H), 2.59 - 2.50 (m, 1H), 2.06 - 1.97 (m, 1H).
**LCMS** Rₜ = 1.21 min in 2.0 min chromatography, 10-80AB, MS ESI calcd. for C₁₇H₁₅ClF₃N₂O₃S [M+H]⁺ 419.0, found 418.8.

### Example 52. Synthesis of Compound II-13

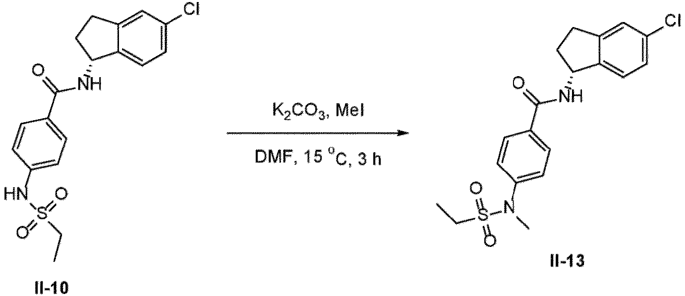

A mixture of*N-*[(1*R*)-5-chloroindan-1-yl]-4-(ethylsulfonylamino)benzamide (120 mg, 0.32 mmol) and K₂CO₃ (87.55 mg, 0.63 mmol) and Mel (134.87 mg, 0.95 mmol) in DMF (5 mL) was stirred at 15 °C for 3 hours. The mixture was diluted with H₂O (5 mL) and the aqueous layer was extracted with EtOAc (5 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 38-68% B over 6 minutes) to give the product (86.8 mg, 0.22 mmol, 70% yield) as a solid.
**¹H NMR** (400MHz, DMSO-*d⁶*) δ_{H} = 8.79 (d, 1H), 7.91 (d, 2H), 7.50 (d, 2H), 7.34 (s, 1H), 7.25 - 7.19 (m, 2H), 5.51 (q, 1H), 3.30 (s, 3H), 3.19 (q, 2H), 3.03 - 2.95 (m, 1H), 2.90 - 2.80 (m, 1H), 2.47 - 2.41 (m, 1H), 2.07 - 1.96 (m, 1H), 1.17 (t, 3H).
**LCMS** Rₜ = 1.18 min in 2.0 min chromatography, 10-80AB, MS ESI calcd. for C₁₉H₂₂ClN₂O₃S [M+H]⁺ 393.1, found 392.9.

### Example 53. Synthesis of Compound II-14

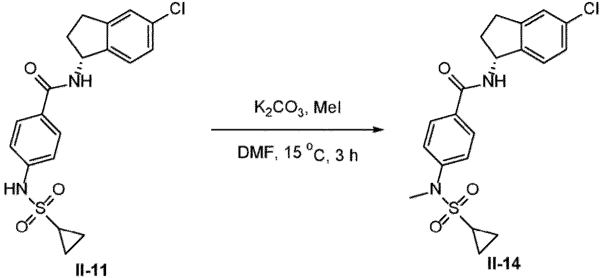

A mixture of*N-*[(1*R*)-5-chloroindan-1-yl]-4-(cyclopropylsulfonylamino)benzamide (140 mg, 0.36 mmol) and K₂CO₃ (99 mg, 0.72 mmol) and Mel (152.51 mg, 1.07 mmol) in DMF (5 mL) was stirred at 15 °C for 3 hours. The mixture was diluted with H₂O (5 mL) and the aqueous layer was extracted with EtOAc (5 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 40-70% B over 6 minutes) to give the product (83.3 mg, 0.21 mmol, 57% yield) as a solid.
**¹H NMR** (400MHz, CD₃CN) δ_{H} = 7.83 (dd, 2H), 7.50 (dd, 2H), 7.32 - 7.24 (m, 3H), 7.22 - 7.18 (m, 1H), 5.57 (q, 1H), 3.33 (s, 3H), 3.07 - 2.99 (m, 1H), 2.94 - 2.85 (m, 1H), 2.61 - 2.45 (m, 2H), 2.07 - 1.98 (m, 1H), 0.98 - 0.87 (m, 4H).
**LCMS** Rₜ = 1.18 min in 2.0 min chromatography, 10-80AB, MS ESI calcd. for C₂₀H₂₂ClN₂O₃S [M+H]⁺ 405.1, found 404.9.

### Example 54. Synthesis of Compound II-15

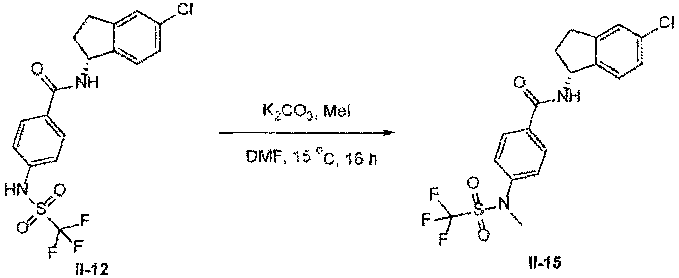

A mixture of*N*-[(1*S*)-5-chloroindan-1-yl]-4-(trifluoromethylsulfonylamino)benzamide (250 mg, 0.60 mmol) and K₂CO₃ (165 mg, 1.19 mmol) and Mel (254.18 mg, 1.79 mmol) in DMF (10mL) was stirred at 20 °C for 3 hours. The mixture was diluted with H₂O (15 mL) and the aqueous layer was extracted with EtOAc (15 mL x 2). The combined organic phase was washed with brine (15 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 55-85 %B over 8 minutes) to give the product (65.9 mg, 0.15 mmol, 25% yield) as a solid.
**¹H NMR** (400MHz, CD₃CN) δ_{H} = 7.89 (d, 2H), 7.52 (d, 2H), 7.38 - 7.26 (m, 3H), 7.23 - 7.18 (m, 1H), 5.56 (q, 1H), 3.48 (s, 3H), 3.09 - 2.97 (m, 1H), 2.96 - 2.83 (m, 1H), 2.62 - 2.50 (m, 1H), 2.07 - 1.98 (m, 1H).
**LCMS** Rₜ = 1.27 min in 2 min chromatography, 10-80AB, MS ESI calcd. for C₁₈H₁₇ClF₃N₂O₃S [M+H]⁺ 433.1, found 432.8.

### Example 55. Synthesis of Compound II-17

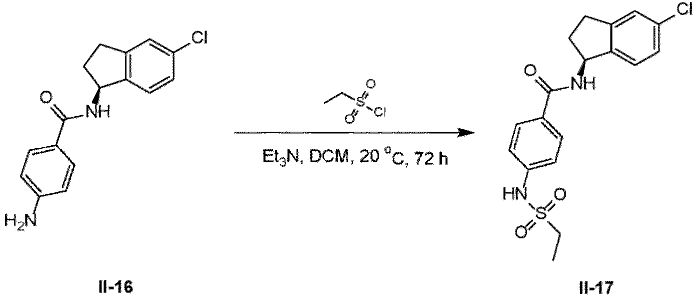

A mixture of 4-amino-*N*-[(1*S*)-5-chloroindan-1-yl]benzamide (200 mg, 0.7 mmol) and ethanesulfonyl chloride (448.39 mg, 3.49 mmol) in pyridine (20 mL) was stirred at 20 °C for 72 hours under N₂. The reaction was concentrated and quenched with sat. NH₄Cl (15 mL). The mixture was extracted with EtOAc (15 mL x 2). The combined organic phase was washed with brine (15 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 37-67%B over 8 minutes) to give the product (35.3 mg, 0.09 mmol, 13% yield) as a solid.
**¹H NMR** (400MHz, CD₃CN) δ_{H} = 7.89 - 7.74 (m, 3H), 7.32 - 7.14 (m, 6H), 5.56 (q, 1H), 3.13 (q, 2H), 3.07 - 2.96 (m, 1H), 2.95 - 2.83 (m, 1H), 2.61 - 2.49 (m, 1H), 2.06 - 1.97 (m, 1H), 1.25 (t, 3H).
**LCMS** Rt = 1.02 min in 2 min chromatography, 10-80AB, MS ESI calcd. for C₁₈H₂₀ClN₂O₃S [M+H]⁺ 379.1, found 379.0.

### Example 56. Synthesis of Compound II-18

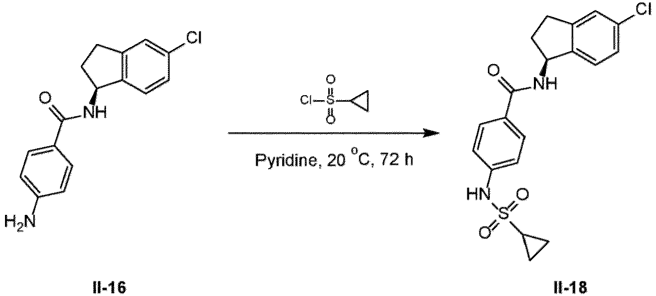

A mixture of 4-amino-*N*-[(1*S*)-5-chloroindan-1-yl]benzamide (200 mg, 0.7 mmol) and cyclopropanesulfonyl chloride (127.47 mg, 0.91 mmol) in pyridine (15 mL) was stirred at 20 °C for 72 hours under N₂. The reaction was concentrated and quenched with sat. NH₄Cl (15 mL). The mixture was extracted with EtOAc (15 mL x 2). The combined organic phase was washed with brine (15 mL), dried over anhydrous Na₂SO₄, filtered and concentrated. The crude product was triturated from Hexane and DCM (5:1, 5 mL) to give the product (49.5 mg, 0.13 mmol, 18% yield) as a solid.
**¹H NMR** (400MHz, DMSO-*d⁶*) δ_{H} = 10.08 (s, 1H), 8.68 (d, 1H), 7.87 (d, 2H), 7.43 - 7.14 (m, 5H), 5.50 (q, 1H), 3.07 - 2.93 (m, 1H), 2.92 - 2.79 (m, 1H), 2.74 - 2.65 (m, 1H), 2.46 - 2.40 (m, 1H), 2.10 - 1.91 (m, 1H), 1.04 - 0.91 (m, 4H).
**LCMS** Rt = 1.05 min in 2 min chromatography, 10-80AB, MS ESI calcd. for C₁₉H₂₀ClN₂O₃S [M+H]⁺ 391.1, found 391.0.

### Example 57. Synthesis of Compound II-19

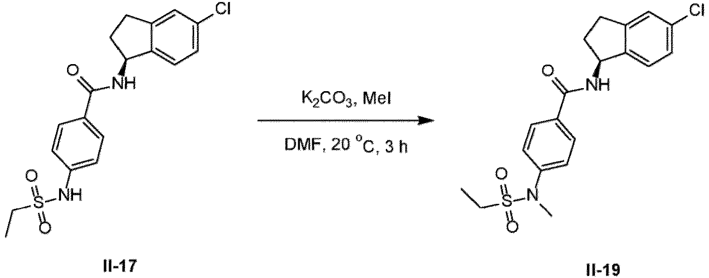

A mixture of *N*-[(1*S*)-5-chloroindan-1-yl]-4-(ethylsulfonylamino)benzamide (220 mg, 0.58 mmol) and K₂CO₃ (160.51mg, 1.16 mmol) and Mel (247.26 mg, 1.74 mmol) in DMF (5mL) was stirred at 20°C for 3 hours. The mixture was diluted with H₂O (5 mL) and the aqueous layer was extracted with EtOAc (15 mL x 2). The combined organic phase was washed with brine (15 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 48-78 %B over 8 minutes) to give the product (119.2 mg, 0.31 mmol, 52% yield) as a solid.
**¹H NMR** (400MHz, CD₃CN) δ_{H} = 7.83 (dd, 2H), 7.47 (dd, 2H), 7.33 - 7.23 (m, 3H), 7.22 - 7.16 (m, 1H), 5.56 (q,1H), 3.32 (s, 3H), 3.10 (q, 2H), 3.07 - 2.97 (m, 1H), 2.95 - 2.84 (m, 1H), 2.61 - 2.51 (m, 1H), 2.07 - 1.97 (m, 1H), 1.25 (t, 3H).
**LCMS** Rt = 1.04 min in 2 min chromatography, 10-80AB, MS ESI calcd. for C₁₉H₂₂ClN₂O₃S [M+H]⁺ 393.1, found 393.0.

### Example 58. Synthesis of Compound II-20

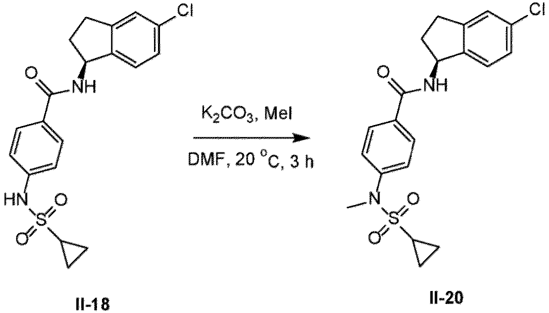

A mixture of N-[(1S)-5-chloroindan-1-yl]-4-(cyclopropylsulfonylamino)benzamide (150 mg, 0.38 mmol), K₂CO₃ (106.08 mg, 0.77 mmol), and Mel (163.41 mg, 1.15 mmol) in DMF (5 mL) was stirred at 20 °C for 3 hours. The mixture was diluted with H₂O (15 mL) and extracted with EtOAc (15 mL x 2). The combined organic phase was washed with brine (15 mL), dried over Na₂SO₄, filtered and concentrated to give the crude product. The crude product was triturated from Hexane and DCM (5:1 5 mL) to give the product (146.5 mg, 0.36 mmol, 94% yield) as a solid.
**¹H NMR** (400MHz, CD₃CN) δ_{H} = 7.83 (d, 2H), 7.50 (d, 2H), 7.34 - 7.23 (m, 3H), 7.23 - 7.17 (m, 1H), 5.57 (q, 1H), 3.33 (s, 3H), 3.09 - 2.98 (m, 1H), 2.95 - 2.83 (m, 1H), 2.62 - 2.43 (m, 2H), 2.07 - 1.97 (m, 1H), 1.00 - 0.83 (m, 4H).
**LCMS** Rt = 1.05 min in 2 min chromatography, 10-80AB, MS ESI calcd. for C₂₀H₂₂ClN₂O₃S [M+H]⁺ 405.1, found 405.0.

### Example 59. Synthesis of Compound II-21

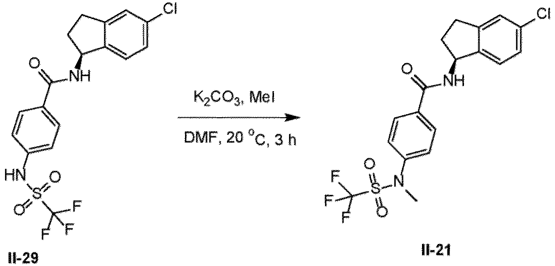

A mixture of *N-*[(1*S*)-5-chloroindan-1-yl]-4-(trifluoromethylsulfonylamino)benzamide (250 mg, 0.60 mmol) and K₂CO₃ (165 mg, 1.19 mmol) and Mel (254.18 mg, 1.79 mmol) in DMF (10mL) was stirred at 20 °C for 3 hours. The mixture was diluted with H₂O (15 mL) and the aqueous layer was extracted with EtOAc (15 mL x 2). The combined organic phase was washed with brine (15 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 55-85 %B over 8 minutes) to give the product (65.9 mg, 0.15 mmol, 25% yield) as a solid.
**¹H NMR** (400MHz, CD₃CN) δ_{H} = 7.89 (d, 2H), 7.52 (d, 2H), 7.38 - 7.26 (m, 3H), 7.23 - 7.18 (m, 1H), 5.56 (q, 1H), 3.48 (s, 3H), 3.09 - 2.97 (m, 1H), 2.96 - 2.83 (m, 1H), 2.62 - 2.50 (m, 1H), 2.07 - 1.98 (m, 1H).
**LCMS** Rₜ = 1.27 min in 2 min chromatography, 10-80AB, MS ESI calcd. for C₁₈H₁₇ClF₃N₂O₃S [M+H]⁺ 433.1, found 432.8.

### Example 60. Synthesis of Compound II-22

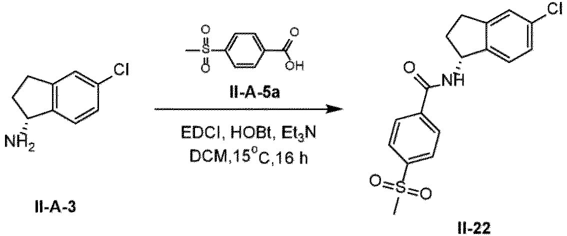

To a mixture of (1*R*)-5-chloroindan-1-amine (50 mg, 0.30 mmol) and HOBt (80.61 mg, 0.60 mmol) and Et₃N (0.21 mL, 1.49 mmol) in CH₂Cl₂ (5 mL) were added EDCI (114.35 mg, 0.60 mmol) and 4-methylsulfonylbenzoic acid (71.66 mg, 0.36 mmol). The reaction mixture was stirred at 15 °C for 16 hours. The mixture was diluted with sat. NH₄Cl solution (5 mL) and the aqueous layer was extracted with CH₂Cl₂ (5 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 32-62% B over 7 minutes) to give the product (13.7 mg, 0.04 mmol, 13% yield) as a solid.
**¹H NMR** (400MHz, CD₃CN) δ_{H} = 8.03 - 7.97 (m, 4H), 7.49 - 7.39 (m, 1H), 7.32 - 7.29 (m, 2H), 7.23 - 7.19 (m, 1H), 5.58 (q, 1H), 3.08 (s, 3H), 3.07 - 3.00 (m, 1H), 2.96 - 2.86 (m, 1H), 2.62 - 2.53 (m, 1H), 2.07 - 1.99 (m, 1H).
**LCMS** Rₜ = 1.12 min in 2.0 min chromatography, 10-80AB, MS ESI calcd. for C₁₇H₁₇ClNO₃S [M+H]⁺ 350.1, found 349.9.

### Example 61. Synthesis of Compound II-23

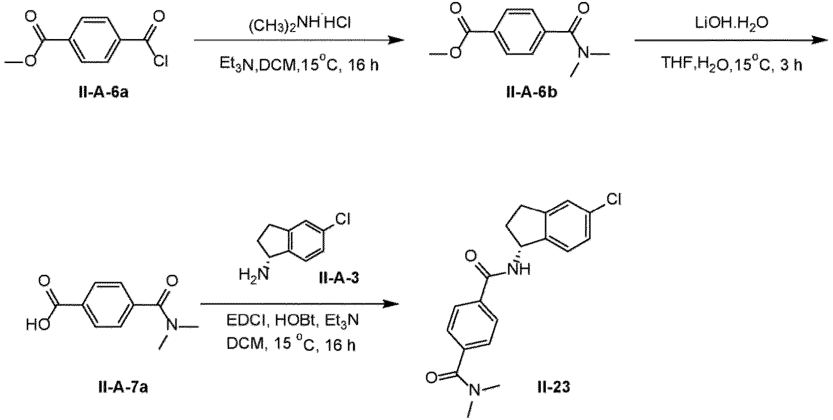

**Synthesis of II-A-6b:** A mixture of *N*-methylmethanamine hydrochloride (500 mg, 6.13 mmol) and Et₃N (2.55 mL, 18.4 mmol) and methyl 4-chlorocarbonylbenzoate (1826.71 mg, 9.2 mmol) in DCM (10 mL) was stirred at 15 °C for 16 hours. The mixture was diluted with sat. NH₄Cl (10 mL) and the aqueous layer was extracted with DCM (10 mL x 2). The combined organic phase was washed with brine (10 mL), dried over anhydrous Na₂SO₄, filtered and concentrated. The crude product was purified by flash chromatography on silica gel (EtOAc in PE = 30% to 60%) to give the product (1100 mg, 4.70 mmol, 77% yield) as a solid.
**LCMS** Rt = 0.65 min in 1.5 min chromatography, 5-95AB, MS ESI calcd. for C₁₁H₁₃NO₃ [M+H]⁺ 208.1, found 207.9.

**Synthesis of II-A-7a:** A mixture of methyl 4-(dimethylcarbamoyl)benzoate (500 mg, 2.41mmol) and LiOH.HzO (202.48 mg, 4.83 mmol) in THF (8 mL) and water (4 mL) was stirred at 15 °C for 3 hours. The mixture was acidified with 1N HCl to pH 2. The mixture was diluted with H₂O (10 mL) and the aqueous layer was extracted with EtOAc (20 mL x 2). The combined organic phase was concentrated to give the crude product (518 mg, 2.45 mmol) as a solid.
**LCMS** Rt = 0.87 min in 2 min chromatography, 0-60AB, MS ESI calcd. for C₁₀H₁₂NO₃ [M+H]⁺ 194.1, found 193.8.

**Synthesis of Compound II-23:** To a mixture of 4-(dimethylcarbamoyl)benzoic acid (80.67 mg, 0.42 mmol) and HOBt (112.85 mg, 0.84 mmol) and Et₃N (0.29 mL, 2.09 mmol) in DCM (5 mL) were added EDCI (120.07 mg, 0.63 mmol) and (1*R*)-5-chloroindan-1-amine (70 mg, 0.42 mmol). The reaction mixture was stirred at 15 °C for 16 hours. The reaction was quenched with sat. NH₄Cl (20 mL) and the mixture was extracted with DCM (20 mL x 2). The combined organic phase was washed with brine (20 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 39-69% B over 8 minutes) to give the product (13.7 mg, 0.04 mmol, 10% yield) as a solid.
**¹H NMR** (400MHz, CD₃CN) δ_{H} = 7.85 (d, 2H), 7.44 (d, 2H), 7.37 - 7.26 (m, 3H), 7.24 - 7.17 (m, 1H), 5.58 (q, 1H), 3.11 - 2.96 (m, 4H), 2.96 - 2.81 (m, 4H), 2.64 - 2.50 (m, 1H), 2.06 - 1.99 (m, 1H).
**LCMS** Rt = 1.15 min in 2 min chromatography, 10-80AB, MS ESI calcd. for C₁₉H₂₀ClN₂O₂ [M+H]⁺ 343.1, found 343.0.

### Example 62. Synthesis of Compound II-24

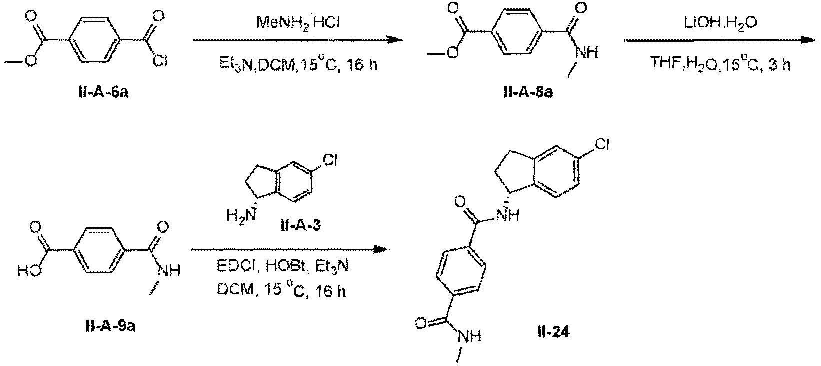

Synthesis of II-A-8a: A mixture of methanamine hydrochloride (500 mg, 7.41 mmol) and Et₃N (3.07 mL, 22.22 mmol) and methyl 4-chlorocarbonylbenzoate (2.21g, 11.11 mmol) in DCM (15 mL) was stirred at 15 °C for 16 hours. The mixture was diluted with sat. NH₄Cl (15 mL) and the aqueous layer was extracted with DCM (15 mL x 2). The combined organic phase was washed with brine (15 mL), dried over anhydrous Na₂SO₄, filtered and concentrated. The crude product was purified by flash chromatography on silica gel (EtOAc in PE = 30% to 60%) to give the product (712 mg, 3.69 mmol, 50% yield) as a solid.
**LCMS** Rt = 0.60 min in 1.5 min chromatography, 5-95AB, MS ESI calcd. for C₁₀H₁₂NO₃ [M+H]⁺ 194.1, found 193.9.

Synthesis of II-A-9a: A mixture of methyl 4-(methylcarbamoyl)benzoate (500 mg, 2.59 mmol) and LiOH·H₂O (217.18 mg, 5.18 mmol) in THF (4 mL) and water (2 mL) was stirred at 15 °C for 3 hours. The mixture was acidified with 1N HCl (5 mL) and diluted with H₂O (10 mL). The aqueous layer was extracted with EtOAc (20 mL x 2). The combined organic phase was concentrated to give the crude product (606 mg, 3.38 mmol) as a solid.
**LCMS** Rt = 0.81 min in 2 min chromatography, 0-60AB, MS ESI calcd. for C₉H₉NO₃ [M+H]⁺ 180.1, found 179.9.

Synthesis of II-24: To a mixture of (1*R*)-5-chloroindan-1-amine (100 mg, 0.6 mmol) and HOBt (161.21 mg, 1.19 mmol) and Et₃N (0.41 mL, 2.98 mmol) in DCM (5 mL) were added EDCI (171.53 mg, 0.89 mmol) and 4-(methylcarbamoyl)benzoic acid (106.88 mg, 0.6 mmol). The reaction mixture was stirred at 15 °C for 16 hours. The reaction was quenched with sat. NH₄Cl (20 mL) and the mixture was extracted with DCM (20 mL x 2). The combined organic phase was washed with brine (20 mL), dried over anhydrous Na₂SO₄, filtered and concentrated. The crude product was purified by prep-HPLC (Boston Prime (150 mm x 30 mm, 5µm) A = H₂O (0.05% NH₄OH) and B = CH₃CN; 35-65% B over 8 minutes) to give the product (8.8 mg, 0.03 mmol, 4% yield) as a solid.
**¹H NMR** (400MHz, DMSO-*d⁶*) δ_{H} = 8.89 (d, 1H), 8.56 (d, 1H), 8.03 - 7.80 (m, 4H), 7.34 (s, 1H), 7.24 (s, 2H), 5.52 (q, 1H), 3.07 - 2.94 (m, 1H), 2.92 - 2.83 (m, 1H), 2.79 (d, 3H), 2.47 - 2.42 (m, 1H), 2.09 - 1.95 (m, 1H).
**LCMS** Rt = 1.09 min in 2 min chromatography, 10-80AB, MS ESI calcd. for C₁₈H₁₈ClN₂O₂ [M+H]⁺ 329.1, found 329.1.

### Example 63. Synthesis of Compound II-25

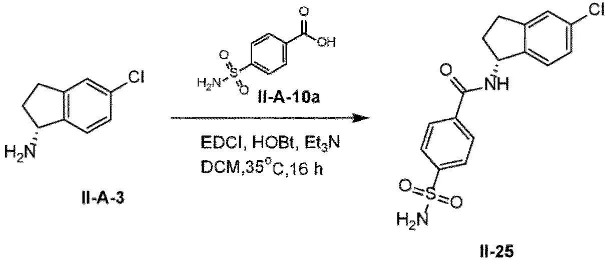

To a mixture of 4-sulfamoylbenzoic acid (72.01 mg, 0.36 mmol) and HOBt (80.61 mg, 0.60 mmol) and Et₃N (0.21 mL, 1.49 mmol) in CH₂Cl₂ (3 mL) were added EDCI (114.35 mg, 0.60 mmol) and (1*R*)-5-chloroindan-1-amine (50 mg, 0.30 mmol). The reaction mixture was stirred at 35 °C for 16 hours. The mixture was diluted with sat. NH₄Cl (5 mL) and extracted with EtOAc (5 mL x 2). The combined organic phase was washed with brine (5 mL), dried over anhydrous Na₂SO₄, filtered and concentrated. The crude product was purified by Prep-HPLC (Xtimate C18 (150 mm x 25 mm, 5 µm) A = H₂O (10 mM NH₄HCO₃) and B = CH₃CN; 40-50% B over 9 minutes) to give the product (20.6 mg, 58.6 µmol, 20% yield) as a solid.
**¹H NMR** (400MHz, CD₃CN) δ_{H} = 7.98 - 7.91 (m, 4H), 7.43 - 7.35 (m, 1H), 7.32 - 7.29 (m, 2H), 7.23 - 7.19 (m, 1H), 5.74 (s, 2H), 5.58 (q, 1H), 3.09 - 3.00 (m, 1H), 2.95 - 2.86 (m, 1H), 2.62 - 2.53 (m, 1H), 2.06 - 2.00 (m, 1H).
**LCMS** Rₜ = 1.11 min in 2.0 min chromatography, 10-80AB, MS ESI calcd. for C₁₆H₁₆ClN₂O₃S [M+H]⁺ 351.0, found 351.1.

### Example 64. Synthesis of Compound II-26

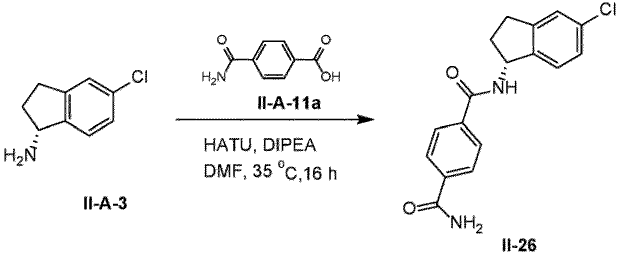

To a mixture of 4-carbamoylbenzoic acid (75.86 mg, 0.46 mmol) and DIPEA (0.22 mL, 1.25 mmol) and HATU (317.54 mg, 0.84 mmol) in DMF (3 mL) was added (1*R*)-5-chloroindan-1-amine (70 mg, 0.42 mmol). The reaction mixture was stirred at 35 °C for 16 hours. The mixture was diluted with sat. NH₄Cl (5 mL) and the aqueous layer was extracted with EtOAc (5 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 33-53% B over 7 minutes) to give the product (7.6 mg, 24.0 µmol, 6% yield) as a solid.
**¹H NMR** (400MHz, DMSO-*d⁶*) δ_{H} = 8.95-8.82 (m, 1H), 8.07 (br s, 1H), 8.02-7.88 (m, 4H), 7.48 (br s, 1H), 7.34 (s, 1H), 7.32-7.22 (m, 2H), 5.52 (q, 1H), 3.06 - 2.96 (m, 1H), 2.92 - 2.82 (m, 1H), 2.46 - 2.42 (m, 1H), 2.08 - 1.98 (m, 1H).
**LCMS** Rₜ = 1.07 min in 2.0 min chromatography, 10-80AB, MS ESI calcd. for C₁₇H₁₆ClN₂O₂ [M+H]⁺ 315.1, found 314.9.

### Example 65. Synthesis of Compound II-27

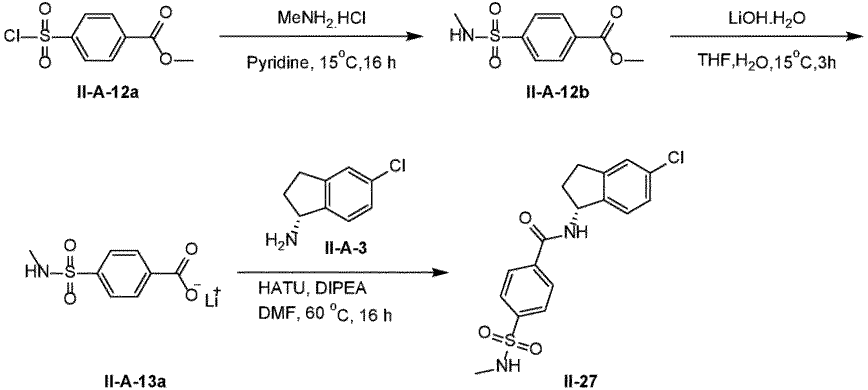

**Synthesis of II-A-12b:** A mixture of methanamine hydrochloride (500 mg, 7.41 mmol) and methyl 4-chlorosulfonylbenzoate (2606.56 mg, 11.11 mmol) in pyridine (10 mL) was stirred at 15 °C for 16 hours. The reaction was concentrated and acidified with 1M HCl to pH~7 and the mixture was extracted with CH₂Cl₂ (80 mL x 2). The combined organic phase was washed with water (80 mL) and brine (80 mL), dried over anhydrous Na₂SO₄, filtered and concentrated to give the crude product (1600 mg, 6.35 mmol) as a solid.
**LCMS** Rt = 0.65 min in 1.5 min chromatography, 5-95AB, MS ESI calcd. for C₉H₁₂NO₄S [M+H]⁺ 230.0, found 229.9.

**Synthesis of II-A-13a:** A mixture of methyl 4-(methylsulfamoyl)benzoate (500 mg, 2.18 mmol) and LiOH.HzO (183.03 mg, 4.36 mmol) in THF (8 mL) and water (4 mL) was stirred at 15 °C for 3 hours. The mixture was concentrated to give the crude product (488 mg, 2.08 mmol) as a solid, which was used for next step directly without further purification.
**LCMS** Rt = 0.87 min in 1.5 min chromatography, 5-95AB, MS ESI calcd. for C₈H₁₀NO₄S [M+H]⁺ 216.0, found 215.8.

**Synthesis of II-27:** To a mixture of (1*R*)-5-chloroindan-1-amine (100 mg, 0.60 mmol) and DIPEA (0.31 mL, 1.79 mmol) and HATU (453.63 mg, 1.19 mmol) in DMF (5 mL) was added lithium 4-(methylsulfamoyl)benzoate (141.23 mg, 0.66 mmol). The reaction mixture was stirred at 60 °C for 16 hours. The mixture was diluted with sat.NH₄Cl (5 mL) and the aqueous layer was extracted with EtOAc (15 mL x 2). The combined organic phase was washed with brine (5 mL), dried over anhydrous Na₂SO₄, filtered and concentrated. The crude product was purified by Prep-HPLC (Waters Xbridge (150 mm x 25 mm, 5 µm) A = H₂O (10 mM NH₄HCO₃) and B = CH₃CN; 35-55% B over 8 minutes) to give the product (26.6 mg, 73.0 µmol, 12% yield) as a solid.
**¹H NMR** (400MHz, CD₃CN) δ_{H} = 7.98 (dd, 2H), 7.88 (dd, 2H), 7.45 - 7.37 (m, 1H), 7.33 - 7.28 (m, 2H), 7.23 - 7.19 (m, 1H), 5.58 (q, 1H), 5.53 - 5.47 (m, 1H), 3.09 - 3.00 (m, 1H), 2.95 - 2.86 (m, 1H), 2.62 - 2.54 (m, 1H), 2.51 (d, 3H), 2.08 - 1.98 (m, 1H).
**LCMS** Rₜ = 1.20 min in 2.0 min chromatography, 10-80AB, MS ESI calcd. for C₁₇H₁₈ClN₂O₃S [M+H]⁺ 365.1, found 364.9.

### Example 66. Synthesis of Compound II-28

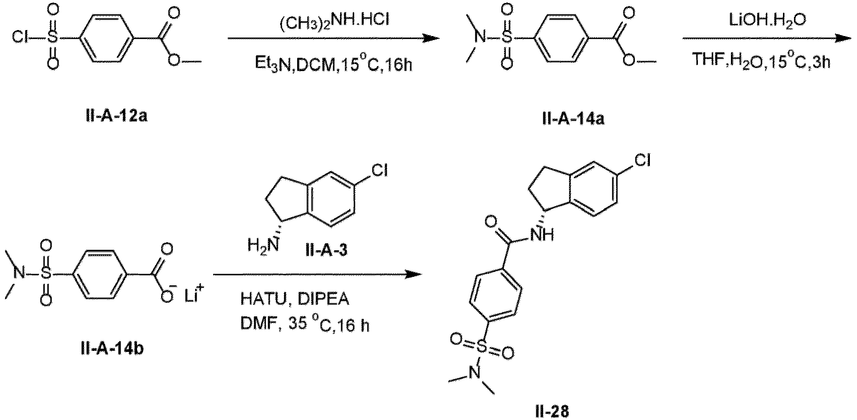

**Synthesis of II-A-14a:** A mixture of *N*-methylmethanamine hydrochloride (500 mg, 6.13 mmol) and Et₃N (2.55 mL, 18.4 mmol) and methyl 4-chlorosulfonylbenzoate (2158.39 mg, 9.2 mmol) in CH₂Cl₂ (10 mL) was stirred at 15 °C for 16 hours. The mixture was quenched with sat. NH₄Cl (10 mL) and the mixture was extracted with CH₂Cl₂ (10 mL x 2). The combined organic phase was washed with brine (10 mL), dried over anhydrous Na₂SO₄, filtered and concentrated to give the crude product (1700 mg, 5.7 mmol).
LCMS Rt = 0.73 min in 1.5 min chromatography, 5-95AB, MS ESI calcd. for C₁₀H₁₄NO₄S [M+H]⁺ 244.1, found 243.9.

**Synthesis of II-A-14b:** A mixture of methyl 4-(dimethylsulfamoyl)benzoate (500 mg, 2.06 mmol) and LiOH.HzO (172.48 mg, 4.11 mmol) in THF (8 mL) and water (4 mL). The mixture was stirred at 15 °C for 3 hours. The mixture was concentrated to give the crude product (510 mg, 2.05 mmol) as a solid.

**Synthesis of II-28:** To a mixture of (1*R*)-5-chloroindan-1-amine (100 mg, 0.60 mmol) and DIPEA (0.31 mL, 1.79 mmol) and HATU (453.63 mg, 1.19 mmol) in DMF (5 mL) was added lithium 4-(dimethylsulfamoyl)benzoate (150.43 mg, 0.66 mmol). The reaction mixture was stirred at 35 °C for 16 hours. The mixture was diluted with sat. NH₄Cl solution (5 mL) and the aqueous layer was extracted with EtOAc (5 mL x 2). The combined organic phase was washed with brine (5 mL), dried over anhydrous Na₂SO₄, filtered and concentrated. The crude product was purified by Prep-HPLC (Xtimate C18 (150 mm x 25 mm, 5 µm) A = H₂O (10 mM NH₄HCO₃) and B = CH₃CN; 35-65% B over 9 minutes) to give the product (74.0 mg, 0.19 mmol, 33% yield) as a solid.
**¹H NMR** (400MHz, CD₃CN) δ_{H} = 8.01 (d, 2H), 7.83 (d, 2H), 7.49 - 7.38 (m, 1H), 7.33 - 7.28 (m, 2H), 7.23 - 7.19 (m, 1H), 5.58 (q, 1H), 3.09 - 3.00 (m, 1H), 2.96 - 2.86 (m, 1H), 2.66 (s, 6H), 2.62 - 2.53 (m, 1H), 2.09 - 2.00 (m, 1H).
**LCMS** Rₜ = 1.25 min in 2.0 min chromatography, 10-80AB, MS ESI calcd. for C₁₈H₂₀ClN₂O₃S [M+H]⁺ 379.1, found 379.0.

### Example 67. Synthesis of Compound II-29

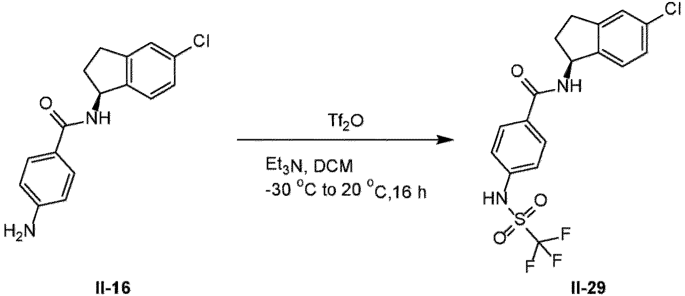

A mixture of 4-amino-*N*-[(1*S*)-5-chloroindan-1-yl]benzamide (140 mg, 0.49 mmol) and Et₃N (0.1 mL, 0.73 mmol) and Tf₂O (165.29 mg, 0.59 mmol) in DCM (8 mL) was stirred at -30 °C for 1 hour under N₂. Then the mixture was stirred at 20 °C for 16 hours. The reaction was concentrated and quenched with sat. NH₄Cl (25 mL). The mixture was extracted with DCM (25 mL x 2). The combined organic phase was washed with brine (25 mL), dried over anhydrous Na₂SO₄, filtered and concentrated. The crude product was purified by Prep-HPLC (Xtimate (150 mm x 25 mm, 5 µm) A = H₂O (0.05% NH₄OH) and B = CH₃CN; 33-63% B over 8 minutes) to give the product (17.2 mg, 0.04 mmol, 8% yield) as a solid.
**¹H NMR** (400MHz, CD₃CN) δ_{H} = 7.83 (dd, 2H), 7.40 - 7.23 (m, 5H), 7.23 - 7.16 (m, 1H), 5.56 (q, 1H), 3.06 - 2.98 (m, 1H), 2.94 - 2.83 (m, 1H), 2.59 - 2.50 (m, 1H), 2.05 - 1.99 (m, 1H).
**LCMS** Rₜ = 1.26 min in 2 min chromatography, 10-80AB, MS ESI calcd. for C₁₇H₁₅ClF₃N₂O₃S [M+H]⁺ 419.0, found 418.9.

### Example 68. Synthesis of Compound II-30

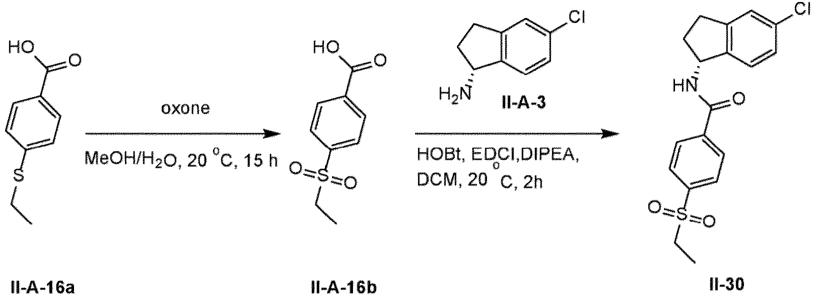

**Synthesis of II-A-16b:** To a mixture of 4-ethylsulfanylbenzoic acid (100 mg, 0.55 mmol) in methanol (3 mL) was added oxone (673.44 mg, 1.1 mmol) in water (3 mL) at 0 °C. The mixure was stirred at 20 °C for 15 hours. Methanol was evaporated under vacuum and the reaction mixture was diluted with water (20 mL). The aqueous layer was extracted with EtOAc (30 mL x 2). The combined organic phase was washed with brine (20 mL), dried over anhydrous Na₂SO₄, filtered and concentrated to give the product (90 mg, 0.42 mmol) as a solid.
**¹H NMR** (400MHz, CDCl₃) δ_{H} = 8.31 (d, 2H), 8.05 (d, 2H), 3.18 (q, 2H), 1.31 (t, 3H).

**Synthesis of II-30:** To a mixture of (1*R*)-5-chloroindan-1-amine (75.12 mg, 0.45 mmol) and HOBt (75.69 mg, 0.56 mmol) and EDCI (85.9 mg, 0.45 mmol) in DCM (3 mL) were added DIPEA (0.2 mL, 1.12 mmol) and 4-ethylsulfonylbenzoic acid (80 mg, 0.37 mmol). The mixture was stirred at 20 °C for 2 hours. The mixture was diluted with water (20 mL) and the aqueous layer was extracted with DCM (30 mL x 2). The combined organic phase was washed with brine (20 mL), dried over anhydrous Na₂SO₄, filtered and concentrated. The crude product was purified by Prep-HPLC (Waters Xbridge 150 mm x 25 mm, 5 µm, A = water (0.05% NH₄OH v/v) and B = CH₃CN; 31-61 %B over 8 minutes) to give the product (25.0 mg, 68.8 µmol, 18% yield) as a solid.
**¹H NMR** (400MHz, CDCl₃) δ_{H} = 8.03 - 7.82 (m, 4H), 7.32 - 7.27 (m, 2H), 7.24 - 7.20 (m, 1H), 6.36 (d, 1H), 5.68 (q, 1H), 3.14 (q, 2H), 3.10 - 3.10 (m, 1H), 3.01 - 2.89 (m, 1H), 2.76 - 2.70 (m, 1H), 2.04 - 1.94 (m, 1H), 1.28 (t, 3H).
**LCMS** Rₜ = 1.19 min in 2.0 min chromatography, 10-80AB, MS ESI calcd. for C₁₈H₁₉ClNO₃S [M+H]⁺364.1, found 364.0.

### Example 69. Synthesis of Compound II-31

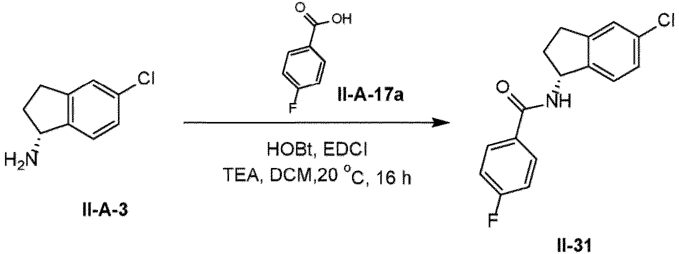

A mixture of (1*R*)-5-chloroindan-1-amine (200 mg, 1.19 mmol) and 4-fluorobenzoic acid (167.16 mg, 1.19 mmol) and HOBt (322.43 mg, 2.39 mmol) and EDCI (343.06 mg, 1.79 mmol) and TEA (603.61 mg, 5.97 mmol) in DCM (20 mL) was stirred at 20 °C for 16 hours. The mixture was diluted with H₂O (10 mL) and the aqueous layer was extracted with EtOAc (20 mL x 2). The combined organic phase was washed with brine (10 mL), dried over anhydrous Na₂SO₄, filtered and concentrated. The crude product was purified by Prep-HPLC (Kromasil (150 mm x 30 mm, 5 µm) A = H₂O (0.05% NH₄OH) and B = CH₃CN; 53-83%B over 8 minutes) to give the product (160.6 mg, 0.55 mmol, 46% yield) as a solid.
**¹H NMR** (400MHz, CD₃CN) δ_{H} = 7.91 - 7.83 (m, 2H), 7.35 - 7.24 (m, 3H), 7.23 - 7.13 (m, 3H), 5.55 (q, 1H), 3.08 - 2.98 (m, 1H), 2.94 - 2.84 (m, 1H), 2.60 - 2.50 (m, 1H), 2.07 - 1.96 (m, 1H)
**LCMS** Rₜ = 1.28 min in 2.0 min chromatography, 10-80AB, MS ESI calcd. for C₁₆H₁₄ClFNO [M+H]⁺290.1, found 289.9.

### Example 70. Synthesis of Compound II-32

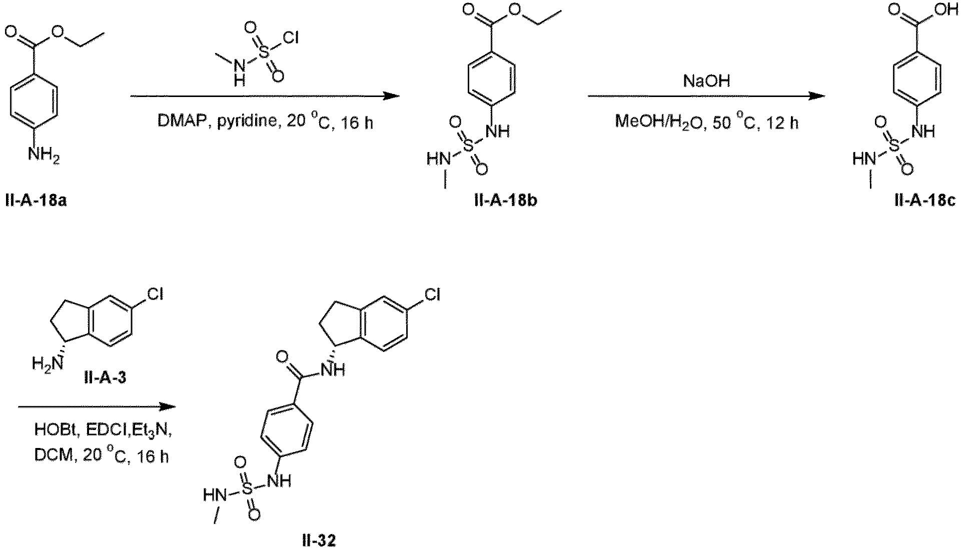

Synthesis of II-A-18b: A mixture of ethyl 4-aminobenzoate (1 g, 6.05 mmol) and DMAP (1.11 g, 9.08 mmol) in pyridine (20 mL) was added N-methylsulfamoyl chloride (1.18 g, 9.08 mmol) at 0 °C. The mixture was stirred at 20 °C for 16 hours under N₂. The mixture was concentrated and diluted with H₂O (10 mL). The aqueous layer was extracted with EtOAc (20 mL x 2). The combined organic phase was washed with brine (10 mL), dried over anhydrous Na₂SO₄, filtered and concentrated. The crude product was triturated from EtOAc (5 mL) to give the product (1100 mg, 4.12 mmol, 68% yield) as a solid.
**LCMS** Rₜ = 0.69 min in 1.5 min chromatography, 5-95AB, MS ESI calcd. for C₁₀H₁₅N₂O₄S [M+H]⁺ 259.1, found 259.0.

Synthesis of II-A-18c: To a solution of ethyl 4-(methylsulfamoylamino)benzoate (400 mg, 1.55 mmol) in methanol (10 mL) was added a solution of NaOH (247.78 mg, 6.19 mmol) in water (10 mL) slowly. The resulting mixture was stirred at 50 °C for 12 hours. The mixture was concentrated to remove MeOH under reduced pressure. 1N HCl (30 mL) was added to adjust to pH 2. The mixture was extracted with EtOAc (20 mL x 2). The combined organic phase was washed with brine (10 mL), dried over anhydrous Na₂SO₄, filtered and concentrated to give the crude product (320 mg, 1.39 mmol) as a solid.
**¹H NMR** (400MHz, DMSO-*d⁶*) δ_{H} = 12.65 (s, 1H), 10.19 (s, 1H), 7.85 (d, 2H), 7.62 - 7.48 (m, 1H), 7.21 (d, 2H), 2.44 (d, 3H)

Synthesis of II-32: A mixture of 4-(methylsulfamoylamino)benzoic acid (123.61 mg, 0.54 mmol) and HOBt (161.21 mg, 1.19 mmol) and Et₃N (0.25 mL, 1.79 mmol) and EDCI (171.53 mg, 0.89 mmol) and (1*R*)-5-chloroindan-1-amine (100 mg, 0.6 mmol) in DCM (10 mL) was stirred at 20 °C for 16 hours under N₂. The mixture was quenched with sat. NH₄Cl (20 mL) and the mixture was extracted with DCM (20 mL x 2). The combined organic phase was washed with brine (20 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 40-70% B over 8 minutes) to give the product (11.4 mg, 0.03 mmol, 5% yield) as a solid.
**¹H NMR** (400MHz, CD₃CN) δ_{H} = 7.94 (br s, 1H), 7.82 (d, 2H), 7.30 (d, 2H), 7.27 - 7.18 (m, 4H), 5.65-5.52 (m, 2H), 3.11 - 2.99 (m, 1H), 2.98 - 2.85 (m, 1H), 2.63 - 2.50 (m, 4H), 2.08 - 2.00 (m, 1H).
**LCMS** Rₜ = 1.14 min in 2.0 min chromatography, 10-80AB, MS ESI calcd. for C₁₇H₁₉ClN₃O₃S [M +H]⁺ 380.1, found 380.0.

### Example 71. Synthesis of Compound II-33

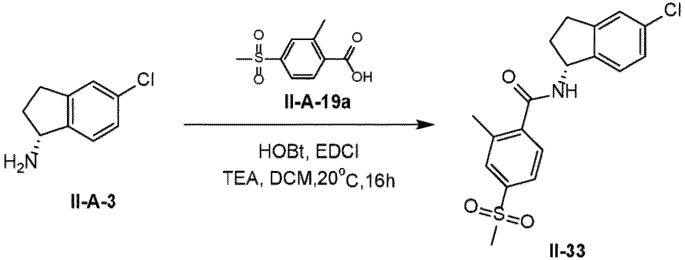

A mixture of (1*R*)-5-chloroindan-1-amine (78.25 mg, 0.47 mmol), 2-methyl-4-methylsulfonyl-benzoic acid (100 mg, 0.47 mmol) and HOBt (126.15 mg, 0.93 mmol) and EDCI (134.22 mg, 0.70 mmol) and TEA (236.16 mg, 2.33 mmol) in DCM (20 mL) was stirred at 20 °C for 16 hours. The mixture was diluted with H₂O (10 mL) and the aqueous layer was extracted with EtOAc (20 mL x 2). The combined organic phase was washed with brine (10 mL), dried over anhydrous Na₂SO₄, filtered and concentrated. The crude product was purified by Prep-HPLC (Kromasil (150 mm x 30 mm, 5 µm) A = H₂O (0.05% NH₄OH) and B = CH₃CN; 45-75% B over 8 minutes) to give the product (73.0 mg, 0.20 mmol, 43% yield) as a solid.
**¹H NMR** (400MHz, DMSO-*d*⁶) δ_{H} = 8.88 (d, 1H), 7.83 (s, 1H), 7.79 (d, 1H), 7.58 (d, 1H), 7.33 (d, 2H), 7.29 - 7.25 (m, 1H), 5.47 (q, 1H), 3.22 (s, 3H), 3.03 - 2.92 (m, 1H), 2.90 - 2.79 (m, 1H), 2.50- 2.40 (m, 4H), 2.01 - 1.89 (m, 1H).
**LCMS** Rₜ = 1.17 min in 2.0 min chromatography, 10-80AB, MS ESI calcd. for C₁₈H₁₉ClNO₃S [M+H]⁺364.1, found 363.9.

### Example 72. Synthesis of Compound II-34

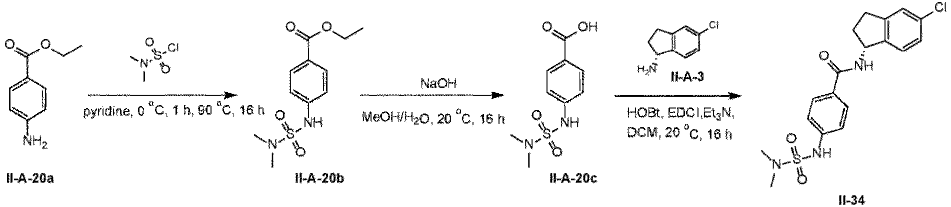

**Synthesis of II-A-20b:** A mixture of ethyl 4-aminobenzoate (500 mg, 3.03 mmol) in pyridine (20 mL) was added *N*,*N*-dimethylsulfamoyl chloride (1303.86 mg, 9.08 mmol) at 0 °C for 1 hour. Then the mixture was stirred at 90 °C for 16 hours under N₂. The mixture was concentrated and diluted with H₂O (10 mL). The aqueous layer was extracted with EtOAc (20 mL x 2). The combined organic phase was washed with brine (10 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 35-65% B over 8 minutes) to give the product (500 mg, 1.84 mmol, 61% yield) as a solid.
**LCMS Rt** = 0.75 min in 1.5 min chromatography, 5-95AB, MS ESI calcd. for C₁₁H₁₇N₂O₄S [M+H]⁺ 273.1, found 273.2.

**Synthesis of II-A-20c:** To a solution of NaOH (88.13 mg, 2.2 mmol) in water (10 mL) was added ethyl 4-(dimethylsulfamoylamino)benzoate (150 mg, 0.55 mmol) in MeOH (10 mL) at 20 °C. The resulting mixture was stirred at 20 °C for 16 hours. The mixture was concentrated and water (20 mL) was added. The aqueous layer was extracted with EtOAc (20 mL x 2). The combined organic phase was washed with 1N HCl (20 mL x 4) and water (20 mL x 2) and brine (20 mL x 2), dried over anhydrous Na₂SO₄, filtered and concentrated to give the crude product (105 mg, 0.41 mmol) as a solid, which was used directly for next step without further purification.
**LCMS** Rₜ = 0.55 min in 1.5 min chromatography, 5-95AB, MS ESI calcd. for C₉H₁₃N₂O₄S [M +H]+ 245.1, found 244.9.

**Synthesis of Compound II-34:** A mixture of 4-(dimethylsulfamoylamino)benzoic acid (118.03 mg, 0.48 mmol) and HOBt (145.09 mg, 1.07 mmol) and Et₃N (0.22 mL, 1.61 mmol) and EDCI (154.38 mg, 0.81 mmol) and (1*R*)-5-chloroindan-1-amine (90 mg, 0.54 mmol) in DCM (10 mL) was stirred at 20 °C for 16 hours under N₂. The reaction was quenched with sat. NH₄Cl (20 mL) and the mixture was extracted with DCM (20 mL x 2). The combined organic phase was washed with brine (20 mL), dried over anhydrous Na₂SO₄, filtered and concentrated. The crude product was purified by prep-HPLC (Boston Prime (150 mm x 30 mm, 5 µm) A = H₂O (0.05% NH₄OH) and B = CH₃CN; 40-70% B over 8 minutes) to give the product (86.5 mg, 0.22 mmol, 41% yield) as a solid.
**¹H NMR** (400MHz, CD₃CN) δ_{H} = 7.91 (br s, 1H), 7.77 (d, 2H), 7.32 - 7.23 (m, 4H), 7.23-7.12 (m, 2H), 5.55 (q, 1H), 3.08 - 2.97 (m, 1H), 2.95 - 2.83 (m, 1H), 2.77 (s, 6H), 2.61 - 2.48 (m, 1H), 2.05 - 1.97 (m, 1H).
**LCMS** Rₜ = 1.19 min in 2.0 min chromatography, 10-80AB, MS ESI calcd. for C₁₈H₂₁ClN₃O₃S [M +H]⁺ 394.1, found 394.0.

### Example 73. Synthesis of Compound II-35

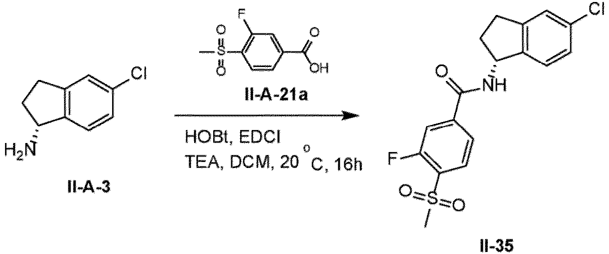

A mixture of (1*R*)-5-chloroindan-1-amine (76.83 mg, 0.46 mmol), 3-fluoro-4-methylsulfonyl-benzoic acid (100 mg, 0.46 mmol) and HOBt (123.86 mg, 0.92 mmol) and EDCI (131.78 mg, 0.69 mmol) and TEA (231.87 mg, 2.29 mmol) in DCM (20 mL) was stirred at 20 °C for 16 hours. The mixture was concentrated and diluted with H₂O (10 mL). The aqueous layer was extracted with EtOAc (20 mL x 2). The combined organic phase was washed with brine (10 mL), dried over anhydrous Na₂SO₄, filtered and concentrated. The crude product was purified by Prep-HPLC (Kromasil (150 mm x 30 mm, 5 µm) A = H₂O (0.05% NH₄OH) and B = CH₃CN; 46-76 %B over 8 minutes) to give the product (60.0 mg, 0.16 mmol, 36% yield) as a solid.
**¹H NMR** (400MHz, DMSO-*d⁶*) δ_{H} = 9.11 (d, 1H), 8.05 - 7.89 (m, 3H), 7.36 (s, 1H), 7.29 - 7.22 (m, 2H), 5.51 (q, 1H), 3.37 (s, 3H), 3.05 - 2.96 (m, 1H), 2.92 - 2.82 (m, 1H), 2.49 - 2.44 (m, 1H), 2.08 - 1.95 (m, 1H).
**LCMS** Rₜ = 1.19 min in 2.0 min chromatography, 10-80AB, MS ESI calcd. for C₁₇H₁₆ClFNO₃S [M+H]⁺ 368.0, found 368.0.

### Example 74. Synthesis of Compound II-36

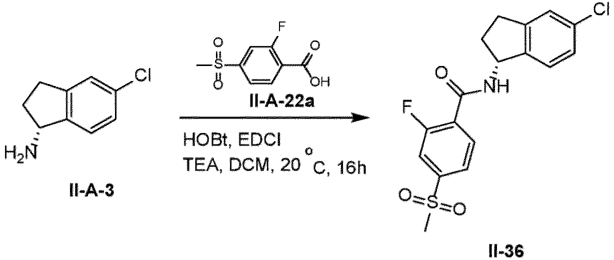

A mixture of (1*R*)-5-chloroindan-1-amine (76.83 mg, 0.46 mmol), 2-fluoro-4-methylsulfonyl-benzoic acid (100 mg, 0.46 mmol) and HOBt (123.86 mg, 0.92 mmol) and EDCI (131.78 mg, 0.69 mmol) and TEA (231.87 mg, 2.29 mmol) in DCM (20 mL) was stirred at 20 °C for 16 hours. The mixture was diluted with H₂O (10 mL) and the aqueous layer was extracted with EtOAc (20 mL x 2). The combined organic phase was washed with brine (10 mL), dried over anhydrous Na₂SO₄, filtered and concentrated. The crude product was purified by Prep-HPLC (Kromasil (150 mm x 30 mm, 5 µm) A = H₂O (0.05% NH₄OH) and B = CH₃CN; 45-75 %B over 8 minutes) to give the product (56.2 mg, 0.15 mmol, 33% yield) as a solid.
**¹H NMR** (400MHz, CD₃OD) δ_{H} = 7.94 - 7.78 (m, 3H), 7.34 - 7.27 (m, 2H), 7.24 - 7.20 (m, 1H), 5.59 (t, 1H), 3.18 (s, 3H), 3.10 - 3.01 (m, 1H), 2.97 - 2.88 (m, 1H), 2.67 - 2.58 (m, 1H), 2.08 - 1.98 (m, 1H).
**LCMS** Rₜ = 1.18 min in 2.0 min chromatography, 10-80AB, MS ESI calcd. for C₁₇H₁₆ClFNO₃S [M+H]⁺368.0, found 368.0.

### Example 75. Synthesis of Compound II-37

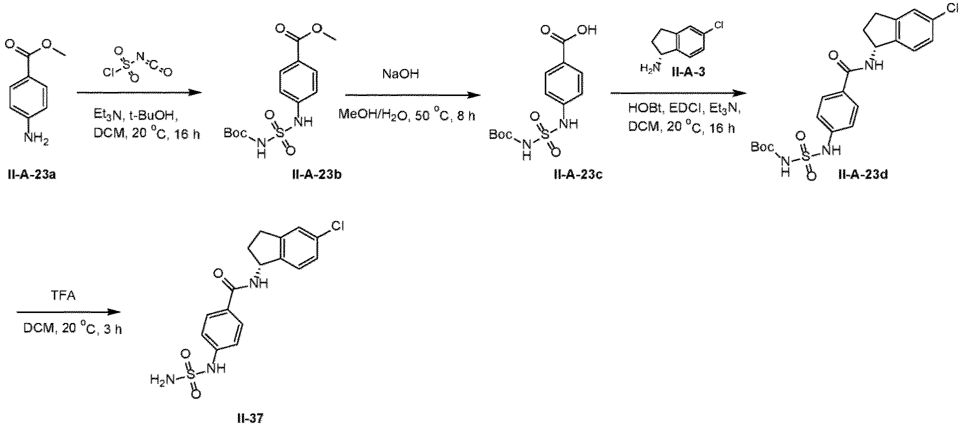

**Synthesis of II-A-23b:** To a solution of CSI (500 mg, 3.53 mmol) and t-BuOH (261.85 mg, 3.53 mmol) in DCM (20 mL) was added methyl 4-aminobenzoate (534.01 mg, 3.53 mmol). The mixture was stirred at 20 °C for 16 hours. The reaction was quenched with sat. NH₄Cl solution (15 mL). The mixture was extracted with EtOAc (20 mL x 2). The combined organic phase was washed with brine (20 mL), dried over anhydrous Na₂SO₄, filtered and concentrated to give the crude product (780 mg, 1.39 mmol) as a solid.
**LCMS** Rₜ = 2.04 min in 4 min chromatography, 10-80AB, MS ESI calcd. for C₁₃H₁₈N₂O₆SNa [M +Na]⁺ 353.1, found 352.9.

**Synthesis of II-A-23c:** To a solution of methyl 4-(tert-butoxysulfamoylamino)benzoate (550 mg, 1.82 mmol) in methanol (10 mL) and water (10 mL) was added NaOH (218.29 mg, 5.46 mmol). The mixture was stirred at 50 °C for 8 h. The mixture was diluted with sat. NH₄Cl solution (15 mL) and the aqueous layer was extracted with EtOAc (20 mL x 2). The combined organic phase was washed with brine (10 mL), dried over anhydrous Na₂SO₄, filtered and concentrated. The aqueous phase was concentrated then the mixture was extracted with EtOAc (20 mL x 2) to give the crude product (400 mg, 1.38 mmol) as a solid.
**¹H NMR** (400MHz, DMSO-*d*⁶) δ_{H} = 12.65 (br s, 1H), 11.52 (s, 1H), 10.83 (s, 1H), 7.88 (d, 2H), 7.23 (d, 2H), 1.31 (s, 9H).

**Synthesis of II-A-23d:** A mixture of 4-(tert-butoxycarbonylsulfamoylamino)benzoic acid (400 mg, 1.26 mmol) and HOBt (341.74 mg, 2.53 mmol) and Et₃N (0.52 mL, 3.79 mmol) and EDCI (363.61 mg, 1.9 mmol) and (1*R*)-5-chloroindan-1-amine (211.98 mg, 1.26 mmol) in DCM (30 mL) was stirred at 20 °C for 16 hours under N₂. The reaction was quenched with sat. NH₄Cl (20 mL) and the mixture was extracted with DCM (20 mL x 2). The combined organic phase was washed with brine (20 mL), dried over anhydrous Na₂SO₄, filtered and concentrated. The crude product was triturated from DCM (1 mL) to give the product (180 mg, 0.39 mmol, 31% yield) as a solid.
**LCMS** Rₜ = 0.88 min in 1.5 min chromatography, 5-95AB, MS ESI calcd. for C₂₁H₂₅ClN₃O₅S [M+H]+ 466.1, found 466.2.

**Synthesis of II-37:** To a solution of tert-butyl *N*-[[4-[[(1*R*)-5-chloroindan-1-yl]carbamoyl]phenyl]sulfamoyl]carbamate (60 mg, 0.13 mmol) in DCM (15 mL) was added TFA (5 mL, 20 mmol). The mixture was stirred at 20 °C for 3 h. The reaction was quenched with sat. Na₂CO₃ solution (15 mL) and concentrated. The mixture was extracted with DCM (15 mL x 2) and the organic layer was concentrated. The crude product was triturated by DCM (1 mL) to give the product (36.4 mg, 0.1 mmol, 77% yield) as a solid.
**¹H NMR** (400MHz, CD₃CN) δ_{H} = 7.97 (s, 1H), 7.80 (d, 2H), 7.31 - 7.17 (m, 6H), 5.63 (s, 2H), 5.56 (q, 1H), 3.09 - 2.96 (m, 1H), 2.95 - 2.82 (m, 1H), 2.61 - 2.49 (m, 1H), 2.05 - 1.97 (m, 1H).
**LCMS** Rₜ = 1.07 min in 2.0 min chromatography, 10-80AB, MS ESI calcd. for C₁₆H₁₇ClN₃O₃S [M+H]⁺ 366.1, found 366.0.

### Example 76. Synthesis of Compound II-38

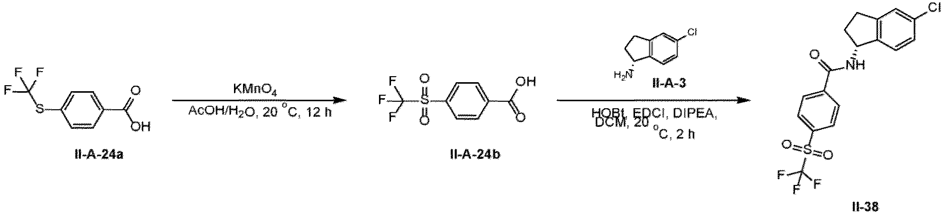

**Synthesis of II-A-24b:** A mixture of KMnO₄ (2000 mg, 12.66 mmol) and 4-(trifluoromethylsulfanyl)benzoic acid (500 mg, 2.25 mmol) in acetic acid (15 mL) and water (5 mL) was stirred at 20 °C for 12 hours. The mixture was diluted with water (20 mL) and the aqueous layer was extracted with EtOAc (20 mL x 2). The combined organic phase was washed with water (20 mL x 2) and brine (20 mL), dried over anhydrous Na₂SO₄, filtered and concentrated to give the product (500 mg, 1.97 mmol) as a solid.
**¹H NMR** (400MHz, CD₃OD) δ_{H} = 8.37 (d, 2H), 8.20 (d, 2H).

**Synthesis of II-38:** To a mixture of DIPEA (0.31 mL, 1.79 mmol) and 4-(trifluoromethylsulfonyl)benzoic acid (151.62 mg, 0.60 mmol) and HOBt (161.21 mg, 1.19 mmol) in DCM (3 mL) were added EDCI (171.53 mg, 0.89 mmol) and (1*R*)-5-chloroindan-1-amine (100 mg, 0.60 mmol). The mixture was stirred at 20 °C for 2 hours. The mixture was diluted with water (20 mL) and the aqueous layer was extracted with EtOAc (20 mL x 2). The combined organic phase was washed with water (20 mL x 2) and brine (20 mL), dried over anhydrous Na₂SO₄, filtered and concentrated. The crude product was purified by flash chromatography on silica gel (EtOAc in PE = 0% to 30%) to give the product (59.9 mg, 145.6 µmol, 24% yield) as a solid.
**¹H NMR** (400MHz, CDCl₃) δ_{H} = 8.16 - 8.10 (m, 2H), 8.09 - 8.03 (m, 2H), 7.29 - 7.27 (m, 2H), 7.24 - 7.20 (m, 1H), 6.40 (d, 1H), 5.67 (q, 1H), 3.11 - 3.01 (m, 1H), 3.00 - 2.90 (m, 1H), 2.78-2.72 (m, 1H), 2.02-1.93 (m, 1H).
**LCMS** Rₜ = 1.32 min in 2.0 min chromatography, 10-80AB, MS ESI calcd. for C₁₇H₁₄ClF₃NO₃S [M+H]⁺404.0, found 404.0.

### Example 77. Synthesis of Compound II-39

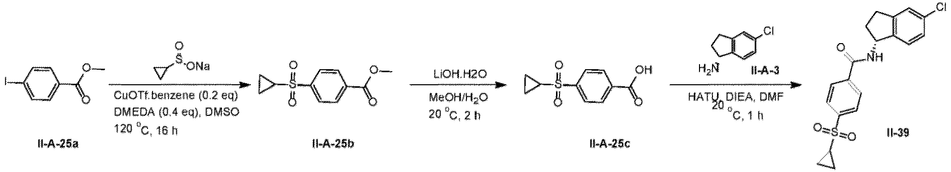

**Synthesis of II-A-25b:** A mixture of methyl 4-iodobenzoate (750 mg, 2.86 mmol) and cyclopropylsulfinyloxysodium (440.07 mg, 3.43 mmol) and copper(I) trifluoromethanesulfonate benzene complex (144.06 mg, 0.57 mmol) and *N,N-*dimethylethane-1,2-diamine (100.92 mg, 1.14 mmol) in DMSO (5 mL) was stirred at 120 °C under N₂ for 16 hours. After cooling to room temperature, the mixture was diluted with H₂O (30 mL) and EtOAc (50 mL). The layers were separated and the aqueous layer was extracted with EtOAc (30 mL). The combined organic phase was washed with brine (20 x 2 mL), dried over anhydrous Na₂SO₄, filtered and concentrated. The crude product was purified by flash chromatography on silica gel (EtOAc in PE = 0% to 15% to 30%) to give the product (480 mg, 1.99 mmol, 70% yield) as a solid.
**¹H NMR** (400MHz, CDCl₃) δ_{H} = 8.22 (dd, 2H), 7.98 (dd, 2H), 3.98 (s, 3H), 2.56-2.43 (m, 1H), 1.43 - 1.35 (m, 2H), 1.12 - 1.03 (m, 2H).

**Synthesis of II-A-25c:** To a solution of methyl 4-cyclopropylsulfonylbenzoate (80 mg, 0.33 mmol) in methanol (2 mL) and water (2 mL) was added LiOH.H₂O (41.91 mg, 1 mmol). The resulting mixture was stirred at 20 °C for 2 hours. 1N HCl aqueous (10 mL) was added and the aqueous layer was extracted with EtOAc (20 mL x 3). The combined organic phase was washed with brine (10 mL), dried over anhydrous Na₂SO₄, filtered and concentrated to give the crude product (75 mg, 331.5 µmol) as a solid.
**¹H NMR** (400MHz, DMSO-*d*⁶) δ_{H} = 13.50 (br s, 1H), 8.17 (d, 2H), 8.02 (d, 2H), 2.98 - 2.88 (m, 1H), 1.19 - 1.03 (m, 4H).

**Syntheis of Compound II-39:** A mixture of 4-cyclopropylsulfonylbenzoic acid (75 mg, 0.33 mmol) and HATU (252.09 mg, 0.66 mmol) and DIPEA (0.17 mL, 0.99 mmol) and (1*R*)-5-chloroindan-1-amine (55.57 mg, 0.33 mmol) in DMF (5 mL) was stirred at 20 °C for 1 hour. Water (20 mL) was added and the aqueous layer was extracted with EtOAc (20 mL x 2). The combined organic phase was washed with brine (20 mL x 2), dried over anhydrous Na₂SO₄, filtered and concentrated. The crude product was purified by flash chromatography on silica gel (EtOAc in PE = 0% to 10% to 30%) to give the product (72.0 mg, 191.4 µmol, 58% yield) as a solid.
**¹H NMR** (400MHz, CD₃CN) δ_{H} = 8.02 - 7.98 (m, 2H), 7.98 - 7.93 (m, 2H), 7.45 (d, 1H), 7.33 - 7.27 (m, 2H), 7.23 - 7.18 (m, 1H), 5.57 (q, 1H), 3.10 - 2.99 (m, 1H), 2.96 - 2.85 (m, 1H), 2.64 - 2.52 (m, 2H), 2.09 - 1.98 (m, 1H), 1.24 - 1.18 (m, 2H), 1.08 - 1.01 (m, 2H).
**LCMS** Rₜ = 1.22 min in 2.0 min chromatography, 10-80AB, MS ESI calcd. for C₁₉H₁₉ClNO₃S [M+H]⁺376.1, found 376.0.

### Example 78. Synthesis of Compound II-40

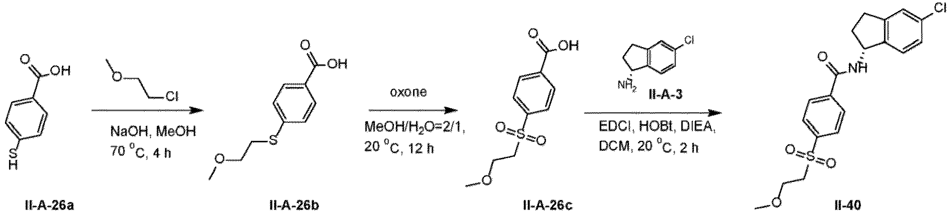

**Synthesis of II-A-26b:** To a mixture of 4-sulfanylbenzoic acid (140 mg, 0.91 mmol) in methanol (3 mL) was added NaOH (108.96 mg, 2.72 mmol) and 1-chloro-2-methoxy-ethane (171.68 mg, 1.82 mmol) at 20 °C. The reaction mixture was stirred at 70 °C for 4 hours. After cooling to room temperature, the mixture was quenched by addition of 1 M HCl (20 mL). The mixture was extracted with EtOAc (20 mL x 2). The combined organic phase was washed with brine (20 mL), dried over anhydrous Na₂SO₄, filtered and concentrated to give the product (140 mg, 659.5 µmol) as a solid.
**¹H NMR** (400MHz, DMSO-*d⁶*) δ_{H} = 12.88 (br s, 1H), 7.84 (d, 2H), 7.40 (d, 2H), 3.56 (t, 2H), 3.29 - 3.21 (m, 5H).

**Synthesis of II-A-26c:** A mixture of oxone (2081.41 mg, 3.39 mmol) and 4-(2-methoxyethylsulfanyl)benzoic acid (120 mg, 0.57 mmol) in water (4 mL) and methanol (6 mL) was stirred at 20 °C for 12 hours. The mixture was diluted with water (20 mL) and the aqueous layer was extracted with EtOAc (20 mL x 2). The combined organic phase was washed with 1M HCl (20 mL) and water (20 mL x 2) and brine (20 mL), dried over anhydrous Na₂SO₄, filtered and concentrated to give the product (140 mg, 573.2 µmol) as a solid.
**¹H NMR** (400MHz, DMSO-*d⁶*) δ_{H} = 13.55 (br s, 1H), 8.15 (d, 2H), 8.01 (d, 2H), 3.70 - 3.60 (m, 4H), 3.06 (s, 3H).

**Synthesis of II-40:** To a mixture of HOBt (14.51 mg, 0.11 mmol) and EDCI (18.87 mg, 0.10 mmol) and 4-(2-methoxyethylsulfonyl)benzoic acid (24.04 mg, 0.10 mmol) in DCM (6 mL) were added DIPEA (0.05 mL, 0.27 mmol) and (1*R*)-5-chloroindan-1-amine (15 mg, 0.09 mmol). The mixure was stirred at 20 °C for 2 hours. The mixture was diluted with water (20 mL) and the aqueous layer was extracted with EtOAc (20 mL x 2). The combined organic phase was washed with 1M HCl (10 mL) and water (20 mL x 2) and brine (20 mL), dried over anhydrous Na₂SO₄, filtered and concentrated. The crude product was purified by Prep-HPLC (Waters Xbridge 150 mm x 25 mm, 5 µm), A = H₂O (10 mM NH₄HCO₃)-ACN) and B = CH₃CN; 36-66 %B over 7 minutes) to give the product (78.6 mg, 197.5 µmol) as a solid.
**¹H NMR** (400MHz, CD₃CN) δ_{H} = 8.02 - 7.92 (m, 4H), 7.54-7.40 (m, 1H), 7.34 - 7.28 (m, 2H), 7.23 - 7.15 (m, 1H), 5.58 (q, 1H), 3.67 (t, 2H), 3.44 (t, 2H), 3.13 (s, 3H), 3.08 - 3.00 (m, 1H), 2.96 - 2.86 (m, 1H), 2.63-2.50 (m, 1H), 2.08 - 2.00 (m, 1H).
**LCMS** Rₜ = 1.18 min in 2.0 min chromatography, 10-80AB, MS ESI calcd. for C₁₉H₂₁ClNO₄S [M+H]⁺ 394.1, found 393.9.

### Example 79. Synthesis of Compounds II-41 and II-31

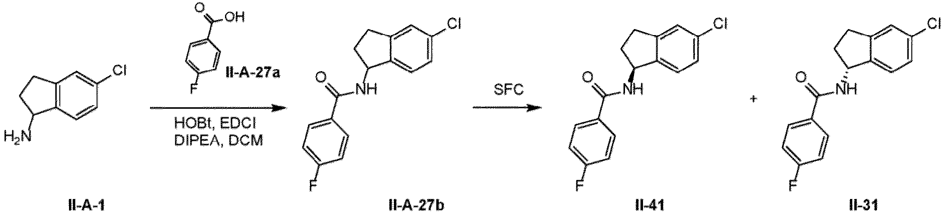

**Synthesis of II-A-27b:** A mixture of 4-fluorobenzoic acid (83.58 mg, 0.60 mmol) and HOBt (161.21 mg, 1.19 mmol) and EDCI (171.53 mg, 0.89 mmol) and TEA (301.81 mg, 2.98 mmol) and 5-chloroindan-1-amine (100 mg, 0.60 mmol) in DCM (20 mL) was stirred at 20 °C for 16 hours. The mixture was diluted with H₂O (10 mL) and the aqueous layer was extracted with EtOAc (20 mL x 2). The combined organic phase was washed with brine (10 mL), dried over anhydrous Na₂SO₄, filtered and concentrated to give the crude product (110 mg, 0.37 mmol, 62% yield) as a solid.
**LCMS** Rt = 0.88 min in 2.0 min chromatography, 10-80AB, MS ESI calcd. for C₁₆H₁₄ClFNO [M+H]⁺ 290.1, found 290.0.
**Analytical SFC:** (Chiralcel OD-3 150 mm x 4.6 mm I.D., 3µm, Mobile phase: A: CO₂ B: ethanol (0.05% DEA), Gradient: from 5% to 40% of B in 5min and from 40% to 5% of B in 0.5 min, hold 5% of B for 1.5 min. Flow rate: 2.5 mL/min, Column temp.: 35°C) showed two peaks at 3.18 min and 3.81 min.

**Synthesis of Compounds II-41 and II-31:** The product (110 mg, 0.37 mmol) was purified by SFC (DAICEL CHIRALCEL OD-H (250 mm x 30 mm, 5µm); A = CO₂ and B = EtOH (0.1% NH₃.H₂O); 38 °C; 50 mL/min; 30% B; 9 min run; 7 injections) to give the enantiomer 1, assigned as Compound II-31 (32 mg, 0.11 mmol) (Rₜ of Peak 1= 3.18 min) as a solid and enantiomer 2, assigned as Compound II-41 (28.0 mg, 0.10 mmol) (Rt of Peak 2= 3.81 min) as a solid. Stereochemistry is randomly assigned.

### Compound II-31:

**¹H NMR** (400MHz, CD₃CN) δ_{H} = 7.91 - 7.82 (m, 2H), 7.35 - 7.22 (m, 3H), 7.22 - 7.14 (m, 3H), 5.55 (q, 1H), 3.07 - 2.98 (m, 1H), 2.94 - 2.84 (m, 1H), 2.60 - 2.50 (m, 1H), 2.06 - 1.97 (m, 1H)
**LCMS** Rₜ = 1.28 min in 2.0 min chromatography, 10-80AB, MS ESI calcd. for C₁₆H₁₄ClFNO [M+H]⁺ 290.1, found 289.9.

### Compound II-41:

**¹H NMR** (400MHz, CD₃CN) δ_{H} = 7.91 - 7.82 (m, 2H), 7.35 - 7.22 (m, 3H), 7.22 - 7.14 (m, 3H), 5.55 (q, 1H), 3.07 - 2.98 (m, 1H), 2.94 - 2.84 (m, 1H), 2.60 - 2.50 (m, 1H), 2.06 - 1.97 (m, 1H)
**LCMS** Rₜ = 1.28 min in 2.0 min chromatography, 10-80AB, MS ESI calcd. for C₁₆H₁₄ClFNO [M+H]⁺ 290.1, found 289.9.

### Example 80. Synthesis of Compound II-42

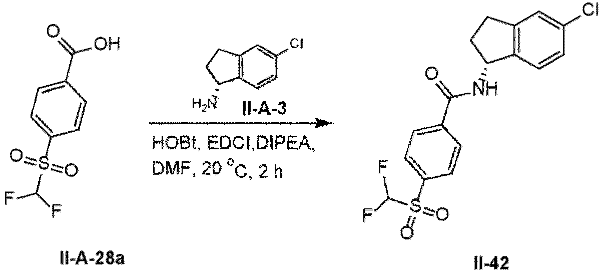

To a mixture of HOBt (58.04 mg, 0.43 mmol) and EDCI (75.47 mg, 0.39 mmol) and 4-(difluoromethylsulfonyl)benzoic acid (92.99 mg, 0.39 mmol) in DMF (3 mL) were added DIPEA (0.19 mL, 1.07 mmol) and (1*R*)-5-chloroindan-1-amine (60 mg, 0.36 mmol). The mixture was stirred at 20 °C for 2 hours. The mixture was diluted with water (20 mL) and the aqueous layer was extracted with EtOAc (30 mL x 2). The combined organic phase was washed with brine (20 mL), dried over anhydrous Na₂SO₄, filtered and concentrated. The crude product was purified by Prep-HPLC (Waters Xbridge (150 mm x 25 mm, 5 µm, A = H₂O (10mM NH₄HCO₃) and B = CH₃CN; 45-75 %B over 7 minutes) to give the product (56.3 mg, 146.0 µmol, 41% yield) as a solid.
**¹H NMR** (400MHz, CD₃CN) δ_{H} = 8.11 - 8.01 (m, 4H), 7.58-7.43 (m, 1H), 7.35 - 7.26 (m, 2H), 7.24 - 7.18 (m, 1H), 6.57 (t, 1H), 5.58 (q, 1H), 3.09 - 3.00 (m, 1H), 2.96 - 2.85 (m, 1H), 2.62-2.52 (m, 1H), 2.09 - 1.99 (m, 1H).
**LCMS** Rₜ = 1.24 min in 2.0 min chromatography, 10-80AB, MS ESI calcd. for C₁₇H₁₅ClF₂NO₃S [M+H]⁺386.0, found 385.9.

### Example 81. Synthesis of Compound II-43

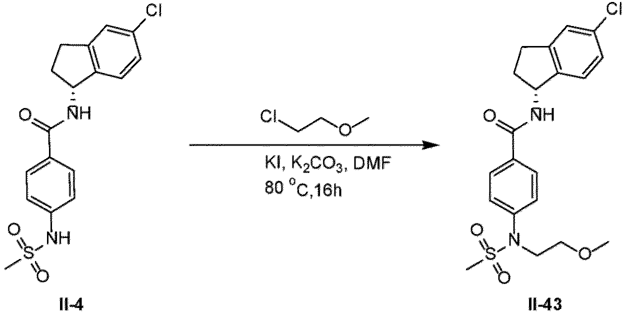

To a mixture of *N*-[(1*R*)-5-chloroindan-1-yl]-4-(methanesulfonamido)benzamide (100 mg, 0.27 mmol) and 1-chloro-2-methoxy-ethane (116.6 mg, 1.23 mmol) and KI (13.65 mg, 0.08 mmol) in DMF (6 mL) was added K₂CO₃ (113.64 mg, 0.82 mmol) at 20 °C. The mixture was stirred under N₂ at 80 °C for 16 hours. After cooling to room temperature, the mixture was concentrated to give the residue. The residue was diluted with H₂O (30 mL) and the mixture was extracted with EtOAc (10 mL x 2). The combined organic phase was washed with brine (10 mL), dried over anhydrous Na₂SO₄, filtered and concentrated. The crude product was purified by Prep-HPLC (Boston Prime (150 mm x 30 mm x 5 µm) A = H₂O (0.05% NH₄OH) and B = CH₃CN; 39-69%B over 9 minutes) to give the product (65.5 mg, 0.16 mmol, 57% yield) as a solid.
**¹H NMR** (400MHz, CD₃CN) δ_{H} = 7.84 (dd, 2H), 7.43 (dd, 2H), 7.35 - 7.25 (m, 3H), 7.22 - 7.17 (m, 1H), 5.56 (q, 1H), 3.83 (t, 2H), 3.37 (t, 2H), 3.23 (s, 3H), 3.07 - 2.98 (m, 1H), 2.96 (s, 3H), 2.93 - 2.84 (m, 1H), 2.60 - 2.50 (m, 1H), 2.07 - 1.97 (m, 1H).
**LCMS** Rₜ = 1.19 min in 2.0 min chromatography, 10-80AB, MS ESI calcd. for C₂₀H₂₄ClN₂O₄S [M+H]⁺ 423.1, found 423.0.

### Example 82. Synthesis of Compound II-44

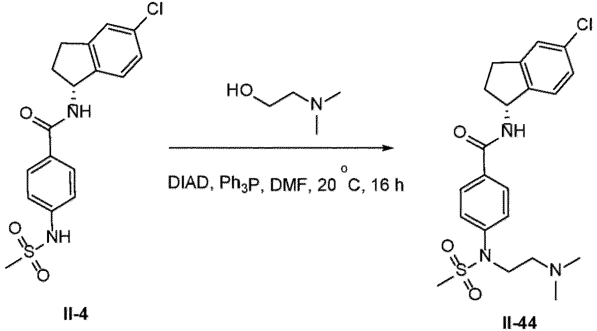

To a mixture of*N*-[(1*R*)-5-chloroindan-1-yl]-4-(methanesulfonamido)benzamide (100 mg, 0.27 mmol) and 2-(dimethylamino)ethanol (73.3 mg, 0.82 mmol) and Ph₃P (143.79 mg, 0.55 mmol) in THF (10 mL) was added DIAD (110.85 mg, 0.55 mmol) at 0 °C. The mixture was stirred under N₂ at 20 °C for 16 hours. The mixture was concentrated to dryness and diluted with H₂O (10 mL). The mixture was extracted with EtOAc (10 mL x 2). The combined organic phase was washed with brine (10 mL), dried over anhydrous Na₂SO₄, filtered and concentrated. The crude product was purified by Prep-HPLC (Boston Prime C18 (150 mm x 30 mm x 10 um) A = H₂O (0.05% ammonia hydroxide) and B = CH₃CN; 45-55%B over 9 minutes) to give the product (23.7 mg, 0.05 mmol, 20% yield) as a solid.
**¹H NMR** (400MHz, CD₃CN) δ_{H} = 7.86 (dd, 2H), 7.45 (dd, 2H), 7.35 - 7.25 (m, 3H), 7.24 - 7.16 (m, 1H), 5.57 (q, 1H), 3.76 (t, 2H), 3.09 - 2.99 (m, 1H), 2.97 (s, 3H), 2.94 - 2.84 (m, 1H), 2.62 - 2.50 (m, 1H), 2.29 (t, 2H), 2.14 (s, 6H), 2.07 - 1.98 (m, 1H).
**LCMS** Rₜ = 0.96 min in 2.0 min chromatography, 10-80AB, MS ESI calcd. for C₂₁H₂₇ClN₃O₃S [M+H]⁺ 436.1, found 436.1.

### Example 83. Synthesis of Compound II-45

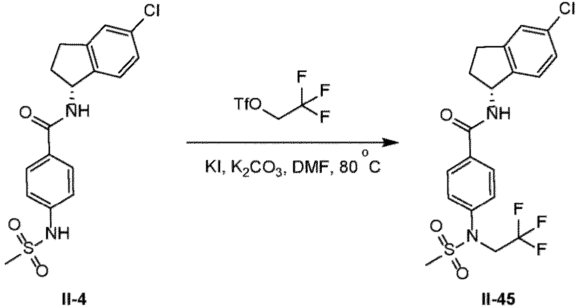

To a mixture of *N*-[(1*R*)-5-chloroindan-1-yl]-4-(methanesulfonamido)benzamide (100 mg, 0.27 mmol), 2,2,2-trifluoroethyl trifluoromethanesulfonate (318.08 mg, 1.37 mmol) and KI (22.75 mg, 0.14 mmol) in DMF (6 mL) was added K₂CO₃ (113.64 mg, 0.82 mmol) at 20 °C. The mixture was stirred under N₂ at 80 °C for 16 hours. After cooling to room temperature, the mixture was concentrated under reduced pressure. The residue was diluted with H₂O (30 mL) and the mixture was extracted with EtOAc (10 mL x 2). The combined organic phase was washed with brine (10 mL), dried over anhydrous Na₂SO₄, filtered and concentrated. The crude product was triturated from i-Pr₂O (10 mL) to give the product (96.3 mg, 0.22 mmol, 79% yield) as a solid.
**¹H NMR** (400MHz, CD₃CN) δ_{H} = 7.89 (d, 2H), 7.52 (d, 2H), 7.38-7.25 (m, 3H), 7.23 - 7.17 (m, 1H), 5.56 (q, 1H), 4.40 (q, 2H), 3.15 - 2.96 (m, 4H), 2.94 - 2.84 (m, 1H), 2.61 - 2.50 (m, 1H), 2.07 - 1.97 (m, 1H).
**LCMS** Rₜ = 1.26 min in 2.0 min chromatography, 10-80AB, MS ESI calcd. for C₁₉H₁₉ClF₃N₂O₃S [M+H]⁺ 447.1, found 447.1.

### Example 84. Synthesis of Compound II-46

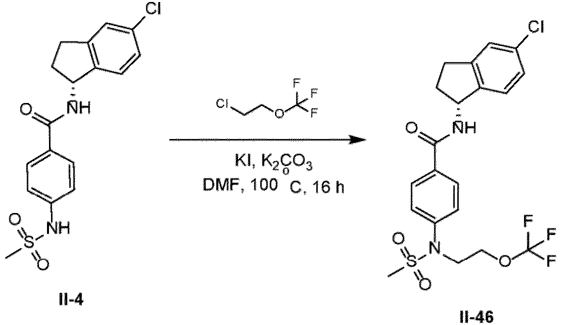

To a mixture of*N*-[(1*R*)-5-chloroindan-1-yl]-4-(methanesulfonamido)benzamide (60 mg, 0.16 mmol), 1-chloro-2-(trifluoromethoxy)ethane (109.9 mg, 0.74 mmol) and KI (13.65 mg, 0.08 mmol) in DMF (6 mL) was added K₂CO₃ (68.19 mg, 0.49 mmol) at 20 °C. The mixture was stirred under N₂ at 100 °C for 16 hours. The mixture was diluted with H₂O (10 mL) and the mixture was extracted with EtOAc (10 mL x 2). The combined organic phase was washed with brine (10 mL), dried over anhydrous Na₂SO₄, filtered and concentrated. The crude product was purified by Prep-HPLC (Boston Prime C18 (150 mm x 30 mm x 5 µm) A = H₂O (0.05% ammonia hydroxide) and B = CH₃CN; 51-71%B over 9 minutes) to give the product (35.71 mg, 0.07 mmol, 46% yield) as a solid.
**¹H NMR** (400MHz, CD₃CN) δ_{H} = 7.88 (dd, 2H), 7.46 (dd, 2H), 7.38 - 7.25 (m, 3H), 7.23 - 7.16 (m, 1H), 5.57 (q, 1H), 4.10 - 4.03 (m, 2H), 4.03 - 3.95 (m, 2H), 3.09 - 2.98 (m, 1H), 2.95 (s, 3H), 2.94 - 2.84 (m, 1H), 2.62 - 2.51 (m, 1H), 2.07 - 1.98 (m, 1H).
**LCMS** Rₜ = 1.23 min in 2.0 min chromatography, 10-80AB, MS ESI calcd. for C₂₀H₂₁ClF₃N₂O₄S [M+H]⁺ 477.1, found 477.0.

### Example 85. Synthesis of Compound II-47

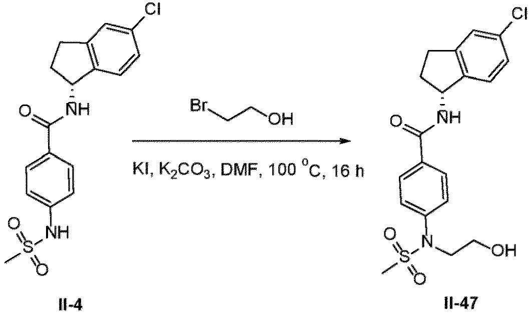

To a mixture of*N-*[(1*R*)-5-chloroindan-1-yl]-4-(methanesulfonamido)benzamide (120 mg, 0.33 mmol) and 2-bromoethanol (184.95 mg, 1.48 mmol) and KI (16.38 mg, 0.1 mmol) in DMF (6 mL) was added K₂CO₃ (136.37 mg, 0.99 mmol) at 20 °C. The mixture was stirred at 100 °C for 16 hours under N₂. The mixture was quenched with sat. NH₄Cl (10 mL) and the mixture was extracted with EtOAc (10 mL x 2). The combined organic phase was washed with brine (10 mL), dried over anhydrous Na₂SO₄, filtered and concentrated. The crude product was purified by Prep-HPLC (Boston Prime (150 mm x 30 mm x 5 µm) A = H₂O (0.05% NH₄OH) and B = CH₃CN; 30-60% B over 9 minutes) to give the product (36.3 mg, 0.09 mmol, 27% yield) as a solid.
**¹H NMR** (400MHz, CD₃CN) δ_{H} = 7.85 (d, 2H), 7.46 (dd, 2H), 7.36 - 7.26 (m, 3H), 7.23 - 7.16 (m, 1H), 5.57 (q, 1H), 3.77 (t, 2H), 3.50 (q, 2H), 3.08 - 2.98 (m, 1H), 2.97 (s, 3H), 2.94 - 2.84 (m, 2H), 2.63 - 2.49 (m, 1H), 2.05 - 1.97 (m, 1H).
**LCMS** Rₜ = 1.11 min in 2.0 min chromatography, 10-80AB, MS ESI calcd. for C₁₉H₂₂ClN₂O₄S [M +H]⁺ 409.1, found 409.0.

### Example 86 . Synthesis of Compound II-48

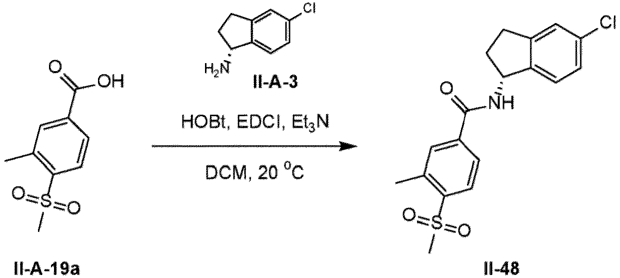

A mixture of 3-methyl-4-methylsulfonyl-benzoic acid (100 mg, 0.47 mmol) and HOBt (126.15 mg, 0.93 mmol) and EDCI (134.22 mg, 0.7 mmol) and TEA (0.32 mL, 2.33 mmol) and (1*R*)-5-chloroindan-1-amine (78.25 mg, 0.47 mmol) in DCM (10 mL) was stirred at 20 °C for 16 hours. The mixture was concentrated under reduced pressure. The residue was diluted with H₂O (20 mL) and the mixture was extracted with EtOAc (20 mL x 2). The combined organic phase was washed with water (20 mL) and brine (20 mL), dried over anhydrous Na₂SO₄, filtered and concentrated. The crude product was purified by Prep-HPLC (Boston Prime (150 mm x 30 mm x 5 µm) A = H₂O (0.05% NH₄OH) and B = CH₃CN; 46-76% B over 9 minutes) to give the product (56.1 mg, 0.15 mmol, 33% yield) as a solid.
**¹H NMR** (400MHz, CD₃CN) δ_{H} = 8.01 (d, 1H), 7.87 - 7.75 (m, 2H), 7.54 - 7.40 (m, 1H), 7.35 - 7.25 (m, 2H), 7.24 - 7.17 (m, 1H), 5.56 (q, 1H), 3.14 - 2.99 (m, 4H), 2.96 - 2.84 (m, 1H), 2.70 (s, 3H), 2.62 - 2.50 (m, 1H), 2.07 - 1.99 (m, 1H).
**LCMS** Rₜ = 1.19 min in 2 min chromatography, 10-80AB, MS ESI calcd. C₁₈H₁₉ClNO₃S [M+H]⁺ 364.1, found 364.0.

### xample 87. Synthesis of Compound II-49

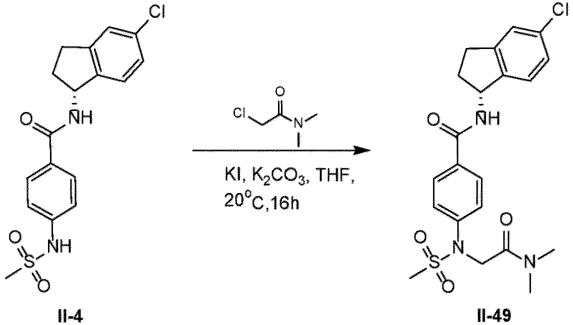

To a mixture of *N*-[(1*R*)-5-chloroindan-1-yl]-4-(methanesulfonamido)benzamide (100 mg, 0.27 mmol) and 2-chloro-*N*,*N*-dimethyl-acetamide (166.6 mg, 1.37 mmol) and KI (13.65 mg, 0.08 mmol) in THF (10 mL) was added K₂CO₃ (113.64 mg, 0.82 mmol) at 0 °C. The mixture was stirred under N₂ at 20 °C for 16 hours. The mixture was concentrated to dryness and diluted with H₂O (10 mL). The mixture was extracted with EtOAc (10 mL x 2). The combined organic phase was washed with brine (10 mL), dried over anhydrous Na₂SO₄, filtered and concentrated. The crude product was purified by Prep-HPLC (Boston Prime (150 mm x 25 mm x 5 µm) A = H₂O (0.05% NH₄OH) and B = CH₃CN; 40-60% B over 9 minutes) to give the product (37.5 mg, 0.08 mmol, 30% yield) as a solid.
**¹H NMR** (400MHz, CD₃CN) δ_{H} = 7.85 (d, 2H), 7.56 (d, 2H), 7.37 - 7.28 (m, 3H), 7.27 - 7.20 (m, 1H), 5.59 (q, 1H), 4.62 (s, 2H), 3.15 (s, 3H), 3.10 - 3.01 (m, 1H), 2.97 (s, 3H), 2.95 - 2.80 (m, 4H), 2.63 - 2.54 (m, 1H), 2.09 - 2.00 (m, 1H).
**LCMS** Rₜ = 1.15 min in 2.0 min chromatography, 10-80AB, MS ESI calcd. for C₂₁H₂₅ClN₃O₄S [M+H]⁺ 450.1, found 450.1.

### Example 88. Synthesis of Compound II-50

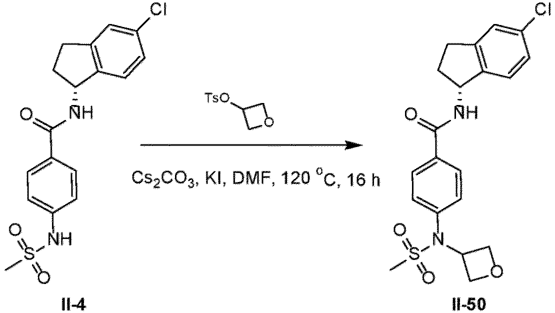

To a mixture of*N-*[(1*R*)-5-chloroindan-1-yl]-4-(methanesulfonamido)benzamide (180 mg, 0.49 mmol) and oxetan-3-yl 4-methylbenzenesulfonate (1.13 g, 4.93 mmol) and Cs₂CO₃ (642.94 mg, 1.97 mmol) in DMF (10 mL) was added KI (409.48 mg, 2.47 mmol) at 0 °C and the mixture was stirred under N₂ at 120 °C for 16 hours. After cooling to room temperature, the reaction was quenched with sat. NH₄Cl (20 mL). The mixture was extracted with EtOAc (20 mL x 2). The combined organic phase was washed with brine (20 mL), dried over anhydrous Na₂SO₄, filtered and concentrated. The crude product was purified by prep-HPLC (Boston Green ODS (150 mm x 30 mm, 5 µm) A = water (0.075%TFA) and B = CH₃CN; 42-62%B over 9 minutes) to give the product which was then neutralized with sat. NaHCO₃ (10 mL) and the mixture was extracted with EtOAc (10 mL x 2). The combined organic phase was washed with brine (10 mL), dried over anhydrous Na₂SO₄, filtered and concentrated to give the product (19.8 mg, 0.05 mmol, 9% yield) as a solid.
**¹H NMR** (400MHz, CD₃CN) δ_{H} = 7.94 - 7.79 (m, 2H), 7.40 - 7.25 (m, 5H), 7.24 - 7.16 (m, 1H), 5.57 (q, 1H), 5.27-5.13 (m, 1H), 4.61 (t, 2H), 4.45 (t, 2H), 3.10 - 2.97 (m, 1H), 2.95 - 2.85 (m, 1H), 2.82 (s, 3H), 2.62 - 2.49 (m, 1H), 2.07 - 1.98 (m, 1H).
**LCMS** Rₜ = 1.15 min in 2.0 min chromatography, 10-80AB, MS ESI calcd. for C₂₀H₂₂ClN₂O₄S [M+H]⁺ 421.1, found 421.2.

### Example 89. Synthesis of Compound II-51

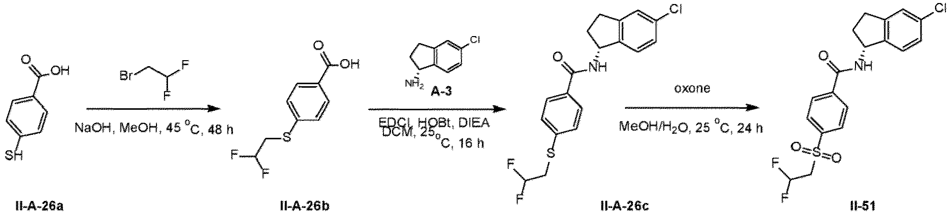

**Synthesis of II-A-26b:** A mixture of 4-sulfanylbenzoic acid (500 mg, 3.24 mmol) and NaOH (389.13 mg, 9.73 mmol) in methanol (15 mL) was stirred at 45 °C for 20 min. Then 2-bromo-1,1-difluoro-ethane (940.07 mg, 6.49 mmol) was added. The reaction mixture was stirred at 45 °C for 48 h. After cooling to r. t., the mixture was diluted with H₂O (20 mL) and the mixture was concentrated to remove MeOH. Then the solution was acidified to pH~3 with 1M HCl. The solid was collected by filtration and the filter cake was dried in oven to give the product (400 mg, 1.83 mmol).
**¹H NMR** (400MHz, DMSO-*d*⁶) δ_{H} = 7.86 (d, 2H), 7.51 (d, 2H), 6.25 (tt, 1H), 3.64 (dt, 2H).

**Synthesis of II-A-26c:** A mixture of 4-(2,2-difluoroethylsulfanyl)benzoic acid (300 mg, 1.37 mmol), HOBt (371.54 mg, 2.75 mmol), EDCI (527.08 mg, 2.75 mmol), DIPEA (0.76 mL, 5.5 mmol) and (1*R*)-5-chloroindan-1-amine (230.46 mg, 1.37 mmol) in DCM (15 mL) was stirred at 25 °C for 16 hours. The reaction was concentrated and diluted with H₂O (20 mL) and the aqueous layer was extracted with DCM (20 mL x 2). The combined organic phase was washed with brine (30 mL), dried over anhydrous Na₂SO₄, filtered and concentrated. The crude product was purified with flash chromatography on silica gel (DCM) to give the product (300mg, 0.8071mmol, 59% yield) as a solid.
**¹H NMR** (400MHz, DMSO-*d*⁶) δ_{H} = 7.75 (d, 2H), 7.46 (d, 2H), 7.28 - 7.25 (m, 2H), 7.25 - 7.18 (m, 1H), 6.34 - 6.22 (m, 1H), 5.92 (tt, 1H), 5.67 (q, 1H), 3.34 (dt, 2H), 3.15 - 2.90 (m, 2H), 2.80 - 2.63 (m, 1H), 2.05-1.92 (m, 1H).
**LCMS Rₜ** = 1.25 min in in 2.0 min chromatography, 10-80AB, MS ESI calcd. for C₁₈H₁₇ClF₂NOS [M+H]⁺ 368.1, found 367.9.

**Synthesis of Compound II-51:** To the solution of*N*-[(1*R*)-5-chloroindan-1-yl]-4-(2,2-difluoroethylsulfanyl)benzamide (80.mg, 0.22 mmol) in methanol (15 mL) and water (10 mL) was added oxone (800.75 mg, 1.3 mmol). The mixture was stirred at 25 °C for 24 hours. The suspension was filtered and the filtrate was concentrated under reduced pressure. The crude product was washed with H₂O (10 mL) and triturated from MeOH (5 mL) to give the product (30.7 mg, 0.07 mmol, 34% yield) as a solid.
**¹H NMR** (400MHz, CD₃CN) δ_{H} = 8.08 - 7.96 (m, 4H), 7.55 - 7.45 (m 1H), 7.36 - 7.26 (m, 2H), 7.25 - 7.17 (m, 1H), 6.24 (tt, 1H), 5.58 (q, 1H), 3.90 (dt, 2H), 3.13 - 2.99 (m, 1H), 2.97 - 2.83 (m, 1H), 2.66 - 2.51 (m, 1H), 2.10-1.98 (m, 1H).
**LCMS** Rₜ = 1.22 min in in 2.0 min chromatography, 10-80AB, MS ESI calcd. for C₁₈H₁₇ClF₂NO₃S [M+H]⁺ 400.1, found 400.0.

### Example 90. Synthesis of Compounds II-52 and II-53

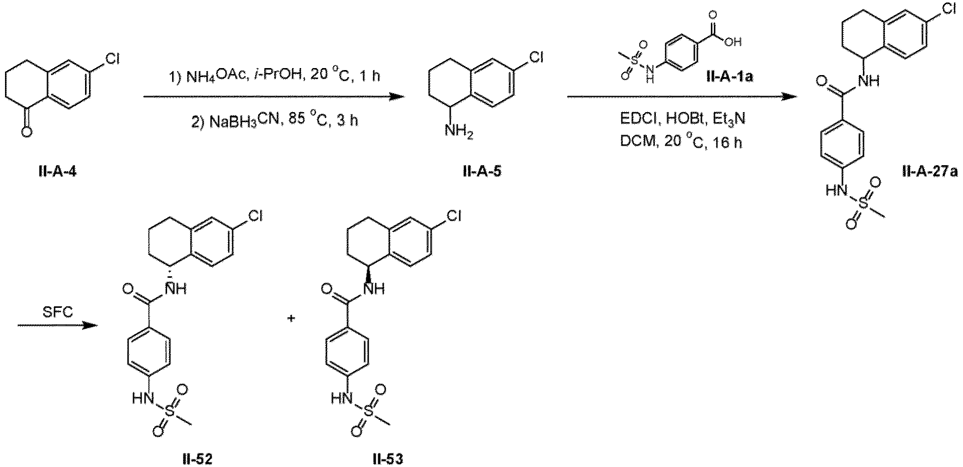

**Synthesis of II-A-5:** A mixture of 6-chlorotetralin-1-one (500 mg, 2.77 mmol) and NH₄OAc (2134.2 mg, 27.68 mmol) in i-PrOH (14 mL) was stirred at 20 °C for 1 hour. Then NaBH₃CN (608.81 mg, 9.69 mmol) was added and the resulting mixture was heated to 85 °C. The mixture was stirred at 85 °C for 3 hours. 5 N NaOH solution (50 mL) was added and the aqueous layer was extracted with EtOAc (30 mL). The organic phase was separated and washed with brine (20 mL x 2), dried over anhydrous Na₂SO₄, filtered and concentrated. The residue was re-dissolved in DCM (30 mL) and extracted with 1N HCl aqueous (30 mL x 2). Saturated Na₂CO₃ solution (30 mL) was added to adjust to pH 9 and the aqueous layer was extracted with EtOAc (30 mL x 2). The combined organic phase was washed with water (30 mL x 2) and brine (30 mL x 2), dried over anhydrous Na₂SO₄, filtered and concentrated to give the crude product (270 mg, 1.49 mmol) as an oil.
**¹H NMR** (400MHz, CDCl₃) δ_{H} = 7.35 (d, 1H), 7.15 (dd, 1H), 7.08 (s, 1H), 3.99-3.89 (m, 1H), 2.85-2.63 (m, 2H), 2.06-1.87 (m, 2H), 1.82-1.62 (m, 2H).

**Synthesis of II-A-27a:** A mixture of 4-(methanesulfonamido)benzoic acid (170.61 mg, 0.79 mmol) and HOBt (238.04 mg, 1.76 mmol) and Et₃N (0.37 mL, 2.64 mmol) and EDCI (253.26 mg, 1.32 mmol) and 6-chlorotetralin-1-amine (160 mg, 0.88 mmol) in DCM (15 mL) was stirred at 20 °C for 16 hours under N₂. The reaction was quenched with sat. NH₄Cl (20 mL) and the mixture was extracted with DCM (30 mL x 2). The combined organic phase was washed with brine (20 mL), dried over anhydrous Na₂SO₄, filtered and concentrated to give the product (301 mg, 0.79 mmol) as a solid.
**LCMS** Rₜ = 1.06 min in 1.5 min chromatography, 5-95AB, MS ESI calcd. for C₁₈H₂₀ClN₂O₃S [M+H]⁺ 379.1, found 379.0.
**Analytical SFC:** (Chiralpak OJ-3 150 mm x 4.6 mm I.D, 3µm, Mobile phase: A: CO₂ B: ethanol (0.05% DEA), Gradient: from 5% to 40% of B in 5 min and hold 40% of B for 2.5 min, then 5% of B for 2.5 min. Flow rate: 2.5 mL/min, Column temp.: 35 °C) showed two peaks at Rₜ = 5.08 min and Rₜ = 5.67 min.

**Synthesis of Compounds II-52 and II-53:** *N*-(6-chlorotetralin-1-yl)-4-(methanesulfonamido)benzamide (300 mg, 0.79 mmol) was purified by SFC [DAICEL CHIRALCEL OJ-H (250 mm x 30 mm I.D., 5 µm); A = CO₂ and B = EtOH (0.1% NH₃H₂O); 38 °C; 60 mL/min; 35% B; 12 min run; 9 injections] to give the the enantiomer 1, randomly assigned as Compound **II-52** (95.6 mg, 0.25 mmol) (Rₜ of Peak 1= 5.08 min) as a solid and the enantiomer 2, randomly assigned as Compound **II-53** (62.12 mg, 0.16 mmol) (Rt of Peak 2= 5.67 min) as a solid. Stereochemistry is randomly assigned.

### Compound II-52

**¹H NMR** (400MHz, DMSO-*d*⁶) δ_{H} = 10.08 (s, 1H), 8.66 (d, 1H), 7.89 (d, 2H), 7.32-7.11 (m, 5H), 5.28-5.10 (m, 1H), 3.05 (s, 3H), 2.84-2.73 (m, 2H), 2.04-1.87 (m, 2H), 1.85-1.65 (m, 2H).
**LCMS** Rₜ = 1.09 min in 2 min chromatography, 10-80AB, MS ESI calcd. for C₁₈H₂₀ClN₂O₃S [M+H]⁺ 379.1, found 379.0.

### Compound II-53

**¹H NMR** (400MHz, DMSO-*d*⁶) δ_{H} = 10.08 (s, 1H), 8.66 (d, 1H), 7.88 (d, 2H), 7.28-7.13 (m, 5H), 5.25-5.12 (m, 1H), 3.05 (s, 3H), 2.84-2.74 (m, 2H), 2.03-1.87 (m, 2H), 1.85-1.66 (m, 2H).
**LCMS** Rt = 1.07 min in 2 min chromatography, 10-80AB, MS ESI calcd. for C₁₈H₂₀ClN₂O₃S [M+H]⁺ 379.1, found 379.0.

### Example 91. Synthesis of Compounds II-54 and II-55

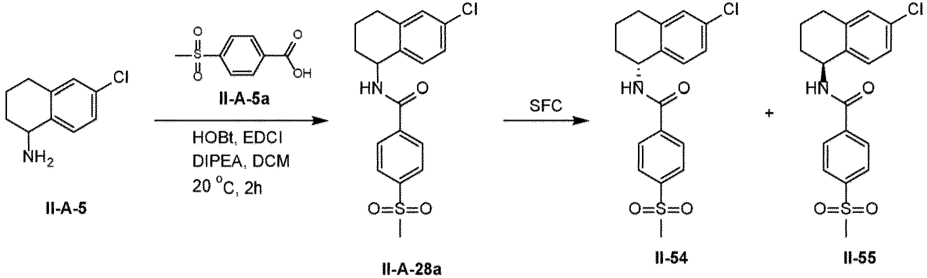

To a mixture of 6-chlorotetralin-1-amine (110 mg, 0.61 mmol) and HOBt (163.65 mg, 1.21 mmol) and EDCI (174.12 mg, 0.91 mmol) in DCM (3 mL) were added DIPEA (0.32 mL, 1.82 mmol) and 4-methylsulfonylbenzoic acid (145.48 mg, 0.73 mmol). The mixture was stirred at 20 °C for 2 hours. The mixture was diluted with water (20 mL) and the aqueous layer was extracted with EtOAc (20 mL x 2). The combined organic phase was washed with 1M HCl (20 mL) and water (20mL x 2) and brine (20 mL), dried over anhydrous Na₂SO₄, filtered and concentrated.
**Analytical SFC:** (Chiralcel OJ-3 150 x 4.6 mm I.D., 3 µm, Mobile phase: A: CO₂, B: ethanol (0.05% DEA), Gradient: from 5% to 40% of B in 5 min and hold 40% of B for 2.5 min, then 5% of B for 2.5 min. Flow rate: 2.5 mL/min, Column temp.: 35 °C) showed two peaks at 1.84 min and 5.65 min.

The product was purified by SFC (DAICEL CHIRALPAK AS (250 mm x 30 mm, 10 µm); A = CO₂ and B = MeOH (0.1% NH₃H₂O); 38 °C; 70 mL/min; 50% B; 13 min run; 6 injections) to give enantiomer 1, randomly assigned as Compound **II-54** (38.1 mg, 103.2 µmol) (Rₜ of Peak 1 = 1.84 min) as a solid and the enantiomer 2, randomly assigned as Compound **II-55** (47.5 mg, 130.5 µmol) (Rₜ of Peak 2 = 5.65 min) as a solid. Stereochemistry is randomly assigned.

### Compound II-54

**¹H NMR** (400MHz, CDCl₃) δ_{H} = 8.05 - 8.00 (m, 2H), 7.99 - 7.93 (m, 2H), 7.25 (s, 1H), 7.20 - 7.12 (m, 2H), 6.37 (d, 1H), 5.43 - 5.34 (m, 1H), 3.07 (s, 3H), 2.91 - 2.76 (m, 2H), 2.21 - 2.11 (m, 1H), 2.00 - 1.84 (m, 3H).
**LCMS** Rₜ = 1.20 min in 2.0 min chromatography, 10-80AB, MS ESI calcd. for C₁₈H₁₉ClNO₃S [M+H]⁺364.1, found 364.0.

### Compound II-55

**¹H NMR** (400MHz, CDCl₃) δ_{H} = 8.08 - 8.00 (m, 2H), 8.00 - 7.94 (m, 2H), 7.25 (s, 1H), 7.20 - 7.13 (m, 2H), 6.35 (d, 1H), 5.46 - 5.31 (m, 1H), 3.07 (s, 3H), 2.90 - 2.74 (m, 2H), 2.22 - 2.09 (m, 1H), 2.01 - 1.84 (m, 3H).
**LCMS** Rₜ = 1.19 min in 2.0 min chromatography, 10-80AB, MS ESI calcd. for C₁₈H₁₉ClNO₃S [M+H]⁺364.1, found 364.0.

### Example 92. Synthesis of Compounds II-56 and II-58

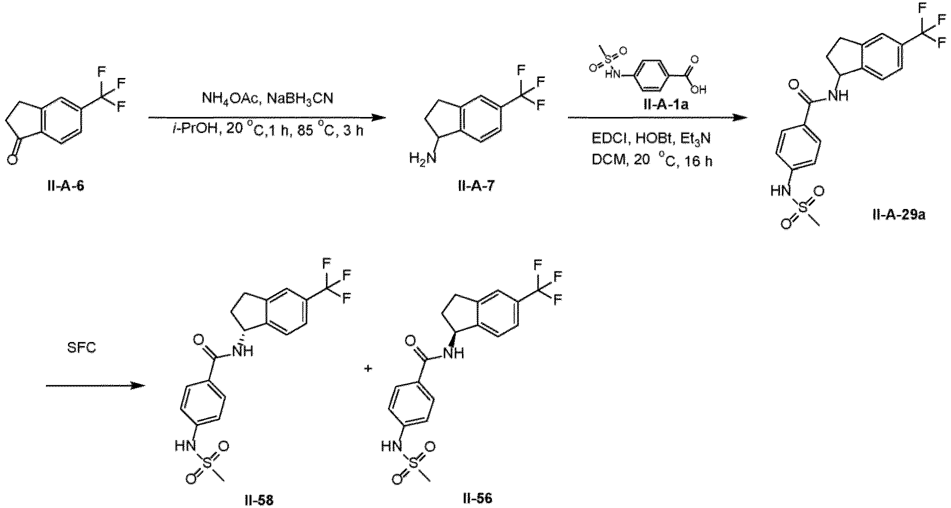

**Synthesis of II-A-7:** A mixture of 5-(trifluoromethyl)indan-1-one (400 mg, 2 mmol) and NH₄OAc (1.54 g, 19.98 mmol) in i-PrOH (10 mL) was stirred at 20 °C for 1 hour and then NaBH₃CN (439.53 mg, 6.99 mmol) was added. The mixture was heated to 85 °C and stirred for 3 hours. 5 N NaOH aqueous (150 mL) was added and the aqueous layer was extracted with EtOAc (100 mL). The organic phase was separated and washed with brine (100 mL x 2), dried over anhydrous Na₂SO₄, filtered and concentrated. The residue was re-dissolved in DCM (100 mL) and extracted with 1N HCl aqueous (100 mL x 2). Saturated Na₂CO₃ aqueous (100 mL) was added to adjust to pH 9 and the aqueous layer was extracted with EtOAc (100 mL x 2). The combined organic phase was washed with water (100 mL x 2) and brine (100 mL x 2), dried over anhydrous Na₂SO₄, filtered and concentrated to give the crude product (210 mg, 0.94 mmol) as an oil, which was used for next step directly without further purification.
**LCMS** Rₜ = 0.74 min in 1.5 min chromatography, 5-95AB, MS ESI calcd. for C₁₀H₁₀F₃N₂ [M-NH₃+H]⁺ 185.1, found 184.8.

**Synthesis of II-A-29a:** A mixture of 4-(methanesulfonamido)benzoic acid (192.56 mg, 0.89 mmol) and HOBt (268.66 mg, 1.99 mmol) and Et₃N (0.41 mL, 2.98 mmol) and EDCI (285.85 mg, 1.49 mmol) and 5-(trifluoromethyl)indan-1-amine (200 mg, 0.99 mmol) in DCM (15 mL) was stirred at 20 °C for 16 hours under N₂. The reaction was quenched with sat. NH₄Cl (30 mL) and the mixture was extracted with DCM (30 mL x 2). The combined organic phase was washed with brine (30 mL), dried over anhydrous Na₂SO₄, filtered and concentrated. The crude product was triturated from DCM/n-hexane (2 mL/8 mL) to give the product (310 mg, 0.77 mmol, 78% yield) as a solid.
**LCMS** Rₜ = 0.94 min in 1.5 min chromatography, 5-95AB, MS ESI calcd. for C₁₈H₁₈F₃N₂O₃S [M +H]⁺ 399.1, found 398.9.
**Analytical SFC:** (Chiralpak AD-3 150 x 4.6 mm I.D, 3 µm, Mobile phase: A: CO₂ B: ethanol (0.05% DEA), Gradient: from 5% to 40% of B in 5 min and hold 40% of B for 2.5 min, then 5% of B for 2.5 min. Flow rate: 2.5 mL/min, Column temp.: 35 °C) showed two peaks at 4.16 min and 4.36 min.

**Synthesis of Compounds II-58 and II-56:** 4-(methanesulfonamido)-N-[5-(trifluoromethyl)indan-1-yl]benzamide (310 mg, 0.77 mmol) was purified by SFC (DAICEL CHIRALPAK AD-H (250 mm x 30 mm I.D., 5 µm); A = CO₂ and B = EtOH (0.1% NH₃H₂O); 38 °C; 50 mL/min; 35% B; 8 min run; 24 injections) to give the enantiomer 1, randomly assigned as Compound **II-58** (41.9 mg, 104.7 µmol) (Rₜ of Peak 1= 4.16 min) as a solid and the enantiomer 2, randomly assigned as Compound **II-56** (43.4 mg, 109.0 µmol) (Rₜ of Peak 2= 4.36 min) as a solid. Stereochemistry is randomly assigned.

### Compound II-58

**¹H NMR** (400MHz, CD₃CN) δ_{H} = 7.91 - 7.73 (m, 3H), 7.59 (s, 1H), 7.54 - 7.43 (m, 2H), 7.37 - 7.18 (m, 3H), 5.65 (q, 1H), 3.16 - 3.05 (m, 1H), 3.03 - 2.91 (m, 4H), 2.65 - 2.54 (m, 1H), 2.09 - 2.01 (m, 1H).
**LCMS** Rₜ = 1.18 min in 2.0 min chromatography, 10-80AB, MS ESI calcd. for C₁₈H₁₈F₃N₂O₃S [M +H]⁺ 399.1, found 399.0.

### Compound II-56

**¹H NMR** (400MHz, CD₃CN) δ_{H} = 7.89 - 7.64 (m, 3H), 7.59 (s, 1H), 7.54 - 7.43 (m, 2H), 7.37 - 7.21 (m, 3H), 5.65 (q, 1H), 3.15 - 3.05 (m, 1H), 3.02 - 2.91 (m, 4H), 2.65 - 2.55 (m, 1H), 2.09 - 2.01 (m, 1H).
**LCMS** Rₜ = 1.17 min in 2.0 min chromatography, 10-80AB, MS ESI calcd. for C₁₈H₁₈F₃N₂O₃S [M +H]⁺ 399.1, found 399.0.

### Example 93. Synthesis of Compounds II-57 and II-59

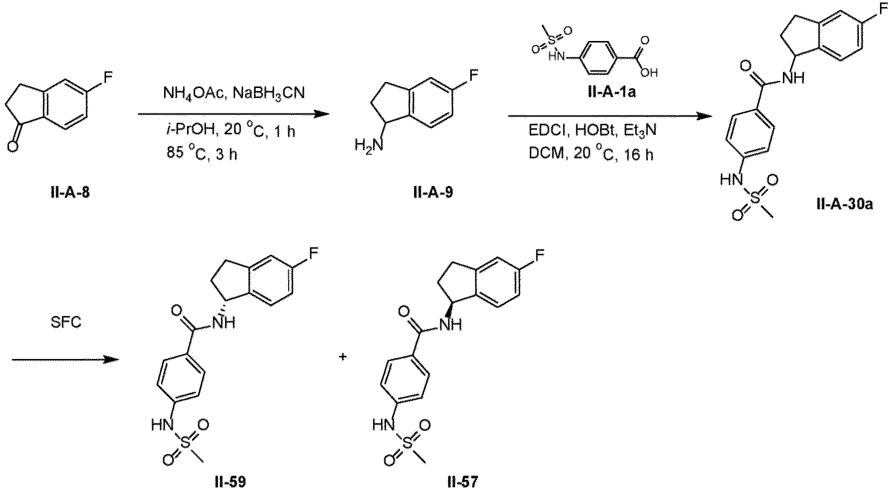

**Synthesis of II-A-9:** A mixture of 5-fluoroindan-1-one (2 g, 13.32 mmol) and NH₄OAc (10.27 g, 133.2 mmol) in i-PrOH (250 mL) was stirred at 20 °C for 1 hour and then NaBH₃CN (2.93 g, 46.62 mmol) was added. The mixture was heated to 85 °C and stirred for 3 hours. 5 N aqueous NaOH (100 mL) was added and the mixture was extracted with EtOAc (100 mL). The organic phase was separated and washed with brine (100 mL x 2), dried over anhydrous Na₂SO₄, filtered and concentrated. The residue was re-dissolved in DCM (100 mL) and the organic layer was extracted with 1M HCl (100 mL x 2). Saturated Na₂CO₃ solution (100 mL) was added to adjust to pH = 9 and the aqueous layer was extracted with EtOAc (100 mL x 2). The combined organic phase was washed with water (100 mL x 2) and brine (100 mL x 2), dried over anhydrous Na₂SO₄, filtered and concentrated to give the crude product (900 mg, 4.99 mmol) as an oil.
**LCMS** Rₜ = 0.27 min in 1.5 min chromatography, 5-95AB, MS ESI calcd. for C₉H₁₀FN₂ [M-NH₂]⁺ 135.1, found 134.9.

**Synthesis of II-A-30a:** A mixture of 4-(methanesulfonamido)benzoic acid (384.39 mg, 1.79 mmol) and HOBt (536.3 mg, 3.97 mmol) and Et₃N (0.82 mL, 5.95 mmol) and EDCI (570.61 mg, 2.98 mmol) and 5-fluoroindan-1-amine (300 mg, 1.98 mmol) in DCM (15 mL) was stirred at 20 °C for 16 hours under N₂. The reaction was quenched with sat. NH₄Cl (30 mL) and the mixture was extracted with DCM (30 mL x 2). The combined organic phase was washed with brine (30 mL), dried over anhydrous Na₂SO₄, filtered and concentrated to give the product (350 mg, 1.0 mmol, 51% yield) as a solid.
**LCMS** Rₜ = 1.08 min in 2 min chromatography, 10-80AB, MS ESI calcd. for C₁₇H₁₈FN₂O₃S [M +H]⁺ 349.1, found 348.9.
**Analytical SFC:** (Chiralcel OJ-3 100 mm x 4.6 mm I.D, 3 µm, Mobile phase: A: CO₂ B: ethanol (0.05% DEA), Gradient: from 5% to 40% of B in 5 min and hold 40% of B for 2.5 min, then 5% of B for 2.5 min. Flow rate: 2.5 mL/min, Column temp.: 35 °C) showed two peaks at 3.32 min and 3.67 min.

**Synthesis of II-A-9:** *N-*[5-fluoroindan-1-yl]-4-(methanesulfonamido)benzamide (200 mg, 0.57 mmol) was purified by SFC (DAICEL CHIRALCEL OJ-H (250 mm x 30 mm I.D., 5 µm); A = CO₂ and B = EtOH (0.1% NH₃H₂O); 38 °C; 50 mL/min; 35% B; 9 min run; 14 injections) to give the enantiomer 1, randomly assigned as Compound **59** (89.7 mg, 0.26 mmol) (Rₜ of Peak 1= 3.32 min) as a solid and the enantiomer 2, randomly assigned as Compound **57** (81.4 mg, 0.23 mmol) (Rₜ of Peak 2= 3.67 min) as a solid. Stereochemistry is randomly assigned.

### Compound II-59

**¹H NMR** (400MHz, CD₃CN) δ_{H} = 7.88 - 7.72 (m, 3H), 7.33 - 7.15 (m, 4H), 7.05 - 6.97 (m, 1H), 6.97 - 6.87 (m, 1H), 5.55 (q, 1H), 3.08 - 2.95 (m, 4H), 2.94 - 2.82 (m, 1H), 2.63 - 2.49 (m, 1H), 2.08 - 1.97 (m, 1H).
**LCMS** Rₜ = 1.09 min in 2.0 min chromatography, 10-80AB, MS ESI calcd. for C₁₇H₁₈FN₂O₃S [M+H]⁺ 349.1, found 348.9.

### Compound II-57

**¹H NMR** (400MHz, CD₃CN) δ_{H} = 7.86 - 7.77 (m, 3H), 7.32 - 7.17 (m, 4H), 7.05 - 6.98 (m, 1H), 6.97 - 6.88 (m, 1H), 5.55 (q, 1H), 3.09 - 2.95 (m, 4H), 2.94 - 2.83 (m, 1H), 2.63 - 2.50 (m, 1H), 2.07 - 1.96 (m, 1H).
**LCMS** Rₜ = 1.08 min in 2.0 min chromatography, 10-80AB, MS ESI calcd. for C₁₇H₁₈FN₂O₃S [M +H]⁺ 349.1, found 348.9.

### Example 94. Synthesis of Compounds III-1 and III-2

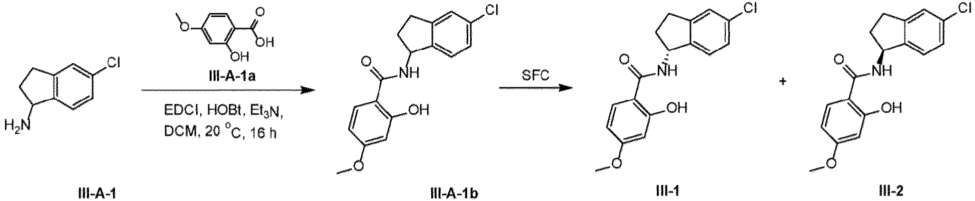

### Synthesis of III-A-1b

A mixture of 2-hydroxy-4-methoxy-benzoic acid (135.41 mg, 0.81 mmol), HOBt (241.82 mg, 1.79 mmol), EDCI (257.29 mg, 1.34 mmol), Et₃N (0.37 mL, 2.68 mmol) and 5-chloroindan-1-amine (150 mg, 0.89 mmol) in DCM (10 mL) was stirred at 20 °C for 16 hours under N₂. The reaction was quenched with sat. NH₄Cl (15 mL) and the mixture was extracted with DCM (20 mL x 2). The combined organic phase was washed with brine (15 mL), dried over anhydrous Na₂SO₄, filtered and concentrated. The crude product was purified by flash chromatography on silica gel (EtOAc in PE = 0 to 15% to 25% to 40%) to give the product (101 mg, 0.32 mmol, 35% yield) as a solid.
**LCMS** Rₜ = 0.89 min in 1.5 min chromatography, 5-95AB, MS ESI calcd. for C₁₇H₁₇ClNO₃ [M+H]⁺ 318.1, found 317.9.
**Analytical SFC:** (Chiralcel OJ-3 100 mm x 4.6 mm I.D, 3 µm, Mobile phase: A: CO₂ B: ethanol (0.05% DEA), Gradient: from 5% to 40% of B in 5 min and hold 40% for 2.5 min, then 5% ofB for 2.5 min. Flow rate: 2.5 mL/min, Column temp.: 35 °C) showed two peaks at 1.97 min (50%) and 7.57 min (50%).

### Synthesis of Compounds III-1 and III-2

*N*-(5-chloroindan-1-yl)-2-hydroxy-4-methoxy-benzamide (100 mg, 0.31 mmol) was purified by SFC [DAICEL CHIRALCEL OJ (250 mm x 30 mm, 10 µm); A = CO₂ and B = EtOH (0.1% NH₃H₂O); 38 °C; 80 mL/min; 55% B; 11 min run; 8 injections] to give the enantiomer 1, randomly assigned as Compound 1 (55.80 mg, 0.18 mmol) (Rₜ of Peak 1= 1.98 min) as a solid and the enantiomer 2, randomly assigned as Compound 2 (70.50 mg, 0.22 mmol) (Rₜ of Peak 2= 7.56 min) as a solid. Stereochemistry is randomly assigned.

### Compound III-1

**¹H NMR** (400MHz, CD₃CN) δ_{H} = 12.94 (s, 1H), 7.51 (d, 1H), 7.41-7.25 (m, 3H), 7.24-7.17 (m, 1H), 6.48-6.38 (m, 2H), 5.59 (q, 1H), 3.80 (s, 3H), 3.11-2.98 (m, 1H), 2.97-2.83 (m, 1H), 2.61-2.50 (m, 1H), 2.10-1.99 (m, 1H).
LCMS Rₜ = 1.21 min in 2 min chromatography, 10-80AB, MS ESI calcd. for C₁₇H₁₇ClNO₃ [M+H]⁺ 318.1, found 317.9.

### Compound III-2

**¹H NMR** (400MHz, CD₃CN) δ_{H} = 12.94 (s, 1H), 7.51 (d, 1H), 7.44-7.25 (m, 3H), 7.24-7.16 (m, 1H), 6.49-6.37 (m, 2H), 5.59 (q, 1H), 3.80 (s, 3H), 3.10-3.00 (m, 1H), 2.97-2.84 (m, 1H), 2.61-2.50 (m, 1H), 2.08-2.00 (m, 1H).
**LCMS** Rₜ = 1.21 min in 2 min chromatography, 10-80AB, MS ESI calcd. for C₁₇H₁₇ClNO₃ [M+H]⁺ 318.1, found 317.9.

### Example 95. Synthesis of Compound III-3

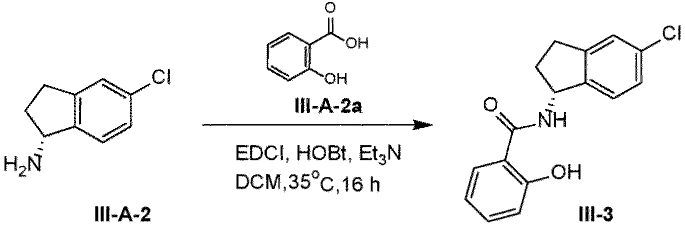

To a mixture of 2-hydroxybenzoic acid (63.44 mg, 0.46 mmol) and HOBt (112.85 mg, 0.84 mmol) and Et₃N (0.29 mL, 2.09 mmol) in CH₂Cl₂ (3 mL) was added EDCI (160.09 mg, 0.84 mmol) and (1*R*)-5-chloroindan-1-amine (70 mg, 0.42 mmol). The reaction mixture was stirred at 35 °C for 16 hours. The mixture was diluted with sat. NH₄Cl (5 mL) and the aqueous layer was extracted with CH₂Cl₂ (5 mL x 2). The combined organic phase was washed with brine (5 mL), dried over anhydrous Na₂SO₄, filtered and concentrated. The crude product was purified by Prep-HPLC (Xtimate C18 (150 mm x 25 mm, 5 µm) A = H₂O (10 mM NH₄HCO₃) and B = CH₃CN; 56 - 76% B over 9 minutes) to give the product (29.1 mg, 0.10 mmol, 24% yield) as a solid.
**¹H NMR** (400MHz, CD₃CN) δ_{H} = 12.66 - 12.46 (m, 1H), 7.62 - 7.48 (m, 2H), 7.44 - 7.39 (m, 1H), 7.32 - 7.28 (m, 2H), 7.23 - 7.19 (m, 1H), 6.93 (dd, 1H), 6.89 - 6.84 (m, 1H), 5.61 (q, 1H), 3.10 - 3.01 (m, 1H), 2.96 - 2.87 (m, 1H), 2.62 - 2.53 (m, 1H), 2.11 - 2.02 (m, 1H).
**LCMS** Rₜ = 1.36 min in 2.0 min chromatography, 10-80AB, MS ESI calcd. for C₁₆H₁₅ClNO₂ [M+H]⁺ 288.1, found 287.9.

### Example 96. Synthesis of Compound III-4

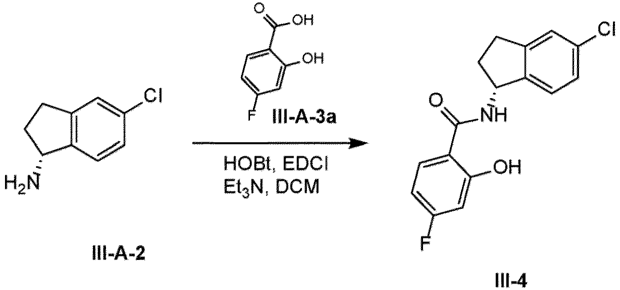

A mixture of 4-fluoro-2-hydroxy-benzoic acid (93.12 mg, 0.60 mmol) and HOBt (161.21 mg, 1.19 mmol) and EDCI (171.53 mg, 0.89 mmol) and TEA (301.81 mg, 2.98 mmol) and (1*R*)-5-chloroindan-1-amine (100 mg, 0.60 mmol) in DCM (20 mL) was stirred at 20 °C for 16 hours. The mixture was diluted with H₂O (10 mL) and the aqueous layer was extracted with EtOAc (20 mL x 2). The combined organic phase was washed with brine (10 mL), dried over anhydrous Na₂SO₄, filtered and concentrated. The crude product was purified by Prep-HPLC (Boston Prime C18 (150 mm x 30 mm, 5 µm) A = H₂O (0.05% NH₄OH) and B = CH₃CN; 55-85 %B over 8 minutes) to give the impure product. The impure product was purified by Prep-HPLC (Boston Green ODS (150 mm x 30 mm, 5 µm) A = H₂O (0.1% TFA) and B = CH₃CN; 65-95 %B over 9 minutes) to give the product (12.1 mg, 0.04 mmol, 7% yield) as a solid.
**¹H NMR** (400MHz, CDCl₃) δ_{H} = 12.66 (d, 1H), 7.34 - 7.20 (m, 4H), 6.71 (dd, 1H), 6.62-6.54 (m, 1H), 6.29 (d, 1H), 5.64 (q, 1H), 3.11 - 3.00 (m, 1H), 2.99 - 2.88 (m, 1H), 2.77 - 2.66 (m, 1H), 2.03 - 1.94 (m, 1H)
**LCMS** Rₜ = 1.36 min in 2.0 min chromatography, 10-80AB, MS ESI calcd. for C₁₆H₁₄ClFNO₂ [M+H]⁺ 306.1, found 305.9.

### Example 97. Synthesis of Compound III-5

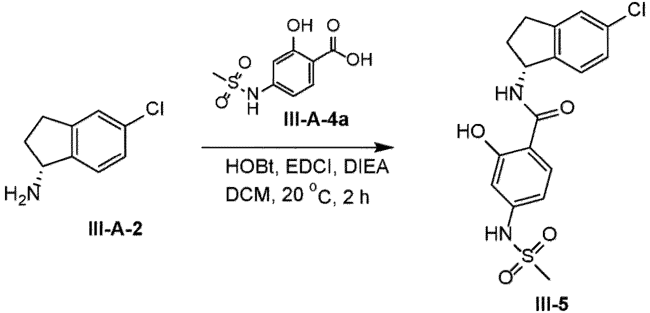

To a mixture of DIPEA (0.5 mL, 2.84 mmol), 2-hydroxy-4-(methanesulfonamido)benzoic acid (218.98 mg, 0.95 mmol) and HOBt (255.94 mg, 1.89 mmol) in DCM (3 mL) were added EDCI (272.32 mg, 1.42 mmol) and (1*R*)-5-chloroindan-1-amine (158.76 mg, 0.95 mmol). The mixture was stirred at 20 °C for 2 hours. The mixture was diluted with 1M HCl (10 mL) and the aqueous layer was extracted with EtOAc (20 mL x 2). The combined organic phase was washed with 1M HCl (20 mL), water (20 mL x 2) and brine (20 mL), dried over anhydrous Na₂SO₄, filtered and concentrated. The crude product was purified by Prep-HPLC (Waters Xbridge (150 mm x 25mm, 5 µm), A = H₂O (10 mM NH₄HCO₃) and B = CH₃CN; 38-58 %B over 8 minutes) to give the product (23.0 mg, 6% yield) as a solid.
**¹H NMR** (400MHz, CDCl₃) δ_{H} = 12.63 (s, 1H), 7.32 - 7.20 (m, 4H), 6.77 (d, 1H), 6.69 (dd, 1H), 6.55 (br s, 1H), 6.30 (d, 1H), 5.64 (q, 1H), 3.10 (s, 3H), 3.08 - 3.01 (m, 1H), 2.99 - 2.91 (m, 1H), 2.76 - 2.67 (m, 1H), 2.04 - 1.93 (m, 1H).
**LCMS** Rₜ = 1.22 min in 2.0 min chromatography, 10-80AB, MS ESI calcd. for C₁₇H₁₈ClN₂O₄S [M+H]⁺381.1, found 380.9.

### Example 98. Synthesis of Compound III-6

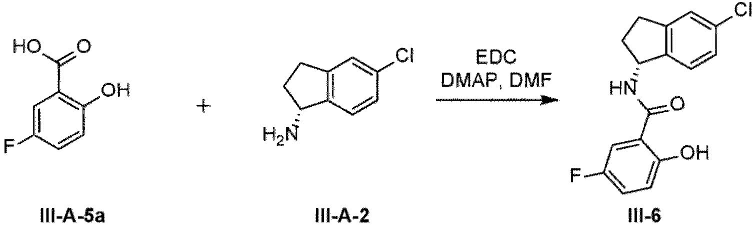

To a stirred solution of 5-fluoro-2-hydroxy-benzoic acid (100.0 mg, 0.64 mmol) in DMF (2.0 mL) was added EDC (184 mg, 0.96 mmol) followed by DMAP (156 mg, 1.28 mmol) and (1*R*)-5-chloroindan-1-amine (88 mg, 0.52 mmol). The reaction mixture was stirred for 12 h at room temperature. The reaction mixture was treated with water (20 mL) and extracted with ethyl acetate (2 x 20 mL). The organic layer was washed with brine (20 mL), dried over Na₂SO₄ and concentrated. The crude product was purified by column chromatography on silica gel with 10% EtOAc/PE to afford Compound **III-6** (10 mg, 0.03 mmol, 6% yield) as a solid.
**LCMS:** 306.0 (M+H), Rₜ 2.69 min. Column: ZORBAX XDB C-18 (50 X 4.6 mm), 3.5 µm. Mobile Phase: A: 0.1% HCOOH in water, B: ACN; Flow Rate:1.5 mL/min
**¹H NMR (400 MHz, DMSO-d₆):** δ 12.33 (s, 1H), 9.14 (s, 1H), 7.79-7.76 (m, 1H), 7.37 (s, 1H), 7.32-7.24 (m, 3H), 6.95-6.92 (m, 1H), 5.56-5.50 (m, 1H), 3.06-2.99 (m, 1H), 2.93-2.84 (m, 1H), 2.55 (m, 1H), 2.07-1.97 (m, 1H).

### Example 99. Synthesis of Compound III-7

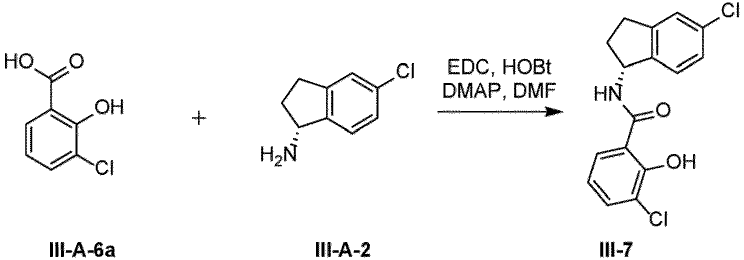

To a stirred solution of 3-chloro-2-hydroxy-benzoic acid (**III-A-6a,** 102 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 7% EtOAc/PE to afford Compound **III-7** (40 mg, 0.13 mmol, 21% yield) as a solid.
**LCMS:** 322.0 (M+H), Rₜ 2.83 min. Column: ZORBAX XDB C-18 (50 X 4.6 mm), 3.5 µm. Mobile Phase: A: 0.1% HCOOH in water, B: ACN; Flow Rate:1.5 mL/min
**¹H NMR (400 MHz, DMSO-d₆):** δ 13.68 (s, 1H), 9.32 (d, 1H), 7.92 (d, 1H), 7.62 (d, 1H), 7.38 (s, 1H), 7.29-7.24 (m, 2H), 6.91 (t, 1H), 5.60-5.55 (m, 1H), 3.07-3.00 (m, 1H), 2.93-2.85 (m, 1H), 2.46 (m, 1H), 2.10-2.05 (m, 1H).

### Example 100. Synthesis of Compound III-8

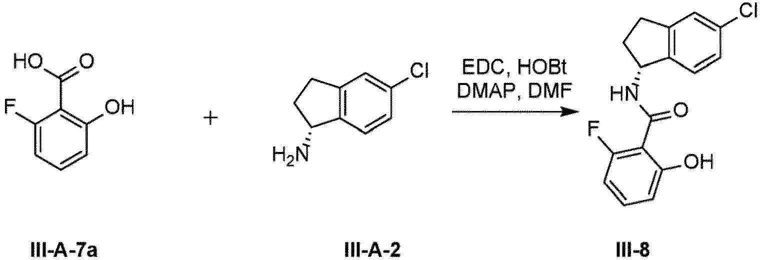

To a stirred solution of 2-fluoro-6-hydroxy-benzoic acid (**III-A-7a**, 92 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 5% EtOAc/PE to afford Compound **III-8** (62 mg, 0.19 mmol, 33% yield) as a solid.
**LCMS:** 306.0 (M+H), Rₜ 2.87 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR(400 MHz, DMSO-d₆):** δ 10.65 (brs, 1H), 8.69 (d, 1H), 7.32-7.22 (m, 4H), 6.73-6.65 (m, 2H), 5.50-5.44 (m, 1H), 2.98-2.80 (m, 2H), 2.48-2.33 (m, 1H), 2.00-1.90 (m, 1H).

### Example 101. Synthesis of Compound III-9

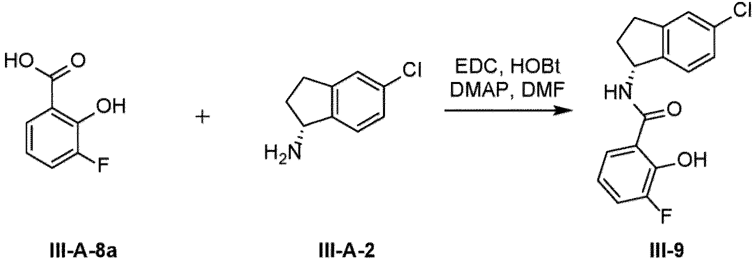

To a stirred solution of 3-fluoro-2-hydroxy-benzoic acid (**III-A-8a,** 92 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 18% EtOAc/PE to afford Compound **III-9** (23 mg, 0.07 mmol, 12% yield) as a solid.
**LCMS:** 304.0 (M-H), Rₜ 2.65 min. Column: ZORBAX XDB C-18 (50 X 4.6 mm), 3.5 µm; Mobile Phase: A: 0.1% HCOOH in water, B: ACN; Flow Rate: 1.5 mL/min
**¹H NMR (400 MHz, DMSO-d₆):** δ 12.94 (brs, 1H), 9.30 (brs, 1H), 7.74 (d, 1H), 7.42-7.37 (m, 2H), 7.29-7.24 (m, 2H), 6.88-6.83 (m, 1H), 5.59-5.53 (m, 1H), 3.03-2.99 (m, 1H), 2.93-2.87 (m, 1H), 2.46 (m, 1H), 2.08-2.03 (m, 1H).

### Example 102. Synthesis of Compound III-10

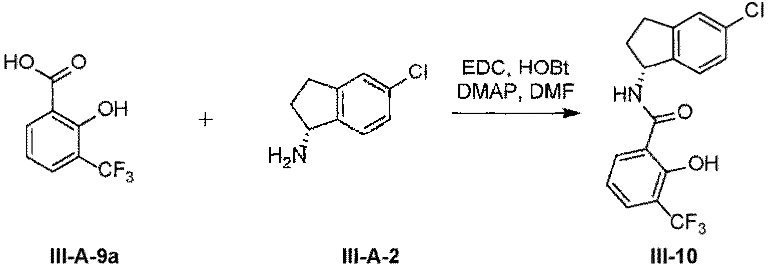

To a stirred solution of 2-hydroxy-3-(trifluoromethyl)benzoic acid (**III-A-9a,** 126 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2,** 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 8% EtOAc/PE to afford Compound **III-10** (21 mg, 0.05 mmol, 9% yield) as a solid. **LCMS:** 356.0 (M+H), Rₜ 2.87 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 14.10 (s, 1H), 9.47 (brs, 1H), 8.21 (d, 1H), 7.77 (d, 1H), 7.38 (s, 1H), 7.31-7.24 (m, 2H), 7.02 (s, 1H), 5.61-5.55 (m, 1H), 3.08-3.01 (m, 1H), 2.93-2.85 (m, 1H), 2.55 (m, 1H), 2.10-2.05 (m, 1H).

### Example 103. Synthesis of Compound III-11

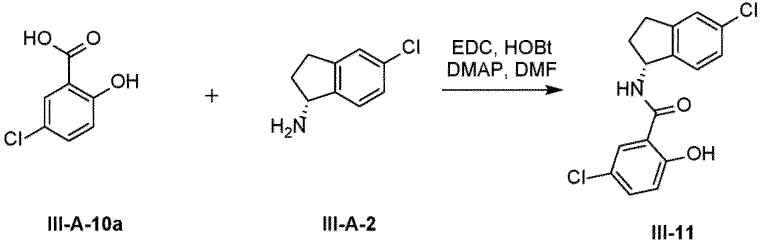

To a stirred solution of 5-chloro-2-hydroxy-benzoic acid (**III-A-10a,** 102 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 5% EtOAc/PE to afford Compound **III-11** (46 mg, 0.14 mmol, 23% yield).
**LCMS:** 322.1 (M+H), Rₜ 2.86 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 12.59 (s, 1H), 9.13 (d, 1H), 8.01 (d, 1H), 7.47-7.44 (dd, 1H), 7.37 (s, 1H), 7.30-7.24 (m, 2H), 6.96 (d, 1H), 5.56-5.50 (m, 1H), 3.05-2.99 (m, 1H), 2.92-2.86 (m, 1H), 2.46 (m, 1H), 2.06-2.00 (m, 1H).

### Example 104. Synthesis of Compound III-12

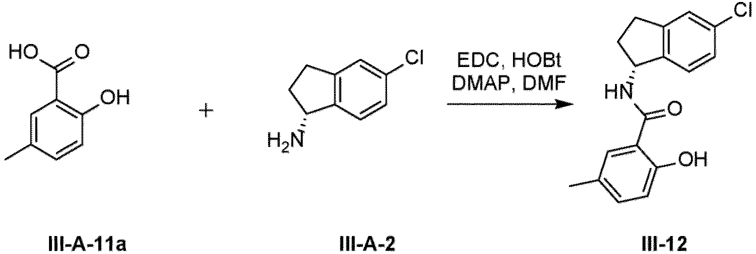

To a stirred solution of 2-hydroxy-5-methyl-benzoic acid (**III-A-11a,** 90 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 20% EtOAc/PE to afford Compound **III-12** (108 mg, 0.34 mmol, 57% yield) as a solid.
**LCMS:** 302.1 (M+H), Rₜ 2.75 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 12.32 (s, 1H), 9.00 (d, 1H), 7.74 (d, 1H), 7.37 (s, 1H), 7.27-7.21 (m, 3H), 6.81 (d, 1H), 5.57-5.52 (m, 1H), 3.06-2.99 (m, 1H), 2.92-2.84 (m, 1H), 2.49 (m, 1H), 2.23 (s, 3H), 2.08-1.99 (m, 1H).

### Example 105. Synthesis of Compound III-13

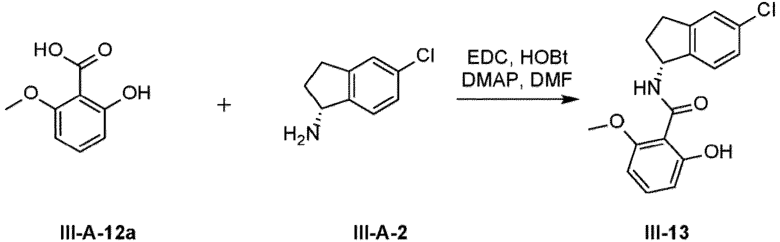

To a stirred solution of 2-hydroxy-6-methoxy-benzoic acid (**III-A-12a**, 100 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2,** 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 15% EtOAc/PE to afford Compound **III-13** (121 mg, 0.37 mmol, 63% yield) as a solid.

**LCMS:** 318.0 (M+H), Rₜ 2.90 min. Column: ZORBAX XDB C-18 (50 X 4.6 mm), 3.5 µm; Mobile Phase: A: 0.1% HCOOH in water, B: ACN; Flow Rate:1.5 mL/min

**¹H NMR (400 MHz, DMSO-d₆):** δ 12.79 (s, 1H), 8.71 (d, 1H), 7.35-7.25 (m, 4H), 6.57-6.52 (m, 2H), 5.54-5.48 (m, 1H), 3.83 (s, 3H), 2.99-2.85 (m, 2H), 2.46 (m, 1H), 2.07-2.01 (m, 1H).

### Example 106. Synthesis of Compound III-14

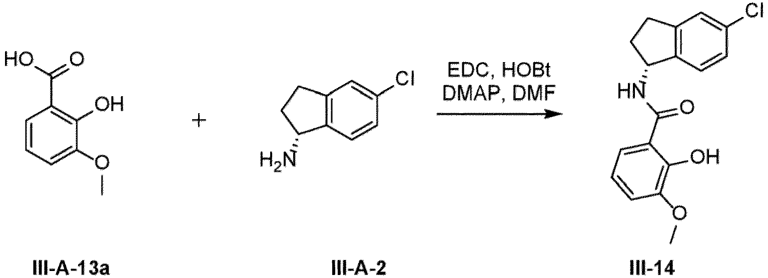

To a stirred solution of 2-hydroxy-3-methoxy-benzoic acid **(III-A-13a,** 100 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 15% EtOAc/PE to afford Compound **III-14** (122 mg, 0.36 mmol, 67% yield) as a solid.
**LCMS:** 318.1 (M+H), Rₜ 2.51 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 12.69 (brs, 1H), 9.05 (d, 1H), 7.50-7.48 (m, 1H), 7.37 (s, 1H), 7.25 (m, 2H), 7.13-7.11 (m, 1H), 6.81 (t, 1H), 5.59-5.53 (m, 1H), 3.79 (s, 3H), 3.06-2.99 (m, 1H), 2.92-2.84 (m, 1H), 2.45 (m, 1H), 2.10-2.03 (m, 1H).

### Example 107. Synthesis of Compound III-15

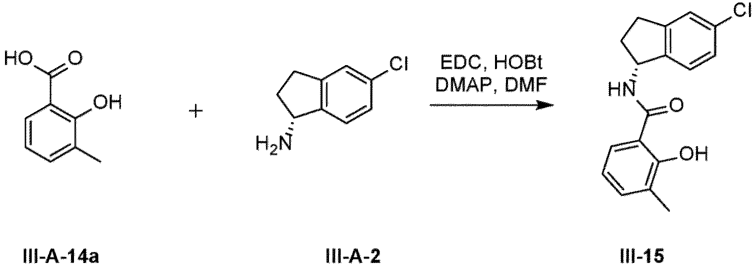

To a stirred solution of 2-hydroxy-3-methyl-benzoic acid (**III-A-14a,** 100 mg, 0.66 mmol) in DMF (3.0 mL) was added EDC (188 mg, 0.99 mmol) followed by HOBt (17 mg, 0.13 mmol), DMAP (120 mg, 0.99 mmol) and (1*R*)-5-chloroindan-1-amine (**III-A-2**, 110 mg, 0.66 mmol). The reaction mixture was stirred for 12 h at room temperature. The reaction mixture was treated with water (20 mL) and extracted with ethyl acetate (2 x 20 mL). The organic layer was washed with brine (20 mL), dried over Na₂SO₄ and concentrated. The crude product was purified by column chromatography on silica gel with 18% EtOAc/PE to afford Compound **III-15** (108 mg, 0.34 mmol, 53% yield) as a solid.
**LCMS:** 302.2 (M+H), Rₜ 2.88 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 13.19 (s, 1H), 9.12 (d, 1H), 7.77-7.75 (m, 1H), 7.37-7.31 (m, 2H), 7.25-7.24 (m, 2H), 6.79-6.75 (m, 1H), 5.60-5.54 (m, 1H), 3.06-2.99 (m, 1H), 2.92-2.84 (m, 1H), 2.45 (m, 1H), 2.18 (s, 3H), 2.11-2.06 (m, 1H).

### Example 108. Synthesis of Compound III-16

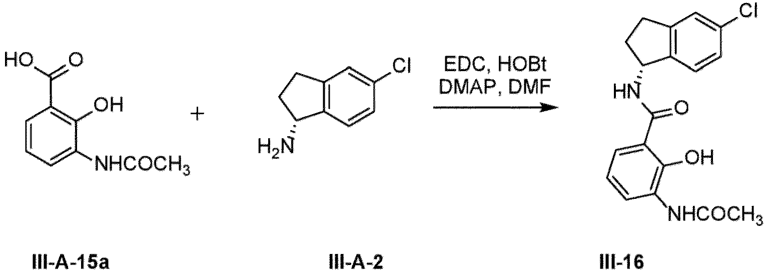

To a stirred solution of 3-acetamido-2-hydroxybenzoic acid (**III-A-15a,** 115 mg, 0.59 mmol) in DMF (3.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 120 mg, 0.72 mmol). The reaction mixture was stirred for 12 h at room temperature. The reaction mixture was treated with water (20 mL) and extracted with ethyl acetate (2 x 20 mL). The organic layer was washed with brine (20 mL), dried over Na₂SO₄ and concentrated. The crude product was purified by column chromatography on silica gel with 60% EtOAc/PE to afford Compound **III-16** (28 mg, 0.07 mmol, 13% yield) as a solid.
**LCMS:** 345.1 (M+H), Rₜ 2.35 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 13.56 (s, 1H), 9.28 (s, 1H), 9.23 (s, 1H), 8.08 (d, 1H), 7.67 (d, 1H), 7.38 (s, 1H), 7.31-7.24 (m, 2H), 6.83 (t, 1H), 5.61-5.55 (m, 1H), 3.07-3.00 (m, 1H), 2.93-2.85 (m, 1H), 2.45 (m, 1H), 2.12-2.05 (m, 4H).

### Example 109. Synthesis of Compound III-17

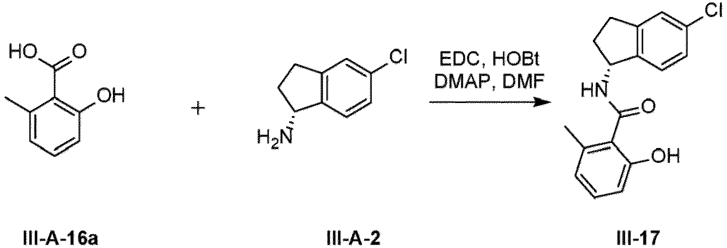

To a stirred solution of 2-hydroxy-6-methyl-benzoic acid (**III-A-16a,** 90 mg, 0.59 mmol) in DMF (2.0 mL) was added EDC (170 mg, 0.89 mmol) followed by HOBt (16 mg, 0.12 mmol), DMAP (108 mg, 0.89 mmol) and (1*R*)-5-chloroindan-1-amine (**III-A-2**, 100 mg, 0.59 mmol). The reaction mixture was stirred for 12 h at room temperature. The reaction mixture was treated with water (15 mL) and extracted with ethyl acetate (2 x 15 mL). The organic layer was washed with brine (20 mL), dried over Na₂SO₄ and concentrated. The crude product was purified by column chromatography on silica gel with 5% EtOAc/PE to afford Compound **III-17** (24 mg, 0.08 mmol, 13% yield) as a solid.
**LCMS:** 302.1 (M+H), Rₜ 2.17 min. Column: ZORBAX XDB C-18 (50 X 4.6 mm), 3.5 µm; Mobile Phase: A: 0.1% HCOOH in water, B: ACN; Flow Rate:1.5 mL/min
**¹H NMR (400 MHz, DMSO-d₆):** δ 9.47 (s, 1H), 8.50 (d, 1H), 7.35 (d, 1H), 7.29 (s, 1H), 7.25-7.23 (m, 1H), 7.03 (t, 1H), 6.67-6.61 (m, 2H), 5.48-5.42 (m, 1H), 2.96-2.77 (m, 2H), 2.44-2.37 (m, 1H), 2.20 (s, 3H), 1.96-1.91 (m, 1H).

### Example 110. Efficacy of exemplary compounds in the inhibition of KCNT1

### KCNT1-WT-Basal - Patch Clamp Assay

Inhibition of KCNT1 (KNa1.1, Slack) was evaluated using a tetracycline inducible cell line (HEK-TREX). Currents were recorded using the SyncroPatch 384PE automated, patch clamp system. Pulse generation and data collection were performed with PatchController384 V1.3.0 and DataController384 V1.2.1 (Nanion Technologies). The access resistance and apparent membrane capacitance were estimated using built-in protocols. Current were recorded in perforated patch mode (10 µM escin) from a population of cells. The cells were lifted, triturated, and resuspended at 800,000 cells/ml. The cells were allowed to recover in the cell hotel prior to experimentation. Currents were recorded at room temperature. The external solution contained the following (in mM): NaCl 105, NMDG 40, KCl 4, MgCh 1, CaCh 5 and HEPES 10 (pH = 7.4, Osmolarity ~300 mOsm). The extracellular solution was used as the wash, reference and compound delivery solution. The internal solution contained the following (in mM): NaCl 70, KF 70, KCl 10, EGTA 5, HEPES 5 and Escin 0.01 (pH = 7.2, Osmolarity ~295 mOsm). Escin is made at a 5mM stock in water, aliquoted, and stored at -20°C. The compound plate was created at 2x concentrated in the extracellular solution. The compound was diluted to 1:2 when added to the recording well. The amount of DMSO in the extracellular solution was held constant at the level used for the highest tested concentration. A holding potential of -80 mV with a 100ms step to 0mV was used. Mean current was measured during the step to 0 mV. 100 µM Bepridil was used to completely inhibit KCNT1 current to allow for offline subtraction of non-KCNT1 current. The average mean current from 3 sweeps was calculated and the % inhibition of each compound was calculated. The % Inhibition as a function of the compound concentration was fit with a Hill equation to derive IC₅₀, slope, min and max parameters. If KCNT1 inhibition was less than 50% at the highest tested concentration or if an IC₅₀ could not be calculated, then a percent inhibition was reported in place of the IC₅₀.

Results from this assay are summarized in Table 1 below. In this table, "A" indicates IC₅₀ of less than or equal to 1 µM; "B" indicates inhibition of between 1 µM to 20 µM; and "C" indicates inhibition of greater than or equal to 20 µM.

**Table 1.**

| **Compound** | **KCNT1 WB IC₅₀ (µM)** |
|---|---|
| **I-1** | B |
| **I-2** | B |
| **I-3** | B |
| **I-4** | A |
| **I-5** | B |
| **I-6** | A |
| **I-7** | C |
| **I-8** | B |
| **I-9** | B |
| **I-10** | B |
| **I-11** | C |
| **I-12** | C |
| **I-13** | C |
| **I-14** | C |
| **I-15** | A |
| **I-16** | A |
| **I-17** | A |
| **I-18** | A |
| **I-19** | A |
| **I-20** | A |
| **I-21** | A |
| **I-22** | A |
| **I-23** | B |
| **I-24** | A |
| **I-25** | C |
| **I-26** | A |
| **I-27** | A |
| **I-28** | A |
| **I-29** | A |
| **I-30** | C |
| **I-31** | A |
| **I-32** | A |
| **I-33** | C |
| **I-34** | A |
| **I-35** | A |
| **I-36** | A |
| **I-37** | A |
| **I-38** | B |
| **I-39** | A |
| **I-40** | A |
| **I-41** | A |
| **I-42** | A |
| **I-43** | A |
| **I-44** | A |
| **I-45** | A |
| **I-46** | A |
| **I-47** | A |
| **II-1** | C |
| **II-2** | A |
| **II-3** | C |
| **II-4** | A |
| **II-5** | C |
| **II-6** | B |
| **II-7** | C |
| **II-8** | B |
| **II-9** | B |
| **II-10** | A |
| **II-11** | B |
| **II-12** | C |
| **II-13** | A |
| **II-14** | A |
| **II-15** | B |
| **II-16** | C |
| **II-17** | C |
| **II-18** | C |
| **II-19** | B |
| **II-20** | B |
| **II-21** | B |
| **II-22** | A |
| **II-23** | B |
| **II-24** | B |
| **II-25** | A |
| **II-26** | B |
| **II-27** | A |
| **II-28** | A |
| **II-29** | C |
| **II-30** | A |
| **II-31** | C |
| **II-32** | A |
| **II-33** | C |
| **II-34** | B |
| **II-35** | A |
| **II-36** | B |
| **II-37** | B |
| **II-38** | A |
| **II-39** | A |
| **II-40** | B |
| **II-41** | C |
| **II-42** | A |
| **II-43** | B |
| **II-44** | B |
| **II-45** | B |
| **II-46** | C |
| **II-47** | B |
| **II-48** | B |
| **II-49** | B |
| **II-50** | B |
| **II-51** | B |
| **II-52** | C |
| **II-53** | A |
| **II-54** | C |
| **II-55** | B |
| **II-56** | A |
| **II-57** | B |
| **II-58** | C |
| **II-59** | C |
| **III-1** | C |
| **III-2** | A |
| **III-3** | A |
| **III-4** | A |
| **III-5** | A |
| **III-6** | A |
| **III-7** | A |
| **III-8** | B |
| **III-9** | A |
| **III-10** | A |
| **III-11** | A |
| **III-12** | A |
| **III-13** | B |
| **III-14** | A |
| **III-15** | A |
| **III-16** | A |
| **III-17** | C |

### Equivalents and Scope

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

### NUMBERED EMBODIMENTS

1. A pharmaceutical composition comprising a compound of Formula **I-I:** 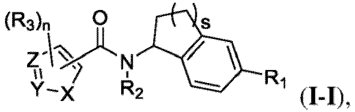
   or a pharmaceutically acceptable salt thereof, wherein
      X is selected from the group consisting of NH, O, and S, wherein the hydrogen of NH may be substituted with R₃;
   Y is selected from N and CH, wherein the hydrogen of CH may be substituted with R₃;
   Z is selected from N and CH, wherein the hydrogen of CH may be substituted with R₃, or Z is C when Z is substituted with the -C(O)N(R₂)- moiety;
      R₁ is selected from the group consisting of -Cl, -F, and C₁₋₆alkyl substituted with one or more substituents independently selected from -Cl and -F;
      R₂ is hydrogen;
      each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆,-C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
   n is selected from the group consisting of 0, 1, 2, 3, and 4;
      R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
      each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and
   s is 1 or 2;
   and a pharmaceutically acceptable excipient.
2. The pharmaceutical composition of embodiment 1, wherein x is NH.
3. The pharmaceutical composition of embodiment 1, wherein x is O.
4. The pharmaceutical composition of embodiment 1, wherein x is S.
5. The pharmaceutical composition of any one of embodiments 1-4, wherein Y is N.
6. The pharmaceutical composition of any one of embodiments 1-4, wherein Y is CH.
7. The pharmaceutical composition of any one of embodiments 1-6, wherein Z is N.
8. The pharmaceutical composition of any one of embodiments 1-6, wherein Z is CH.
9. The pharmaceutical composition of embodiment 1, wherein the compound is a compound of Formula **I-Ia** or Formula **I-Ial:** 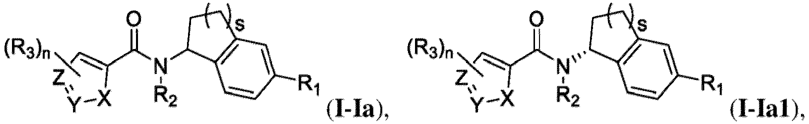 or a pharmaceutically acceptable salt thereof, wherein the variables are as defined in embodiment 1.
10. The pharmaceutical composition of embodiment 1 or 9, wherein the compound is a compound of Formula **I-Ib** or Formula **I-Ib1:** 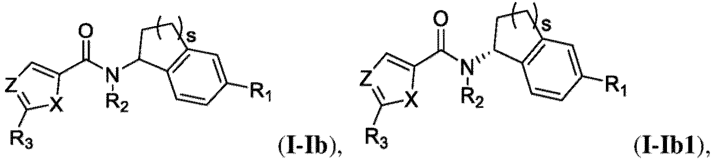 or a pharmaceutically acceptable salt thereof, wherein the variables are as defined in embodiment 1.
11. The pharmaceutical composition of embodiment 1 or 9, wherein the compound is a compound of Formula **I-a** or Formula **I-a1:** 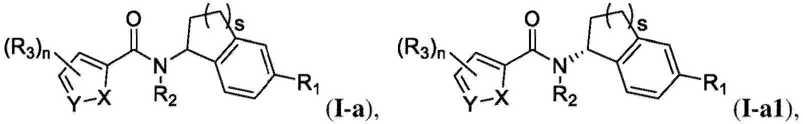 or a pharmaceutically acceptable salt thereof, wherein the variables are as defined in embodiment 1.
12. The pharmaceutical composition of embodiment 1, wherein the compound is a compound of Formula **I-Ic** or Formula **I-Ic1:** 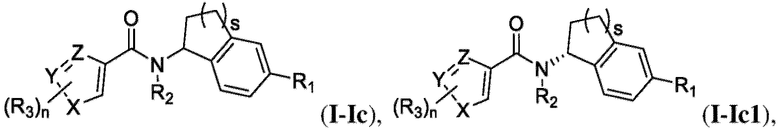 or a pharmaceutically acceptable salt thereof, wherein the variables are as defined in embodiment 1.
13. The pharmaceutical composition of embodiment 1, wherein the compound is a compound of Formula **I-c** or Formula **I-c1:** 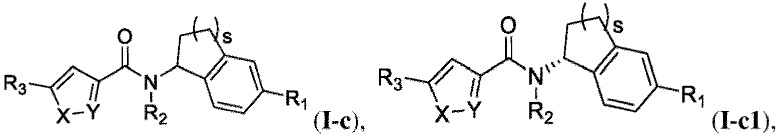 or a pharmaceutically acceptable salt thereof, wherein the variables are as defined in embodiment 1.
14. The pharmaceutical composition of any one of embodiments 1, 9 and 11, wherein the compound is a compound of Formula **I-d** or Formula **I-dl:** 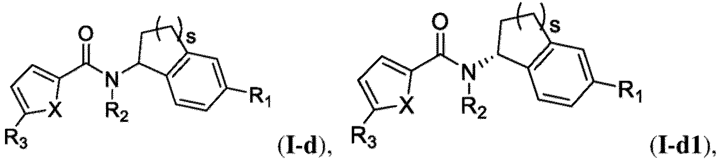 or a pharmaceutically acceptable salt thereof, wherein the variables are as defined in embodiment 1.
15. The pharmaceutical composition of any one of embodiments 1-14, wherein R₁ is selected from the group consisting of -Cl, -F, and -CF₃.
16. The pharmaceutical composition of any one of embodiments 1-15, wherein R₁ is -Cl.
17. The pharmaceutical composition of any one of embodiments 1-15, wherein Ri is -F.
18. The pharmaceutical composition of any one of embodiments 1-15, wherein R₁ is-CF₃.
19. The pharmaceutical composition of any one of embodiments 1-18, wherein each R₃ is selected from the group consisting of -Cl, methyl, methyl substituted with -NR₄R₅ or -S(O)-₂R₆, methoxymethyl, trifluoromethyl, ethyl, cyclopropyl, cyclohexyl, -S(O)₂R₆, -C(O)NR₄R₅, and -S(O)₂NR₄R₅.
20. The pharmaceutical composition of any one of embodiments 1-19, wherein each R₃ is selected from the group consisting of methyl, ethyl, cyclopropyl, cyclohexyl, and-S(O)₂NR₄R₅.
21. The pharmaceutical composition of any one of embodiments 1-20, wherein each R₃ is selected from the group consisting of methyl, cyclopropyl, and -S(O)₂NR₄R₅.
22. The pharmaceutical composition of any one of embodiments 1-21, wherein each R₃ is methyl.
23. The pharmaceutical composition of any one of embodiments 1-21, wherein each R₃ is cyclopropyl.
24. The pharmaceutical composition of any one of embodiments 1-21, wherein each R₃ is -S(O)₂NR₄R₅.
25. The pharmaceutical composition of any one of embodiments 1-18, wherein R₃ is C₁₋₆alkyl substituted with -NR₄R₅ or -S(O)₂R₆.
26. The pharmaceutical composition of any one of embodiments 1-9, 11, 12, and 15-25, wherein n is 1 or 2.
27. The pharmaceutical composition of any one of embodiments 1-9, 11, 12, and 15-26, wherein n is 2.
28. The pharmaceutical composition of any one of embodiments 1-9, 11, 12, and 15-26, wherein n is 1.
29. The pharmaceutical composition of any one of embodiments 1-28, wherein each of R₄ and R₅ are independently selected from the group consisting of hydrogen, methyl, ethyl, cyclopropyl, and -C(O)CH₃.
30. The pharmaceutical composition of any one of embodiments 1-29, wherein each of R₄ and R₅ are independently hydrogen or methyl.
31. The pharmaceutical composition of any one of embodiments 1-30, wherein each R₄ and R₅ are hydrogen.
32. The pharmaceutical composition of any one of embodiments 1-30, wherein each R₄ and R₅ are methyl.
33. The pharmaceutical composition of any one of embodiments 1-30, wherein R₄ is H and R₅ is methyl.
34. The pharmaceutical composition of any one of embodiments 1-28, wherein R₄ and R₅ are taken together with the nitrogen to which R₄ and R₅ are attached to form a 4-6 membered heterocyclyl optionally substituted with -OH, methyl, or -OCH₃.
35. The pharmaceutical composition of any one of embodiments 1-28, wherein R₆ is selected from the group consisting of methyl, ethyl, methoxyethyl, and cyclopropyl.
36. The pharmaceutical composition of any one of embodiments 1-35, wherein s is 2.
37. The pharmaceutical composition of any one of embodiments 1-35, wherein s is 1.
38. The pharmaceutical composition of embodiment 1, wherein the compound is selected from the group consisting of: 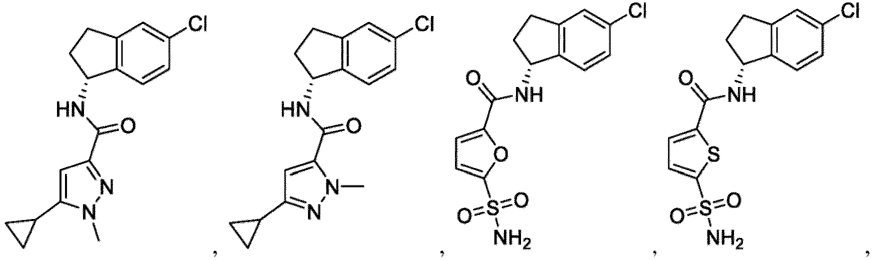 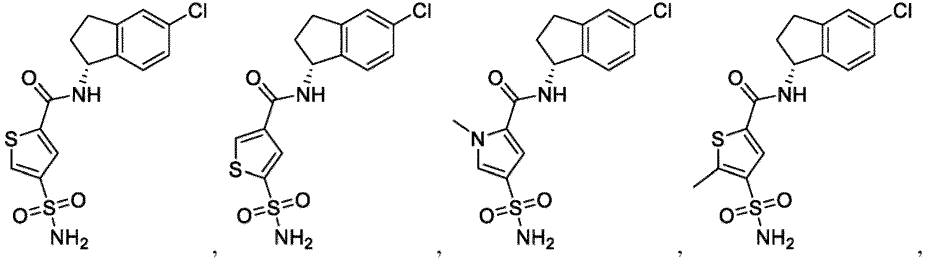 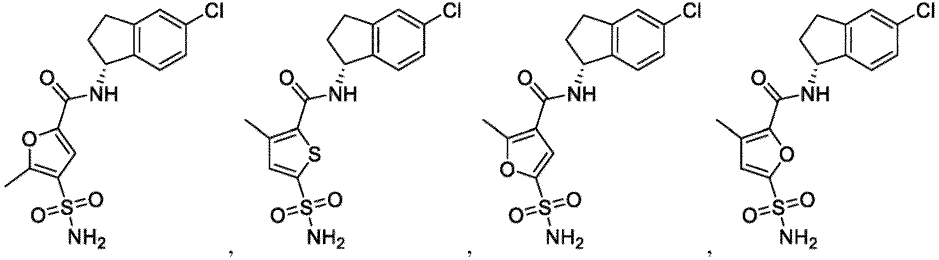 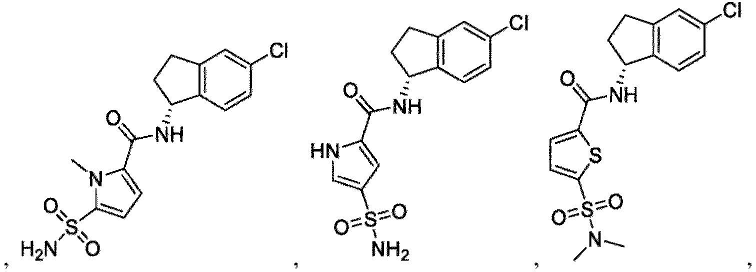 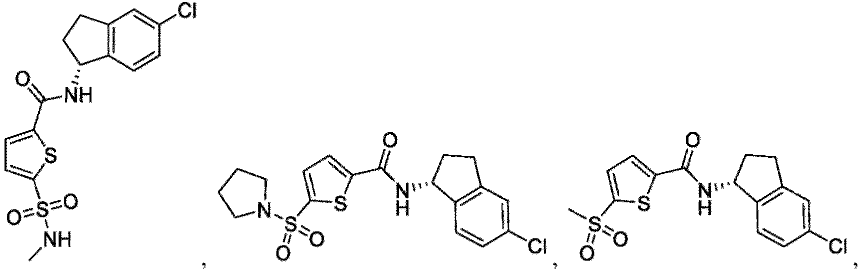 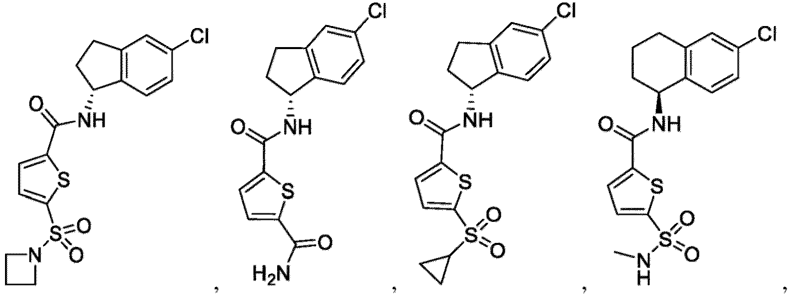 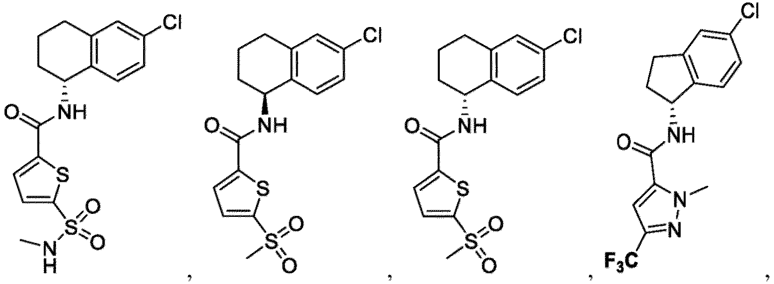 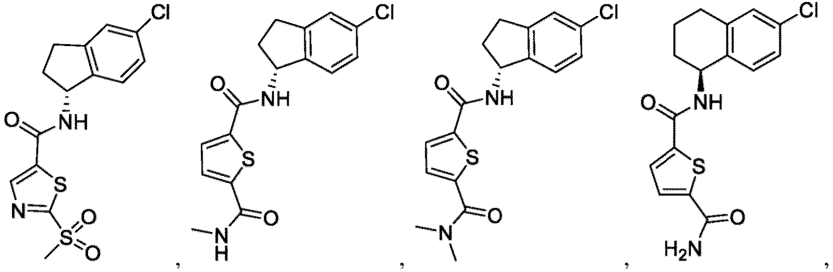 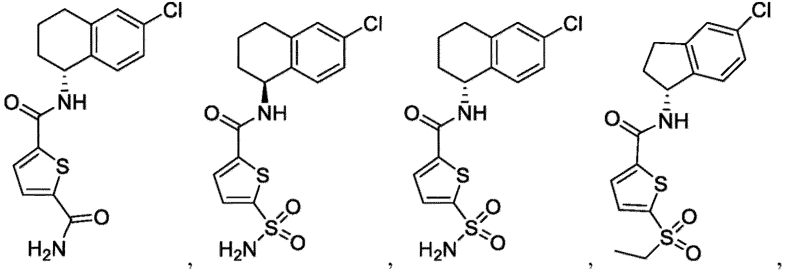 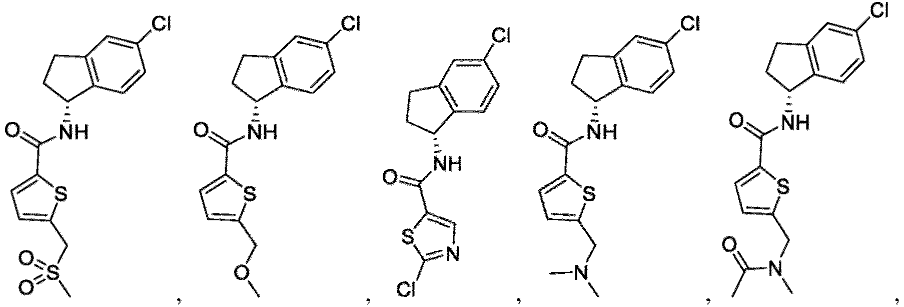 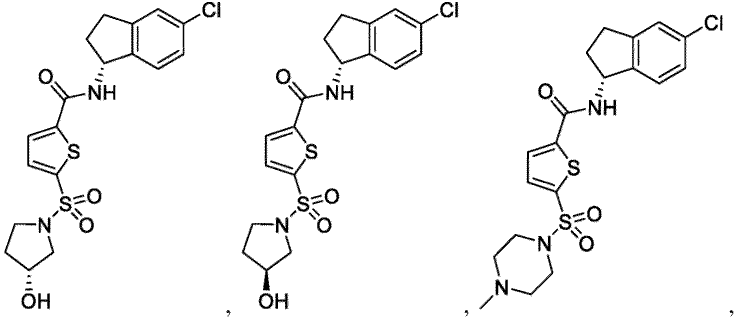 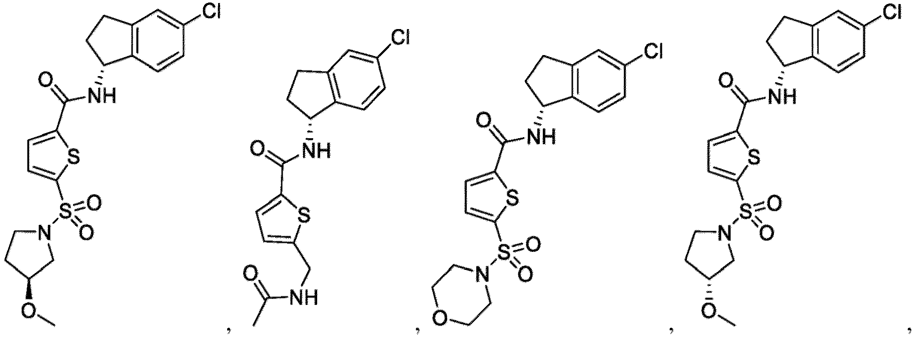 and 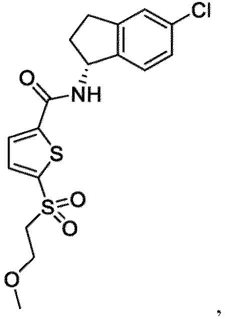 or a pharmaceutically acceptable salt thereof.
39. A method of treating a neurological disease or disorder, wherein the method comprises administering to a subject in need thereof a compound of Formula **I-I:** 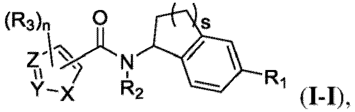 or a pharmaceutically acceptable salt thereof, wherein
   X is selected from the group consisting of NH, O, and S, wherein the hydrogen of NH may be substituted with R₃;
   Y is selected from N and CH, wherein the hydrogen of CH may be substituted with R₃;
   Z is selected from N and CH, wherein the hydrogen of CH may be substituted with R₃, or Z is C when Z is substituted with the -C(O)N(R₂)- moiety;
   R₁ is selected from the group consisting of -Cl, -F, and C₁₋₆alkyl substituted with one or more substituents independently selected from -Cl and -F;
   R₂ is hydrogen;
   each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆,-C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
   n is selected from the group consisting of 0, 1, 2, 3, and 4;
   R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
   each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and
   s is 1 or 2.
40. A method of treating a disease or condition associated with excessive neuronal excitability, wherein the method comprises administering to a subject in need thereof a compound of Formula **I-I:** 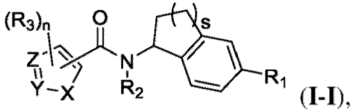 or a pharmaceutically acceptable salt thereof, wherein
   X is selected from the group consisting of NH, O, and S, wherein the hydrogen of NH may be substituted with R₃;
   Y is selected from N and CH, wherein the hydrogen of CH may be substituted with R₃;
   Z is selected from N and CH, wherein the hydrogen of CH may be substituted with R₃, or Z is C when Z is substituted with the -C(O)N(R₂)- moiety;
   R₁ is selected from the group consisting of -Cl, -F, and C₁₋₆alkyl substituted with one or more substituents independently selected from -Cl and -F;
   R₂ is hydrogen;
   each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆,-C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
   n is selected from the group consisting of 0, 1, 2, 3, and 4;
   R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
   each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and
   s is 1 or 2.
41. A method of treating a disease or condition associated with a gain-of-function mutation of a gene (e.g., KCNT1), wherein the method comprises administering to a subject in need thereof a compound of Formula **I-I:** 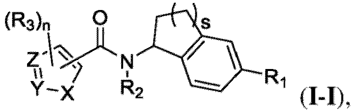 or a pharmaceutically acceptable salt thereof, wherein
   X is selected from the group consisting of NH, O, and S, wherein the hydrogen of NH may be substituted with R₃;
   Y is selected from N and CH, wherein the hydrogen of CH may be substituted with R₃;
   Z is selected from N and CH, wherein the hydrogen of CH may be substituted with R₃, or Z is C when Z is substituted with the -C(O)N(R₂)- moiety;
   R₁ is selected from the group consisting of -Cl, -F, and C₁₋₆alkyl substituted with one or more substituents independently selected from -Cl and -F;
   R₂ is hydrogen;
   each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆,-C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
   n is selected from the group consisting of 0, 1, 2, 3, and 4;
   R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
   each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and
   s is 1 or 2.
42. A method of treating a neurological disease or disorder, wherein the method comprises administering to a subject in need thereof a pharmaceutical composition of any one of embodiments 1-38.
43. A method of treating a disease or condition associated with excessive neuronal excitability, wherein the method comprises administering to a subject in need thereof a pharmaceutical composition of any one of embodiments 1-38.
44. A method of treating a disease or condition associated with a gain-of-function mutation of a gene (e.g., KCNT1), wherein the method comprises administering to a subject in need thereof a pharmaceutical composition of any one of embodiments 1-38.
45. The method of any one of embodiments 39-44, wherein the neurological disease or disorder, the disease or condition associated with excessive neuronal excitability, or the disease or condition associated with a gain-of-function mutation of a gene (e.g., KCNT1) is epilepsy, an epilepsy syndrome, or an encephalopathy.
46. The method of any one of embodiments 39-44, wherein the neurological disease or disorder, the disease or condition associated with excessive neuronal excitability, or the disease or condition associated with a gain-of-function mutation of a gene (e.g., KCNT1) is a genetic or pediatric epilepsy or a genetic or pediatric epilepsy syndrome.
47. The method of any one of embodiments 39-44, wherein the neurological disease or disorder, the disease or condition associated with excessive neuronal excitability, or the disease or condition associated with a gain-of-function mutation of a gene (e.g., KCNT1) is a cardiac dysfunction.
48. The method of any one of embodiments 39-44, wherein the neurological disease or disorder, the disease or condition associated with excessive neuronal excitability, or the disease or condition associated with a gain-of-function mutation of a gene (e.g., KCNT1) is selected from the group consisting of epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, Lennox Gastaut syndrome, seizures (e.g., Generalized tonic clonic seizures, Asymmetric Tonic Seizures), leukodystrophy, leukoencephalopathy, intellectual disability, Multifocal Epilepsy, Drug resistant epilepsy, Temporal lobe epilepsy, or cerebellar ataxia).
49. The method of any one of embodiments 39-44, wherein the neurological disease or disorder, the disease or condition associated with excessive neuronal excitability, or the disease or condition associated with a gain-of-function mutation of a gene (e.g., KCNT1) is selected from the group consisting of cardiac arrhythmia, sudden unexpected death in epilepsy, Brugada syndrome, and myocardial infarction.
50. The methof of any one of embodiments 39-44, wherein the neurological disease or disorder, the disease or condition associated with excessive neuronal excitability, or the disease or condition associated with a gain-of-function mutation of a gene (e.g., KCNT1) is selected from pain and related conditions (e.g. neuropathic pain, acute/chronic pain, migraine).
51. The method of any one of embodiments 39-44, the neurological disease or disorder, the disease or condition associated with excessive neuronal excitability, or the disease or condition associated with a gain-of-function mutation of a gene (e.g., KCNT1) is a muscle disorder (e.g. myotonia, neuromyotonia, cramp muscle spasms, spasticity).
52. The method of any one of embodiments 39-44, wherein the neurological disease or disorder, the disease or condition associated with excessive neuronal excitability, or the disease or condition associated with a gain-of-function mutation of a gene (e.g., KCNT1) is selected from itch and pruritis, ataxia and cerebellar ataxias.
53. The method of any one of embodiments 39-44, wherein the neurological disease or disorder, the disease or condition associated with excessive neuronal excitability, or the disease or condition associated with a gain-of-function mutation of a gene (e.g., KCNT1) is selected from psychiatric disorders (e.g. major depression, anxiety, bipolar disorder, schizophrenia).
54. The method of any one of embodiments 39-44, wherein the neurological disease or disorder or the disease or condition associated with excessive neuronal excitability and/or a gain-of-function mutation in a gene (e.g., KCNT1) is selected from the group consisting of learning disorders, Fragile X, neuronal plasticity, and autism spectrum disorders.
55. The method of any one of embodiments 39-44, wherein the neurological disease or disorder, the disease or condition associated with excessive neuronal excitability, or the disease or condition associated with a gain-of-function mutation of a gene (e.g., KCNT1) is selected from the group consisting of epileptic encephalopathy with SCN1A, SCN2A, SCN8A mutations, early infantile epileptic encephalopathy, Dravet syndrome, Dravet syndrome with SCN1A mutation, generalized epilepsy with febrile seizures, intractable childhood epilepsy with generalized tonic-clonic seizures, infantile spasms, benign familial neonatal-infantile seizures, SCN2A epileptic encephalopathy, focal epilepsy with SCN3A mutation, cryptogenic pediatric partial epilepsy with SCN3A mutation, SCN8A epileptic encephalopathy, sudden unexpected death in epilepsy, Rasmussen encephalitis, malignant migrating partial seizures of infancy, autosomal dominant nocturnal frontal lobe epilepsy, sudden expected death in epilepsy (SUDEP), KCNQ2 epileptic encephalopathy, and KCNT1 epileptic encephalopathy.
56. A pharmaceutical composition comprising a compound of Formula **II:** 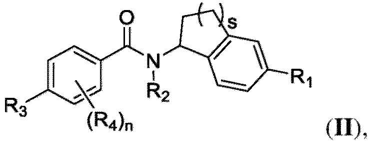 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl,-NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and-S(O)₂-C₁₋₆alkyl, wherein the C₁₋₆alkyl is optionally substituted with one or more halogen or C₁₋₆alkoxy;
   each R₄ is independently selected from the group consisting of C₁₋₆alkyl, halogen, and -OH; wherein R₄ is substituted at the carbon adjacent to R₃ when R₄ is -OH;
   n is selected from the group consisting of 0, 1, 2, 3, and 4;
   R₅, R₆, R₉, and R₁₀ are each independently hydrogen or C₁₋₆alkyl;
   each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
   s is 1 or 2;
   and a pharmaceutically acceptable excipient.
57. The pharmaceutical composition of embodiment 56, wherein the compound is a compound of Formula **II-a,** Formula **II-a1,** or Formula **II-a2:** 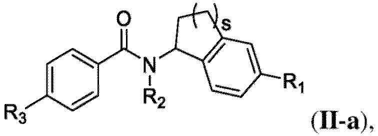 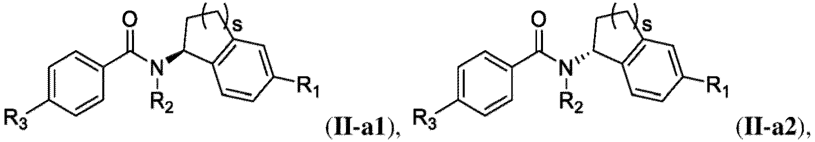 or a pharmaceutically acceptable salt thereof, wherein the variables are as defined in embodiment 56.
58. The pharmaceutical composition of embodiment 56, wherein the compound is a compound of Formula **II-b,** Formula **II-b1**, or Formula **II-b2:** 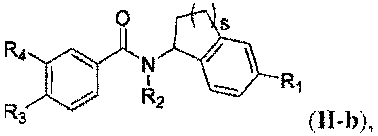 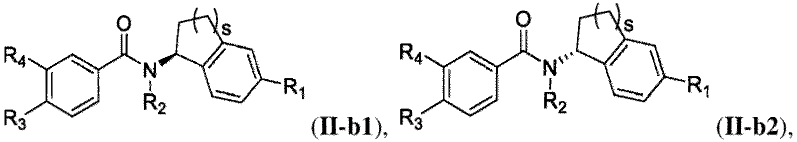 or a pharmaceutically acceptable salt thereof, wherein the variables are as defined in embodiment 56.
59. The pharmaceutical composition of embodiment 56, wherein the compound is a compound of Formula **II-c,** Formula **II-c1**, or Formula **II-c2:** 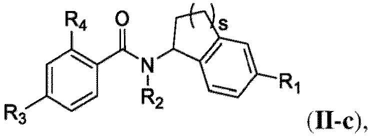 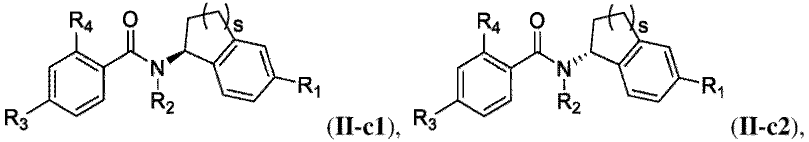 or a pharmaceutically acceptable salt thereof, wherein the variables are as defined in embodiment 56.
60. The pharmaceutical composition of any one of embodiments 56-59, wherein R₁ is selected from the group consisting of -Cl, -F, and -CF₃.
61. The pharmaceutical composition of any one of embodiments 56-60, wherein R₁ is -Cl.
62. The pharmaceutical composition of any one of embodiments 56-60, wherein R₁ is -F.
63. The pharmaceutical composition of any one of embodiments 56-60, wherein R₁ is-CF₃.
64. The pharmaceutical composition of any one of embodiments 56-63, wherein R₃ is selected from the group consisting of -F, methoxy, -NH₂, 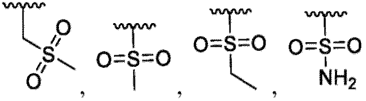 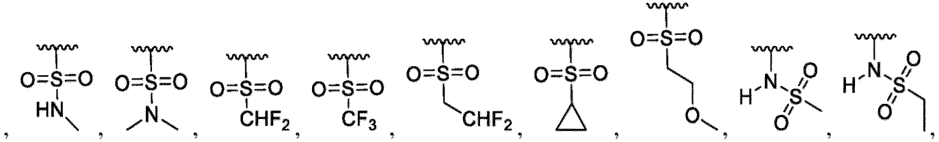 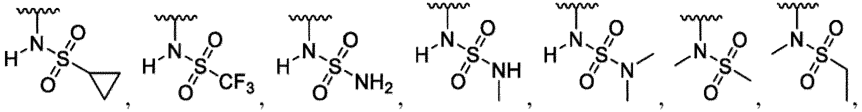 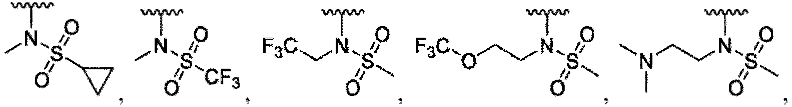 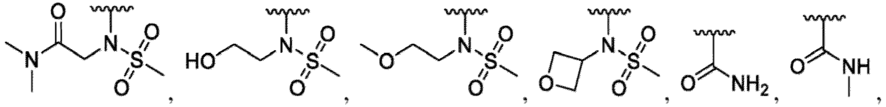 and 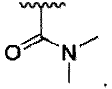
65. The pharmaceutical composition of any one of embodiments 56-64, wherein R₃ is selected from the group consisting of 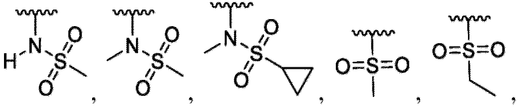 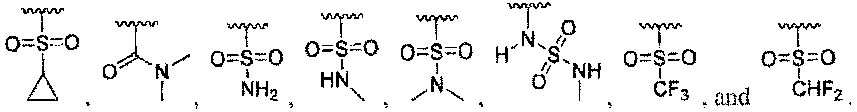
66. The pharmaceutical composition of any one of embodiments 56-64, wherein R₃ is 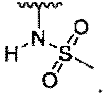
67. The pharmaceutical composition of any one of embodiments 56-64, wherein R₃ is 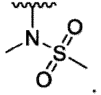
68. The pharmaceutical composition of any one of embodiments 56-64, wherein R₃ is 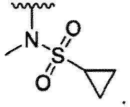
69. The pharmaceutical composition of any one of embodiments 56-64, wherein R₃ is 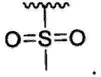
70. The pharmaceutical composition of any one of embodiments 56-64, wherein R₃ is 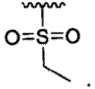
71. The pharmaceutical composition of any one of embodiments 56-64, wherein R₃ is 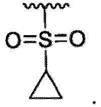
72. The pharmaceutical composition of any one of embodiments 56-64, wherein R₃ is 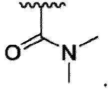
73. The pharmaceutical composition of any one of embodiments 56-64, wherein R₃ is 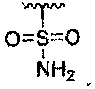
74. The pharmaceutical composition of any one of embodiments 56-64, wherein R₃ is 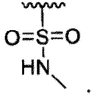
75. The pharmaceutical composition of any one of embodiments 56-64, wherein R₃ is 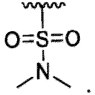
76. The pharmaceutical composition of any one of embodiments 56-64, wherein R₃ is 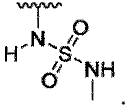
77. The pharmaceutical composition of any one of embodiments 56-64, wherein R₃ is 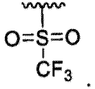
78. The pharmaceutical composition of any one of embodiments 56-64, wherein R₃ is 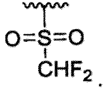
79. The pharmaceutical composition of any one of embodiments 56 and 58-78, wherein R₄ is -F or methyl.
80. The pharmaceutical composition of any one of embodiments 56 and 58-79, wherein R₄ is -F.
81. The pharmaceutical composition of any one of embodiments 56 and 58-79, wherein R₄ is methyl.
82. The pharmaceutical composition of any one of embodiments 56 and 60-81, wherein n is 1.
83. The pharmaceutical composition of any one of embodiments 56-82, wherein each R₅, R₆, R₉, and R₁₀ are hydrogen.
84. The pharmaceutical composition of any one of embodiments 56-82, wherein each R₅, R₆, R₉, and R₁₀ are methyl.
85. The pharmaceutical composition of any one of embodiments 56-82, wherein R₅ is H and R₆ is methyl.
86. The pharmaceutical composition of any one of embodiments 56-85, wherein R₇ is selected from the group consisting of hydrogen, methyl, ethyl, and 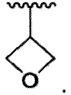
87. The pharmaceutical composition of any one of embodiments 56-86, wherein R₇ is hydrogen.
88. The pharmaceutical composition of any one of embodiments 56-86, wherein R₇ is methyl.
89. The pharmaceutical composition of any one of embodiments 56-86, wherein R₇ is ethyl.
90. The pharmaceutical composition of any one of embodiments 56-86, wherein R₇ is 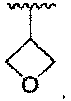
91. The pharmaceutical composition of any one of embodiments 56-90, wherein s is 1.
92. The pharmaceutical composition of any one of embodiments 56-90, wherein s is 2.
93. The pharmaceutical composition of embodiment 56, wherein the compound is selected from the group consisting of: 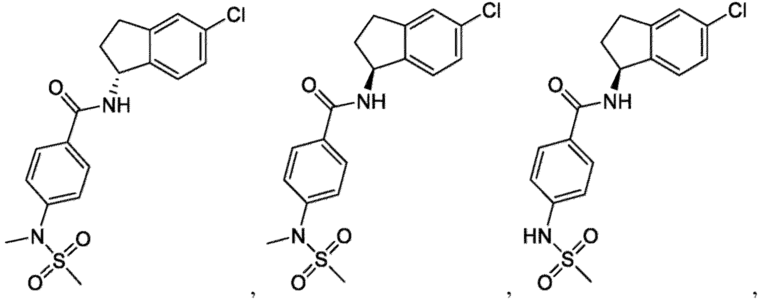 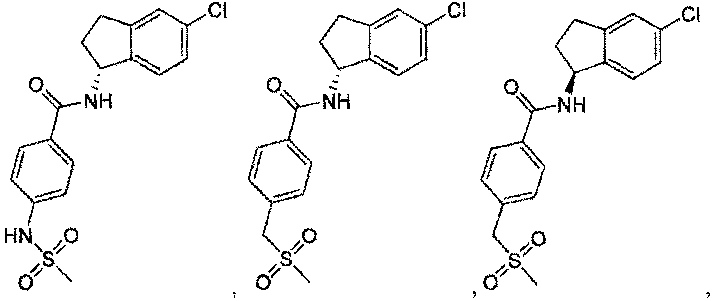 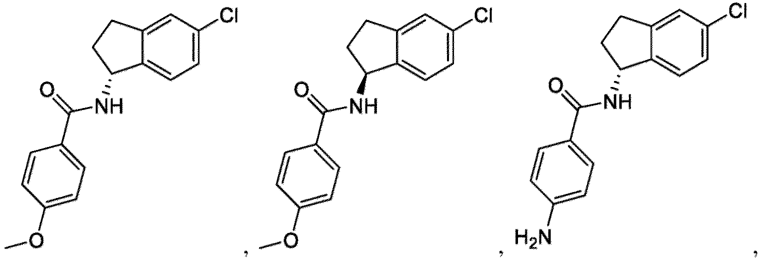 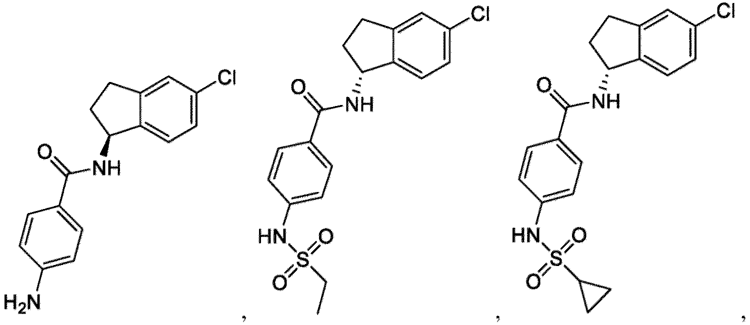 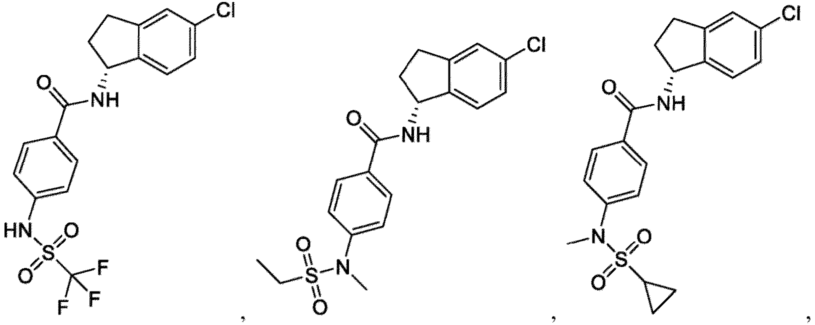 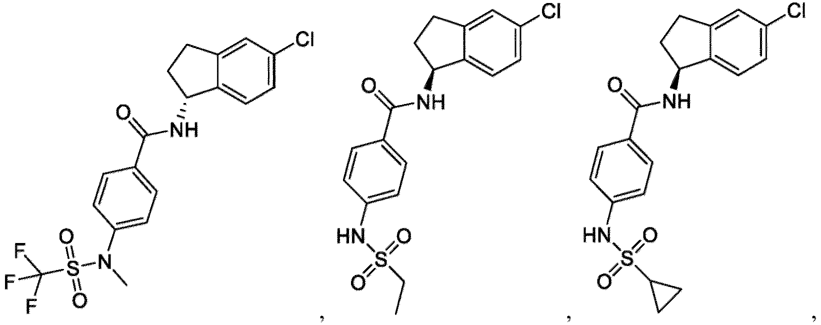 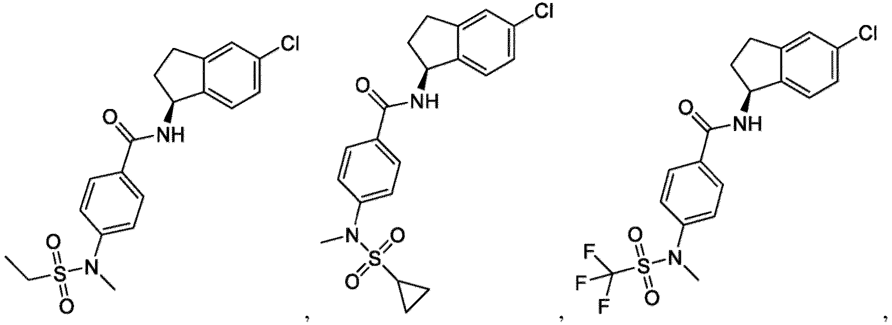 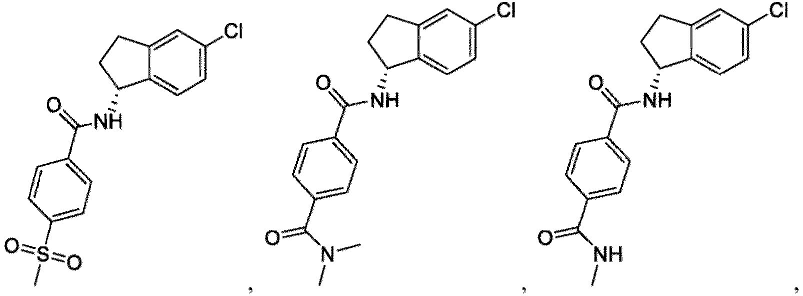 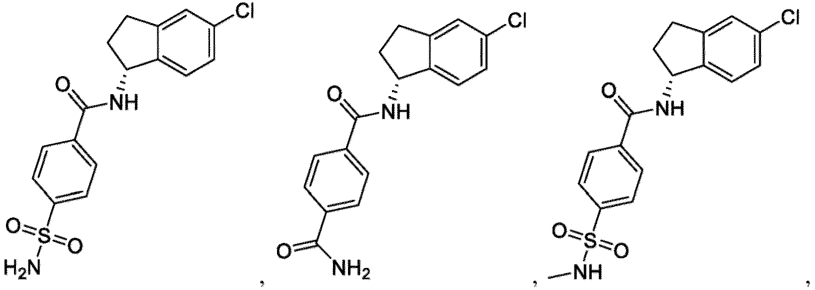 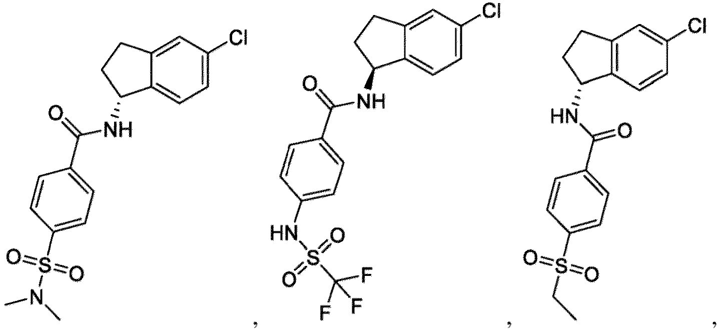 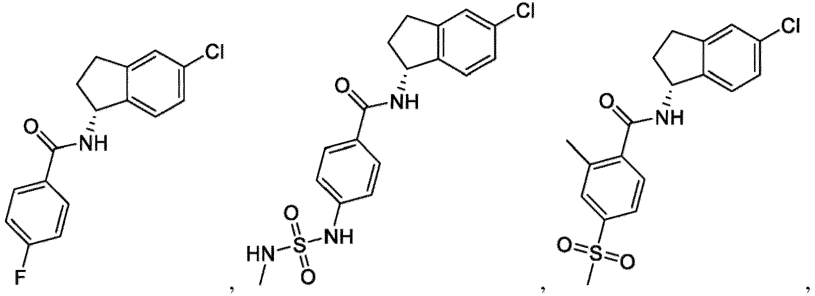 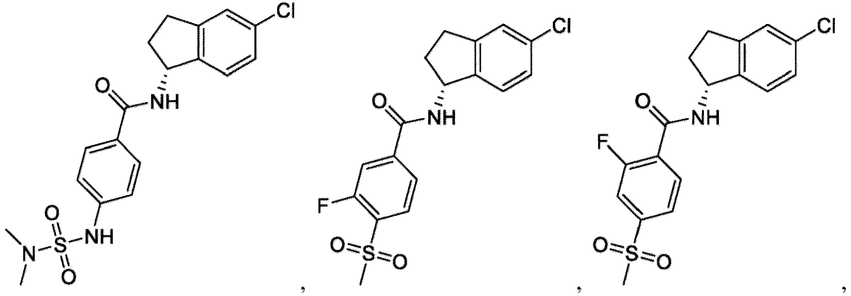 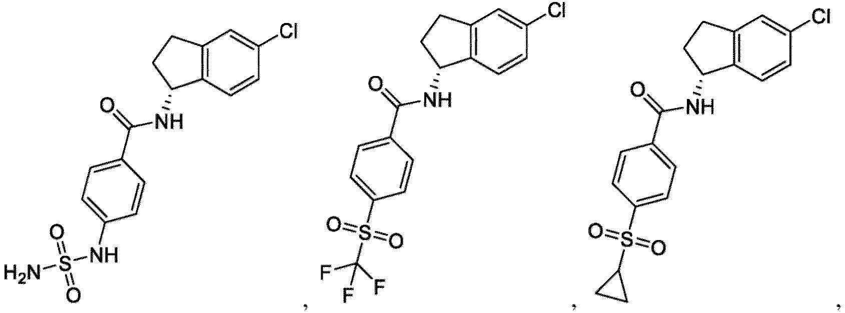 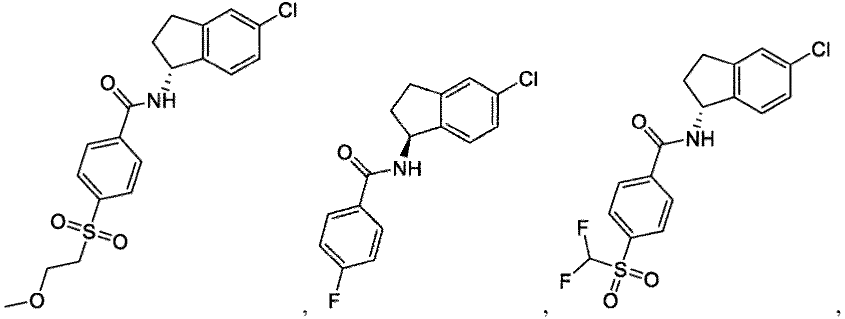 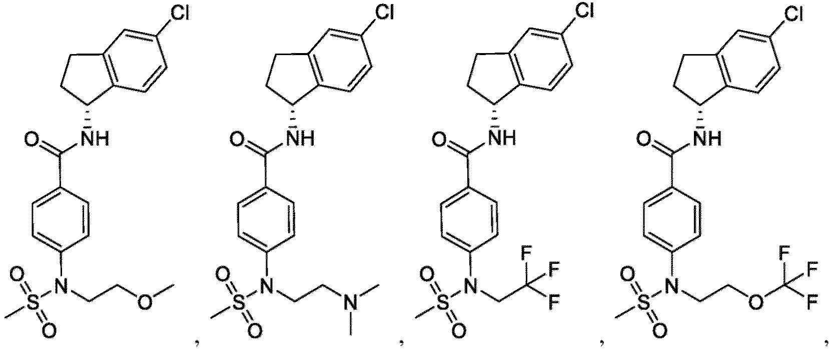 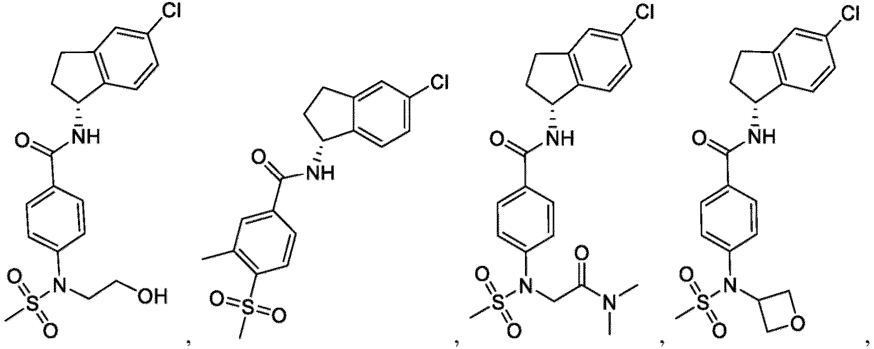 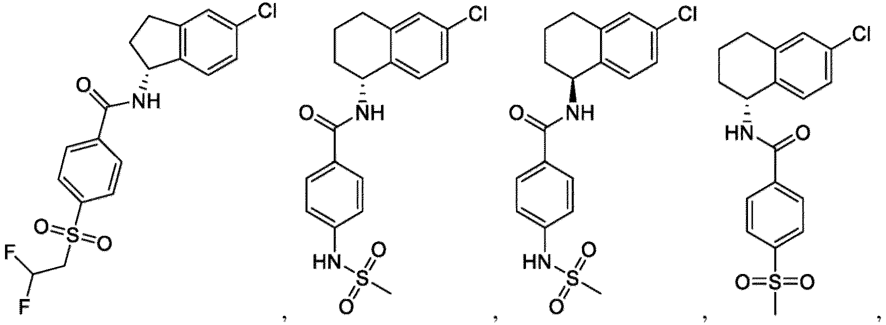 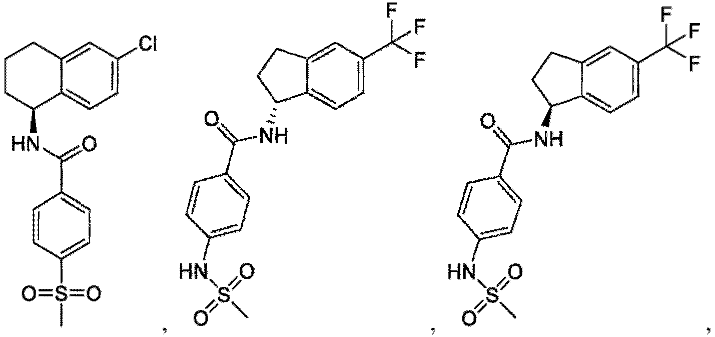 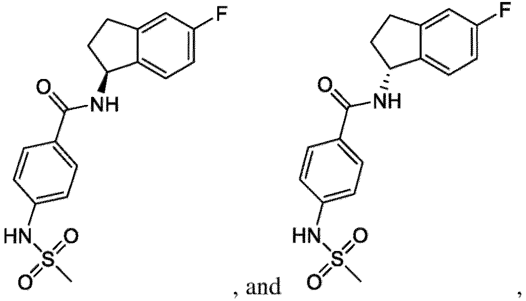 or a pharmaceutically acceptable salt thereof.
94. A method of treating a neurological disease or disorder, wherein the method comprises administering to a subject in need thereof a compound of Formula **II:** 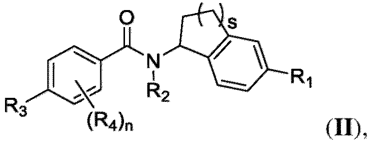 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl,-NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and-S(O)₂-C₁₋₆alkyl, wherein the C₁₋₆alkyl is optionally substituted with one or more halogen or C₁₋₆alkoxy;
   each R₄ is independently selected from the group consisting of C₁₋₆alkyl, halogen, and -OH; wherein R₄ is substituted at the carbon adjacent to R₃ when R₄ is -OH;
   n is selected from the group consisting of 0, 1, 2, 3, and 4;
   R₅, R₆, R₉, and R₁₀ are each independently hydrogen or C₁₋₆alkyl;
   each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
   s is 1 or 2.
95. A method of treating a disease or condition associated with excessive neuronal excitability, wherein the method comprises administering to a subject in need thereof a compound of Formula II: 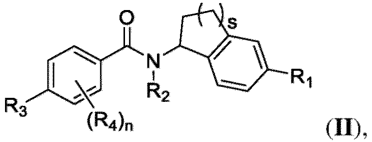 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl,-NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and-S(O)₂-C₁₋₆alkyl, wherein the C₁₋₆alkyl is optionally substituted with one or more halogen or C₁₋₆alkoxy;
   each R₄ is independently selected from the group consisting of C₁₋₆alkyl, halogen, and -OH; wherein R₄ is substituted at the carbon adjacent to R₃ when R₄ is -OH;
   n is selected from the group consisting of 0, 1, 2, 3, and 4;
   R₅, R₆, R₉, and R₁₀ are each independently hydrogen or C₁₋₆alkyl;
   each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
   s is 1 or 2.
96. A method of treating a disease or condition associated with a gain-of-function mutation of a gene (e.g., KCNT1), wherein the method comprises administering to a subject in need thereof a compound of Formula II: 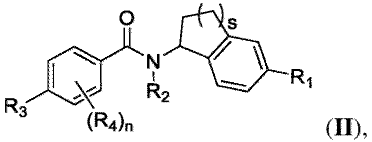 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl,-NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and-S(O)₂-C₁₋₆alkyl, wherein the C₁₋₆alkyl is optionally substituted with one or more halogen or C₁₋₆alkoxy;
   each R₄ is independently selected from the group consisting of C₁₋₆alkyl, halogen, and -OH; wherein R₄ is substituted at the carbon adjacent to R₃ when R₄ is -OH;
   n is selected from the group consisting of 0, 1, 2, 3, and 4;
   R₅, R₆, R₉, and R₁₀ are each independently hydrogen or C₁₋₆alkyl;
   each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
   s is 1 or 2.
97. A method of treating a neurological disease or disorder, wherein the method comprises administering to a subject in need thereof a pharmaceutical composition of any one of embodiments 56-93.
98. A method of treating a disease or condition associated with excessive neuronal excitability, wherein the method comprises administering to a subject in need thereof a pharmaceutical composition of any one of embodiments 56-93.
99. A method of treating a disease or condition associated with a gain-of-function mutation of a gene (e.g., KCNT1), wherein the method comprises administering to a subject in need thereof a pharmaceutical composition of any one of embodiments 56-93.
100. The method of any one of embodiments 39-44, wherein the neurological disease or disorder, the disease or condition associated with excessive neuronal excitability, or the disease or condition associated with a gain-of-function mutation of a gene (e.g., KCNT1) is epilepsy, an epilepsy syndrome, or an encephalopathy.
101. The method of any one of embodiments 39-44, wherein the neurological disease or disorder, the disease or condition associated with excessive neuronal excitability, or the disease or condition associated with a gain-of-function mutation of a gene (e.g., KCNT1) is a genetic or pediatric epilepsy or a genetic or pediatric epilepsy syndrome.
102. The method of any one of embodiments 39-44, wherein the neurological disease or disorder, the disease or condition associated with excessive neuronal excitability, or the disease or condition associated with a gain-of-function mutation of a gene (e.g., KCNT1) is a cardiac dysfunction.
103. The method of any one of embodiments 39-44, wherein the neurological disease or disorder, the disease or condition associated with excessive neuronal excitability, or the disease or condition associated with a gain-of-function mutation of a gene (e.g., KCNT1) is selected from the group consisting of epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, Lennox Gastaut syndrome, seizures (e.g., Generalized tonic clonic seizures, Asymmetric Tonic Seizures), leukodystrophy, leukoencephalopathy, intellectual disability, Multifocal Epilepsy, Drug resistant epilepsy, Temporal lobe epilepsy, or cerebellar ataxia).
104. The method of any one of embodiments 39-44, wherein the neurological disease or disorder, the disease or condition associated with excessive neuronal excitability, or the disease or condition associated with a gain-of-function mutation of a gene (e.g., KCNT1) is selected from the group consisting of cardiac arrhythmia, sudden unexpected death in epilepsy, Brugada syndrome, and myocardial infarction.
105. The methof of any one of embodiments 39-44, wherein the neurological disease or disorder, the disease or condition associated with excessive neuronal excitability, or the disease or condition associated with a gain-of-function mutation of a gene (e.g., KCNT1) is selected from pain and related conditions (e.g. neuropathic pain, acute/chronic pain, migraine).
106. The method of any one of embodiments 39-44, the neurological disease or disorder, the disease or condition associated with excessive neuronal excitability, or the disease or condition associated with a gain-of-function mutation of a gene (e.g., KCNT1) is a muscle disorder (e.g. myotonia, neuromyotonia, cramp muscle spasms, spasticity).
107. The method of any one of embodiments 39-44, wherein the neurological disease or disorder, the disease or condition associated with excessive neuronal excitability, or the disease or condition associated with a gain-of-function mutation of a gene (e.g., KCNT1) is selected from itch and pruritis, ataxia and cerebellar ataxias.
108. The method of any one of embodiments 39-44, wherein the neurological disease or disorder, the disease or condition associated with excessive neuronal excitability, or the disease or condition associated with a gain-of-function mutation of a gene (e.g., KCNT1) is selected from psychiatric disorders (e.g. major depression, anxiety, bipolar disorder, schizophrenia).
109. The method of any one of embodiments 39-44, wherein the neurological disease or disorder or the disease or condition associated with excessive neuronal excitability and/or a gain-of-function mutation in a gene (e.g., KCNT1) is selected from the group consisting of learning disorders, Fragile X, neuronal plasticity, and autism spectrum disorders.
110. The method of any one of embodiments 39-44, wherein the neurological disease or disorder, the disease or condition associated with excessive neuronal excitability, or the disease or condition associated with a gain-of-function mutation of a gene (e.g., KCNT1) is selected from the group consisting of epileptic encephalopathy with SCN1A, SCN2A, SCN8A mutations, early infantile epileptic encephalopathy, Dravet syndrome, Dravet syndrome with SCN1A mutation, generalized epilepsy with febrile seizures, intractable childhood epilepsy with generalized tonic-clonic seizures, infantile spasms, benign familial neonatal-infantile seizures, SCN2A epileptic encephalopathy, focal epilepsy with SCN3A mutation, cryptogenic pediatric partial epilepsy with SCN3A mutation, SCN8A epileptic encephalopathy, sudden unexpected death in epilepsy, Rasmussen encephalitis, malignant migrating partial seizures of infancy, autosomal dominant nocturnal frontal lobe epilepsy, sudden expected death in epilepsy (SUDEP), KCNQ2 epileptic encephalopathy, and KCNT1 epileptic encephalopathy.
111. A pharmaceutical composition comprising a compound of Formula **III:** 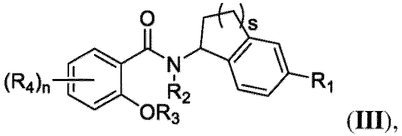 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is hydrogen;
   R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
   n is selected from the group consisting of 0, 1, 2, 3, and 4;
   R₇ and R₈ are each independently hydrogen or C₁₋₆alkyl; and
   s is 1 or 2;
   and a pharmaceutically acceptable excipient.
112. The pharmaceutical composition of embodiment 111, wherein the compound is a compound of Formula **III-a,** Formula **III-a1,** or Formula **III-a2:** 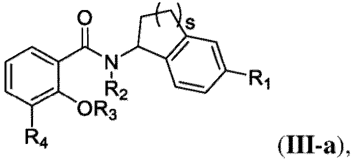 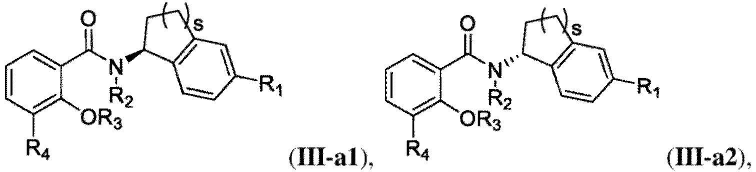 or a pharmaceutically acceptable salt thereof, wherein the variables are as defined in embodiment 111.
113. The pharmaceutical composition of embodiment 111, wherein the compound is a compound of Formula **III-b,** Formula **III-b1**, or Formula **III-b2:** 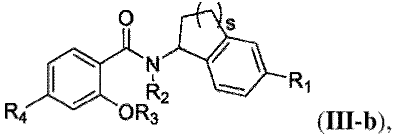 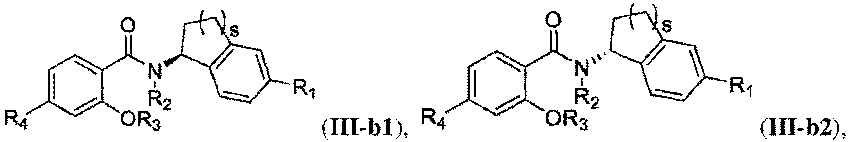 or a pharmaceutically acceptable salt thereof, wherein the variables are as defined in embodiment 111.
114. The pharmaceutical composition of embodiment 111, wherein the compound is a compound of Formula **III-c,** Formula **III-c1**, or Formula **III-c2:** 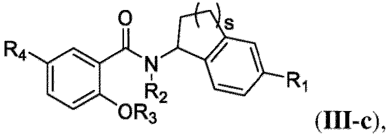 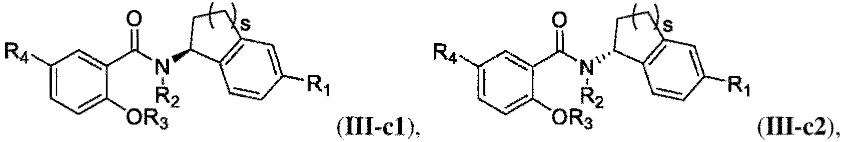 or a pharmaceutically acceptable salt thereof, wherein the variables are as defined in embodiment 111.
115. The pharmaceutical composition of any one of embodiments 111-114, wherein R₁ is selected from the group consisting of -Cl, -F, and -CF₃.
116. The pharmaceutical composition of any one of embodiments 111-115, wherein R₁ is-Cl.
117. The pharmaceutical composition of any one of embodiments 111-115, wherein R₁ is-F.
118. The pharmaceutical composition of any one of embodiments 111-15, wherein R₁ is-CF₃.
119. The pharmaceutical composition of any one of embodiments 111-118, wherein R₄ is selected from the group consisting of methyl, methoxy, -F, -Cl, -CF₃, methoxy, 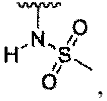 and 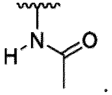
120. The pharmaceutical composition of any one of embodiments 111-119, wherein R₄ is - F.
121. The pharmaceutical composition of any one of embodiments 111-119, wherein R₄ is - Cl.
122. The pharmaceutical composition of any one of embodiments 111-119, wherein R₄ is-CF₃.
123. The pharmaceutical composition of any one of embodiments 111-119, wherein R₄ is methoxy.
124. The pharmaceutical composition of any one of embodiments 111-119, wherein R₄ is 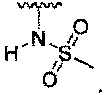
125. The pharmaceutical composition of any one of embodiments 111-119, wherein R₄ is 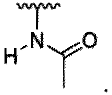
126. The pharmaceutical composition of any one of embodiments 111 and 115-1125, wherein n is selected from the group consisting of 0, 1, and 2.
127. The pharmaceutical composition of any one of embodiments 111 and 115-126, wherein n is 0 or 1.
128. The pharmaceutical composition of any one of embodiments 111 and 115-127, wherein n is 1.
129. The pharmaceutical composition of any one of embodiments 111 and 115-127, wherein n is 0.
130. The pharmaceutical composition of any one of embodiments 111-129, wherein each R₇ and R₈ are independently hydrogen.
131. The pharmaceutical composition of any one of embodiments 111-130, wherein s is 2.
132. The pharmaceutical composition of any one of embodiments 111-130, wherein s is 1.
133. The pharmaceutical composition of embodiment 111, wherein the compound is selected from the group consisting of: 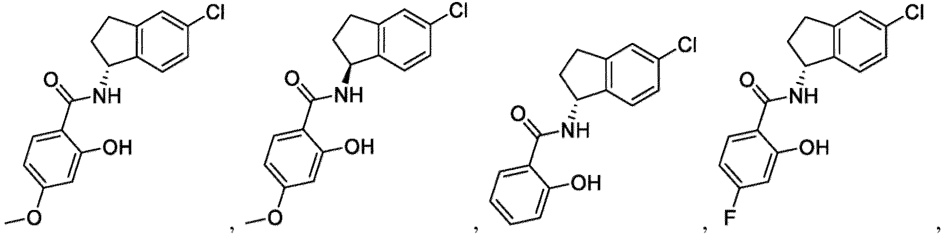 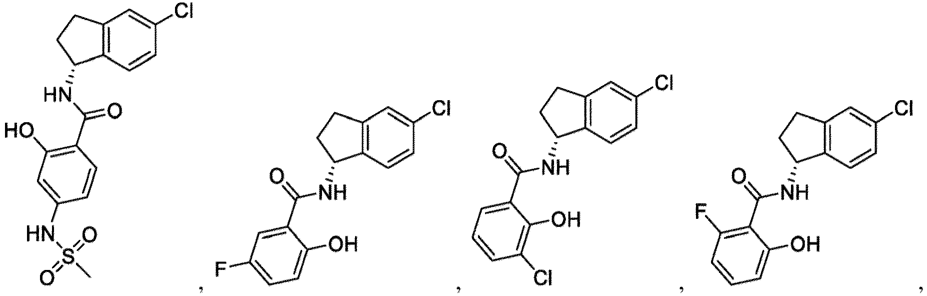 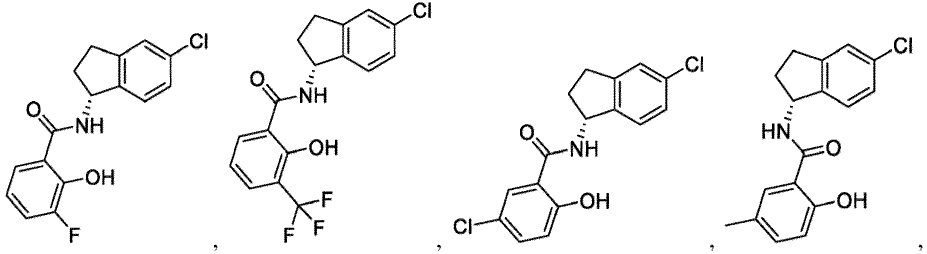 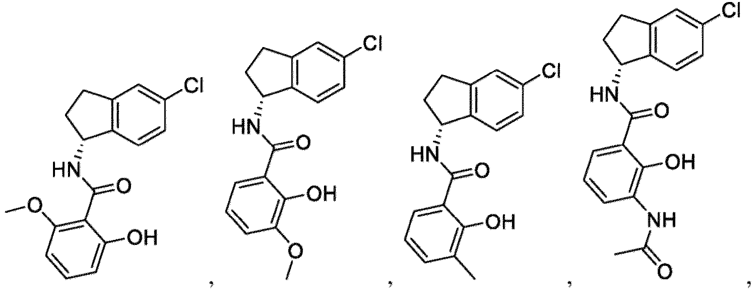 and 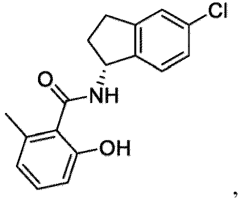 or a pharmaceutically acceptable salt thereof.
134. A method of treating a neurological disease or disorder, wherein the method comprises administering to a subject in need thereof a compound of Formula **III:** 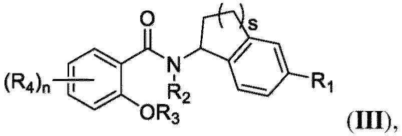 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is hydrogen;
   R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
   n is selected from the group consisting of 0, 1, 2, 3, and 4;
   R₇ and R₈ are each independently hydrogen or C₁₋₆alkyl; and
   s is 1 or 2.
135. A method of treating a disease or condition associated with excessive neuronal excitability, wherein the method comprises administering to a subject in need thereof a compound of Formula **III:** 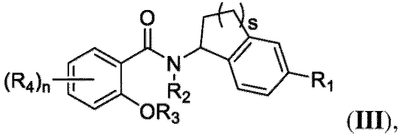 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is hydrogen;
   R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
   n is selected from the group consisting of 0, 1, 2, 3, and 4;
   R₇ and R₈ are each independently hydrogen or C₁₋₆alkyl; and
   s is 1 or 2.
136. A method of treating a disease or condition associated with a gain-of-function mutation of a gene (e.g., KCNT1), wherein the method comprises administering to a subject in need thereof a compound of Formula **III:** 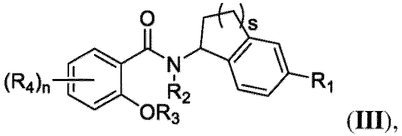 or a pharmaceutically acceptable salt thereof, wherein
   R₁ is selected from the group consisting of -Cl, -F, and C₁₋₆alkyl substituted with one or more substituents independently selected from -Cl and -F;
   R₂ is hydrogen;
   R₃ is hydrogen;
   R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl;
   n is selected from the group consisting of 0, 1, 2, 3, and 4;
   R₇ and R₈ are each independently hydrogen or C₁₋₆alkyl; and
   s is 1 or 2.
137. A method of treating a neurological disease or disorder, wherein the method comprises administering to a subject in need thereof a pharmaceutical composition of any one of embodiments 111-133.
138. A method of treating a disease or condition associated with excessive neuronal excitability, wherein the method comprises administering to a subject in need thereof a pharmaceutical composition of any one of embodiments 111-133.
139. A method of treating a disease or condition associated with a gain-of-function mutation of a gene (e.g., KCNT1), wherein the method comprises administering to a subject in need thereof a pharmaceutical composition of any one of embodiments 111-133.
140. The method of any one of embodiments 134-139, wherein the neurological disease or disorder, the disease or condition associated with excessive neuronal excitability, or the disease or condition associated with a gain-of-function mutation of a gene (e.g., KCNT1) is epilepsy, an epilepsy syndrome, or an encephalopathy.
141. The method of any one of embodiments 134-139, wherein the neurological disease or disorder, the disease or condition associated with excessive neuronal excitability, or the disease or condition associated with a gain-of-function mutation of a gene (e.g., KCNT1) is a genetic or pediatric epilepsy or a genetic or pediatric epilepsy syndrome.
142. The method of any one of embodiments 134-139, wherein the neurological disease or disorder, the disease or condition associated with excessive neuronal excitability, or the disease or condition associated with a gain-of-function mutation of a gene (e.g., KCNT1) is a cardiac dysfunction.
143. The method of any one of embodiments 134-139, wherein the neurological disease or disorder, the disease or condition associated with excessive neuronal excitability, or the disease or condition associated with a gain-of-function mutation of a gene (e.g., KCNT1) is selected from the group consisting of epilepsy and other encephalopathies (e.g., epilepsy of infancy with migrating focal seizures (MMFSI, EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, Lennox Gastaut syndrome, seizures (e.g., Generalized tonic clonic seizures, Asymmetric Tonic Seizures), leukodystrophy, leukoencephalopathy, intellectual disability, Multifocal Epilepsy, Drug resistant epilepsy, Temporal lobe epilepsy, or cerebellar ataxia).
144. The method of any one of embodiments 134-139, wherein the neurological disease or disorder, the disease or condition associated with excessive neuronal excitability, or the disease or condition associated with a gain-of-function mutation of a gene (e.g., KCNT1) is selected from the group consisting of cardiac arrhythmia, sudden unexpected death in epilepsy, Brugada syndrome, and myocardial infarction.
145. The method of any one of embodiments 134-139, wherein the neurological disease or disorder, the disease or condition associated with excessive neuronal excitability, or the disease or condition associated with a gain-of-function mutation of a gene (e.g., KCNT1) is selected from pain and related conditions (e.g. neuropathic pain, acute/chronic pain, migraine).
146. The method of any one of embodiments 134-139, the neurological disease or disorder, the disease or condition associated with excessive neuronal excitability, or the disease or condition associated with a gain-of-function mutation of a gene (e.g., KCNT1) is a muscle disorder (e.g. myotonia, neuromyotonia, cramp muscle spasms, spasticity).
147. The method of any one of embodiments 134-139, wherein the neurological disease or disorder, the disease or condition associated with excessive neuronal excitability, or the disease or condition associated with a gain-of-function mutation of a gene (e.g., KCNT1) is selected from itch and pruritis, ataxia and cerebellar ataxias.
148. The method of any one of embodiments 134-139, wherein the neurological disease or disorder, the disease or condition associated with excessive neuronal excitability, or the disease or condition associated with a gain-of-function mutation of a gene (e.g., KCNT1) is selected from psychiatric disorders (e.g. major depression, anxiety, bipolar disorder, schizophrenia).
149. The method of any one of embodiments 134-139, wherein the neurological disease or disorder or the disease or condition associated with excessive neuronal excitability and/or a gain-of-function mutation in a gene (e.g., KCNT1) is selected from the group consisting of learning disorders, Fragile X, neuronal plasticity, and autism spectrum disorders.
150. The method of any one of embodiments 134-139, wherein the neurological disease or disorder, the disease or condition associated with excessive neuronal excitability, or the disease or condition associated with a gain-of-function mutation of a gene (e.g., KCNT1) is selected from the group consisting of epileptic encephalopathy with SCN1A, SCN2A, SCN8A mutations, early infantile epileptic encephalopathy, Dravet syndrome, Dravet syndrome with SCN1A mutation, generalized epilepsy with febrile seizures, intractable childhood epilepsy with generalized tonic-clonic seizures, infantile spasms, benign familial neonatal-infantile seizures, SCN2A epileptic encephalopathy, focal epilepsy with SCN3A mutation, cryptogenic pediatric partial epilepsy with SCN3A mutation, SCN8A epileptic encephalopathy, sudden unexpected death in epilepsy, Rasmussen encephalitis, malignant migrating partial seizures of infancy, autosomal dominant nocturnal frontal lobe epilepsy, sudden expected death in epilepsy (SUDEP), KCNQ2 epileptic encephalopathy, and KCNT1 epileptic encephalopathy.

## Claims

1. A compound of Formula **I-I:** 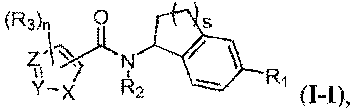
or a pharmaceutically acceptable salt thereof, wherein
X is selected from the group consisting of NH, O, and S, wherein the hydrogen of NH may be substituted with R₃;
Y is selected from N and CH, wherein the hydrogen of CH may be substituted with R₃;
Z is selected from N and CH, wherein the hydrogen of CH may be substituted with R₃, or Z is C when Z is substituted with the -C(O)N(R₂)- moiety;
R₁ is selected from the group consisting of -Cl, -F, and C₁₋₆alkyl substituted with one or more substituents independently selected from -Cl and -F, such as R₁ is -Cl, -F, or -CF₃;
R₂ is hydrogen;
each R₃ is independently selected from the group consisting of halogen, C₁₋₆alkyl, C₁₋₆heteroalkyl, C₃-₇cycloalkyl, 3-7 membered heterocyclyl, -S(O)₂NR₄R₅, -NR₄S(O)R₆,-C(O)NR₄R₅, -S(O)₂R₆, and -O-R₆, wherein C₁₋₆alkyl is optionally substituted with one or more substituents independently selected from halogen, -NR₄R₅, and -S(O)₂R₆;
n is selected from the group consisting of 0, 1, 2, 3, and 4;
R₄ and R₅ are each independently hydrogen or C₁₋₆alkyl, wherein C₁₋₆alkyl is optionally substituted with oxo; or R₄ and R₅ may be taken together with the nitrogen to which R₄ and R₅ are attached to form a 4-7 membered heterocyclyl optionally substituted with one or more substituents independently selected from halogen, -OH, C₁₋₆alkyl, and C₁₋₆heteroalkyl;
each R₆ is independently selected from the group consisting of C₁₋₆alkyl, C₁₋₆heteroalkyl, C₂₋₆alkenyl, C₂₋₆alkynyl, C₃₋₇cycloalkyl, phenyl, and benzyl; and
s is 1 or 2; such as a compound of Formula **I-d** or Formula **I-d1:** 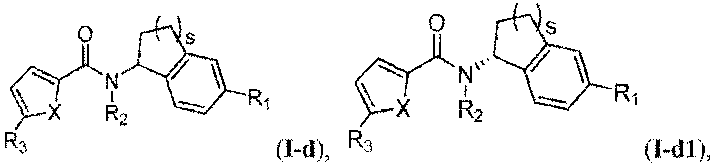 or a compound of Formula **I-Ic** or Formula **I-Ic1:**

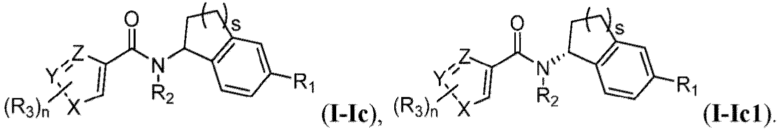

2. The compound of claim 1, wherein x is S.

3. The compound of claim 1 or 2, wherein Z is CH.

4. The compound of claim 1, wherein the compound is a compound of Formula **I-Ia** or Formula **I-Ia1:** 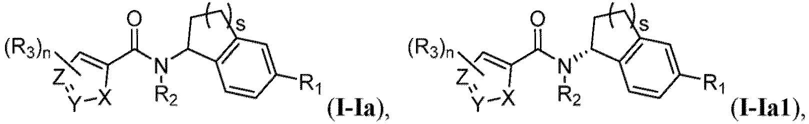 such as a compound of Formula **I-Ib** or Formula **I-Ib1:** 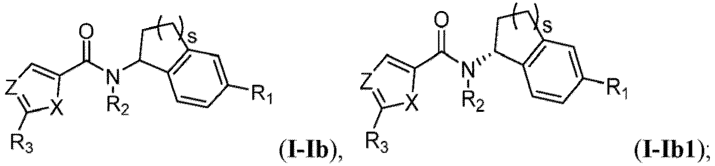 or a compound of Formula **I-a** or Formula **I-a1:** 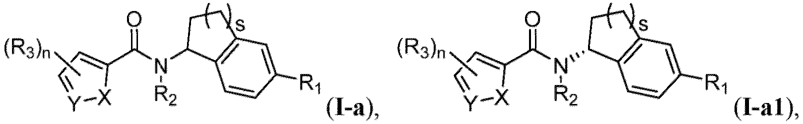 or a pharmaceutically acceptable salt thereof, wherein the variables are as defined in claim 1.

5. The compound of any one of claims 1-4, wherein R₁ is -Cl.

6. The compound of any one of claims 1-5, wherein each R₃ is selected from the group consisting of -Cl, methyl, methyl substituted with -NR₄R₅ or -S(O)₂R₆, methoxymethyl, trifluoromethyl, ethyl, cyclopropyl, cyclohexyl, -S(O)₂R₆, -C(O)NR₄R₅, -S(O)₂NR₄R₅, and C₁₋₆alkyl substituted with -NR₄R₅ or -S(O)₂R₆.

7. The compound of any one of claims 1-6, wherein n is 1 or 2.

8. The compound of any one of claims 1-7, wherein each of R₄ and R₅ are independently selected from the group consisting of hydrogen, methyl, ethyl, cyclopropyl, and -C(O)CH₃, such as each of R₄ and R₅ are hydrogen or methyl or R₄ is H and R₅ is methyl, or wherein R₄ and R₅ are taken together with the nitrogen to which R₄ and R₅ are attached to form a 4-6 membered heterocyclyl optionally substituted with -OH, methyl, or -OCH₃.

9. The compound of any one of claims 1-8, wherein R₆ is selected from the group consisting of methyl, ethyl, methoxyethyl, and cyclopropyl.

10. The compound of claim 1, wherein the compound is selected from the group consisting of: 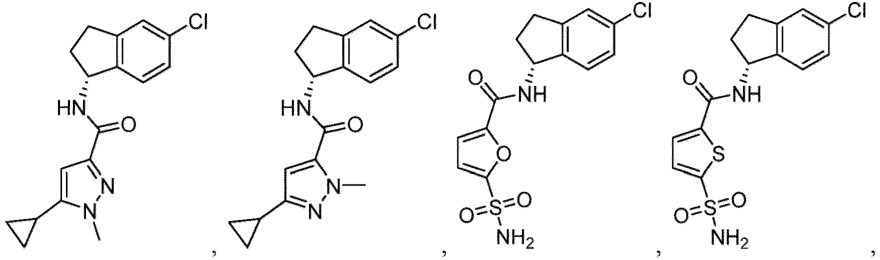 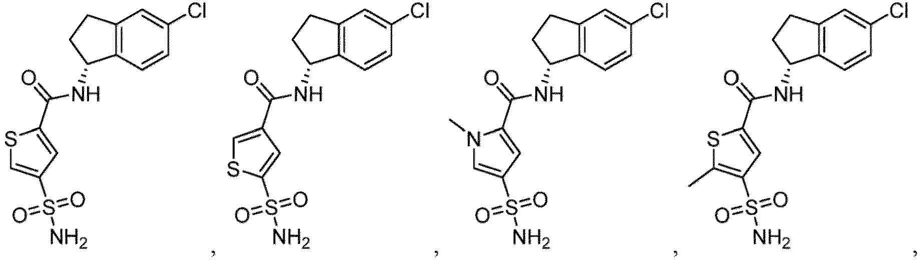 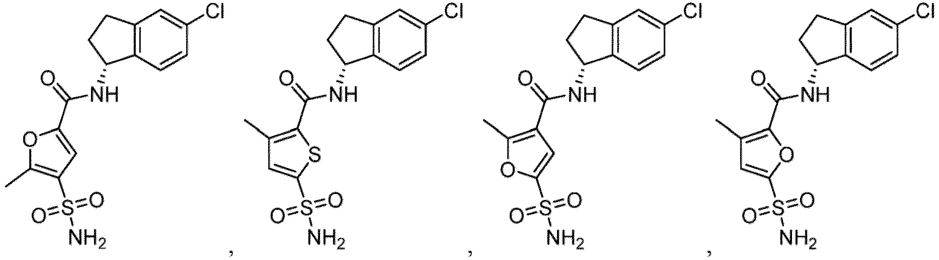 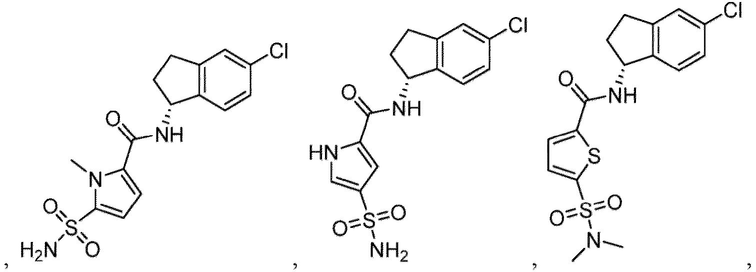 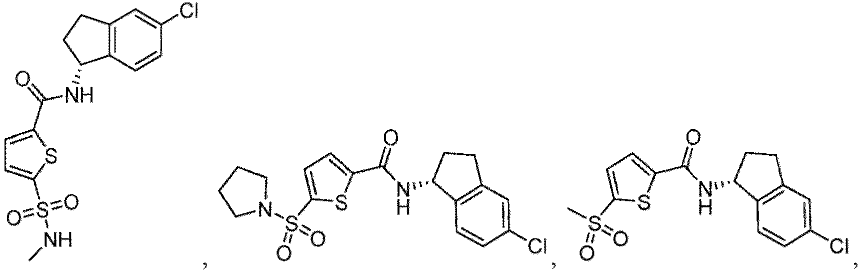 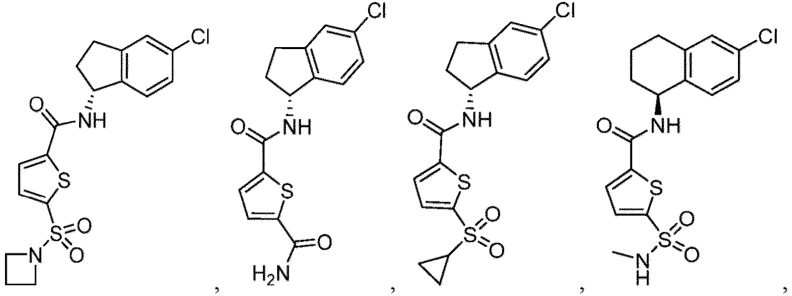 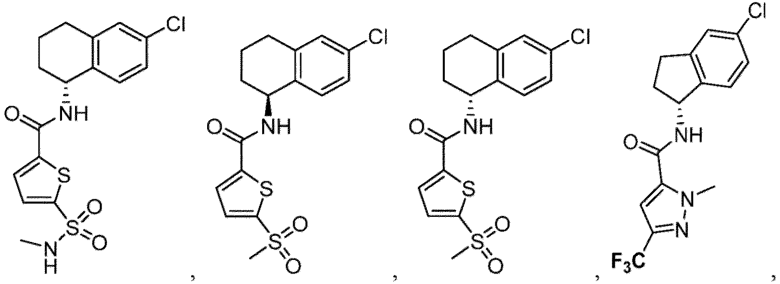 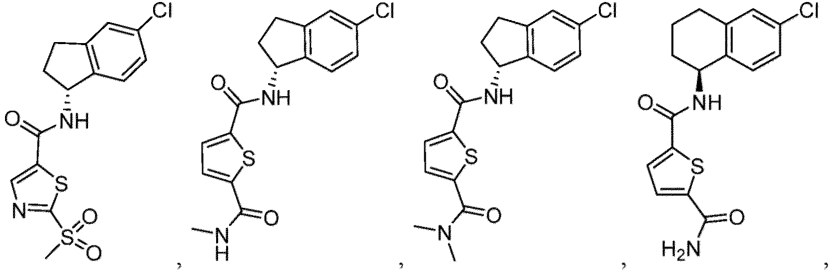 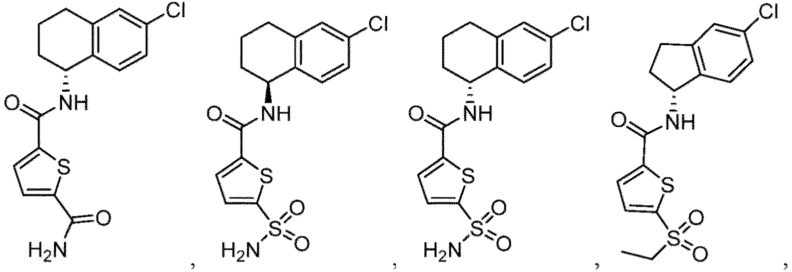 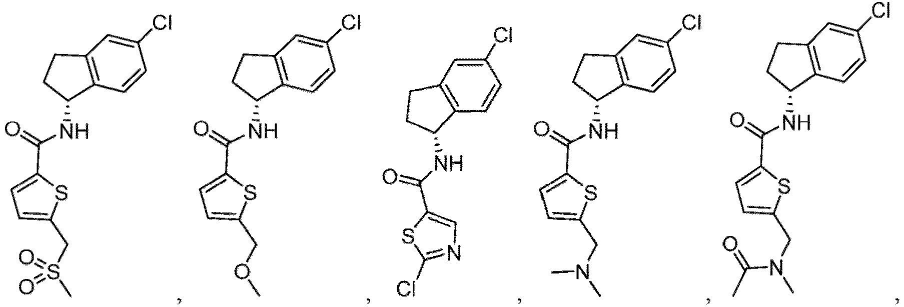 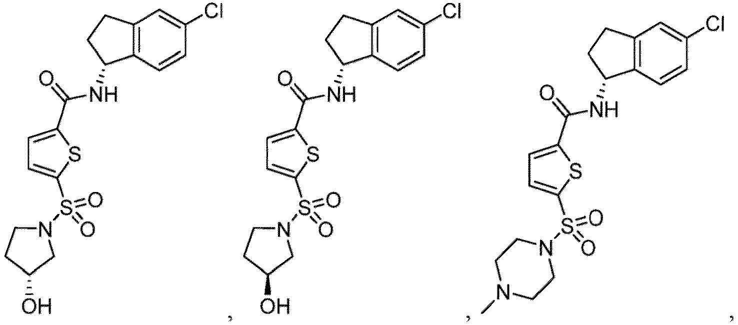 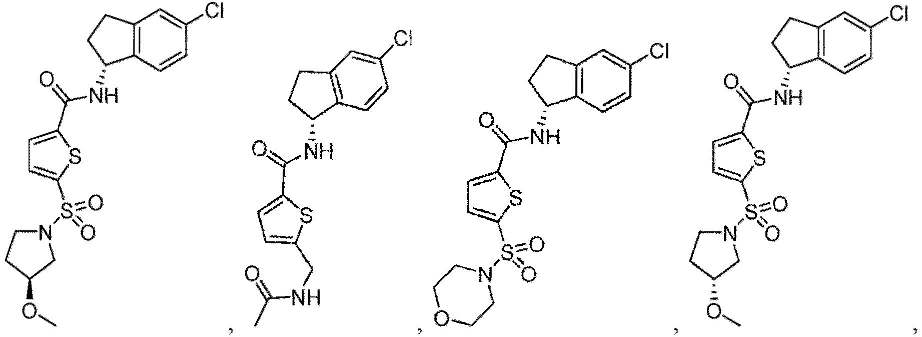 and 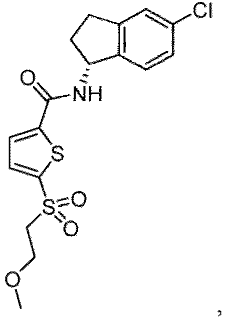 or a pharmaceutically acceptable salt thereof.

11. A compound of Formula **II:** 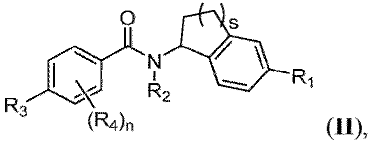
or a pharmaceutically acceptable salt thereof, wherein
R₁ is selected from the group consisting of -Cl, -F, and C₁₋₆alkyl substituted with one or more substituents independently selected from -Cl and -F;
R₂ is hydrogen;
R₃ is independently selected from the group consisting of halogen, C₁₋₆alkoxy, C₁₋₆alkylene-S(O)₂-C₁₋₆alkyl, -C(O)NR₅R₆, -NR₇S(O)₂C₁₋₆alkyl, -NR₇S(O)₂C₃₋₇ cycloalkyl,-NR₇S(O)₂NR₅R₆, -NR₉R₁₀, -S(O)₂-C₃₋₆cycloalkyl, -S(O)₂-NR₅R₆, -S(O)₂-C₁₋₆alkoxy, and-S(O)₂-C₁₋₆alkyl, wherein the C₁₋₆alkyl is optionally substituted with one or more halogen or C₁₋₆alkoxy;
each R₄ is independently selected from the group consisting of C₁₋₆alkyl, halogen, and -OH; wherein R₄ is substituted at the carbon adjacent to R₃ when R₄ is -OH;
n is selected from the group consisting of 0, 1, 2, 3, and 4;
R₅, R₆, R₉, and R₁₀ are each independently hydrogen or C₁₋₆alkyl;
each R₇ is independently selected from the group consisting of hydrogen, C₁₋₆alkyl, and 3-7 membered heterocyclyl, wherein the C₁₋₆alkyl is optionally substituted with one or more substituents independently selected from the group consisting of halogen, C₁₋₆alkoxy, C₁₋₆haloalkoxy, -OH, -NR₅R₆, and -C(O)NR₅R₆; and
s is 1 or 2;
such as a compound of Formula **II-a**, Formula **II-a1,** or Formula **II-a2:** 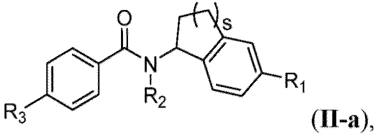 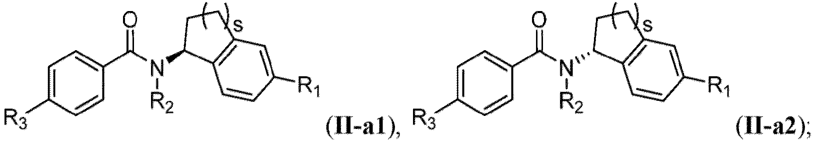
a compound of Formula **II-b,** Formula **II-b1**, or Formula **II-b2:** 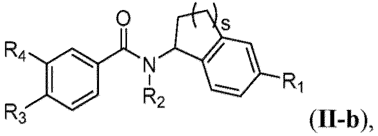 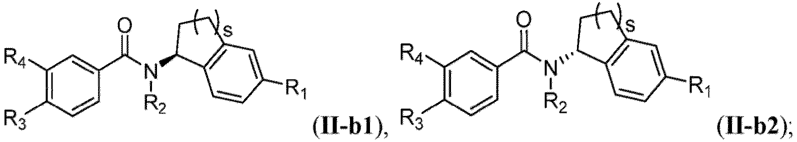
or a compound of of Formula **II-c,** Formula **II-c1**, or Formula **II-c2:**

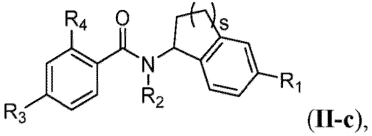

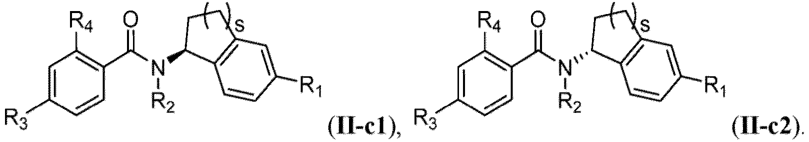

12. The compound of claim 11, wherein R₃ is selected from the group consisting of -F, methoxy, -NH₂,

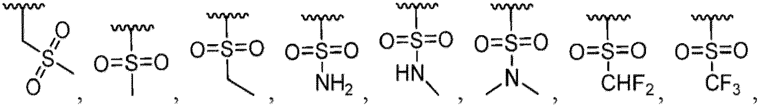

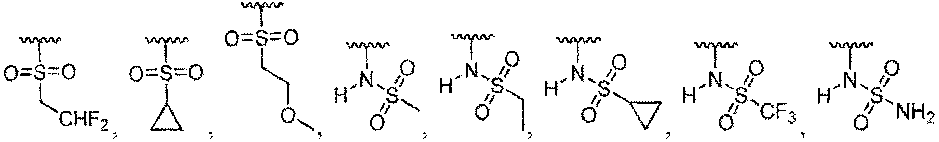

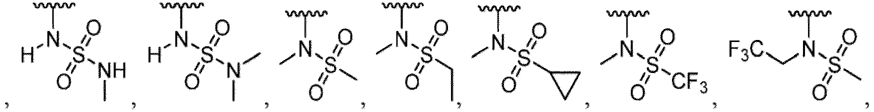

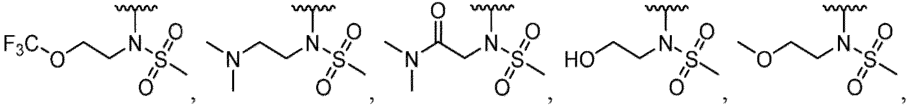

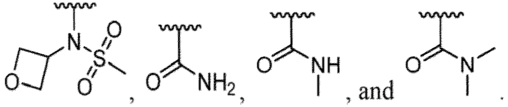

13. The compound of claim 11 or 12, wherein the compound is selected from the group consisting of: 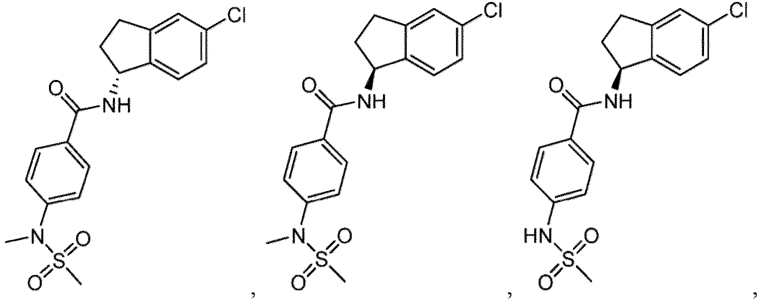 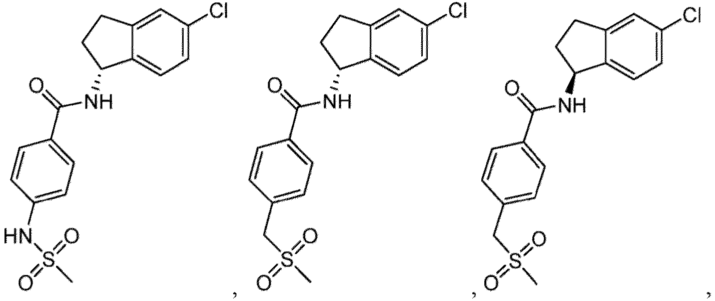 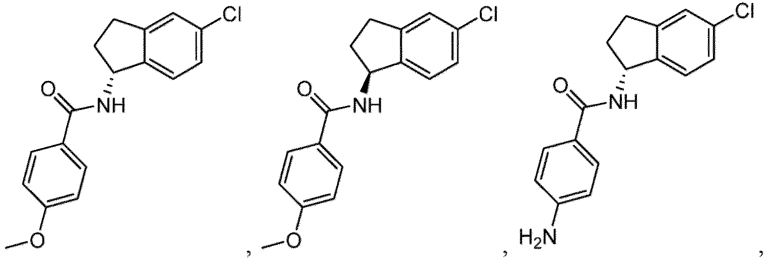 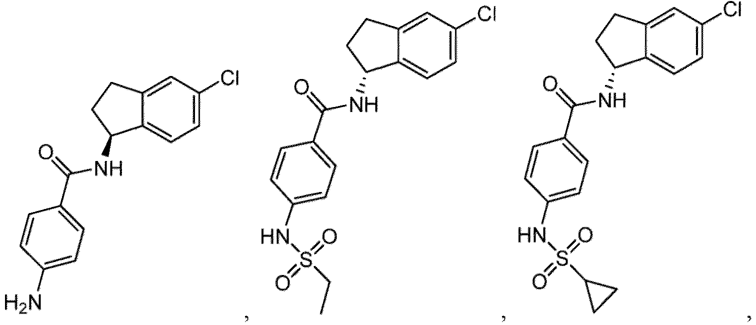 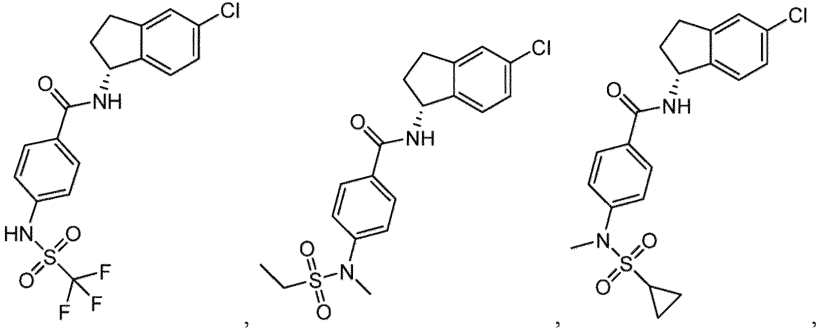 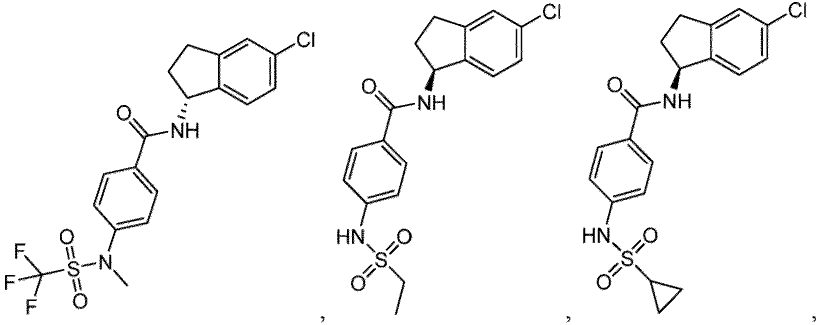 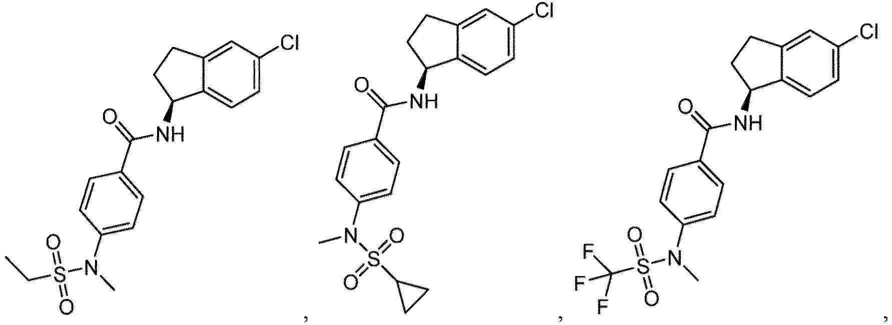 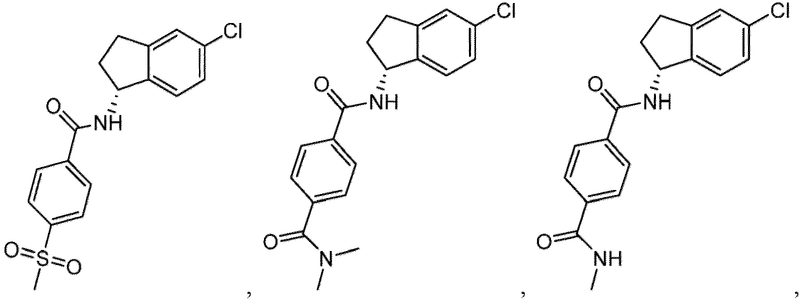 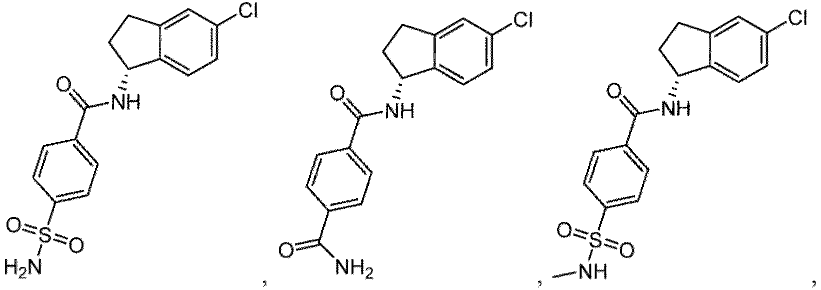 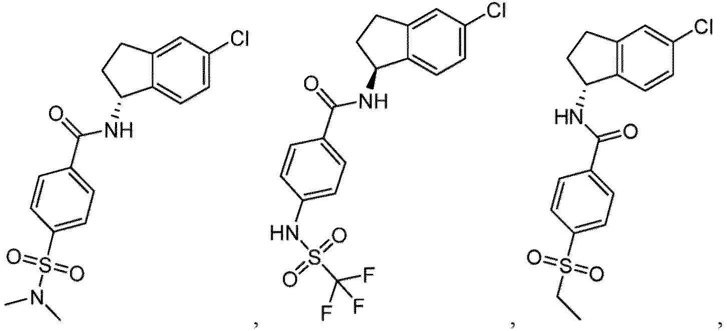 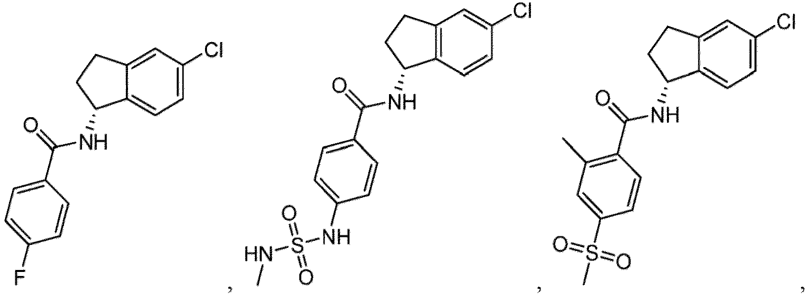 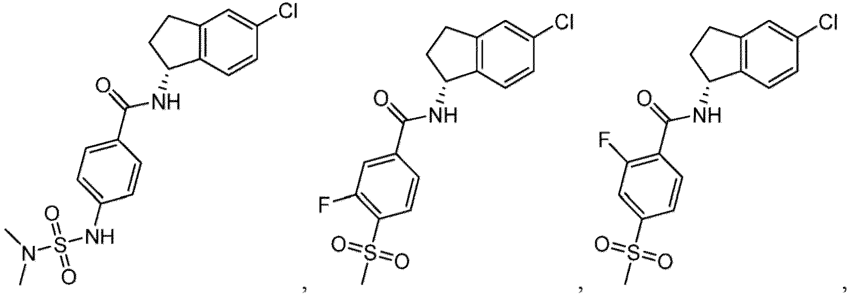 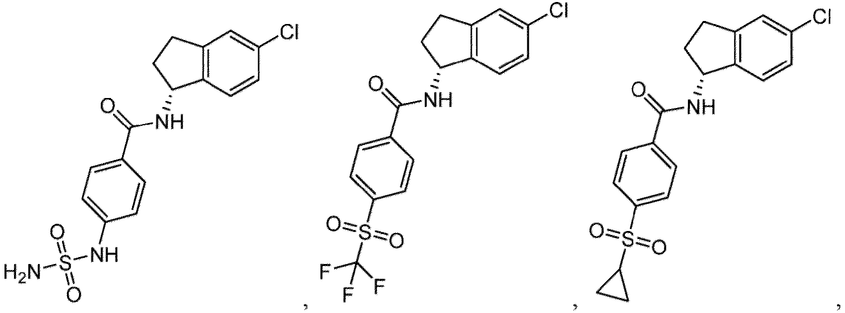 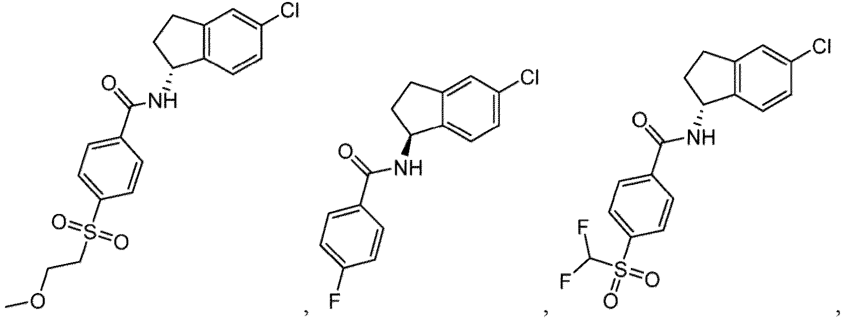 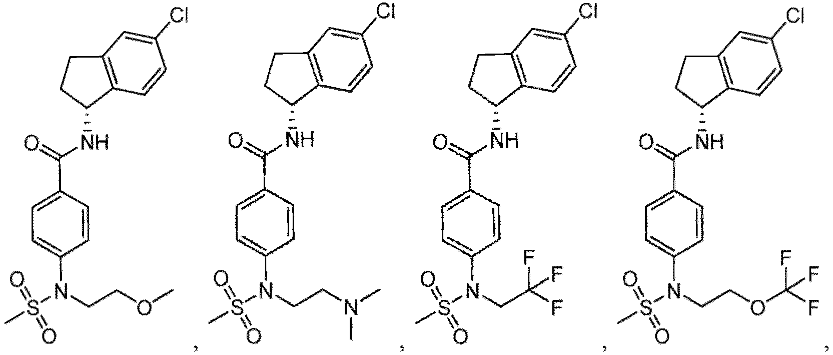 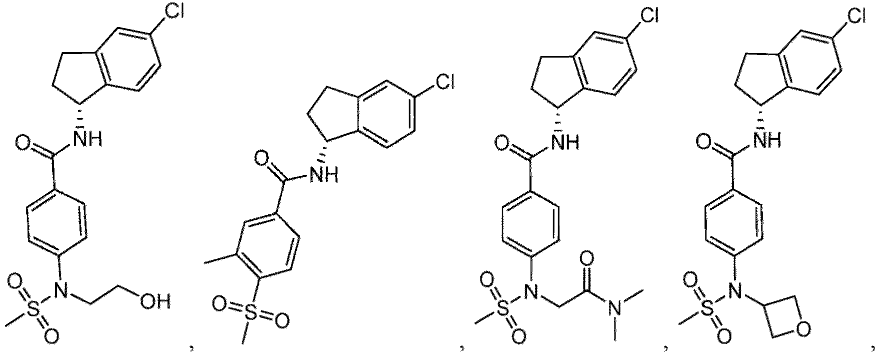 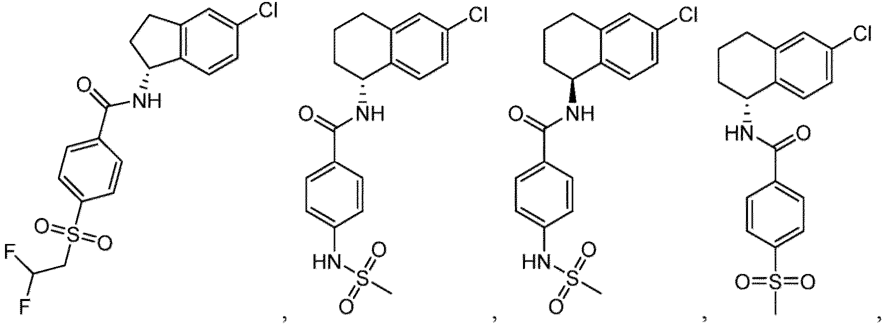 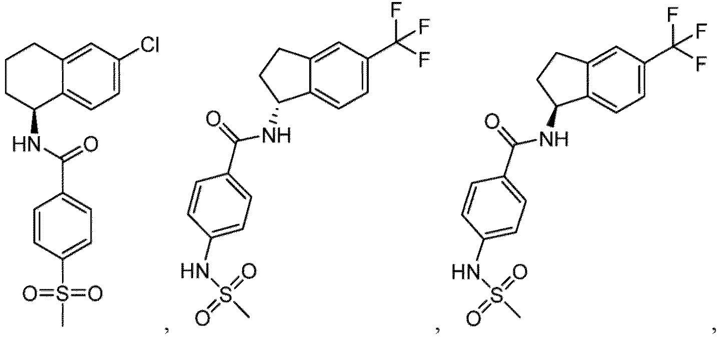 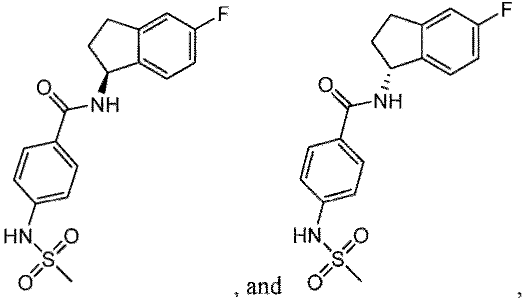 or a pharmaceutically acceptable salt thereof.

14. A compound of Formula **III:** 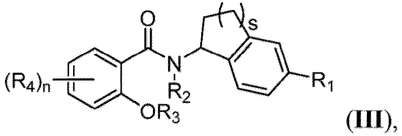
or a pharmaceutically acceptable salt thereof, wherein
R₁ is selected from the group consisting of -Cl, -F, and C₁₋₆alkyl substituted with one or more substituents independently selected from -Cl and -F, such as R₁ is -Cl, -F, or -CF₃;
R₂ is hydrogen;
R₃ is hydrogen;
R₄ is each independently selected from the group consisting of C₁₋₆alkyl, C₁₋₆alkoxy, halogen, C₁₋₆haloalkyl, -NR₇S(O)₂C₁₋₆alkyl, and -NR₈C(O)-C₁₋₆alkyl, such as R₄ is selected from methyl, methoxy, -F, -Cl, -CF₃, methoxy, 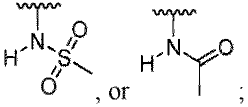
n is selected from the group consisting of 0, 1, 2, 3, and 4;
R₇ and R₈ are each independently hydrogen or C₁₋₆alkyl; and
s is 1 or 2;
such as a compound of Formula **III-a,** Formula **III-a1**, or Formula **III-a2:** 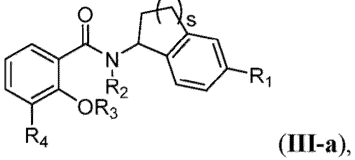 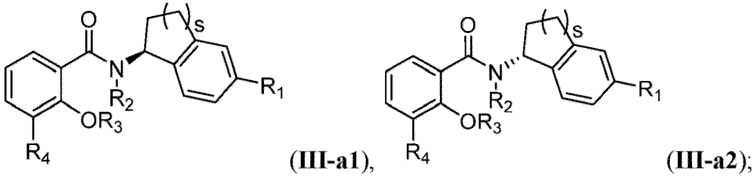
a compound of Formula **III-b,** Formula **III-b1**, or Formula **III-b2:** 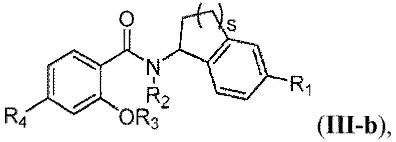 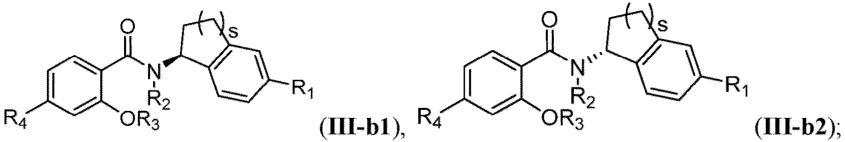 or
a compound of Formula **III-c,** Formula **III-c1,** or Formula **III-c2:**

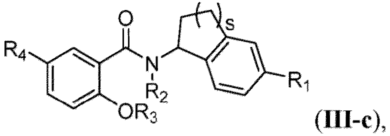

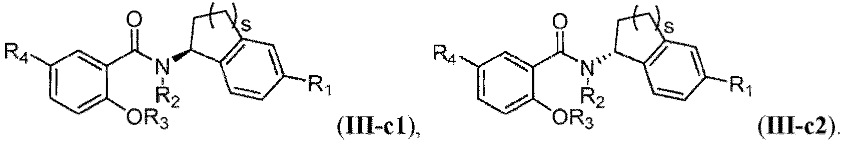

15. The compound of claim 14, wherein the compound is selected from the group consisting of: 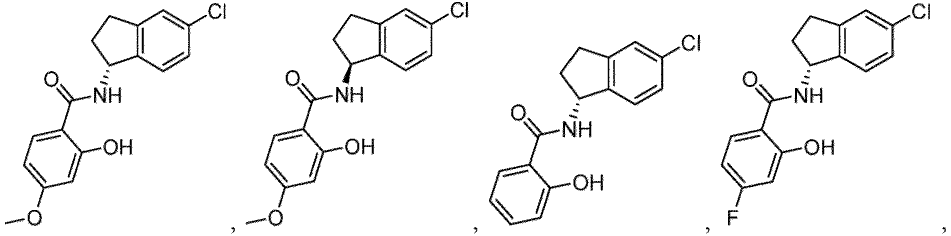 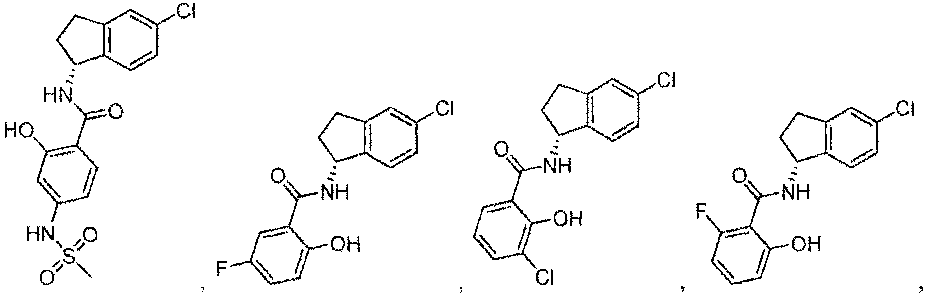 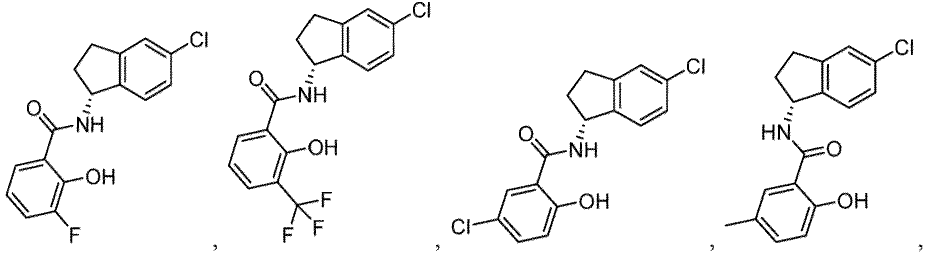 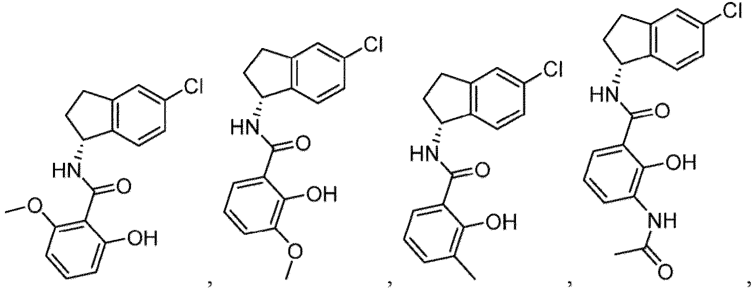 and 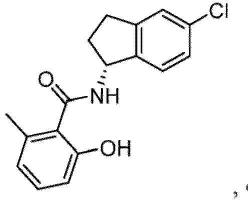 or a pharmaceutically acceptable salt thereof.
